# EUROPEAN PATENT APPLICATION

(11) **EP 1 829 965 A1**
(43) Date of publication of application: **05.09.2007**
(21) Application number: 05809612.4
(22) Date of filing: 25.11.2005
(51) Int. Cl.: C12N 15/09, C12N 1/20, C12N 1/21, C12P 7/64, C12P 13/00, C12P 13/04, C12P 17/00, C12P 19/00, C12P 19/30, C12P 19/38, C12P 21/02

(54) **INDUSTRIALLY USEFUL MICROORGANISM**

(30) Priority: 26.11.2004 JP 2004341706; 13.07.2005 JP 2005203965
(71) Applicant: KYOWA HAKKO KOGYO CO., LTD., Chiyoda-ku, Tokyo 100-8185 (JP)
(72) Inventor: MIZOGUCHI, Hiroshi, (JP); HARA, Kiyotaka, (JP); MORI, Hideo, (JP)
(74) Representative: Simcox, Michael Thomas
(86) International application number: PCT/JP2005/021691
(87) International publication number: WO 2006/057341

(57) **Abstract**

According to the present invention, the *E*. *coli* mutant strain having chromosomal DNA that is at least 470 kbp shorter than that of a wild-type *E*. *coli* strain exhibits such a property that the number of the cells after a certain period of culture is greater than a wild-type strain. Thus, by culturing the mutant strain in a medium so as to generate and accumulate a useful substance in the culture, a useful substance such as proteins, peptides, amino acids, nucleic acids, vitamins, saccharides, organic acids, and lipids can be efficiently produced.

## Description

### Technical Field

The present invention relates to an *E*. *coli* mutant strain having chromosomal DNA shorter than that of a wild-type *E*. *coli* strain and a process for producing a useful substance using such mutant strain.

### Background Art

All nucleotide sequences of the chromosomal DNAs of *E. coli* have been elucidated (Non-Patent Documents 1 and 2). Also, a method wherein a given gene or a given region on chromosomal DNA of *E*. *coli* is deleted as designed by homologous recombination has been known (Non-Patent Document 3). With the utilization of all of the nucleotide sequence information of chromosomal DNA and homologous recombination, a library of mutant *E*. *coli* in which genes of chromosomal DNA are comprehensively disrupted, a library of mutant *E*. *coli* in which approximately 20-kbp deletable regions of chromosomal DNAs are comprehensively deleted, and the like have been constructed (Non-Patent Documents 4 and 5).

As example of *E. coli* mutants that lack multiple regions on chromosomal DNA, a mutant that lacks a 376-kbp region (Non-Patent Document 6), a mutant that lacks a 262-kbp region (Non-Patent Document 7), and a mutant that lacks a 313-kbp region (Non-Patent Document 5) have been reported. However, the growth (the amount of cells or the growth rate) of such mutant strains is merely described as equivalent to that of their parental wild-type strains, and the influence of the introduction of deletions that are 400 kbp or longer into chromosomal DNA imposed upon the growth of *E. coli* is not described.

That is, an *E. coli* mutant, which lacks multiple regions on chromosomal DNA and the growth of which is improved over that of a wild-type *E. coli* strain, and a process for producing a useful substance using such mutant have not yet been known.
Non-Patent Document 1: Science, 277, 1453, 1997
Non-Patent Document 2 : DNA Data Bank of Japan, "Escherichia coli K12 W3110," online, October 29, 2004, Internet <URL: http:llgib.genes.nig.ac.jp/single/index.php?spid=Ecol_K12_W3110> Non-Patent Document 3: J. Bacteriol., 180, 2063, 1998
Non-Patent Document 4: Tanpakushitsu, kakusan, kouso (Protein, nucleic acid, enzyme), vol. 46, p. 2386, 2001
Non-Patent Document 5: Nature Biotechnol., 20, 1018, 2002
Non-Patent Document 6: Genome Research, 12, 640, 2002
Non-Patent Document 7: Genome Research, 13, 644, 2003

### Disclosure of the Invention

### Object of the Invention

It is an object of the present invention to provide an *E. coli* mutant strain that lacks a plurality of regions on chromosomal DNA and a process for producing a useful substance using such mutant.

### Means for Attaining the Object

The present invention relates to the following (1) to (47).
(1) An *E. coli* mutant strain comprising the following genes [1] and [2] or homologous genes thereof and comprising chromosomal DNA that is at least 470 kbp shorter than that of a wild-type *E*. *coli* strain, provided that when the following genes [1] and [2] or homologous genes thereof comprise genes that wild-type *E. coli* strains do not originally contain (hereafter referred to as "NC genes"), the aforementioned *E*. *coli* mutant strain derived from the wild-type *E. coli* strain does not necessarily have the NC gene.
   [1] thrA gene, thrB gene, thrC gene, talB gene, mog gene, htgA gene, dnaK gene, dnaJ gene, hokC gene, nhaA gene, nhaR gene, rpsT gene, ribF gene, ileS gene, lspA gene, fkpB gene, lytB gene, dapB gene, carA gene, carB gene, caiF gene, caiE gene, caiD gene, caiC gene, caiB gene, caiA gene, caiT gene, fixA gene, fixB gene, fixC gene, fixX gene, kefC gene, folA gene, apaH gene, apaG gene, ksgA gene, pdxA gene, surA gene, imp gene, djlA gene, rluA gene, hepA gene, polB gene, araD gene, araA gene, araB gene, araC gene, tbpA gene, leuD gene, leuC gene, leuB gene, leuA gene, leuL gene, leuO gene, ilvI gene, ilvH gene, fruR gene, ftsL gene, ftsI gene, murE gene, murF gene, mraY gene, murD gene, ftsW gene, murG gene, murC gene, ddlB gene, ftsQ gene, ftsA gene, ftsZ gene, IpxC gene, secA gene, mutT gene, yacE gene, guaC gene, hofC gene, hofB gene, ppdD gene, nadC gene, ampD gene, ampE gene, aroP gene, pdhR gene, aceE gene, aceF gene, IpdA gene, acnB gene, speD gene, speE gene, gcd gene, hpt gene, yadF gene, panD gene, panC gene, panB gene, htrE gene, ecpD gene, folK gene, pcnB gene, dksA gene, sfsA gene, ligT gene, hrpB gene, ponB gene, hemL gene, pfs gene, dgt gene, htrA gene, dapD gene, glnD gene, map gene, rpsB gene, tsf gene, smb gene, rrf gene, dxr gene, rth gene, cdsA gene, yaeL gene, hlpA gene, lpxD gene, fabZ gene, lpxA gene, lpxB gene, rnhB gene, dnaE gene, accA gene, ldcC gene, mesJ gene, rof gene, cutF gene, proS gene, rcsF gene, abc gene, dniR gene, gloB gene, rnhA gene, dnaQ gene, up 18 gene, gmhA gene, pepD gene, gpt gene, crl gene, phoE gene, proB gene, proA gene, hemB gene, proC gene, aroL gene, aroM gene, araJ gene, sbcC gene, sbcD gene, phoB gene, phoR gene, brnQ gene, proY gene, malZ gene, queA gene, tgt gene, secD gene, secF gene, tsx gene, ribD gene, ribH gene, nusB gene, thiL gene, pgpA gene, dxs gene, ispA gene, xseB gene, thiI gene, thiJ gene, apbA gene, cyoE gene, cyoD gene, cyoC gene, cyoB gene, cyoA gene, ampG gene, bolA gene, tig gene, clpP gene, clpX gene, Ion gene, hupB gene, cof gene, mdlA gene, mdlB gene, glnK gene, amtB gene, tesB gene, maa gene, hha gene, acrB gene, acrA gene, acrR gene, aefA gene, priC gene, apt gene, dnaX gene, recR gene, htpG gene, adk gene, hemH gene, aes gene, gsk gene, fsr gene, ushA gene, cueR gene, gcl gene, gip gene, purK gene, purE gene, ybbF gene, ppiB gene, cysS gene, folD gene, sfmA gene, sfmC gene, sfmD gene, sfmH gene, sfmF gene, fimZ gene, entD gene, fepA gene, fes gene, entF gene, fepE gene, fepC gene, fepG gene, fepD gene, fepB gene, entC gene, entE gene, entB gene, entA gene, cstA gene, dsbG gene, ahpC gene, ahpF gene, rnk gene, rna gene, citT gene, citG gene, citX gene, citF gene, citE gene, citD gene, citC gene, citA gene, citB gene, dcuC gene, dcuC gene, crcA gene, cspE gene, crcB gene, lipA gene, lipB gene, dacA gene, rlpA gene, mrdB gene, mrdA gene, phpB gene, nadD gene, holA gene, rlpB gene, leuS gene, Int gene, phoL gene, ubiF gene, asnB gene, nagD gene, nagC gene, nagA gene, nagB gene, nagE gene, glnS gene, fur gene, fldA gene, seqA gene, pgm gene, potE gene, speF gene, kdpE gene, kdpD gene, kdpC gene, kdpB gene, kdpA gene, phrB gene, end8 gene, abrB gene, gltA gene, sdhC gene, sdhD gene, sdhA gene, sdhB gene, sucA gene, sucB gene, sucC gene, sucD gene, farR gene, hrsA gene, cydA gene, cydB gene, tolQ gene, tolR gene, tolA gene, tolB gene, pal gene, nadA gene, pnuC gene, czcD gene, aroG gene, gpmA gene, galM gene, galK gene, galT gene, galE gene, modF gene, modE gene, modA gene, modB gene, modC gene, bioA gene, bioB gene, bioF gene, bioC gene, bioD gene, uvrB gene, moaA gene, moaB gene, moaC gene, moaD gene, moaE gene, rhlE gene, dps gene, ompX gene, mipB gene, moeB gene, moeA gene, mdfA gene, grxA gene, poxB gene, aqpZ gene, cspD gene, clpA gene, infA gene, aat gene, cydC gene, cydD gene, trxB gene, lrp gene, ftsK gene, lolA gene, serS gene, dmsA gene, dmsB gene, dmsC gene, pflA gene, pflB gene, focA gene, serC gene, aroA gene, cmk gene, rpsA gene, himD gene, msbA gene, lpxK gene, kdsB gene, smtA gene, mukF gene, kicA gene, mukB gene, aspC gene, ompF gene, asnS gene, pncB gene, pepN gene, ssuB gene, ssuC gene, ssuD gene, ssuA gene, ssuE gene, pyrD gene, uup gene, pqiA gene, pqiB gene, rmf gene, fabA gene, ompA gene, sulA gene, helD gene, mgsA gene, acyP gene, hyaA gene, hyaB gene, hyaC gene, hyaD gene, hyaE gene, hyaF gene, appC gene, appB gene, appA gene, csgG gene, csgF gene, csgE gene, csgD gene, csgB gene, csgA gene, csgC gene, sfaA gene, mdoG gene, mdoH gene, msyB gene, htrB gene, solA gene, dinI gene, pyrC gene, grxB gene, rimJ gene, mviM gene, mviN gene, flgN gene, flgM gene, flgA gene, flgB gene, flgC gene, flgD gene, flgE gene, flgF gene, flgG gene, flgH gene, flgI gene, flgJ gene, flgK gene, flgL gene, rne gene, rluC gene, rpmF gene, plsX gene, fabH gene, fabD gene, fabG gene, acpP gene, fabF gene, pabC gene, tmk gene, holB gene, ptsG gene, fhuE gene, nagZ gene, ndh gene, mfd gene, ycfU gene, ycfV gene, ycfW gene, cobB gene, potD gene, potC gene, potB gene, potA gene, pepT gene, phoQ gene, phoP gene, purB gene, trmU gene, icdA gene, minE gene, minD gene, minC gene, clyA gene, umuD gene, umuC gene, dsbB gene, nhaB gene, fadR gene, dadA gene, dadX gene, ldcA gene, mltE gene, tagl gene, treA gene, dhaR gene, pth gene, prsA gene, ychB gene, hemM gene, hemA gene, prfA gene, hemK gene, kdsA gene, chaA gene, chaB gene, chaC gene, narL gene, narX gene, narK gene, narG gene, narH gene, narJ gene, narI gene, tpr gene, purU gene, sprE gene, galU gene, hns gene, tdk gene, adhE gene, oppA gene, oppB gene, oppC gene, oppD gene, oppF gene, cls gene, kch gene, tonB gene, trpA gene, trpB gene, trpC gene, trpD gene, trpE gene, trpL gene, btuR gene, sohB gene, topA gene, cysB gene, acnA gene, ribA gene, pgpB gene, pyrF gene, osmB gene, rnb gene, envM gene, sapF gene, sapD gene, sapC gene, sapB gene, sapA gene, aldH gene, ordL gene, goaG gene, pspF gene, pspA gene, pspB gene, pspC gene, pspD gene, pspE gene, sucP gene, ompG gene, tyrR gene, tpx gene, mppA gene, fnr gene, ogt gene, dbpA gene, intR gene, lar gene, recT gene, recE gene, racC gene, kil gene, sieB gene, racR gene, dnaC gene, enpP gene, trkG gene, VMTH_LAMBD gene, VLOM_LAMBD gene, tnpO gene, upo3 gene, ompN gene, nifJ gene, hslJ gene, IdhA gene, feaR gene, feaB gene, tynA gene, paaZ gene, paaA gene, paaB gene, paaC gene, paaD gene, paaE gene, paaF gene, paaG gene, paaH gene, paaI gene, paaJ gene, paaK gene, paaX gene, paaY gene, acpD gene, hrpA gene, aldA gene, gapC gene, gapC gene, cybB gene, trg gene, rimL gene, tehA gene, tehB gene, prtC gene, potA gene, potC gene, ansP gene, nhoA gene, narV gene, narW gene, narY gene, narZ gene, narU gene, fdnG gene, fdnH gene, fdnI gene, adhP gene, sfcA gene, rpsV gene, osmC gene, dos gene, xasA gene, gadB gene, pqqL gene, chuR gene, hipA gene, hipB gene, tam gene, uxaB gene, marC gene, marR gene, marA gene, marB gene, dcp gene, lycV gene, dicA gene, dicB gene, rspB gene, rspA gene, speG gene, dmsB gene, dmsC gene, bioD gene, mlc gene, asr gene, ebr gene, pntB gene, pntA gene, arcD gene, rstA gene, rstB gene, tus gene, fumC gene, fumA gene, manA gene, uidC gene, uidB gene, uidA gene, uidR gene, hdhA gene, malI gene, malX gene, malY gene, add gene, blr gene, nth gene, gst gene, pdxY gene, tyrS gene, pdxH gene, slyB gene, slyA gene, sodC gene, nemA gene, gloA gene, rnt gene, lhr gene, sodB gene, araJ gene, purR gene, cfa gene, ribE gene, ag43 gene, phsC gene, nrfC gene, pykF gene, lpp gene, sufS gene, aroD gene, ppsA gene, aroH gene, nlpC gene, btuD gene, btuE gene, btuC gene, himA gene, pheT gene, pheS gene, pheM gene, rplT gene, rpmI gene, infC gene, thrS gene, arp gene, pfkB gene, cedA gene, katE gene, celF gene, celD gene, celC gene, celB gene, celA gene, osmE gene, nadE gene, spy gene, astE gene, astB gene, astD gene, astA gene, cstC gene, xthA gene, gdhA gene, topB gene, selD gene, sppA gene, ansA gene, tdh gene, gapA gene, ttuC gene, caiT gene, rnd gene, fadD gene, slp gene, pabB gene, sdaA gene, manX gene, manY gene, manZ gene, rrmA gene, cspC gene, kdgR gene, tcmA gene, htpX gene, prc gene, pqiB gene, pphA gene, holE gene, exoX gene, ptrB gene, purT gene, eda gene, edd gene, zwf gene, pykA gene, mlt gene, znuA gene, znuC gene, ruvB gene, ruvA gene, ruvC gene, ntpA gene, aspS gene, bisZ gene, torY gene, cutC gene, argS gene, pgsA gene, amn gene, cbl gene, nac gene, erfK gene, cobT gene, cobS gene, cobU gene, gyrI gene, phsE gene, sbcB gene, hisL gene, hisG gene, hisD gene, hisC gene, hisB gene, hisH gene, hisA gene, hisF gene, hisI gene, wzz gene, ugd gene, gnd gene, galF gene, wcaM gene, wcaL gene, wcaK gene, wzxC gene, wcaJ gene, cpsG gene, cpsB gene, wcaI gene, wcaH gene, wcaG gene, gmd gene, wcaF gene, wcaE gene, wcaD gene, wcaC gene, wcaB gene, wcaA gene, wzb gene, wza gene, asmA gene, dcd gene, udk gene, thiD gene, thiM gene, mrp gene, metG gene, cdd gene, sanA gene, mglC gene, mglA gene, mglB gene, galS gene, folE gene, cirA gene, lysP gene, nfo gene, fruA gene, fruK gene, fruB gene, rplY gene, narP gene, ccmH gene, dsbE gene, ccmF gene, ccmE gene, ccmD gene, ccmC gene, ccmB gene, ccmA gene, napC gene, napB gene, napH gene, napG gene, napA gene, napD gene, napF gene, eco gene, alkB gene, ada gene, apbE gene, rcsB gene, rcsC gene, atoS gene, atoC gene, atoD gene, atoA gene, atoE gene, atoB gene, gyrA gene, ubiG gene, nrdA gene, nrdB gene, inaA gene, glpQ gene, glpT gene, glpA gene, glpB gene, glpC gene, cinA gene, ais gene, pmrD gene, menE gene, menC gene, menB gene, menD gene, menF gene, nuoN gene, nuoM gene, nuoL gene, nuoK gene, nuoJ gene, nuol gene, nuoH gene, nuoG gene, nuoF gene, nuoE gene, nuoD gene, nuoB gene, nuoA gene, lrhA gene, ackA gene, pta gene, ubiX gene, purF gene, dedE gene, dedD gene, foIC gene, accD gene, dedA gene, truA gene, usg1 gene, pdxB gene, div gene, fabB gene, ddpX gene, mepA gene, aroC gene, sixA gene, fadL gene, vacJ gene, dsdC gene, dsdX gene, dsdA gene, emrY gene, emrK gene, evgA gene, evgS gene, bifF gene, ddg gene, glk gene, mntH gene, nupC gene, gItX gene, xapR gene, xapB gene, xapA gene, lig gene, zipA gene, cysZ gene, cysK gene, ptsH gene, ptsI gene, gsr gene, pdxK gene, cysM gene, cysA gene, cysW gene, cysU gene, cysP gene, ucpA gene, ptbA gene, amiA gene, hemF gene, cchA gene, eutC gene, eutB gene, eutH gene, eutG gene, eutJ gene, eutE gene, cchB gene, eutI gene, talA gene, tktB gene, aegA gene, narQ gene, acrD gene, dapE gene, purC gene, nlpB gene, dapA gene, gcvR gene, bcp gene, hyfA gene, hyfB gene, hyfC gene, hyfD gene, hyfE gene, hyfF gene, hyfG gene, hyfH gene, hyfI gene, hyfJ gene, hyfR gene, focB gene, perM gene, yfgE gene, uraA gene, upp gene, purM gene, purN gene, ppk gene, ppx gene, guaA gene, guaB gene, xseA gene, hiss gene, gcpE gene, ndk gene, pbpC gene, sseA gene, sseB gene, pepB gene, fdx gene, hscA gene, hscB gene, iscU gene, iscS gene, iscR gene, suhB gene, csiE gene, dgxA gene, dgxB gene, hcaC gene, hcaB gene, hcaD gene, glyA gene, hmpA gene, glnB gene, purL gene, acpS gene, pdxJ gene, recO gene, era gene, rnc gene, lepB gene, lepA gene, rseC gene, rseB gene, rseA gene, rpoE gene, nadB gene, srmB gene, ung gene, trx2 gene, pssA gene, kgtP gene, clpB gene, rluD gene, pheL gene, pheA gene, tyrA gene, aroF gene, rplS gene, trmD gene, rimM gene, rpsP gene, ffh gene, grpE gene, recN gene, smpA gene, smpB gene, gabD gene, gabT gene, gabP gene, nrdF gene, emrR gene, emrA gene, emrB gene, gshA gene, csrA gene, alaS gene, oraA gene, recA gene, mltB gene, hypF gene, hydN gene, ascG gene, ascF gene, ascB gene, hycI gene, hycH gene, hycG gene, hycF gene, hycE gene, hycD gene, hycC gene, hycB gene, hycA gene, hypA gene, hypB gene, hypC gene, hypD gene, hypE gene, fhlA gene, mutS gene, prpB gene, rpoS gene, pcm gene, surE gene, ygbB gene, ygbP gene, ygbQ gene, cysC gene, cysN gene, cysD gene, iap gene, cysH gene, cysI gene, cysJ gene, ptpS gene, eno gene, pyrG gene, mazG gene, chpR gene, relA gene, barA gene, gud2 gene, gudT gene, mioC gene, syd gene, sdaB gene, exo gene, fucO gene, fucA gene, fucP gene, fucI gene, fucK gene, fucU gene, fucR gene, gcvA gene, nifS gene, mltA gene, amiC gene, argA gene, thyA gene, lgt gene, ptsP gene, mutH gene, tas gene, aas gene, galR gene, lysA gene, lysR gene, araE gene, kduD gene, kduI gene, lysS gene, prfB gene, recJ gene, dsbC gene, xerD gene, fldB gene, up14 gene, bglA gene, gcvP gene, gcvH gene, gcvT gene, visC gene, ubiH gene, pepP gene, serA gene, rpiA gene, iciA gene, sbm gene, argK gene, fba gene, pgk gene, epd gene, tktA gene, speB gene, speA gene, metK gene, galP gene, sprT gene, endA gene, gshB gene, yqgF gene, ansB gene, mutY gene, mltC gene, nupG gene, speC gene, pitB gene, gsp gene, hybG gene, hybF gene, hybE gene, hybD gene, hybC gene, hybB gene, hybA gene, hoxK gene, exbD gene, exbB gene, metC gene, exsA gene, sufI gene, plsC gene, parC gene, parE gene, icc gene, tolC gene, ribB gene, glgS gene, rfaE gene, glnE gene, cca gene, bacA gene, folB gene, ttdA gene, ttdB gene, ygjD gene, rpsU gene, dnaG gene, rpoD gene, aer gene, ebgR gene, ebgA gene, ebgC gene, fadH gene, uxaA gene, uxaC gene, exuT gene, exuR gene, tdcE gene, tdcD gene, tdcC gene, tdcB gene, tdcA gene, tdcR gene, sohA gene, agaR gene, agaZ gene, agaV gene, agaW gene, agaA gene, agaS gene, agaY gene, agaB gene, agaC gene, agaD gene, agaI gene, mtr gene, deaD gene, nlpI gene, pnp gene, rpsO gene, truB gene, rbfA gene, infB gene, nusA gene, argG gene, secG gene, mrsA gene, folP gene, fisH gene, ftsJ gene, greA gene, dacB gene, yhbZ gene, rpmA gene, rplU gene, ispB gene, nlp gene, murA gene, yrbI gene, yrbK gene, rpoN gene, ptsN gene, ptsO gene, mtgA gene, arcB gene, gltB gene, nanT gene, npl gene, nanR gene, dcuD gene, sspB gene, sspA gene, rpsI gene, rplM gene, hhoA gene, hhoB gene, mdh gene, argR gene, tldD gene, rng gene, mreD gene, mreC gene, mreB gene, accB gene, accC gene, panF gene, prmA gene, fis gene, aroE gene, smg gene, smf gene, def gene, fmt gene, sun gene, trkA gene, mscL gene, zntR gene, rplQ gene, rpoA gene, rpsD gene, rpsK gene, rpsM gene, rpmJ gene, secY gene, rplo gene, rpmD gene, rpsE gene, rplR gene, rplF gene, rpsH gene, rpsN gene, rplE gene, rplX gene, rplN gene, rpsQ gene, rpmC gene, rplP gene, rpsC gene, rplV gene, rpsS gene, rplB gene, rplW gene, rplD gene, rplC gene, rpsJ gene, tufA gene, fusA gene, rpsG gene, rpsL gene, fkpA gene, slyX gene, slyD gene, kefB gene, prkB gene, crp gene, argD gene, pabA gene, fic gene, ppiA gene, nirB gene, nirD gene, nirC gene, cysG gene, php gene, trpS gene, gph gene, rpe gene, dam gene, damX gene, aroB gene, aroK gene, hofQ gene, mrcA gene, pckA gene, envZ gene, ompR gene, greB gene, feoA gene, feoB gene, bioH gene, gntT gene, malQ gene, malP gene, malT gene, rtcA gene, rtcB gene, rtcR gene, glpR gene, glpG gene, glpE gene, glpD gene, glgP gene, glgA gene, glgC gene, glgX gene, glgB gene, asd gene, gntU gene, gntU gene, gntK gene, gntR gene, ggt gene, ugpQ gene, ugpC gene, ugpE gene, ugpA gene, ugpB gene, livF gene, livG gene, livM gene, livH gene, livK gene, livJ gene, rpoH gene, ftsX gene, ftsE gene, ftsY gene, atzN gene, nikA gene, nikB gene, nikC gene, nikD gene, nikE gene, pitA gene, uspB gene, uspA gene, prlC gene, gor gene, arsR gene, arsB gene, arsC gene, slp gene, hdeB gene, hdeA gene, hdeD gene, gadA gene, treF gene, kdgK gene, dctA gene, dppF gene, dppD gene, dppC gene, dppB gene, dppA gene, tag gene, bisC gene, cspA gene, glyS gene, glyQ gene, xylB gene, xylA gene, xylF gene, xylG gene, xylH gene, xylR gene, bax gene, malS gene, avtA gene, lyx gene, sgbH gene, sgbU gene, sgbE gene, aldB gene, selB gene, selA gene, mtlA gene, mtld gene, mtlR gene, lldP gene, lldR gene, lldD gene, cysE gene, gpsA gene, secB gene, grxC gene, tdh gene, kbl gene, htrL gene, rfaD gene, rfaF gene, rfaC gene, rfaL gene, rfaK gene, rfaZ gene, rfaY gene, rfaJ gene, rfaI gene, rfaB gene, rfaS gene, rfaP gene, rfaG gene, rfaQ gene, kdtA gene, kdtB gene, mutM gene, rpmG gene, rpmB gene, radC gene, dfp gene, dut gene, ttk gene, pyrE gene, rph gene, dinD gene, gmk gene, rpoZ gene, spoT gene, spoU gene, recG gene, gItS gene, nlpA gene, uhpT gene, uhpC gene, uhpB gene, uhpA gene, ilvN gene, ilvB gene, ivbL gene, emrD gene, ibpB gene, ibpA gene, gyrB gene, recF gene, dnaN gene, dnaA gene, rpmH gene, rnpA gene, yidC gene, thdF gene, tnaL gene, tnaA gene, tnaB gene, bglB gene, bglF gene, bglG gene, phoU gene, pstB gene, pstA gene, pstC gene, pstS gene, glmS gene, glmU gene, atpC gene, uncD gene, atpG gene, atpA gene, atpH gene, atpF gene, atpE gene, atpB gene, atpI gene, gidB gene, gidA gene, mioC gene, asnC gene, pssR gene, ilvL gene, ilvG gene, ilvM gene, ilvE gene, ilvD gene, ilvA gene, ilvY gene, ilvC gene, ppiC gene, rep gene, gppA gene, rhlb gene, trxA gene, rhoL gene, rho gene, rfe gene, wzzE gene, nfrC gene, rffD gene, rffG gene, rffH gene, rffA gene, wzxE gene, rffT gene, wecG gene, aslB gene, aslA gene, hemY gene, hemX gene, hemD gene, hemC gene, cyaA gene, cyaY gene, dapF gene, xerC gene, uvrD gene, corA gene, radD gene, pldA gene, recQ gene, pldB gene, metR gene, metE gene, udp gene, ubiE gene, aarF gene, tatC gene, tatD gene, rfaH gene, ubiD gene, ubiB gene, fadA gene, fadB gene, pepQ gene, trkH gene, trkH gene, hemG gene, mobB gene, mobA gene, dsbA gene, polA gene, yihA gene, hemN gene, ntrC gene, ntrB gene, glnA gene, fdhE gene, fdoI gene, fdoH gene, fdoG gene, fdhD gene, sodA gene, kdgT gene, cpxA gene, cpxR gene, cpxP gene, pfkA gene, sbp gene, cdh gene, tpiA gene, fpr gene, glpX gene, glpK gene, glpF gene, menG gene, menA gene, hslU gene, hsIV gene, ftsN gene, cytR gene, priA gene, rpmE gene, metJ gene, metB gene, metL gene, metF gene, katG gene, gldA gene, talC gene, ppc gene, argE gene, argC gene, argB gene, argH gene, oxyR gene, udhA gene, trmA gene, btuB gene, murI gene, murB gene, birA gene, coaA gene, tufB gene, secE gene, nusG gene, rplK gene, rplA gene, rplJ gene, rplL gene, rpoB gene, rpoC gene, htrC gene, thiH gene, thiG gene, thiS gene, thiF gene, thiE gene, thiC gene, rsd gene, hemE gene, nfi gene, hupA gene, hydH gene, hydG gene, purD gene, purH gene, metA gene, aceB gene, aceA gene, aceK gene, arp gene, iclR gene, metH gene, pepE gene, lysC gene, pgi gene, ubiC gene, ubiA gene, plsB gene, dgkA gene, lexA gene, dinF gene, qor gene, dnaB gene, alr gene, tyrB gene, napA gene, hobH gene, uvrA gene, ssb gene, soxS gene, soxR gene, acs gene, nrfA gene, nrfB gene, nrfC gene, nrfD gene, nrfE gene, nrfF gene, nrfG gene, gltP gene, fdhF gene, alsR gene, rpiB gene, proP gene, basS gene, basR gene, adiY gene, adi gene, melR gene, melA gene, melB gene, fumB gene, dcuB gene, dcuR gene, dcuS gene, lysU gene, cadA gene, cadB gene, cadC gene, cutA3 gene, cutA2 gene, cutA gene, dcuA gene, aspA gene, fxsA gene, groES gene, groEL gene, efp gene, ecnB gene, sugE gene, blc gene, ampC gene, frdD gene, frdC gene, frdB gene, frdA gene, genX gene, psd gene, yjeQ gene, yjeE gene, amiB gene, mutL gene, miaA gene, hfq gene, hflX gene, hflK gene, hflC gene, purA gene, vacB gene, sgaE gene, rpsF gene, priB gene, rpsR gene, rplI gene, fklB gene, cycA gene, cpdB gene, cysQ gene, msrA gene, chpS gene, chpB gene, ppa gene, fbp gene, mpl gene, pmbA gene, cybC gene, nrdG gene, nrdD gene, treC gene, treB gene, treR gene, mgtA gene, pyrI gene, pyrB gene, pyrBI gene, argI gene, valS gene, holC gene, xerB gene, idnR gene, idnT gene, idnO gene, idnD gene, idnK gene, fecE gene, fecD gene, fecC gene, fecB gene, fecA gene, fecR gene, fecI gene, dnaC gene, dnaT gene, bglJ gene, fhuF gene, rsmC gene, holD gene, rimI gene, prfC gene, osmY gene, deoC gene, deoA gene, deoB gene, deoD gene, IplA gene, smp gene, serB gene, radA gene, nadR gene, slt gene, trpR gene, gpmG gene, rob gene, creA gene, creB gene, creC gene, creD gene, arcA gene, lasT gene, thrL gene;
   [2] acrE gene, argT gene, artI gene, artJ gene, artM gene, artP gene, artQ gene, bfr gene, chiA gene, chpA gene, dacC gene, deoR gene, dinG gene, elaA gene, elaB gene, elaC gene, elaD gene, envD gene, envR gene, fhuA gene, fhuB gene, fhuC gene, fhuD gene, folX gene, glnH gene, glnP gene, glnQ gene, gltJ gene, gltK gene, gltL gene, gspA gene, gspC gene, gspD gene, gspE gene, gspI gene, gspJ gene, gspK gene, gspL gene, hisJ gene, hisM gene, hisP gene, hisQ gene, hofD gene, hofF gene, hofG gene, hofH gene, ltaA gene, mdaA gene, mdaB gene, pinO gene, potF gene, potG gene, potH gene, potI gene, pshM gene, ptwB gene, ptwC gene, rhsB gene, rimK gene, xylU gene.
(2) The *E*. *coli* mutant strain according to above (1) comprising chromosomal DNA that comprises DNA having the nucleotide sequences as shown in [1] to [69] below of the nucleotide sequence as shown in SEQ ID NO: 1 or DNA equivalent thereto.
   [1] a nucleotide sequence of nucleotides 1 to 167064
   [2] a nucleotide sequence of nucleotides 172980 to 243964
   [3] a nucleotide sequence of nucleotides 253411 to 261963
   [4] a nucleotide sequence of nucleotides 387532 to 389138
   [5] a nucleotide sequence of nucleotides 403704 to 518029
   [6] a nucleotide sequence of nucleotides 532712 to 535504
   [7] a nucleotide sequence of nucleotides 550216 to 563940
   [8] a nucleotide sequence of nucleotides 608119 to 675103
   [9] a nucleotide sequence of nucleotides 689375 to 728820
   [10] a nucleotide sequence of nucleotides 739594 to 832551
   [11] a nucleotide sequence of nucleotides 848090 to 867637
   [12] a nucleotide sequence of nucleotides 883474 to 890997
   [13] a nucleotide sequence of nucleotides 909380 to 1048887
   [14] a nucleotide sequence of nucleotides 1097020 to 1198105
   [15] a nucleotide sequence of nucleotides 1225147 to 1630911
   [16] a nucleotide sequence of nucleotides 1641039 to 1642631
   [17] a nucleotide sequence of nucleotides 1650223 to 1964523
   [18] a nucleotide sequence of nucleotides 1993076 to 1996661
   [19] a nucleotide sequence of nucleotides 2028210 to 2033448
   [20] a nucleotide sequence of nucleotides 2044377 to 2069148
   [21] a nucleotide sequence of nucleotides 2081927 to 2104392
   [22] a nucleotide sequence of nucleotides 2116065 to 2146263
   [23] a nucleotide sequence of nucleotides 2187856 to 2200668
   [24] a nucleotide sequence of nucleotides 2229851 to 2255894
   [25] a nucleotide sequence of nucleotides 2263492 to 2270439
   [26] a nucleotide sequence of nucleotides 2286589 to 2386261
   [27] a nucleotide sequence of nucleotides 2395472 to 2423364
   [28] a nucleotide sequence of nucleotides 2434099 to 2472851
   [29] a nucleotide sequence of nucleotides 2482483 to 2505074
   [30] a nucleotide sequence of nucleotides 2514558 to 2755676
   [31] a nucleotide sequence of nucleotides 2789481 to 2795173
   [32] a nucleotide sequence of nucleotides 2800891 to 2804307
   [33] a nucleotide sequence of nucleotides 2810148 to 2825313
   [34] a nucleotide sequence of nucleotides 2830263 to 2910273
   [35] a nucleotide sequence of nucleotides 2910574 to 2985361
   [36] a nucleotide sequence of nucleotides 3033129 to 3077084
   [37] a nucleotide sequence of nucleotides 3078917 to 3110109
   [38] a nucleotide sequence of nucleotides 3134342 to 3165627
   [39] a nucleotide sequence of nucleotides 3172941 to 3360736
   [40] a nucleotide sequence of nucleotides 3370548 to 3411159
   [41] a nucleotide sequence of nucleotides 3420879 to 3453020
   [42] a nucleotide sequence of nucleotides 3469367 to 3618575
   [43] a nucleotide sequence of nucleotides 3636917 to 3854661
   [44] a nucleotide sequence of nucleotides 3865832 to 3867101
   [45] a nucleotide sequence of nucleotides 3877011 to 3926658
   [46] a nucleotide sequence of nucleotides 3938879 to 4059897
   [47] a nucleotide sequence of nucleotides 4079660 to 4086450
   [48] a nucleotide sequence of nucleotides 4099983 to 4139231
   [49] a nucleotide sequence of nucleotides 4149710 to 4235358
   [50] a nucleotide sequence of nucleotides 4251736 to 4298976
   [51] a nucleotide sequence of nucleotides 4311512 to 4313317
   [52] a nucleotide sequence of nucleotides 4329722 to 4409532
   [53] a nucleotide sequence of nucleotides 4423152 to 4496201
   [54] a nucleotide sequence of nucleotides 4509535 to 4517875
   [55] a nucleotide sequence of nucleotides 4599638 to 4641433
   [56] a nucleotide sequence of nucleotides 167065 to 172979
   [57] a nucleotide sequence of nucleotides 675104 to 689374
   [58] a nucleotide sequence of nucleotides 832552 to 848089
   [59] a nucleotide sequence of nucleotides 867638 to 883473
   [60] a nucleotide sequence of nucleotides 890998 to 909379
   [61] a nucleotide sequence of nucleotides 2386262 to 2395471
   [62] a nucleotide sequence of nucleotides 2423365 to 2434098
   [63] a nucleotide sequence of nucleotides 2472852 to 2482482
   [64] a nucleotide sequence of nucleotides 2505075 to 2514557
   [65] a nucleotide sequence of nucleotides 2910274 to 2910573
   [66] a nucleotide sequence of nucleotides 3165628 to 3172940
   [67] a nucleotide sequence of nucleotides 3411160 to 3420878
   [68] a nucleotide sequence of nucleotides 3453021 to 3469366
   [69] a nucleotide sequence of nucleotides 3618576 to 3636916
(3) The *E*. *coli* mutant strain according to (1) or (2) comprising chromosomal DNA that does not comprise yafJ gene, yafK gene, yaflQ gene, dinJ gene, yafL gene, yafM gene, fhiA gene, lafU gene, dinP gene, yafN gene, yafO gene, yafP gene, JW0225 gene, prfH gene, ykfI gene, yafW gene, yeeT gene, ykfG gene, yafX gene, ykfF gene, ykfB gene, yafY gene, ypjK gene, yafZ gene, ykfA gene, perR gene, JW0245 gene, JW0246 gene, JW0247 gene, ykfC gene, JW0249 gene, is5B gene, yi51 gene, mmuP gene, mmuM gene, afuC gene, JW0255 gene, insB gene, insA gene, JW0258 gene, yagB gene, yagA gene, yagE gene, yagF gene, yagG gene, yagH gene, yagI gene, argF gene, yfji gene, yagJ gene, yagK gene, yagL gene, yagM gene, yagN gene, intF gene, yagP gene, yagQ gene, yagR gene, yagS gene, yagT gene, yagU gene, ykgJ gene, yagV gene, yagW gene, yagX gene, yagY gene, yagZ gene, ykgK gene, ykgL gene, ykgM gene, eaeH gene, JW0292 gene, JW0293 gene, ykgA gene, ykgB gene, ykgI gene, merA gene, ykgD gene, ykgE gene, ykgF gene, ykgG gene, ykgH gene, betA gene, betB gene, betI gene, betT gene, yahA gene, yahB gene, yahC gene, yahD gene, yahE gene, yahF gene, yahG gene, yahH gene, arcM gene, yahJ gene, yahK gene, yahL gene, yahM gene, yahN gene, yahO gene, prpR gene, bcpA gene, prpC gene, prpD gene, prpE gene, codB gene, codA gene, cynR gene, cynT gene, cynS gene, cynX gene, lacA gene, lacY gene, lacZ gene, lacI gene, mhpR gene, mhpA gene, mhpB gene, mhpC gene, mhpD gene, mhpF gene, mhpE gene, mhpT gene, yaiL gene, yaiM gene, adhC gene, yaiN gene, yaiO gene, JW0350 gene, yi2A gene, yi22 gene, yi24 gene, yi23 gene, yaiP gene, yaiS gene, tauA gene, tauB gene, tauC gene, ssiD gene, yaiT gene, JW0363 gene, JW0364 gene, yaiU gene, yaiV gene, ampH gene, sbmA gene, yaiW gene, yaiY gene, yaiZ gene, ddlA gene, yaiB gene, phoA gene, psiF gene, yaiC gene, ybbO gene, tesA gene, ybbA gene, ybbP gene, rhsD gene, ybbC gene, rhsE gene, ybbD gene, JW0490 gene, ybbB gene, ybbS gene, ybbT gene, ybbU gene, ybbQ gene, ybbV gene, allP gene, allA gene, ybbY gene, ybbZ gene, ylbA gene, allC gene, ylbC gene, fdrA gene, ylbE gene, ylbF gene, arcC gene, intD gene, EXO_LAMBD gene, VRPP_LAMBD gene, JW0528 gene, JW0529 gene, VREN_LAMBD gene, emrE gene, ybcK gene, ybcL gene, ybcM gene, ybcN gene, ninE gene, ybcO gene, rus gene, ybcQ gene, JW0539.5 gene, nmpC gene, vlyS gene, ybcS gene, ybcT gene, vboR gene, ybcV gene, ybcW gene, nohB gene, ybcX gene, ybcY gene, JW0552 gene, appY gene, ompT gene, envY gene, ybcH gene, nfrA gene, nfrB gene, cusS gene, cusR gene, cusC gene, ylcC gene, cusB gene, cusA gene, pheP gene, ybdG gene, nfnB gene, ybdF gene, ybdJ gene, ybdK gene, hokE gene, yi81 gene, yi82 gene, ymcC gene, ymcD gene, cspH gene, cspG gene, sfa gene, yccL gene, yccM gene, torS gene, torT gene, torR gene, torC gene, torA gene, torD gene, yccD gene, cbpA gene, yccE gene, agp gene, yccJ gene, wrbA gene, yciG gene, ycdG gene, JW0992 gene, JW0993 gene, JW0994 gene, ycdK gene, JW0996 gene, JW0997 gene, ycdC gene, putA gene, yzpU gene, putP gene, JW1002 gene, ycdO gene, ycdB gene, phoH gene, ycdP gene, ycdQ gene, ycdR gene, ycdS gene, ycdT gene, JW1012 gene, JW 1013 gene, JW1014 gene, ycdU gene, ymfD gene, ymfE gene, lit gene, intE gene, VXIS_BPP21 gene, ymfH gene, ymfI gene, ymfJ gene, RPC2_BPP22 gene, JW1132 gene, ymfL gene, ymfM gene, ymfN gene, ymfR gene, ymfO gene, JW 1138 gene, JW 113 9 gene, ycfK gene, ymfS gene, ycfA gene, ycfE gene, pin gene, mcrA gene, JW1146 gene, ycgW gene, ycgX gene, ycgE gene, JW1150 gene, ycgZ gene, ymgA gene, JW1153 gene, ymgC gene, yahA gene, JW1156 gene, JW1157 gene, aidA gene, ymgD gene, JW1160 gene, JW1161 gene, JW1162 gene, ydfH gene, ydfZ gene, ydfI gene, shiA gene, JW1537 gene, tnpR gene, ydfM gene, Y314_LAMBD gene, nohA gene, ydfO gene, yccL gene, cspI gene, ydfR gene, vlyS gene, cspB gene, cspF gene, regQ_LAMBD gene, JW1552 gene, rem gene, hokD gene, relE gene, relB gene, ydfV gene, flxA gene, ydfW gene, ydfX gene, dicC gene, sdaC gene, yqeH gene, yqeI gene, yqeJ gene, yqeK gene, ygeF gene, ygeG gene, ygeH gene, ygeI gene, JW2822 gene, ygeK gene, JW2824 gene, JW2825 gene, JW2830 gene, ygeP gene, JW2832 gene, nlpD gene, yagR gene, ygeT gene, JW2836 gene, ygeV gene, ygeW gene, dpaL gene, ygeY gene, ygeZ gene, arcL gene, yqeB gene, JW2844 gene, ygfJ gene, gltD gene, JW2847 gene, ygfM gene, yagR gene, ygfO gene, ygfP gene, ygfQ gene, ygfR gene, ygfS gene, ygfT gene, pbuX gene, idi gene, epsM gene, exeL gene, exeC gene, JW2938 gene, JW2939 gene, JW2940 gene, JW2941 gene, yghK gene, glcB gene, glcG gene, glcF gene, glcD gene, glcC gene, JW2948 gene, yghQ gene, yghR gene, yghS gene, yghT gene, yihL gene, yihM gene, yihN gene, yshA gene, yihO gene, yihP gene, yihQ gene, yihR gene; yihS gene, yihT gene, yihU gene, yihV gene, yihW gene, yihX gene, rbn gene, yihZ gene, yiiD gene, yiiE gene, yiiF gene, yiiG gene, frvR gene, frvX gene, frvB gene, fivA gene, yiiL gene, rhaD gene, rhaA gene, rhaB gene, rhaS gene, rhaR gene, rhaT gene, ptsA gene, JW3920 gene, frwC gene, frwB gene, pfID gene, pfIC gene, frwD gene, yijO gene, yijP gene, JW3986 gene, yjbF gene, yjbG gene, yjbH gene, JW3989.5 gene, yjbA gene, xylE gene, malG gene, malF gene, malE gene, malK gene, lamB gene, malM gene, yjbI gene, yjcP gene, yjcQ gene, yjcR gene, yjcS gene, alsK gene, alsE gene, alsC gene, alsA gene, alsB gene, phnQ gene, phnP gene, phnO gene, phnN gene, phnM gene, phnL gene, phnK gene, phnJ gene, phnI gene, phnH gene, phnG gene, phnF gene, phnE gene, phnD gene, phnC gene, phnB gene, phnA gene, yjdA gene and yjcZ gene, or homologous gene thereof.
(4) The *E*. *coli* mutant strain according to any one of (1) to (3) comprising chromosomal DNA that does not comprise DNA having the nucleotide sequences as shown in [1] to [18] below of the nucleotide sequence as shown in SEQ ID NO: 1 or DNA equivalent thereto.
   [1] a nucleotide sequence of nucleotides 243965 to 253410
   [2] a nucleotide sequence of nucleotides 261964 to 387531
   [3] a nucleotide sequence of nucleotides 389139 to 403703
   [4] a nucleotide sequence of nucleotides 518030 to 532711
   [5] a nucleotide sequence of nucleotides 535505 to 550215
   [6] a nucleotide sequence of nucleotides 563941 to 608118
   [7] a nucleotide sequence of nucleotides 1048888 to 1097019
   [8] a nucleotide sequence of nucleotides 1198106 to 1225146
   [9] a nucleotide sequence of nucleotides 1630912 to 1641038
   [10] a nucleotide sequence of nucleotides 1642632 to 1650222
   [11] a nucleotide sequence of nucleotides 2985362 to 3033128
   [12] a nucleotide sequence of nucleotides 3110110 to 3134341
   [13] a nucleotide sequence of nucleotides 4059898 to 4079659
   [14] a nucleotide sequence of nucleotides 4086451 to 4099982
   [15] a nucleotide sequence of nucleotides 4139232 to 4149709
   [16] a nucleotide sequence of nucleotides 4235359 to 4251735
   [17] a nucleotide sequence of nucleotides 4298977 to 4311511
   [18] a nucleotide sequence of nucleotides 4313318 to 4329721
(5) The *E*. *coli* mutant strain according to any one of (1) to (4) comprising chromosomal DNA that does not comprise yidF gene, yidG gene, yidH gene, yidI gene, yidJ gene, yidK gene, yidL gene, JW3657 gene, glvG gene, glvB gene, glvC gene, yidP gene, yidE gene, yidQ gene, yidR gene, yidS gene, dgoT gene, dgoA gene, dgoK gene, dgoR gene, yidW gene, yidX gene, yidA gene, yidB gene, asnA gene, yieM gene, JW3724 gene, yieN gene, kup gene, rbsD gene, rbsA gene, rbsC gene, rbsB gene, rbsK gene, rbsR gene and yieO gene, or homologous gene thereof.
(6) The *E*. *coli* mutant strain according to any one of (1) to (5) comprising chromosomal DNA that does not comprise DNA having the nucleotide sequences as shown in [1] to [3] below of the nucleotide sequence as shown in SEQ ID NO: 1 or DNA equivalent thereto.
   [1] a nucleotide sequence of nucleotides3854662 to 3865831
   [2] a nucleotide sequence of nucleotides3867102 to 3877010
   [3] a nucleotide sequence of nucleotides 3926659 to 3938878
(7) The *E*. *coli* mutant strain according to any one of (1) to (6) comprising chromosomal DNA that does not comprise yecT gene, flhE gene, flhA gene, flhB gene, cheZ gene, cheY gene, cheB gene, cheR gene, tap gene, tar gene, cheW gene, cheA gene, motB gene, motA gene, flhC gene, flhD gene, JW1882 gene, JW1883 gene, yecG gene, otsA gene, otsB gene, araH gene, araG gene, araF gene, yecI gene, yecJ gene, JW1892 gene, ftn gene, yecH gene, tyrP gene, uvrC gene, uvrY gene, yecF gene, sdiA gene, yecC gene, yecS gene, yedO gene, fliY gene, fliZ gene, fliA gene, flic gene, fliD gene, fliS gene, fliT gene, amyA gene, yedD gene, yedE gene, yedF gene, yedK gene, yedL gene, yedN gene, yedM gene, fliE gene, flip gene, fliG gene, fliH gene, fliI gene, fliJ gene, fliK gene, fliL gene, fliM gene, fliN gene, fliO gene, fliP gene, fliQ gene, fliR gene, rcsA gene, dsrB gene, JW 1937 gene, yedA gene, vsr gene, dcm gene, yedJ gene, JW1946 gene, ompC gene, JW1948 gene, yedS gene, yedU gene, yedV gene, copR gene, yedX gene, JW1954 gene, JW1955 gene, JW1956 gene, JW1974 gene, JW1979 gene, JW1980 gene, yeeP gene, flu gene, JW1983 gene, yeeS gene, yeeU gene, yeeV gene, yeeW gene, yefJ gene, JW2013 gene, yefJ gene, yefI gene, wbbJ gene, yefG gene, rfc gene, glf gene, rfbX gene, rfbC gene, rfbA gene, rfbD gene and rfbB gene, or homologous gene thereof.
(8) The *E. coli* mutant strain according to any one of (1) to (7) comprising chromosomal DNA that does not comprise DNA having any of the nucleotide sequences as shown in [1] to [5] below of the nucleotide sequence as shown in SEQ ID NO: 1 or DNA equivalent thereto.
   [1] a nucleotide sequence of nucleotides1964524 to 1993075
   [2] a nucleotide sequence of nucleotides 1996662 to 2028209
   [3] a nucleotide sequence of nucleotides 2033449 to 2044376
   [4] a nucleotide sequence of nucleotides 2069149t to 2081926
   [5] a nucleotide sequence of nucleotides 2104393 to 2116064
(9) The *E*. *coli* mutant strain according to any one of (1) to (8) comprising chromosomal DNA that does not comprise intA gene, yfjH gene, alpA gene, yfjI gene, JW2606 gene, yfjJ gene, yfjK gene, yfjL gene, yfjM gene, yfjn gene, yfjO gene, yfjP gene, yfjQ gene, yfjR gene, ypjK gene, yfjS gene, yfjT gene, yfjU gene, yfjV gene, JW2622 gene, yfjW gene, JW2623.5 gene, yfjX gene, yfjY gene, yfjZ gene, ypjF gene, ypjA gene, JW2629 gene, JW2630 gene, JW2631 gene, JW2632 gene, JW2633 gene, JW2634 gene, ygaE gene, yzzM gene, yqaE gene, JW2642 gene, ygaP gene, stpA gene, JW2645 gene, ygaC gene, ygaM gene, nrdH gene, nrdI gene, nrdE gene, proV gene, proW gene, proX gene, JW2655 gene, JW2656 gene, ygaZ gene, ygaH gene, srlA gene, srlB gene, srlD gene, gutM gene, srlR gene and gutQ gene, or homologous gene thereof.
(10) The *E. coli* mutant strain according to any one of (1) to (9) comprising chromosomal DNA that does not copmrise DNA having the nucleotide sequences as shown in [1] to [4] below of the nucleotide sequence as shown in SEQ ID NO: 1 or DNA equivalent thereto.
   [1] a nucleotide sequence of nucleotides 2755677 to 2789480
   [2] a nucleotide sequence of nucleotides 2795174 to 2800890
   [3] a nucleotide sequence of nucleotides 2804308 to 2810147
   [4] a nucleotide sequence of nucleotides 2825314 to 2830262
(11) The *E*. *coli* mutant strain according to any one of (1) to (10) comprising chromosomal DNA that does not comprise gltF gene, yhcA gene, yhcD gene, yhcE gene, yhcF gene, yhcG gene, yhcH gene, nanK gene and nanE gene, or homologous gene thereof.
(12) The *E*. *coli* mutant strain according to any one of (1) to (11) comprising chromosomal DNA that does not comprise DNA having a nucleotide sequence comprising nucleotides 3360737 to 3370547 of the nucleotide sequence as shown in SEQ ID NO: 1 or DNA equivalent thereto.
(13) The *E*. *coli* mutant strain according to any one of (1) to (12) comprising chromosomal DNA that does not comprise yegE gene, alkA gene, yegD gene, JW2055 gene, JW2056 gene, JW2057 gene, JW2058 gene, yegM gene, yegN gene, yegO gene, yegB gene, baeS gene, baeR gene, JW2065 gene, yegQ gene, ogrK gene, JW2068 gene, JW2069 gene, yegS gene, gatR gene, JW2072 gene, JW2073 gene, gatR gene, gatD gene, gatC gene, gatB gene, gatA gene, gatZ gene, gatY gene, fbaB gene, xegT gene, yegU gene, yegV gene, yegW gene, yegX gene, molR gene, yehI gene, yehK gene, yehL gene, yehO gene, yehP gene, yehQ gene, yehR gene, yehS gene, yehT gene, yehU gene, yehV gene, yehW gene, yehX gene, yehY gene, yehZ gene, bglX gene, dld gene, pbpG gene, yohC gene, yeiQ gene, yeiR gene, yeiU gene, spr gene, rtn gene, yejA gene, yejB gene, yejE gene, yejF gene, yejG gene, bcr gene, rsuA gene, yejH gene, yeiI gene, yeiJ gene, yeiK gene, yeiL gene, yeiM gene, yeiN gene and yeiC gene, or homologous gene thereof.
(14) The *E. coli* mutant strain according to any one of (1) to (13) comprising chromosomal DNA that does not comprise DNA having the nucleotide sequences as shown in [1] to [4] below of the nucleotide sequence as shown in SEQ ID NO: 1 or DNA equivalent thereto.
   [1] a nucleotide sequence of nucleotides 2146264 to 2187855
   [2] a nucleotide sequence of nucleotides 2200669 to 2229850
   [3] a nucleotide sequence of nucleotides 2270440 to 2286588
   [4] a nucleotide sequence of nucleotides 2255895 to 2263491
(15) The *E. coli* mutant strain according to any one of (1) to (14) comprising chromosomal DNA that does not comprise cmtA gene, cmtB gene, or homologous gene thereof.
(16) The *E. coli* mutant strain according to any one of (1) to (15) comprising chromosomal DNA that does not comprise DNA having a nucleotide sequence comprising nucleotides 3077085 to 3078916 of the nucleotide sequence as shown in SEQ ID NO: 1 or DNA equivalent thereto.
(17) The *E. coli* mutant strain according to any one of (1) to (16) comprising chromosomal DNA that does not comprise intB gene, JW4228 gene, yjgW gene, yjgX gene, yjgY gene, yjgZ gene, yi41 gene, yi42 gene, yjhB gene, yjhC gene, yjhD gene, yjhE gene, yi91 gene, JW4244 gene, JW4245 gene, JW4246 gene, yjhU gene, yjhF gene, yjhG gene, yjhH gene, yjhI gene, sgcR gene, sgcE gene, sgcA gene, sgcQ gene, sgcC gene, JW4266.5 gene, sgcX gene, yjhP gene, yjhQ gene, JW4269.5 gene, JW4270 gene, JW4270.5 gene, yjhR gene, yjhS gene, yjhT gene, yjhA gene, fimB gene, fimE gene, fimA gene, fimI gene, fimC gene, fimD gene, fimF gene, fimG gene, fimH gene, gntP gene, uxuA gene, uxuB gene, uxuR gene, yjiC gene, yjiD gene, yjiE gene, iadA gene, yjiG gene, yjiH gene, yjiI gene, yjiJ gene, yjiK gene, JW4296.5 gene, yjiL gene, yjiM gene, yjiN gene, yjiO gene, yjiP gene, yjiQ gene, yjiR gene, yjiS gene, yjiT gene, yjiV gene, mcrD gene, mcrC gene, mcrB gene, yjiW gene, hsdS gene, hsdM gene, hsdR gene, mrr gene, yjiA gene, yjiX gene, yjiY gene, tsr gene, yjjL gene, JW4320 gene, yjjM gene, yjjN gene, mdoB gene and yjjA gene, or homologous gene thereof.
(18) The *E. coli* mutant strain according to any one of (1) to (17) comprising chromosomal DNA that does not comprise DNA having the nucleotide sequences as shown in [1] or [2] below of the nucleotide sequence as shown in SEQ ID NO: 1 or DNA equivalent thereto.
   [1] a nucleotide sequence of nucleotides 4496202 to 4509534
   [2] a nucleotide sequence of nucleotides 4517876 to 4599637
(19) The *E. coli* mutant strain according to any one of (1) to (18) comprising chromosomal DNA that does not comprise kdpF gene, ybfA gene, rhsC gene, JW0690 gene, ybfB gene, rhsA gene, ybfC gene, ybfD gene, ybfL gene, ybfD gene and ybgA gene, or homologous gene thereof.
(20) The *E. coli* mutant strain according to any one of (1) to (19) comprising chromosomal DNA that does not comprise DNA having a nucleotide sequence comprising nucleotides 728821 to 739593 of the nucleotide sequence as shown in SEQ ID NO: 1 or DNA equivalent thereto.
(21) The *E. coli* mutant strain according to any one of (1) to (20) comprising chromosomal DNA that does not comprise yjfI gene, yjfJ gene, yjfK gene, yjfL gene, yjfM gene, yjfC gene, aidB gene, yjfN gene, yjfO gene, yjfP gene, yjfQ gene, yjfR gene, sgaT gene, sgaB gene, sgaA gene, sgaH gene and sgaU gene, or homologous gene thereof.
(22) The *E. coli* mutant strain according to any one of (1) to (21) comprising chromosomal DNA that does not comprise DNA having a nucleotide sequence comprising nucleotides 4409533 to 4423151 of the nucleotide sequence as shown in SEQ ID NO: 1 or DNA equivalent thereto.
(23) An *E*. *coli* mutant strain comprising the gene (1) [1] above, spr gene, bcr gene, rsuA gene, or homologous gene thereof and comprising chromosomal DNA that is at least 933 kbp shorter than that of a wild-type *E. coli* strain.
(24) The *E. coli* mutant strain according to (23) comprising chromosomal DNA that comprise DNA having the nucleotide sequences as shown in [1] to [55] of (2) above and DNA having a nucleotide sequence comprising nucleotides 2270440 to 2286588 of the nucleotide sequence as shown in SEQ ID NO: 1 or DNA equivalent thereto.
(25) The *E*. *coli* mutant strain according to (23) or (24) comprising chromosomal DNA that does not comprise yafJ gene, yafK gene, yafQ gene, dinJ gene, yafL gene, yafM gene, fhiA gene, lafU gene, dinP gene, yafN gene, yafO gene, yafP gene, JW0225 gene, prfH gene, ykfI gene, yafW gene, yeeT gene, ykfG gene, yafX gene, ykfF gene, ykfB gene, yafY gene, ypjK gene, yafZ gene, ykfA gene, perR gene, JW0245 gene, JW0246 gene, JW0247 gene, ykfC gene, JW0249 gene, is5B gene, yi51 gene, mmuP gene, mmuM gene, afuC gene, JW0255 gene, insB gene, insA gene, JW0258 gene, yagB gene, yagA gene, yagE gene, yagF gene, yagG gene, yagH gene, yagI gene, argF gene, yfjI gene, yagJ gene, yagK gene, yagL gene, yagM gene, yagN gene, intF gene, yagP gene, yagQ gene, yagR gene, yagS gene, yagT gene, yagU gene, ykgJ gene, yagV gene, yagW gene, yagX gene, yagY gene, yagZ gene, ykgK gene, ykgL gene, ykgM gene, eaeH gene, JW0292 gene, JW0293 gene, ykgA gene, ykgB gene, ykgI gene, merA gene, ykgD gene, ykgE gene, ykgF gene, ykgG gene, ykgH gene, betA gene, betB gene, betI gene, betT gene, yahA gene, yahB gene, yahC gene, yahD gene, yahE gene, yahF gene, yahG gene, yahH gene, arcM gene, yahJ gene, yahK gene, yahL gene, yahM gene, yahN gene, yahO gene, prpR gene, bcpA gene, prpC gene, prpD gene, prpE gene, codB gene, codA gene, cynR gene, cynT gene, cynS gene, cynX gene, lacA gene, lacY gene, lacZ gene, lacI gene, mhpR gene, mhpA gene, mhpB gene, mhpC gene, mhpD gene, mhpF gene, mhpE gene, mhpT gene, yaiL gene, yaiM gene, adhC gene, yaiN gene, yaiO gene, JW0350 gene, yi2A gene, yi22 gene, yi24 gene, yi23 gene, yaiP gene, yaiS gene, tauA gene, tauB gene, tauC gene, ssiD gene, yaiT gene, JW0363 gene, JW0364 gene, yaiU gene, yaiV gene, ampH gene, sbmA gene, yaiW gene, yaiY gene, yaiZ gene, ddlA gene, yaiB gene, phoA gene, psiF gene, yaiC gene, ybbO gene, tesA gene, ybbA gene, ybbP gene, rhsD gene, ybbC gene, rhsE gene, ybbD gene, JW0490 gene, ybbB gene, ybbS gene, ybbT gene, ybbU gene, ybbQ gene, ybbV gene, allP gene, allA gene, ybbY gene, ybbZ gene, ylbA gene, allC gene, ylbC gene, fdrA gene, ylbE gene, ylbE gene, ylbE gene, ylbF gene, arcC gene, intD gene, EXO_LAMBD gene, VRPP_LAMBD gene, JW0528 gene, JW0529 gene, VREN_LAMBD gene, emrE gene, ybcK gene, ybcL gene, ybcM gene, ybcN gene, ninE gene, ybcO gene, rus gene, ybcQ gene, JW0539.5 gene, nmpC gene, vlyS gene, ybcS gene, ybcT gene, vboR gene, ybcV gene, ybcW gene, nohB gene, ybcX gene, ybcY gene, JW0552 gene, appY gene, ompT gene, envY gene, ybcH gene, nfrA gene, nfrB gene, cusS gene, cusR gene, cusC gene, ylcC gene, cusB gene, cusA gene, pheP gene, ybdG gene, nfnB gene, ybdF gene, ybdJ gene, ybdK gene, hokE gene, yi81 gene, yi82 gene, ymcC gene, ymcD gene, cspH gene, cspG gene, sfa gene, yccL gene, yccM gene, torS gene, torT gene, torR gene, torC gene, torA gene, torD gene, yccD gene, cbpA gene, yccE gene, agp gene, yccJ gene, wrbA gene, yciG gene, ycdG gene, JW0992 gene, JW0993 gene, JW0994 gene, ycdK gene, JW0996 gene, JW0997 gene, ycdC gene, putA gene, yzpU gene, putP gene, JW1002 gene, ycdO gene, ycdB gene, phoH gene, ycdP gene, ycdQ gene, ycdR gene, ycdR gene, ycdS gene, ycdT gene, JW1012 gene, JW1013 gene, JW1014 gene, ycdU gene, ymfD gene, ymfE gene, lit gene, intE gene, VXIS_BPP21 gene, ymfH gene, ymfI gene, ymfJ gene, RPC2_BPP22 gene, JW1132 gene, ymfL, gene, ymfM gene, ymfN gene, ymfR gene, ymfO gene, JW1138 gene, JW1139 gene, ycfK gene, ymfS gene, ycfA gene, ycfE gene, pin gene, mcrA gene, JW 1146 gene, ycgW gene, ycgX gene, ycgE gene, JW1150 gene, ycgZ gene, ymgA gene, JW1153 gene, ymgC gene, yahA gene, JW1156 gene, JW1157 gene, aidA gene, ymgD gene, JW1160 gene, JW 1161 gene, JW 1162 gene, ydfH gene, ydfZ gene, ydfI gene, shiA gene, JW1537 gene, tnpR gene, ydfM gene, Y314_LAMBD gene, nohA gene, ydfO gene, yccL gene, cspI gene, ydfR gene, vlyS gene, cspB gene, cspF gene, regQ_LAMBD gene, JW1552 gene, rem gene, hokD gene, relE gene, relB gene, ydfV gene, flxA gene, ydfW gene, ydfX gene, dicC gene, sdaC gene, yqeH gene, yqeI gene, yqeJ gene, yqeK gene, ygeF gene, ygeG gene, ygeH gene, ygeI gene, JW2822 gene, ygeK gene, JW2824 gene, JW2825 gene, JW2830 gene, ygeP gene, JW2832 gene, nlpD gene, yagR gene, ygeT gene, JW2836 gene, ygeV gene, ygeW gene, dpaL gene, ygeY gene, ygeZ gene, arcL gene, yqeB gene, JW2844 gene, ygfJ gene, gltD gene, JW2847 gene, ygfM gene, yagR gene, ygfO gene, ygfP gene, ygfQ gene, ygfR gene, ygfS gene, ygfT gene, pbuX gene, idi gene, epsM gene, exeL gene, exeC gene, JW2938 gene, JW2939 gene, JW2940 gene, JW2941 gene, yghK gene, glcB gene, glcG gene, glcF gene, glcD gene, glcC gene, JW2948 gene, yghQ gene, yghR gene, yghS gene, yghT gene, yihL gene, yihM gene, yihN gene, yshA gene, yihO gene, yihP gene, yihQ gene, yihR gene, yihS gene, yihT gene, yihU gene, yihV gene, yihW gene, yihX gene, rbn gene, yihZ gene, yiiD gene, yiiE gene, yiiF gene, yiiG gene, frvR gene, frvX gene, frvB gene, frvA gene, yiiL gene, rhaD gene, rhaA gene, rhaB gene, rhaS gene, rhaR gene, rhaT gene, ptsA gene, JW3920 gene, frwC gene, frwB gene, pflD gene, pflC gene, frwD gene, yijO gene, yijP gene, JW3986 gene, yjbF gene, yjbG gene, yjbH gene, JW3989.5 gene, yjbA gene, xylE gene, malG gene, malF gene, malE gene, malK gene, lamB gene, malM gene, yjbI gene, yjcP gene, yjcQ gene, yjcR gene, yjcS gene, alsK gene, alsE gene, alsC gene, alsA gene, alsB gene, phnQ gene, phnP gene, phnO gene, phnN gene, phnM gene, phnL gene, phnK gene, phnJ gene, phnI gene, phnH gene, phnG gene, phnF gene, phnE gene, phnD gene, phnC gene, phnB gene, phnA gene, yjdA gene, yjcZ gene, yidF gene, yidG gene, yidH gene, yidI gene, yidJ gene, yidK gene, yidL gene, JW3657 gene, glvG gene, glvB gene, glvC gene, yidP gene, yidE gene, yidQ gene, yidR gene, yidS gene, dgoT gene, dgoA gene, dgoK gene, dgoR gene, yidW gene, yidX gene, yidA gene, yidB gene, asnA gene, yieM gene, JW3724 gene, yieN gene, kup gene, rbsD gene, rbsA gene, rbsC gene, rbsB gene, rbsK gene, rbsR gene, yieO gene, yecT gene, flhE gene, flhA gene, flhB gene, cheZ gene, cheY gene, cheB gene, cheR gene, tap gene, tar gene, cheW gene, cheA gene, motB gene, motA gene, flhC gene, flhD gene, JW1882 gene, JW1883 gene, yecG gene, otsA gene, otsB gene, araH gene, araG gene, araF gene, yecI gene, yecJ gene, JW1892 gene, ftn gene, yecH gene, tyrP gene, uvrC gene, uvrY gene, yecF gene, sdiA gene, yecC gene, yecS gene, yedO gene, fliY gene, fliZ gene, fliA gene, fliC gene, fliD gene, fliS gene, fliT gene, amyA gene, yedD gene, yedE gene, yedF gene, yedK gene, yedL gene, yedN gene, yedM gene, fliE gene, fliF gene, fliG gene, fliH gene, fliI gene, fliJ gene, fliK gene, fliL gene, fliM gene, fliN gene, fliO gene, fliP gene, fliQ gene, fliR gene, rcsA gene, dsrB gene, JW1937 gene, yedA gene, vsr gene, dcm gene, yedJ gene, JW1946 gene, ompC gene, JW1948 gene, yedS gene, yedU gene, yedV gene, copR gene, yedX gene, JW1954 gene, JW1955 gene, JW1956 gene, JW1974 gene, JW1979 gene, JW1980 gene, yeeP gene, flu gene, JW1983 gene, yeeS gene, yeeT gene, yeeU gene, yeeV gene, yeeW gene, yefJ gene, JW2013 gene, yefJ gene, yefI gene, wbbJ gene, yefG gene, rfc gene, glf gene, rfbX gene, rfbC gene, rfbA gene, rfbD gene, rfbB gene, intA gene, yfjH gene, alpA gene, yfjI gene, JW2606 gene, yfjJ gene, yfjK gene, yfjL gene, yfjM gene, yfjN gene, yfjO gene, yfjP gene, yfjQ gene, yfjR gene, ypjK gene, yfjS gene, yfjT gene, yfjU gene, yfjV gene, JW2622 gene, yfjW gene, JW2623.5 gene, yfjX gene, yfjY gene, yfjZ gene, ypjF gene, ypjA gene, JW2629 gene, JW2630 gene, JW2631 gene, JW2632 gene, JW2633 gene, JW2634 gene, ygaE gene, yzzM gene, yqaE gene, JW2642 gene, ygaP gene, stpA gene, JW2645 gene, ygaC gene, ygaM gene, nrdH gene, nrdi gene, nrdE gene, proV gene, proW gene, proX gene, JW2655 gene, JW2656 gene, ygaZ gene, ygaH gene, srlA gene, srlB gene, srlD gene, gutM gene, srlR gene, gutQ gene, gltF gene, yhcA gene, yhcD gene, yhcE gene, yhcE gene, yhcF gene, yhcG gene, yhcH gene, nanK gene, nanE gene, yegE gene, alkA gene, yegD gene, JW2055 gene, JW2056 gene, JW2057 gene, JW2058 gene, yegM gene, yegN gene, yegO gene, yegB gene, baeS gene, baeR gene, JW2065 gene, yegQ gene, ogrK gene, JW2068 gene, JW2069 gene, yegS gene, gatR gene, JW2072 gene, JW2073 gene, gatR gene, gatD gene, gatC gene, gatB gene, gatA gene, gatZ gene, gatY gene, fbaB gene, xegT gene, yegU gene, yegV gene, yegW gene, yegX gene, molR gene, yehI gene, yehK gene, yehL gene, yehO gene, yehP gene, yehQ gene, yehR gene, yehS gene, yehT gene, yehU gene, yehV gene, yehW gene, yehX gene, yehY gene, yehZ gene, bglX gene, dld gene, pbpG gene, yohC gene, yeiI gene, yeiJ gene, yeiK gene, yeiL gene, yeiM gene, yeiN gene, yeiC gene, cmtA gene, cmtB gene, intB gene, JW4228 gene, yjgW gene, yj gX gene, yjgY gene, yjgZ gene, yi41 gene, yi42 gene, yjhB gene, yjhC gene, yjhD gene, yjhE gene, yi91 gene, JW4244 gene, JW4245 gene, JW4246 gene, yjhU gene, yjhF gene, yjhG gene, yjhH gene, yjhI gene, sgcR gene, sgcE gene, sgcA gene, sgcQ gene, sgcC gene, JW4266.5 gene, sgcX gene, yjhP gene, yjhQ gene, JW4269.5 gene, JW4270 gene, JW4270.5 gene, yjhR gene, yjhS gene, yjhT gene, yjhA gene, fimB gene, fimE gene, fimA gene, fimI gene, fimC gene, fimD gene, fimF gene, fimG gene, fimH gene, gntP gene, uxuA gene, uxuB gene, uxuR gene, yjiC gene, yjiD gene, yjiE gene, iadA gene, yjiG gene, yjiH gene, yjiI gene, yjiJ gene, yjiK gene, JW4296.5 gene, yjiL gene, yjiM gene, yjiN gene, yjiO gene, yjiP gene, yjiQ gene, yjiR gene, yjiS gene, yjiT gene, yjiV gene, mcrD gene, mcrC gene, mcrB gene, yjiW gene, hsdS gene, hsdM gene, hsdR gene, mrr gene, yjiA gene, yjiX gene, yjiY gene, tsr gene, yjjL gene, JW4320 gene, yjjM gene, yjjN gene, mdoB gene, yjjA gene, kdpF gene, ybfA gene, rhsC gene, JW0690 gene, ybfB gene, rhsA gene, ybfC gene, ybfL gene, ybfD gene, ybgA gene, yjfI gene, yjfJ gene, yjfK gene, yjfL gene, yjfM gene, yjfC gene, aidB gene, yjfN gene, yjfO gene, yjfP gene, yjfQ gene, yjfR gene, sgaT gene, sgaB gene, sgaA gene, sgaH gene, sgaU gene, rhsB gene, JW3448 gene, yhhH gene, yibA gene, yhhl gene, yhhJ gene, yhiH gene, yhil gene, yhiJ gene, yhiK gene, yhiL gene, yhiM gene, yhiN gene, JW0782 gene, ybiA gene, dinG gene, ybiB gene, ybiC gene, ybiJ gene, ybil gene, ybiX gene, ybiL gene, ybiM gene, ybiN gene, ybiO gene, glnQ gene, glnP gene, glnH gene, yliA gene, yliB gene, yliC gene, yliD gene, JW0817 gene, JW0818 gene, yliG gene, yliH gene, ylil gene, yliJ gene, dacC gene, deoR gene, ybjG gene, ybjC gene, mdaA gene, rimK gene, ybjN gene, potF gene, potG gene, potH gene, potI gene, ybjO gene, ybjF gene, artJ gene, artM gene, artQ gene, artI gene, artP gene, ybjP gene, JW0850 gene, JW0851 gene, JW0852 gene, ybjT gene and ltaA gene, or homologous gene thereof.
(26) The *E*. *coli* mutant strain according to any one of (23) to (25) comprising chromosomal DNA that does not comprise DNA having the nucleotide sequences as shown in [1] to [43] of the nucleotide sequence as shown in SEQ ID NO: 1 or DNA equivalent thereto.
   [1] a nucleotide sequence of nucleotides 243965 to 253410
   [2] a nucleotide sequence of nucleotides 261964 to 387531
   [3] a nucleotide sequence of nucleotides 389139 to 403703
   [4] a nucleotide sequence of nucleotides 518030 to 532711
   [5] a nucleotide sequence of nucleotides 535505 to 550215
   [6] a nucleotide sequence of nucleotides 563941 to 608118
   [7] a nucleotide sequence of nucleotides 728821 to 739593
   [8] a nucleotide sequence of nucleotides 832552 to 848089
   [9] a nucleotide sequence of nucleotides 867638 to 883473
   [10] a nucleotide sequence of nucleotides 890998 to 909379
   [11] a nucleotide sequence of nucleotides 1048888 to 1097019
   [12] a nucleotide sequence of nucleotides 1198106 to 1225146
   [13] a nucleotide sequence of nucleotides 1630912 to 1641038
   [14] a nucleotide sequence of nucleotides 1642632 to 1650222
   [15] a nucleotide sequence of nucleotides 1964524 to 1993075
   [16] a nucleotide sequence of nucleotides 1996662 to 2028209
   [17] a nucleotide sequence of nucleotides 2033449 to 2044376
   [18] a nucleotide sequence of nucleotides 2069149 to 2081926
   [19] a nucleotide sequence of nucleotides 2104393 to 2116064
   [20] a nucleotide sequence of nucleotides 2146264 to 2187855
   [21] a nucleotide sequence of nucleotides 2200669 to 2229850
   [22] a nucleotide sequence of nucleotides 2255895 to 2263491
   [23] a nucleotide sequence of nucleotides 2755677 to 2789480
   [24] a nucleotide sequence of nucleotides 2795174 to 2800890
   [25] a nucleotide sequence of nucleotides 2804308 to 2810147
   [26] a nucleotide sequence of nucleotides 2825314 to 2830262
   [27] a nucleotide sequence of nucleotides 2985362 to 3033128
   [28] a nucleotide sequence of nucleotides 3077085 to 3078916
   [29] a nucleotide sequence of nucleotides 3110110 to 3134341
   [30] a nucleotide sequence of nucleotides 3360737 to 3370547
   [31] a nucleotide sequence of nucleotides 3618576 to 3636916
   [32] a nucleotide sequence of nucleotides 3854662 to 3865831
   [33] a nucleotide sequence of nucleotides 3867102 to 3877010
   [34] a nucleotide sequence of nucleotides 3926659 to 3938878
   [35] a nucleotide sequence of nucleotides 4059898 to 4079659
   [36] a nucleotide sequence of nucleotides 4086451 to 4099982
   [37] a nucleotide sequence of nucleotides 4139232 to 4149709
   [38] a nucleotide sequence of nucleotides 4235359 to 4251735
   [39] a nucleotide sequence of nucleotides 4298977 to 4311511
   [40] a nucleotide sequence of nucleotides 4313318 to 4329721
   [41] a nucleotide sequence of nucleotides 4409533 to 4423151
   [42] a nucleotide sequence of nucleotides 4496202 to 4509534
   [43] a nucleotide sequence of nucleotides 4517876 to 4599637
(27) The *E*. *coli* mutant strain according to any one of (23) to (26) comprising chromosomal DNA that does not comprise ygiS gene, ygiT gene, ygiU gene, JW2991 gene, ygiW gene, ygiX gene, ygiY gene, ygiZ gene, mdaB gene and ygiN gene, or homologous gene thereof.
(28) The *E*. *coli* mutant strain according to any one of (23) to (27) comprising chromosomal DNA that does not comprise DNA having a nucleotide sequence comprising nucleotides 3165628 to 3172940 of the nucleotide sequence as shown in SEQ ID NO: 1 or DNA equivalent thereto.
(29) The *E. coli* mutant strain according to any one of (23) to (28) comprising chromosomal DNA that does not comprise ybeL gene, ybeQ gene, ybeR gene, ybeS gene, ybeT gene, ybeU gene, ybeV gene, ybeW gene, ybeK gene, gltL gene, gltK gene, gltJ gene, ybeJ gene and JW0651 gene, or homologous gene thereof.
(30) The *E. coli* mutant strain according to any one of (23) to (29) comprising chromosomal DNA that does not comprise DNA having a nucleotide sequence comprising nucleotides 675104 to 689374 of the nucleotide sequence as shown in SEQ ID NO: 1 or DNA equivalent thereto.
(31) The *E*. *coli* mutant strain according to any one of (23) to (30) comprising chromosomal DNA that does not comprise yfcC gene, JW2296 gene, yfcE gene, yfcF gene, yfcG gene, folX gene, JW2301 gene, yfcl gene, hisP gene, hisM gene, hisQ gene, hisJ gene, argT gene, yfdB gene, JW2346 gene, yfdH gene, JW2348 gene, yfdK gene, yfdL gene, yfdm gene, xylU gene, yfdN gene, yfdO gene, JW2356 gene, JW2357 gene, JW2358 gene, JW2359 gene, JW2360 gene, JW2378 gene, yqhT gene, ptwC gene andptwB gene, or homologous gene thereof.
(32) The *E*. *coli* mutant strain according to any one of (23) to (31) comprising chromosomal DNA that does not comprise DNA having the nucleotide sequences as shown in [1] to [3] below of the nucleotide sequence as shown in SEQ ID NO: 1 or DNA equivalent thereto.
   [1] a nucleotide sequence of nucleotides 2423365 to 2434098
   [2] a nucleotide sequence of nucleotides 2472852 to 2482482
   [3] a nucleotide sequence of nucleotides 2505075 to 2514557
(33) The *E. coli* mutant strain according to any one of (23) to (32) comprising chromosomal DNA that does not comprise pinO gene, gspA gene, gspC gene, gspD gene, gspE gene, hofF gene, hofG gene, hofH gene, gspI gene, gspJ gene, gspK gene, gspL gene, pshM gene, hofD gene, bfr gene, yheA gene and chiA gene, or homologous gene thereof.
(34) The *E. coli* mutant strain according to any one of (23) to (33) comprising chromosomal DNA that does not comprise DNA having a nucleotide sequence comprising nucleotides 3453021 to 3469366 of the nucleotide sequence as shown in SEQ ID NO: 1 or DNA equivalent thereto.
(35) The *E. coli* mutant strain according to any one of (23) to (34) comprising chromosomal DNA that does not comprise elaB gene, elaA gene, elaC gene, elaD gene, yfbK gene, yfbL gene, yfbM gene, yfbN gene, JW2269 gene and JW2270 gene, or homologous gene thereof.
(36) The *E. coli* mutant strain according to any one of (23) to (35) comprising chromosomal DNA that does not comprise DNA having a nucleotide sequence comprising nucleotides 2386262 to 2395471 of the nucleotide sequence as shown in SEQ ID NO: 1 or DNA equivalent thereto.
(37) The *E. coli* mutant strain according to any one of (23) to (36) comprising chromosomal DNA that does not comprise yhdJ gene, yhdU gene, envR gene, acrE gene, envD gene, yhdV gene, yhdW gene, yhdX gene and yhdY gene, or homologous gene thereof.
(38) The *E. coli* mutant strain according to any one of (23) to (37) comprising chromosomal DNA that does not comprise DNA having a nucleotide sequence comprising nucleotides 3411160 to 3420878 of the nucleotide sequence as shown in SEQ ID NO: 1 or DNA equivalent thereto.
(39) The *E*. *coli* mutant strain according to any one of (23) to (38) comprising chromosomal DNA that does not comprise chpA gene or homologous gene thereof.
(40) The *E. coli* mutant strain according to any one of (23) to (39) comprising chromosomal DNA that does not comprise DNA having a nucleotide sequence comprising nucleotides 2910274 to 2910573 of the nucleotide sequence as shown in SEQ ID NO: 1 or DNA equivalent thereto.
(41) The *E*. *coli* mutant strain according to any one of (23) to (40) comprising chromosomal DNA that does not comprise fhuA gene, fhuC gene, fhuD gene fhuB gene, or homologous gene thereof.
(42) The *E. coli* mutant strain according to any one of (23) to (41) comprising chromosomal DNA that does not comprise DNA having a nucleotide sequence comprising nucleotides 167065 to 172979 of the nucleotide sequence as shown in SEQ ID NO: 1 or DNA equivalent thereto.
(43) The *E. coli* mutant strain according to any one of (1) to (42) obtained via modification by a method selected from among the following [1] to [5]:
   [1] a method for reducing or removing at least one mechanism for regulating biosynthesis of the useful substance;
   [2] a method for increasing the expression level of at least one enzyme associated with biosynthesis of the useful substance;
   [3] a method for increasing the copy number of at least one enzyme gene associated with biosynthesis of the useful substance;
   [4] a method for attenuating or blocking at least one metabolic pathway which branches from the biosynthetic pathway of the useful substance into metabolites other than the useful substance; or
   [5] a method for selecting a strain that exhibits higher resistance to an analogue of a useful substance than a wild-type *E. coli* strain.
(44) The *E. coli* mutant strain according to any one of (1) to (43), wherein the *E. coli* strain is the *E. coli* K-12, B, or W strain.
(45) The *E. coli* mutant strain according to (44), wherein the *E. coli* K-12 strain is the *E. coli* W3110 strain (ATCC27325) or the *E. coli* MG1655 strain (ATCC47076).
(46) A method for producing a useful substance comprising culturing an *E. coli* mutant strain according to any one of (1) to (45) in a medium so as to generate and accumulate such useful substance in the culture and recovering the useful substance from the culture
(47) The method according to any one of (43) to (46), wherein the useful substance is selected from the group consisting of proteins, peptides, amino acids, nucleic acids, vitamins, saccharides, organic acids, and lipids.

### Effects of the Invention

The present invention can provide an *E. coli* mutant, the amount of cells of which, after a given period of culture, is larger than that of a wild-type *E*. *coli* strain, and a process for producing a useful substance using such mutant.

### Brief Description of the Drawings

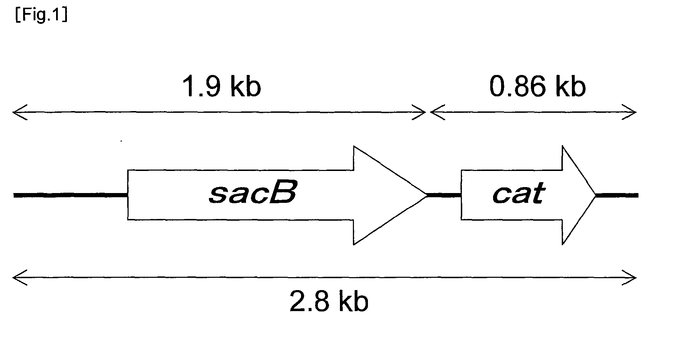

Fig. 1 shows the structure of a DNA fragment comprising sacB gene and cat gene ligated thereto.

### Description of Reference Symbols

*sacB* represents the sacB gene, and *cat* represents the cat gene.

### Best Modes for Carrying out the Invention

Wild-type *E. coli* strains may be any *E. coli* strains that are found in natural populations at the highest frequency. Examples thereof include the *E. coli* W3110 strain (ATCC27325) and the *E. coli* MG1655 strain (ATCC47076) that belong to the *E. coli* K-12 strain, the ATCC11303 strain that belongs to the *E*. *coli* B strain, and the ATCC9637 strain that belongs to the *E. coli* W strain.

Table 1, Table 2, and SEQ ID NO: 1 of the present description and the Internet site URL::http://gib.genes.nig.ac.jp/single/index.php?spid=Ecol_K12_W3110 include nucleotide sequences of the genes identified with JW numbers. The numbers with "JW" represent gene numbers registered at the DNA Data Bank of Japan.

The nucleotide sequence as shown in SEQ ID NO: 1 is the nucleotide sequence of chromosomal DNA of the *E. coli* W3110 strain registered as of October 29, 2004 on the above Internet site.

The term "chromosomal DNA that contains genes" refers to chromosomal DNA that contains a structural gene and a region containing a specific regulatory function such as a prmoter, operator or the like of a given gene.

The term "chromosomal DNA that does not contain gene" refers to chromosomal DNA that does not contain a part of or an entire structural gene of a given gene or chromosomal DNA that does not contain a region having a specific regulatory function, such as a promoter and/or operator of such gene.

The "chromosomal DNA that does not contain part of a structural gene" may be any chromosomal DNA that does not contain part of a structural gene, and a functional protein is not transcribed and expressed from the structural gene. Examples thereof include DNA that has no initiation codon due to a lack of the 5' end region of the structural gene and chromosomal DNA that lacks most of the 3' end region, such as at least 10%, preferably at least 20%, more preferably at least 30%, and further preferably at least 40% of the entire 3' end region. Chromosomal DNA that does not contain two adjacent genes may be chromosomal DNA that does not contain DNA comprising a nucleotide sequence located between such genes.

The term "homologous genes" refers to genes that have homology in terms of the nucleotide sequences of such genes or genes encoding the proteins which have homology in terms of amino acid sequences among *E. coli* species. Having homology refers to having similarity of at least 85%, preferably at least 90%, more preferably at least 95%, further preferably at least 98%, and particularly preferably at least 99%, in the case of nucleotide sequences. In the case of amino acid sequences, such term refers to having similarity of at least 80%, preferably at least 90%, more preferably at least 95%, further preferably at least 98%, and particularly preferably at least 99%.

"DNA equivalent to DNA having a nucleotide sequence represented by given nucleotide numbers in the nucleotide sequence as shown in SEQ ID NO: 1" refers to DNA that is substantially the same as DNA having a nucleotide sequence represented by such given nucleotide numbers of an *E. coli* species in terms of the structure of the gene contained and the position on chromosomal DNA. When structures of genes contained in DNA are substantially the same as each other, genes contained in such DNA are identical or homologous to each other, and arranged in the same order. In that case, nucleotide sequences may differ among genes. When the DNA having a nucleotide sequence represented by given nucleotide numbers contains an insertion sequence, however, such insertion sequence may be absent in DNA equivalent to the aforementioned DNA. "Substantially the same positions on chromosomal DNA" refers to the following conditions. When adenine of the initiation codon of the ThrA gene on *E. coli* chromosomal DNA is designated as No. 1 and the nucleotide sequence of chromosomal DNA is numbered along the coding chain of the ThrA gene, differences between such nucleotide numbers and the given nucleotide numbers are 50 or smaller, preferably 30 or smaller, more preferably 10 or smaller, and further preferably 5 or smaller. When the DNA having a nucleotide sequence represented by given nucleotide numbers contains an insertion sequence, however, DNA equivalent to the aforementioned DNA does not necessarily contain such insertion sequence. Thus, such insertion sequence and nucleotide numbers may be different. An example of DNA equivalent to DNA having a nucleotide sequence represented by given nucleotide numbers of the nucleotide sequence as shown in SEQ ID NO: 1 is DNA having sequence similarity of at least 80%, preferably at least 90%, more preferably at least 95%, further preferably at least 98%, and particularly preferably at least 99% with the nucleotide sequence represented by given nucleotide numbers.

Amino acid sequence or nucleotide sequence homology can be determined with the utilization of the BLAST algorithm of Karlin and Altschul (Pro. Natl. Acad. Sci., U.S.A., 90, 5873, 1993) or the FASTA algorithm (Methods Enzymol., 183, 63, 1990). Based on the BLAST algorithm, programs referred to as BLASTN or BLASTX have been developed (J. Mol. Biol., 215, 403, 1990). When analyzing nucleotide sequences by BLASTN based on BLAST, the score parameter is set to 100 and the word length parameter is set to 12, for example. When analyzing amino acid sequences via BLASTX based on BLAST, the score parameter is set to 50 and the word length parameter is set to 3, for example. When using BLAST and Gapped BLAST programs, the default parameters thereof are used. Specific procedures of such analytical methods are known (http://www.ncbi.nlm.nih.gov.).

### 1. E. coli mutant strain of the present invention

The *E. coli* mutant strain of the present invention may be any *E. coli* mutant strain comprising chromosomal DNA that does not comprise any gene excluding the genes [1] and [2] that are present on chromosomal DNA of a wild-type *E. coli* strain or homologous genes thereof, provided that it comprises the following genes [1] and [2] or homologous genes thereof and having chromosomal DNA that is at least 470 kbp shorter than that of a wild-type *E. coli* strain,
[1] thrA gene, thrB gene, thrC gene, talB gene, mog gene, htgA gene, dnaK gene, dnaJ gene, hokC gene, nhaA gene, nhaR gene, rpsT gene, ribF gene, ileS gene, lspA gene, fkpB gene, lytB gene, dapB gene, carA gene, carB gene, caiF gene, caiE gene, caiD gene, caiC gene, caiB gene, caiA gene, caiT gene, fixA gene, fixB gene, fixC gene, fixX gene, kefC gene, folA gene, apaH gene, apaG gene, ksgA gene, pdxA gene, surA gene, imp gene, djlA gene, rluA gene, hepA gene, polB gene, araD gene, araA gene, araB gene, araC gene, tbpA gene, leuD gene, leuC gene, leuB gene, leuA gene, leuL gene, leuO gene, ilvl gene, ilvH gene, fruR gene, ftsL gene, ftsI gene, murE gene, murF gene, mraY gene, murD gene, ftsW gene, murG gene, murC gene, ddlB gene, ftsQ gene, ftsA gene, ftsZ gene, IpxC gene, secA gene, mutT gene, yacE gene, guaC gene, hofC gene, hofB gene, ppdD gene, nadC gene, ampD gene, ampE gene, aroP gene, pdhR gene, aceE gene, aceF gene, lpdA gene, acnB gene, speD gene, speE gene, gcd gene, hpt gene, yadF gene, panD gene, panC gene, panB gene, htrE gene, ecpD gene, folK gene, pcnB gene, dksA gene, sfsA gene, ligT gene, hrpB gene, ponB gene, hemL gene, pfs gene, dgt gene, htrA gene, dapD gene, glnD gene, map gene, rpsB gene, tsf gene, smb gene, rrf gene, dxr gene, rth gene, cdsA gene, yaeL gene, hlpA gene, lpxD gene, fabZ gene, lpxA gene, lpxB gene, rnhB gene, dnaE gene, accA gene, ldcC gene, mesJ gene, rof gene, cutF gene, proS gene, rcsF gene, abc gene, dniR gene, gloB gene, rnhA gene, dnaQ gene, up 18 gene, gmhA gene, pepD gene, gpt gene, crl gene, phoE gene, proB gene, proA gene, hemB gene, proC gene, aroL gene, aroM gene, araJ gene, sbcC gene, sbcD gene, phoB gene, phoR gene, brnQ gene, proY gene, malZ gene, queA gene, tgt gene, secD gene, secF gene, tsx gene, ribD gene, ribH gene, nusB gene, thiL gene, pgpA gene, dxs gene, ispA gene, xseB gene, thiI gene, thiJ gene, apbA gene, cyoE gene, cyoD gene, cyoC gene, cyoB gene, cyoA gene, ampG gene, bolA gene, tig gene, clpP gene, clpX gene, lon gene, hupB gene, cof gene, mdlA gene, mdlB gene, gInK gene, amtB gene, tesB gene, maa gene, hha gene, acrB gene, acrA gene, acrR gene, aefA gene, priC gene, apt gene, dnaX gene, recR gene, htpG gene, adk gene, hemH gene, aes gene, gsk gene, fsr gene, ushA gene, cueR gene, gel gene, gip gene, purK gene, purE gene, ybbF gene, ppiB gene, cysS gene, folD gene, sfmA gene, sfmC gene, sfmD gene, sfmH gene, sfmF gene, fimZ gene, entD gene, fepA gene, fes gene, entF gene, fepE gene, fepC gene, fepG gene, fepD gene, fepB gene, entC gene, entE gene, entB gene, entA gene, cstA gene, dsbG gene, ahpC gene, ahpF gene, rnk gene, rna gene, citT gene, citG gene, citX gene, citF gene, citE gene, citD gene, citC gene, citA gene, citB gene, dcuC gene, dcuC gene, crcA gene, cspE gene, crcB gene, lipA gene, lipB gene, dacA gene, rlpA gene, mrdB gene, mrdA gene, phpB gene, nadD gene, holA gene, rlpB gene, leuS gene, Int gene, phoL gene, ubiF gene, asnB gene, nagD gene, nagC gene, nagA gene, nagB gene, nagE gene, glnS gene, fur gene, fldA gene, seqA gene, pgm gene, potE gene, speF gene, kdpE gene, kdpD gene, kdpC gene, kdpB gene, kdpA gene, phrB gene, end8 gene, abrB gene, gltA gene, sdhC gene, sdhD gene, sdhA gene, sdhB gene, sucA gene, sucB gene, sucC gene, sucD gene, farR gene, hrsA gene, cydA gene, cydB gene, tolQ gene, tolR gene, tolA gene, tolB gene, pal gene, nadA gene, pnuC gene, czcD gene, aroG gene, gpmA gene, galM gene, galK gene, galT gene, galE gene, modF gene, modE gene, modA gene, modB gene, modC gene, bioA gene, bioB gene, bioF gene, bioC gene, bioD gene, uvrB gene, moaA gene, moaB gene, moaC gene, moaD gene, moaE gene, rhlE gene, dps gene, ompX gene, mipB gene, moeB gene, moeA gene, mdfA gene, grxA gene, poxB gene, aqpZ gene, cspD gene, clpA gene, infA gene, aat gene, cydC gene, cydD gene, trxB gene, lrp gene, ftsk gene, lolA gene, serS gene, dmsA gene, dmsB gene, dmsC gene, pflA gene, pflB gene, focA gene, serC gene, aroA gene, cmk gene, rpsA gene, himD gene, msbA gene, lpxK gene, kdsB gene, smtA gene, mukF gene, kicA gene, mukB gene, aspC gene, ompF gene, asnS gene, pncB gene, pepN gene, ssuB gene, ssuC gene, ssuD gene, ssuA gene, ssuE gene, pyrD gene, uup gene, pqiA gene, pqiB gene, rmf gene, fabA gene, ompA gene, sulA gene, helD gene, mgsA gene, acyP gene, hyaA gene, hyaB gene, hyaC gene, hyaD gene, hyaE gene, hyaF gene, appC gene, appB gene, appA gene, csgG gene, csgF gene, csgE gene, csgD gene, csgB gene, csgA gene, csgC gene, sfaA gene, mdoG gene, mdoH gene, msyB gene, htrB gene, solA gene, dinI gene, pyrC gene, grxB gene, rimJ gene, mviM gene, mviN gene, flgN gene, flgM gene, flgA gene, flgB gene, flgC gene, flgD gene, flgE gene, flgF gene, flgG gene, flgH gene, flgI gene, flgJ gene, flgK gene, flgL gene, rne gene, rluC gene, rpmF gene, plsX gene, fabH gene, fabD gene, fabG gene, acpP gene, fabF gene, pabC gene, tmk gene, holB gene, ptsG gene, fhuE gene, nagZ gene, ndh gene, mfd gene, ycfU gene, ycfV gene, ycfW gene, cobB gene, potD gene, potC gene, potB gene, potA gene, pepT gene, phoQ gene, phoP gene, purB gene, trmU gene, icdA gene, minE gene, minD gene, minC gene, clyA gene, umuD gene, umuC gene, dsbB gene, nhaB gene, fadR gene, dadA gene, dadX gene, ldcA gene, mltE gene, tagl gene, treA gene, dhaR gene, pth gene, prsA gene, ychB gene, hemM gene, hemA gene, prfA gene, hemK gene, kdsA gene, chaA gene, chaB gene, chaC gene, narL gene, narX gene, narK gene, narG gene, narH gene, narJ gene, narI gene, tpr gene, purU gene, sprE gene, galU gene, hns gene, tdk gene, adhE gene, oppA gene, oppB gene, oppC gene, oppD gene, oppF gene, cls gene, kch gene, tonB gene, trpA gene, trpB gene, trpC gene, trpD gene, trpE gene, trpL gene, btuR gene, sohB gene, topA gene, cysB gene, acnA gene, ribA gene, pgpB gene, pyrF gene, osmB gene, rnb gene, envM gene, sapF gene, sapD gene, sapC gene, sapB gene, sapA gene, aldH gene, ordL gene, goaG gene, pspF gene, pspA gene, pspB gene, pspC gene, pspD gene, pspE gene, sucP gene, ompG gene, tyrR gene, tpx gene, mppA gene, fnr gene, ogt gene, dbpA gene, intR gene, lar gene, recT gene, recE gene, racC gene, kil gene, sieB gene, racR gene, dnaC gene, enpP gene, trkG gene, VMTH_LAMBD gene, VLOM_LAMBD gene, tnpO gene, upo3 gene, ompN gene, nifJ gene, hslJ gene, ldhA gene, feaR gene, feaB gene, tynA gene, paaZ gene, paaA gene, paaB gene, paaC gene, paaD gene, paaE gene, paaF gene, paaG gene, paaH gene, paaI gene, paaJ gene, paaK gene, paaX gene, paaY gene, acpD gene, hrpA gene, aldA gene, gapC gene, gapC gene, cybB gene, trg gene, rimL gene, tehA gene, tehB gene, prtC gene, potA gene, potC gene, ansP gene, nhoA gene, narV gene, narW gene, narY gene, narZ gene, narU gene, fdnG gene, fdnH gene, fdnI gene, adhP gene, sfcA gene, rpsV gene, osmC gene, dos gene, xasA gene, gadB gene, pqqL gene, chuR gene, hipA gene, hipB gene, tam gene, uxaB gene, marC gene, marR gene, marA gene, marB gene, dcp gene, lycV gene, dicA gene, dicB gene, rspB gene, rspA gene, speG gene, dmsB gene, dmsC gene, bioD gene, mlc gene, asr gene, ebr gene, pntB gene, pntA gene, arcD gene, rstA gene, rstB gene, tus gene, fumC gene, fumA gene, manA gene, uidC gene, uidB gene, uidA gene, uidR gene, hdhA gene, malI gene, malX gene, malY gene, add gene, blr gene, nth gene, gst gene, pdxY gene, tyrS gene, pdxH gene, slyB gene, slyA gene, sodC gene, nemA gene, gloA gene, rnt gene, lhr gene, sodB gene, araJ gene, purR gene, cfa gene, ribE gene, ag43 gene, phsC gene, nrfC gene, pykF gene, lpp gene, sufS gene, aroD gene, ppsA gene, aroH gene, nlpC gene, btuD gene, btuE gene, btuC gene, himA gene, pheT gene, pheS gene, pheM gene, rplT gene, rpmI gene, infC gene, thrS gene, arp gene, pfkB gene, cedA gene, katE gene, celF gene, celD gene, celC gene, celB gene, celA gene, osmE gene, nadE gene, spy gene, astE gene, astB gene, astD gene, astA gene, cstC gene, xthA gene, gdhA gene, topB gene, selD gene, sppA gene, ansA gene, tdh gene, gapA gene, ttuC gene, caiT gene, rnd gene, fadD gene, slp gene, pabB gene, sdaA gene, manX gene, manY gene, manZ gene, rrmA gene, cspC gene, kdgR gene, tcmA gene, htpX gene, prc gene, pqiB gene, pphA gene, holE gene, exoX gene, ptrB gene, purT gene, eda gene, edd gene, zwf gene, pykA gene, mlt gene, znuA gene, znuC gene, ruvB gene, ruvA gene, ruvC gene, ntpA gene, aspS gene, bisZ gene, torY gene, cutC gene, argS gene, pgsA gene, amn gene, cbl gene, nac gene, erfK gene, cobT gene, cobS gene, cobU gene, gyrI gene, phsE gene, sbcB gene, hisL gene, hisG gene, hisD gene, hisC gene, hisB gene, hisH gene, hisA gene, hisF gene, hisI gene, wzz gene, ugd gene, gnd gene, galF gene, wcaM gene, wcaL gene, wcaK gene, wzxC gene, wcaJ gene, cpsG gene, cpsB gene, wcaI gene, wcaH gene, wcaG gene, gmd gene, wcaF gene, wcaE gene, wcaD gene, wcaC gene, wcaB gene, wcaA gene, wzb gene, wza gene, asmA gene, dcd gene, udk gene, thiD gene, thiM gene, mrp gene, metG gene, cdd gene, sanA gene, mglC gene, mglA gene, mglB gene, galS gene, folE gene, cirA gene, lysP gene, nfo gene, fruA gene, fruK gene, fruB gene, rplY gene, narP gene, ccmH gene, dsbE gene, ccmF gene, ccmE gene, ccmD gene, ccmC gene, ccmB gene, ccmA gene, napC gene, napB gene, napH gene, napG gene, napA gene, napD gene, napF gene, eco gene, alkB gene, ada gene, apbE gene, rcsB gene, rcsC gene, atoS gene, atoC gene, atoD gene, atoA gene, atoE gene, atoB gene, gyrA gene, ubiG gene, nrdA gene, nrdB gene, inaA gene, glpQ gene, glpT gene, glpA gene, glpB gene, glpC gene, cinA gene, ais gene, pmrD gene, menE gene, menC gene, menB gene, menD gene, menF gene, nuoN gene, nuoM gene, nuoL gene, nuoK gene, nuoJ gene, nuoI gene, nuoH gene, nuoG gene, nuoF gene, nuoE gene, nuoD gene, nuoB gene, nuoA gene, IrhA gene, ackA gene, pta gene, ubiX gene, purF gene, dedE gene, dedD gene, folC gene, accD gene, dedA gene, truA gene, usg1 gene, pdxB gene, div gene, fabB gene, ddpX gene, mepA gene, aroC gene, sixA gene, fadL gene, vacJ gene, dsdC gene, dsdX gene, dsdA gene, emrY gene, emrK gene, evgA gene, evgS gene, bifF gene, ddg gene, glk gene, mntH gene, nupC gene, gltX gene, xapR gene, xapB gene, xapA gene, lig gene, zipA gene, cysZ gene, cysK gene, ptsH gene, ptsI gene, gsr gene, pdxK gene, cysM gene, cysA gene, cysW gene, cysU gene, cysP gene, ucpA gene, ptbA gene, amiA gene, hemF gene, cchA gene, eutC gene, eutB gene, eutH gene, eutG gene, eutJ gene, eutE gene, cchB gene, eutI gene, talA gene, tktB gene, aegA gene, narQ gene, acrD gene, dapE gene, purC gene, nlpB gene, dapA gene, gcvR gene, bcp gene, hyfA gene, hyfB gene, hyfC gene, hyfD gene, hyfE gene, hyfF gene, hyfG gene, hyfH gene, hyfI gene, hyfJ gene, hyfR gene, focB gene, perM gene, yfgE gene, uraA gene, upp gene, purM gene, purN gene, ppk gene, ppx gene, guaA gene, guaB gene, xseA gene, hisS gene, gcpE gene, ndk gene, pbpC gene, sseA gene, sseB gene, pepB gene, fdx gene, hscA gene, hscB gene, iscU gene, iscS gene, iscR gene, suhB gene, csiE gene, dgxA gene, dgxB gene, hcaC gene, hcaB gene, hcaD gene, glyA gene, hmpA gene, glnB gene, purL gene, acpS gene, pdxJ gene, recO gene, era gene, rnc gene, lepB gene, lepA gene, rseC gene, rseB gene, rseA gene, rpoE gene, nadB gene, srmB gene, ung gene, trx2 gene, pssA gene, kgtP gene, clpB gene, rluD gene, pheL gene, pheA gene, tyrA gene, aroF gene, rpIS gene, trmD gene, rimM gene, rpsP gene, ffh gene, grpE gene, recN gene, smpA gene, smpB gene, gabD gene, gabT gene, gabP gene, nrdF gene, emrR gene, emrA gene, emrB gene, gshA gene, csrA gene, alaS gene, oraA gene, recA gene, mltB gene, hypF gene, hydN gene, ascG gene, ascF gene, ascB gene, hycI gene, hycH gene, hycG gene, hycF gene, hycE gene, hycD gene, hycC gene, hycB gene, hycA gene, hypA gene, hypB gene, hypC gene, hypD gene, hypE gene, fhlA gene, mutS gene, prpB gene, rpoS gene, pcm gene, surE gene, ygbB gene, ygbP gene, ygbQ gene, cysC gene, cysN gene, cysD gene, iap gene, cysH gene, cysI gene, cysJ gene, ptpS gene, eno gene, pyrG gene, mazG gene, chpR gene, relA gene, barA gene, gud2 gene, gudT gene, mioC gene, syd gene, sdaB gene, exo gene, fucO gene, fucA gene, fucP gene, fucI gene, fucK gene, fucU gene, fucR gene, gcvA gene, nifS gene, mltA gene, amiC gene, argA gene, thyA gene, lgt gene, ptsP gene, mutH gene, tas gene, aas gene, galR gene, lysA gene, lysR gene, araE gene, kduD gene, kduI gene, lysS gene, prfB gene, recJ gene, dsbC gene, xerD gene, fldB gene, upl4 gene, bglA gene, gcvP gene, gcvH gene, gcvT gene, visC gene, ubiH gene, pepP gene, serA gene, rpiA gene, iciA gene, sbm gene, argK gene, fba gene, pgk gene, epd gene, tktA gene, speB gene, speA gene, metK gene, galP gene, sprT gene, endA gene, gshB gene, yqgF gene, ansB gene, mutY gene, mltC gene, nupG gene, speC gene, pitB gene, gsp gene, hybG gene, hybF gene, hybE gene, hybD gene, hybC gene, hybB gene, hybA gene, hoxK gene, exbD gene, exbB gene, metC gene, exsA gene, sufI gene, plsC gene, parC gene, parE gene, icc gene, tolC gene, ribB gene, glgS gene, rfaE gene, glnE gene, cca gene, bacA gene, folB gene, ttdA gene, ttdB gene, ygjD gene, rpsU gene, dnaG gene, rpoD gene, aer gene, ebgR gene, ebgA gene, ebgC gene, fadH gene, uxaA gene, uxaC gene, exuT gene, exuR gene, tdcE gene, tdcD gene, tdcC gene, tdcB gene, tdcA gene, tdcR gene, sohA gene, agaR gene, agaZ gene, agaV gene, agaW gene, agaA gene, agaS gene, agaY gene, agaB gene, agaC gene, agaD gene, agaI gene, mtr gene, deaD gene, nlpI gene, pnp gene, rpsO gene, truB gene, rbfA gene, infB gene, nusA gene, argG gene, secG gene, mrsA gene, folP gene, ftsH gene, ftsJ gene, greA gene, dacB gene, yhbZ gene, rpmA gene, rplU gene, ispB gene, nlp gene, murA gene, yrbI gene, yrbK gene, rpoN gene, ptsN gene, ptsO gene, mtgA gene, arcB gene, gltB gene, nanT gene, npl gene, nanR gene, dcuD gene, sspB gene, sspA gene, rpsI gene, rplM gene, hhoA gene, hhoB gene, mdh gene, argR gene, tldD gene, rng gene, mreD gene, mreC gene, mreB gene, accB gene, accC gene, panF gene, prmA gene, fis gene, aroE gene, smg gene, smf gene, def gene, fmt gene, sun gene, trkA gene, mscL gene, zntR gene, rplQ gene, rpoA gene, rpsD gene, rpsK gene, rpsM gene, rpmJ gene, secY gene, rplO gene, rpmD gene, rpsE gene, rplR gene, rplF gene, rpsH gene, rpsN gene, rplE gene, rplX gene, rplN gene, rpsQ gene, rpmC gene, rplP gene, rpsC gene, rplV gene, rpsS gene, rplB gene, rplW gene, rplD gene, rplC gene, rpsJ gene, tufA gene, fusA gene, rpsG gene, rpsL gene, fkpA gene, slyX gene, slyD gene, kefB gene, prkB gene, crp gene, argD gene, pabA gene, fic gene, ppiA gene, nirB gene, nirD gene, nirC gene, cysG gene, php gene, trpS gene, gph gene, rpe gene, dam gene, damX gene, aroB gene, aroK gene, hofQ gene, mrcA gene, pckA gene, envZ gene, ompR gene, greB gene, feoA gene, feoB gene, bioH gene, gntT gene, malQ gene, malP gene, malT gene, rtcA gene, rtcB gene, rtcR gene, glpR gene, glpG gene, glpE gene, glpD gene, glgP gene, glgA gene, glgC gene, glgX gene, glgB gene, asd gene, gntU gene, gntU gene, gntK gene, gntR gene, ggt gene, ugpQ gene, ugpC gene, ugpE gene, ugpA gene, ugpB gene, livF gene, livG gene, livM gene, livH gene, livK gene, livJ gene, rpoH gene, ftsX gene, ftsE gene, ftsY gene, atzN gene, nikA gene, nikB gene, nikC gene, nikD gene, nikE gene, pitA gene, uspB gene, uspA gene, prlC gene, gor gene, arsR gene, arsB gene, arsC gene, slp gene, hdeB gene, hdeA gene, hdeD gene, gadA gene, treF gene, kdgK gene, dctA gene, dppF gene, dppD gene, dppC gene, dppB gene, dppA gene, tag gene, bisC gene, cspA gene, glyS gene, glyQ gene, xylB gene, xylA gene, xylF gene, xylG gene, xylH gene, xylR gene, bax gene, malS gene, avtA gene, lyx gene, sgbH gene, sgbU gene, sgbE gene, aldB gene, selB gene, selA gene, mtlA gene, mtld gene, mtlR gene, lldP gene, lldR gene, lldD gene, cysE gene, gpsA gene, secB gene, grxC gene, tdh gene, kbl gene, htrL gene, rfaD gene, rfaF gene, rfaC gene, rfaL gene, rfaK gene, rfaZ gene, rfaY gene, rfaJ gene, rfaI gene, rfaB gene, rfaS gene, rfaP gene, rfaG gene, rfaQ gene, kdtA gene, kdtB gene, mutM gene, rpmG gene, rpmB gene, radC gene, dfp gene, dut gene, ttk gene, pyrE gene, rph gene, dinD gene, gmk gene, rpoZ gene, spoT gene, spoU gene, recG gene, gltS gene, nlpA gene, uhpT gene, uhpC gene, uhpB gene, uhpA gene, ilvN gene, ilvB gene, ivbL gene, emrD gene, ibpB gene, ibpA gene, gyrB gene, recF gene, dnaN gene, dnaA gene, rpmH gene, rnpA gene, yidC gene, thdF gene, tnaL gene, tnaA gene, tnaB gene, bglB gene, bglF gene, bglG gene, phoU gene, pstB gene, pstA gene, pstC gene, pstS gene, glmS gene, glmU gene, atpC gene, uncD gene, atpG gene, atpA gene, atpH gene, atpF gene, atpE gene, atpB gene, atpI gene, gidB gene, gidA gene, mioC gene, asnC gene, pssR gene, ilvL gene, ilvG gene, ilvM gene, ilvE gene, ilvD gene, ilvA gene, ilvY gene, ilvC gene, ppiC gene, rep gene, gppA gene, rhlB gene, trxA gene, rhoL gene, rho gene, rfe gene, wzzE gene, nfrC gene, rffD gene, rffG gene, rffH gene, rffA gene, wzxE gene, rffT gene, wecG gene, aslB gene, aslA gene, hemY gene, hemX gene, hemD gene, hemC gene, cyaA gene, cyaY gene, dapF gene, xerC gene, uvrD gene, corA gene, radD gene, pldA gene, recQ gene, pldB gene, metR gene, metE gene, udp gene, ubiE gene, aarF gene, tatC gene, tatD gene, rfaH gene, ubiD gene, ubiB gene, fadA gene, fadB gene, pepQ gene, trkH gene, trkH gene, hemG gene, mobB gene, mobA gene, dsbA gene, polA gene, yihA gene, hemN gene, ntrC gene, ntrB gene, glnA gene, fdhE gene, fdoI gene, fdoH gene, fdoG gene, fdhD gene, sodA gene, kdgT gene, cpxA gene, cpxR gene, cpxP gene, pfkA gene, sbp gene, cdh gene, tpiA gene, fpr gene, glpX gene, glpK gene, glpF gene, menG gene, menA gene, hslU gene, hslV gene, ftsN gene, cytR gene, priA gene, rpmE gene, metJ gene, metB gene, metL gene, metF gene, katG gene, gldA gene, talC gene, ppc gene, argE gene, argC gene, argB gene, argH gene, oxyR gene, udhA gene, trmA gene, btuB gene, murI gene, murB gene, birA gene, coaA gene, tufB gene, secE gene, nusG gene, rplK gene, rplA gene, rplJ gene, rplL gene, rpoB gene, rpoC gene, htrC gene, thiH gene, thiG gene, thiS gene, thiF gene, thiE gene, thiC gene, rsd gene, hemE gene, nfi gene, hupA gene, hydH gene, hydG gene, purD gene, purH gene, metA gene, aceB gene, aceA gene, aceK gene, arp gene, iclR gene, metH gene, pepE gene, lysC gene, pgi gene, ubiC gene, ubiA gene, plsB gene, dgkA gene, lexA gene, dinF gene, qor gene, dnaB gene, alr gene, tyrB gene, napA gene, hobH gene, uvrA gene, ssb gene, soxS gene, soxR gene, acs gene, nrfA gene, nrfB gene, nrfC gene, nrfD gene, nrfE gene, nrfF gene, nrfG gene, gltP gene, fdhF gene, alsR gene, rpiB gene, proP gene, basS gene, basR gene, adiY gene, adi gene, melR gene, melA gene, melB gene, fumB gene, dcuB gene, dcuR gene, dcuS gene, lysU gene, cadA gene, cadB gene, cadC gene, cutA3 gene, cutA2 gene, cutA gene, dcuA gene, aspA gene, fxsA gene, groES gene, groEL gene, efp gene, ecnB gene, sugE gene, blc gene, ampC gene, frdD gene, frdC gene, frdb gene, frdA gene, genX gene, psd gene, yjeQ gene, yjeE gene, amiB gene, mutL gene, miaA gene, hfq gene, hflX gene, hflK gene, hflC gene, purA gene, vacB gene, sgaE gene, rpsF gene, priB gene, rpsR gene, rpll gene, fklB gene, cycA gene, cpdB gene, cysQ gene, msrA gene, chpS gene, chpB gene, ppa gene, fbp gene, mpl gene, pmbA gene, cybC gene, nrdG gene, nrdD gene, treC gene, treB gene, treR gene, mgtA gene, pyrI gene, pyrB gene, pyrBI gene, argI gene, valS gene, holC gene, xerB gene, idnR gene, idnT gene, idnO gene, idnD gene, idnK gene, fecE gene, fecD gene, fecC gene, fecB gene, fecA gene, fecR gene, fecI gene, dnaC gene, dnaT gene, bglJ gene, fhuF gene, rsmC gene, holD gene, rimI gene, prfC gene, osmY gene, deoC gene, deoA gene, deoB gene, deoD gene, lplA gene, smp gene, serB gene, radA gene, nadR gene, slt gene, trpR gene, gpmG gene, rob gene, creA gene, creB gene, creC gene, creD gene, arcA gene, lasT gene, thrL gene, and
[2] acrE gene, argT gene, artI gene, artJ gene, artM gene, artP gene, artQ gene, bfr gene, chiA gene, chpA gene, dacC gene, deoR gene, dinG gene, elaA gene, elaB gene, elaC gene, elaD gene, envD gene, envR gene, fhuA gene, fhuB gene, fhuC gene, fhuD gene, folX gene, glnH gene, glnP gene, glnQ gene, gltJ gene, gltK gene, gltL gene, gspA gene, gspC gene, gspD gene, gspE gene, gspI gene, gspJ gene, gspK gene, gspL gene, hisJ gene, hisM gene, hisP gene, hisQ gene, hofD gene, hofF gene, hofG gene, hofH gene, ltaA gene, mdaA gene, mdaB gene, pinO gene, potF gene, potG gene, potH gene, potI gene, pshM gene, ptwB gene, ptwC gene, rhsB gene, rimK gene, xylU gene.
A specific example thereof is an *E*. *coli* mutant strain comprising chromosomal DNA that does not contain any gene selected from among genes excluding the above genes [1] and [2] among the genes shown in Table 1 or homologous genes thereof. {When the above genes [1] and [2] or homologous genes thereof comprise genes that wild-type *E*. *coli* strains do not originally contain (hereafter referred to as "NC genes"), however, the aforementioned *E*. *coli* mutant strain derived from the wild-type *E*. *coli* strain does not necessarily have the NC gene.} A more preferable example of the *E*. *coli* mutant strain of the present invention is an *E. coli* mutant strain comprising chromosomal DNA that does not contain DNA excluding the nucleotide sequences of the above genes [1] and [2] among the nucleotide sequences as shown in Table 1 and SEQ ID NO: 1 or DNA equivalent thereto. More preferred examples include the followings.
(2) The *E*. *coli* mutant strain according to (1) comprising chromosomal DNA that comprises DNA having the nucleotide sequences as shown in [1] to [69] below of the nucleotide sequence as shown in SEQ ID NO: 1 or DNA equivalent thereto.
[1] a nucleotide sequence of nucleotides 1 to 167064
[2] a nucleotide sequence of nucleotides 172980 to 243964
[3] a nucleotide sequence of nucleotides 253411 to 261963
[4] a nucleotide sequence of nucleotides 387532 to 389138
[5] a nucleotide sequence of nucleotides 403704 to 518029
[6] a nucleotide sequence of nucleotides 532712 to 535504
[7] a nucleotide sequence of nucleotides 550216 to 563940
[8] a nucleotide sequence of nucleotides 608119 to 675103
[9] a nucleotide sequence of nucleotides 689375 to 728820
[10] a nucleotide sequence of nucleotides 739594 to 832551
[11] a nucleotide sequence of nucleotides 848090 to 867637
[12] a nucleotide sequence of nucleotides 883474 to 890997
[13] a nucleotide sequence of nucleotides 909380 to 1048887
[14] a nucleotide sequence of nucleotides 1097020 to 1198105
[15] a nucleotide sequence of nucleotides 1225147 to 1630911
[16] a nucleotide sequence of nucleotides 1641039 to 1642631
[17] a nucleotide sequence of nucleotides 1650223 to 1964523
[18] a nucleotide sequence of nucleotides 1993076 to 1996661
[19] a nucleotide sequence of nucleotides 2028210 to 2033448
[20] a nucleotide sequence of nucleotides 2044377 to 2069148
[21] a nucleotide sequence of nucleotides 2081927 to 2104392
[22] a nucleotide sequence of nucleotides 2116065 to 2146263
[23] a nucleotide sequence of nucleotides 2187856 to 2200668
[24] a nucleotide sequence of nucleotides 2229851 to 2255894
[25] a nucleotide sequence of nucleotides 2263492 to 2270439
[26] a nucleotide sequence of nucleotides 2286589 to 2386261
[27] a nucleotide sequence of nucleotides 2395472 to 2423364
[28] a nucleotide sequence of nucleotides 2434099 to 2472851
[29] a nucleotide sequence of nucleotides 2482483 to 2505074
[30] a nucleotide sequence of nucleotides 2514558 to 2755676
[31] a nucleotide sequence of nucleotides 2789481 to 2795173
[32] a nucleotide sequence of nucleotides 2800891 to 2804307
[33] a nucleotide sequence of nucleotides 2810148 to 2825313
[34] a nucleotide sequence of nucleotides 2830263 to 2910273
[35] a nucleotide sequence of nucleotides 2910574 to 2985361
[36] a nucleotide sequence of nucleotides 3033129 to 3077084
[37] a nucleotide sequence of nucleotides 3078917 to 3110109
[38] a nucleotide sequence of nucleotides 3134342 to 3165627
[39] a nucleotide sequence of nucleotides 3172941 to 3360736
[40] a nucleotide sequence of nucleotides 3370548 to 3411159
[41] a nucleotide sequence of nucleotides 3420879 to 3453020
[42] a nucleotide sequence of nucleotides 3469367 to 3618575
[43] a nucleotide sequence of nucleotides 3636917 to 3854661
[44] a nucleotide sequence of nucleotides 3865832 to 3867101
[45] a nucleotide sequence of nucleotides 3877011 to 3926658
[46] a nucleotide sequence of nucleotides 3938879 to 4059897
[47] a nucleotide sequence of nucleotides 4079660 to 4086450
[48] a nucleotide sequence of nucleotides 4099983 to 4139231
[49] a nucleotide sequence of nucleotides 4149710 to 4235358
[50] a nucleotide sequence of nucleotides 4251736 to 4298976
[51] a nucleotide sequence of nucleotides 4311512 to 4313317
[52] a nucleotide sequence of nucleotides 4329722 to 4409532
[53] a nucleotide sequence of nucleotides 4423152 to 4496201
[54] a nucleotide sequence of nucleotides 4509535 to 4517875
[55] a nucleotide sequence of nucleotides 4599638 to 4641433
[56] a nucleotide sequence of nucleotides 167065 to 172979
[57] a nucleotide sequence of nucleotides 675104 to 689374
[58] a nucleotide sequence of nucleotides 832552 to 848089
[59] a nucleotide sequence of nucleotides 867638 to 883473
[60] a nucleotide sequence of nucleotides 890998 to 909379
[61] a nucleotide sequence of nucleotides 2386262 to 2395471
[62] a nucleotide sequence of nucleotides 2423365 to 2434098
[63] a nucleotide sequence of nucleotides 2472852 to 2482482
[64] a nucleotide sequence of nucleotides 2505075 to 2514557
[65] a nucleotide sequence of nucleotides 2910274 to 2910573
[66] a nucleotide sequence of nucleotides 3165628 to 3172940
[67] a nucleotide sequence of nucleotides 3411160 to 3420878
[68] a nucleotide sequence of nucleotides 3453021 to 3469366
[69] a nucleotide sequence of nucleotides 3618576 to 3636916

(3) The *E*. *coli* mutant strain according to (1) or (2) comprising chromosomal DNA that does not comprise yafJ gene, yafK gene, yafQ gene, dinJ gene, yafL gene, yafM gene, fhiA gene, lafU gene, dinP gene, yafN gene, yafO gene, yafP gene, JW0225 gene, prfH gene, ykfl gene, yafW gene, yeeT gene, ykfG gene, yafX gene, ykfF gene, ykfB gene, yafY gene, ypjK gene, yafZ gene, ykfA gene, perR gene, JW0245 gene, JW0246 gene, JW0247 gene, ykfC gene, JW0249 gene, is5B gene, yi51 gene, mmuP gene, mmuM gene, afuC gene, JW0255 gene, insB gene, insA gene, JW0258 gene, yagB gene, yagA gene, yagE gene, yagF gene, yagG gene, yagH gene, yagI gene, argF gene, yfjI gene, yagJ gene, yagK gene, yagL gene, yagM gene, yagN gene, intF gene, yagP gene, yagQ gene, yagR gene, yagS gene, yagT gene, yagU gene, ykgJ gene, yagV gene, yagW gene, yagX gene, yagY gene, yagZ gene, ykgK gene, ykgL gene, ykgM gene, eaeH gene, JW0292 gene, JW0293 gene, ykgA gene, ykgB gene, ykgI gene, merA gene, ykgD gene, ykgE gene, ykgF gene, ykgG gene, ykgH gene, betA gene, betB gene, betI gene, betT gene, yahA gene, yahB gene, yahC gene, yahD gene, yahE gene, yahF gene, yahG gene, yahH gene, arcM gene, yahJ gene, yahK gene, yahL gene, yahM gene, yahN gene, yahO gene, prpR gene, bcpA gene, prpC gene, prpD gene, prpE gene, codB gene, codA gene, cynR gene, cynT gene, cynS gene, cynX gene, lacA gene, lacY gene, lacZ gene, lacI gene, mhpR gene, mhpA gene, mhpB gene, mhpC gene, mhpD gene, mhpF gene, mhpE gene, mhpT gene, yaiL gene, yaiM gene, adhC gene, yaiN gene, yaiO gene, JW0350 gene, yi2A gene, yi22 gene, yi24 gene, yi23 gene, yaiP gene, yaiS gene, tauA gene, tauB gene, tauC gene, ssiD gene, yaiT gene, JW0363 gene, JW0364 gene, yaiU gene, yaiV gene, ampH gene, sbmA gene, yaiW gene, yaiY gene, yaiZ gene, ddlA gene, yaiB gene, phoA gene, psiF gene, yaiC gene, ybbO gene, tesA gene, ybbA gene, ybbP gene, rhsD gene, ybbC gene, rhsE gene, ybbD gene, JW0490 gene, ybbB gene, ybbS gene, ybbT gene, ybbU gene, ybbQ gene, ybbV gene, allP gene, allA gene, ybbY gene, ybbZ gene, ylbA gene, allC gene, ylbC gene, fdrA gene, ylbE gene, ylbF gene, arcC gene, intD gene, EXO_LAMBD gene, VRPP_LAMBD gene, JW0528 gene, JW0529 gene, VREN_LAMBD gene, emrE gene, ybcK gene, ybcL gene, ybcM gene, ybcN gene, ninE gene, ybcO gene, rus gene, ybcQ gene, JW0539.5 gene, nmpC gene, vlyS gene, ybcS gene, ybcT gene, vboR gene, ybcV gene, ybcW gene, nohB gene, ybcX gene, ybcY gene, JW0552 gene, appY gene, ompT gene, envY gene, ybcH gene, nfrA gene, nfrB gene, cusS gene, cusR gene, cusC gene, ylcC gene, cusB gene, cusA gene, pheP gene, ybdG gene, nfnB gene, ybdF gene, ybdJ gene, ybdK gene, hokE gene, yi81 gene, yi82 gene, ymcC gene, ymcD gene, cspH gene, cspG gene, sfa gene, yccL gene, yccM gene, torS gene, torT gene, torR gene, torC gene, torA gene, torD gene, yccD gene, cbpA gene, yccE gene, agp gene, yccJ gene, wrbA gene, yciG gene, ycdG gene, JW0992 gene, JW0993 gene, JW0994 gene, ycdK gene, JW0996 gene, JW0997 gene, ycdC gene, putA gene, yzpU gene, putP gene, JW1002 gene, ycdO gene, ycdB gene, phoH gene, ycdP gene, ycdQ gene, ycdR gene, ycdS gene, ycdT gene, JW1012 gene, JW1013 gene, JW1014 gene, ycdU gene, ymfD gene, ymfE gene, lit gene, intE gene, VXIS_BPP21 gene, ymfH gene, ymfI gene, ymfJ gene, RPC2_BPP22 gene, JW1132 gene, ymfL gene, ymfM gene, ymfN gene, ymfR gene, ymfO gene, JW1138 gene, JW1139 gene, ycfK gene, ymfS gene, ycfA gene, ycfE gene, pin gene, mcrA gene, JW1146 gene, ycgW gene, ycgX gene, ycgE gene, JW1150 gene, ycgZ gene, ymgA gene, JW1153 gene, ymgC gene, yahA gene, JW1156 gene, JW1157 gene, aidA gene, ymgD gene, JW1160 gene, JW1161 gene, JW1162 gene, ydfH gene, ydfZ gene, ydfI gene, shiA gene, JW1537 gene, tnpR gene, ydfM gene, Y314_LAMBD gene, nohA gene, ydfO gene, yccL gene, cspl gene, ydfR gene, vlyS gene, cspB gene, cspF gene, regQ_LAMBD gene, JW1552 gene, rem gene, hokD gene, relE gene, relB gene, ydfV gene, flxA gene, ydfW gene, ydfX gene, dicC gene, sdaC gene, yqeH gene, yqeI gene, yqeJ gene, yqeK gene, ygeF gene, ygeG gene, ygeH gene, ygeI gene, JW2822 gene, ygeK gene, JW2824 gene, JW2825 gene, JW2830 gene, ygeP gene, JW2832 gene, nlpD gene, yagR gene, ygeT gene, JW2836 gene, ygeV gene, ygeW gene, dpaL gene, ygeY gene, ygeZ gene, arcL gene, yqeB gene, JW2844 gene, ygfJ gene, gltD gene, JW2847 gene, ygfM gene, yagR gene, ygfO gene, ygfP gene, ygfQ gene, ygfR gene, ygfS gene, ygfT gene, pbuX gene, idi gene, epsM gene, exeL gene, exeC gene, JW2938 gene, JW2939 gene, JW2940 gene, JW2941 gene, yghK gene, glcB gene, glcG gene, glcF gene, glcD gene, glcC gene, JW2948 gene, yghQ gene, yghR gene, yghS gene, yghT gene, yihL gene, yihM gene, yihN gene, yshA gene, yihO gene, yihP gene, yihQ gene, yihR gene, yihS gene, yihT gene, yihU gene, yihV gene, yihW gene, yihX gene, rbn gene, yihZ gene, yiiD gene, yiiE gene, yiiF gene, yiiG gene, frvR gene, frvX gene, frvB gene, frvA gene, yiiL gene, rhaD gene, rhaA gene, rhaB gene, rhaS gene, rhaR gene, rhaT gene, ptsA gene, JW3920 gene, frwC gene, frwB gene, pflD gene, pflC gene, frwD gene, yijO gene, yijP gene, JW3986 gene, yjbF gene, yjbG gene, yjbH gene, JW3989.5 gene, yjbA gene, xylE gene, malG gene, malF gene, malE gene, malK gene, lamB gene, malM gene, yjbI gene, yjcP gene, yjcQ gene, yjcR gene, yjcS gene, alsK gene, alsE gene, alsC gene, alsA gene, alsB gene, phnQ gene, phnP gene, phnO gene, phnN gene, phnM gene, phnL gene, phnK gene, phnJ gene, phnI gene, phnH gene, phnG gene, phnF gene, phnE gene, phnD gene, phnC gene, phnB gene, phnA gene, yjdA gene and yjcZ gene, or homologous gene thereof.
(4) The *E*. *coli* mutant strain according to any one of (1) to (3) comprising chromosomal DNA that does not comprise DNA having the nucleotide sequences as shown in [1] to [18] below of the nucleotide sequence as shown in SEQ ID NO: 1 or DNA equivalent thereto.
[1] a nucleotide sequence of nucleotides 243965 to 253410
[2] a nucleotide sequence of nucleotides 261964 to 387531
[3] a nucleotide sequence of nucleotides 389139 to 403703
[4] a nucleotide sequence of nucleotides 518030 to 532711
[5] a nucleotide sequence of nucleotides 535505 to 550215
[6] a nucleotide sequence of nucleotides 563941 to 608118
[7] a nucleotide sequence of nucleotides 1048888 to 1097019
[8] a nucleotide sequence of nucleotides 1198106 to 1225146
[9] a nucleotide sequence of nucleotides 1630912 to 1641038
[10] a nucleotide sequence of nucleotides 1642632 to 1650222
[11] a nucleotide sequence of nucleotides 2985362 to 3033128
[12] a nucleotide sequence of nucleotides 3110110 to 3134341
[13] a nucleotide sequence of nucleotides 4059898 to 4079659
[14] a nucleotide sequence of nucleotides 4086451 to 4099982
[15] a nucleotide sequence of nucleotides 4139232 to 4149709
[16] a nucleotide sequence of nucleotides 4235359 to 4251735
[17] a nucleotide sequence of nucleotides 4298977 to 4311511
[18] a nucleotide sequence of nucleotides 4313318 to 4329721

(5) The *E*. *coli* mutant strain according to any one of (1) to (4) comprising chromosomal DNA that does not comprise yidF gene, yidG gene, yidH gene, yidI gene, yidJ gene, yidK gene, yidL gene, JW3657 gene, glvG gene, glvB gene, glvC gene, yidP gene, yidE gene, yidQ gene, yidR gene, yidS gene, dgoT gene, dgoA gene, dgoK gene, dgoR gene, yidW gene, yidX gene, yidA gene, yidB gene, asnA gene, yieM gene, JW3724 gene, yieN gene, kup gene, rbsD gene, rbsA gene, rbsC gene, rbsB gene, rbsK gene, rbsR gene and yieO gene, or homologous gene thereof.
(6) The *E*. *coli* mutant strain according to any one of (1) to (5) comprising chromosomal DNA that does not comprise DNA having the nucleotide sequences as shown in [1] to [3] below of the nucleotide sequence as shown in SEQ ID NO: 1 or DNA equivalent thereto.
[1] a nucleotide sequence of nucleotides3854662 to 3865831
[2] a nucleotide sequence of nucleotides3867102 to 3877010
[3] a nucleotide sequence of nucleotides 3926659 to 3938878

(7) The *E. coli* mutant strain according to any one of (1) to (6) comprising chromosomal DNA that does not comprise yecT gene, flhE gene, flhA gene, flhB gene, cheZ gene, cheY gene, cheB gene, cheR gene, tap gene, tar gene, cheW gene, cheA gene, motB gene, motA gene, flhC gene, flhD gene, JW1882 gene, JW1883 gene, yecG gene, otsA gene, otsB gene, araH gene, araG gene, araF gene, yecI gene, yecJ gene, JW1892 gene, ftn gene, yecH gene, tyrP gene, uvrC gene, uvrY gene, yecF gene, sdiA gene, yecC gene, yecS gene, yedO gene, fliY gene, fliZ gene, fliA gene, fliC gene, fliD gene, fliS gene, fliT gene, amyA gene, yedD gene, yedE gene, yedF gene, yedK gene, yedL gene, yedN gene, yedM gene, fliE gene, fliF gene, fliG gene, fliH gene, fliI gene, fliJ gene, fliK gene, fliL gene, fliM gene, fliN gene, fliO gene, fliP gene, fliQ gene, fliR gene, rcsA gene, dsrB gene, JW1937 gene, yedA gene, vsr gene, dcm gene, yedJ gene, JW 1946 gene, ompC gene, JW1948 gene, yedS gene, yedU gene, yedV gene, copR gene, yedX gene, JW1954 gene, JW1955 gene, JW1956 gene, JW1974 gene, JW1979 gene, JW1980 gene, yeeP gene, flu gene, JW1983 gene, yeeS gene, yeeU gene, yeeV gene, yeeW gene, yefJ gene, JW2013 gene, yefJ gene, yefI gene, wbbJ gene, yefG gene, rfc gene, glf gene, rfbX gene, rfbC gene, rfbA gene, rfbD gene and rfbB gene, or homologous gene thereof.
(8) The *E*. *coli* mutant strain according to any one of (1) to (7) comprising chromosomal DNA that does not comprise DNA having the nucleotide sequences as shown in [1] to [5] below of the nucleotide sequence as shown in SEQ ID NO: 1 or DNA equivalent thereto.
[1] a nucleotide sequence of nucleotides1964524 to 1993075
[2] a nucleotide sequence of nucleotides 1996662 to 2028209
[3] a nucleotide sequence of nucleotides 2033449 to 2044376
[4] a nucleotide sequence of nucleotides 2069149t to 2081926
[5] a nucleotide sequence of nucleotides 2104393 to 2116064

(9) The *E*. *coli* mutant strain according to any one of (1) to (8) comprising chromosomal DNA that does not comprise intA gene, yfjH gene, alpA gene, yfjI gene, JW2606 gene, yfjJ gene, yfjK gene, yfjL gene, yfjM gene, yfjN gene, yfjO gene, yfjP gene, yfjQ gene, yfjR gene, ypjK gene, yfjS gene, yfjT gene, yfjU gene, yfjV gene, JW2622 gene, yfjW gene, JW2623.5 gene, yfjX gene, yfjY gene, yfjZ gene, ypjF gene, ypjA gene, JW2629 gene, JW2630 gene, JW2631 gene, JW2632 gene, JW2633 gene, JW2634 gene, ygaE gene, yzzM gene, yqaE gene, JW2642 gene, ygaP gene, stpA gene, JW2645 gene, ygaC gene, ygaM gene, nrdH gene, nrdI gene, nrdE gene, proV gene, proW gene, proX gene, JW2655 gene, JW2656 gene, ygaZ gene, ygaH gene, srlA gene, srlB gene, srlD gene, gutM gene, srlR gene and gutQ gene, or homologous gene thereof.
(10) The *E. coli* mutant strain according to any one of (1) to (9) comprising chromosomal DNA that does not comprise DNA having the nucleotide sequences as shown in [1] to [4] below of the nucleotide sequence as shown in SEQ ID NO: 1 or DNA equivalent thereto.
[1] a nucleotide sequence of nucleotides 2755677 to 2789480
[2] a nucleotide sequence of nucleotides 2795174 to 2800890
[3] a nucleotide sequence of nucleotides 2804308 to 2810147
[4] a nucleotide sequence of nucleotides 2825314 to 2830262

(11) The *E*. *coli* mutant strain according to any one of (1) to (10) comprising chromosomal DNA that does not comprise gltF gene, yhcA gene, yhcD gene, yhcE gene, yhcF gene, yhcG gene, yhcH gene, nanK gene and nanE gene, or homologous gene thereof.
(12) The *E*. *coli* mutant strain according to any one of (1) to (11) comprising chromosomal DNA that does not comprise DNA having a nucleotide sequence comprising nucleotides 3360737 to 3370547 of the nucleotide sequence as shown in SEQ ID NO: 1 or DNA equivalent thereto.
(13) The *E*. *coli* mutant strain according to any one of (1) to (12) comprising chromosomal DNA that does not comprise yegE gene, alkA gene, yegD gene, JW2055 gene, JW2056 gene, JW2057 gene, JW2058 gene, yegM gene, yegN gene, yegO gene, yegB gene, baeS gene, baeR gene, JW2065 gene, yegQ gene, ogrK gene, JW2068 gene, JW2069 gene, yegS gene, gatR gene, JW2072 gene, JW2073 gene, gatR gene, gatD gene, gatC gene, gatB gene, gatA gene, gatZ gene, gatY gene, fbaB gene, xegT gene, yegU gene, yegV gene, yegW gene, yegX gene, molR gene, yehI gene, yehK gene, yehL gene, yehO gene, yehP gene, yehQ gene, yehR gene, yehS gene, yehT gene, yehU gene, yehV gene, yehW gene, yehX gene, yehY gene, yehZ gene, bglX gene, dld gene, pbpG gene, yohC gene, yeiQ gene, yeiR gene, yeiU gene, spr gene, rtn gene, yejA gene, yejB gene, yejE gene, yejF gene, yejG gene, bcr gene, rsuA gene, yejH gene, yeiI gene, yeiJ gene, yeiK gene, yeiL gene, yeiM gene, yeiN gene and yeiC gene, or homologous gene thereof.
(14) The *E. coli* mutant strain according to any one of (1) to (13) comprising chromosomal DNA that does not comprise DNA having the nucleotide sequences as shown in [1] to [4] below of the nucleotide sequence as shown in SEQ ID NO: 1 or DNA equivalent thereto.
[1] a nucleotide sequence of nucleotides2146264 to 2187855
[2] a nucleotide sequence of nucleotides 2200669 to 2229850
[3] a nucleotide sequence of nucleotides 2270440 to 2286588
[4] a nucleotide sequence of nucleotides 2255895 to 2263491

(15) The *E. coli* mutant strain according to any one of (1) to (14) comprising chromosomal DNA that does not comprise cmtA gene, cmtB gene, or homologous gene thereof.
(16) The *E. coli* mutant strain according to any one of (1) to (15) comprising chromosomal DNA that does not comprise DNA having a nucleotide sequence comprising nucleotides 3077085 to 3078916 of the nucleotide sequence as shown in SEQ ID NO: 1 or DNA equivalent thereto.
(17) The *E*. *coli* mutant strain according to any one of (1) to (16) comprising chromosomal DNA that does not comprise intB gene, JW4228 gene, yjgW gene, yjgX gene, yjgY gene, yjgZ gene, yi41 gene, yi42 gene, yjhB gene, yjhC gene, yjhD gene, yjhE gene, yi91 gene, JW4244 gene, JW4245 gene, JW4246 gene, yjhU gene, yjhF gene, yjhG gene, yjhH gene, yjhI gene, sgcR gene, sgcE gene, sgcA gene, sgcQ gene, sgcC gene, JW4266.5 gene, sgcX gene, yjhP gene, yjhQ gene, JW4269.5 gene, JW4270 gene, JW4270.5 gene, yjhR gene, yjhS gene, yjhT gene, yjhA gene, fimB gene, fimE gene, fimA gene, fimI gene, fimC gene, fimD gene, fimF gene, fimG gene, fimH gene, gntP gene, uxuA gene, uxuB gene, uxuR gene, yjiC gene, yjiD gene, yjiE gene, iadA gene, yjiG gene, yjiH gene, yjiI gene, yj iJ gene, yjiK gene, JW4296.5 gene, yjiL gene, yjiM gene, yjiN gene, yjiO gene, yjiP gene, yjiQ gene, yjiR gene, yjiS gene, yjiT gene, yjiV gene, mcrD gene, mcrC gene, mcrB gene, yjiW gene, hsdS gene, hsdM gene, hsdR gene, mrr gene, yjiA gene, yjiX gene, yjiY gene, tsr gene, yjjL gene, JW4320 gene, yjjM gene, yjjN gene, mdoB gene and yjjA gene, or homologous gene thereof.
(18) The *E*. *coli* mutant strain according to any one of (1) to (17) comprising chromosomal DNA that does not comprise DNA having the nucleotide sequences as shown in [1] and/or [2] below of the nucleotide sequence as shown in SEQ ID NO: 1 or DNA equivalent thereto.
[1] a nucleotide sequence of nucleotides 4496202 to 4509534
[2] a nucleotide sequence of nucleotides 4517876 to 4599637

(19) The *E. coli* mutant strain according to any one of (1) to (18) comprising chromosomal DNA that does not comprise kdpF gene, ybfA gene, rhsC gene, JW0690 gene, ybfB gene, rhsA gene, ybfC gene, ybfD gene, ybfL gene, ybfD gene and ybgA gene, or homologous gene thereof.
(20) The *E*. *coli* mutant strain according to any one of (1) to (19) comprising chromosomal DNA that does not comprise DNA having a nucleotide sequence comprising nucleotides 728821 to 739593 of the nucleotide sequence as shown in SEQ ID NO: 1 or DNA equivalent thereto.
(21) The *E. coli* mutant strain according to any one of (1) to (20) comprising chromosomal DNA that does not comprise yjfI gene, yjfJ gene, yjfK gene, yjfL gene, yjfM gene, yjfC gene, aidB gene, yjfN gene, yjfO gene, yjfP gene, yjfQ gene, yjfR gene, sgaT gene, sgaB gene, sgaA gene, sgaH gene and sgaU gene, or homologous gene thereof.
(22) The *E*. *coli* mutant strain according to any one of (1) to (21) comprising chromosomal DNA that does not comprise DNA having a nucleotide sequence comprising nucleotides 4409533 to 4423151 of the nucleotide sequence as shown in SEQ ID NO: 1 or DNA equivalent thereto.
Further, the mutant of the present invention includes the followings. (23) An *E. coli* mutant strain comprising the gene (1) [1] above, spr gene, bcr gene, rsuA gene, or homologous gene thereof and comprising chromosomal DNA that is at least 933 kbp shorter than that of a wild-type *E*. *coli* strain, which may be the mutant strain comprising chromosomal DNA that does not comprise any gene excluding the above genes that are present on chromosomal DNA of a wild-type *E*. *coli* strain or homologous genes thereof, preferably the mutant strain comprising chromosomal DNA that does not contain gene other than genes contained in Δ43 region of Table 1; more preferably the mutant strain comprising chromosomal DNA that does not contain DNA other than DNA comprising nucleotide sequence corresponding to Δ43 region of Table 1 or DNA equivalent thereto. Further more preferably, the mutant of the present invention includes the followings.
(24) The *E. coli* mutant strain according to (23) comprising chromosomal DNA that comprises DNA having the nucleotide sequences as shown in [1] to [55] of (2) above and DNA having a nucleotide sequence comprising nucleotides 2270440 to 2286588 of the nucleotide sequence as shown in SEQ ID NO: 1 or DNA equivalent thereto.
(25) The *E. coli* mutant strain according to (23) or (24) comprising chromosomal DNA that does not comprise yafJ gene, yafK gene, yafQ gene, dinJ gene, yafL gene, yafM gene, fhiA gene, lafU gene, dinP gene, yafN gene, yafO gene, yafP gene, JW0225 gene, prfH gene, ykfI gene, yafW gene, yeeT gene, ykfG gene, yafX gene, ykfF gene, ykfB gene, yafY gene, ypjK gene, yafZ gene, ykfA gene, perR gene, JW0245 gene, JW0246 gene, JW0247 gene, ykfC gene, JW0249 gene, is5B gene, yi51 gene, mmuP gene, mmuM gene, afuC gene, JW0255 gene, insB gene, insA gene, JW0258 gene, yagB gene, yagA gene, yagE gene, yagF gene, yagG gene, yagH gene, yagI gene, argF gene, yfjI gene, yagJ gene, yagK gene, yagL gene, yagM gene, yagN gene, intF gene, yagP gene, yagQ gene, yagR gene, yagS gene, yagT gene, yagU gene, ykgJ gene, yagV gene, yagW gene, yagX gene, yagY gene, yagZ gene, ykgK gene, ykgL gene, ykgM gene, eaeH gene, JW0292 gene, JW0293 gene, ykgA gene, ykgB gene, ykgI gene, merA gene, ykgD gene, ykgE gene, ykgF gene, ykgG gene, ykgH gene, betA gene, betB gene, betI gene, betT gene, yahA gene, yahB gene, yahC gene, yahD gene, yahE gene, yahF gene, yahG gene, yahH gene, arcM gene, yahJ gene, yahK gene, yahL gene, yahM gene, yahN gene, yahO gene, prpR gene, bcpA gene, prpC gene, prpD gene, prpE gene, codB gene, codA gene, cynR gene, cynT gene, cynS gene, cynX gene, lacA gene, lacY gene, lacZ gene, lacI gene, mhpR gene, mhpA gene, mhpB gene, mhpC gene, mhpD gene, mhpF gene, mhpE gene, mhpT gene, yaiL gene, yaiM gene, adhC gene, yaiN gene, yaiO gene, JW0350 gene, yi2A gene, yi22 gene, yi24 gene, yi23 gene, yaiP gene, yaiS gene, tauA gene, tauB gene, tauC gene, ssiD gene, yaiT gene, JW0363 gene, JW0364 gene, yaiU gene, yaiV gene, ampH gene, sbmA gene, yaiW gene, yaiY gene, yaiZ gene, ddlA gene, yaiB gene, phoA gene, psiF gene, yaiC gene, ybbO gene, tesA gene, ybbA gene, ybbP gene, rhsD gene, ybbC gene, rhsE gene, ybbD gene, JW0490 gene, ybbB gene, ybbS gene, ybbT gene, ybbU gene, ybbQ gene, ybbV gene, allP gene, allA gene, ybbY gene, ybbZ gene, ylbA gene, allC gene, ylbC gene, fdrA gene, ylbE gene, ylbE gene, ylbE gene, ylbF gene, arcC gene, intD gene, EXO_LAMBD gene, VRPP _LAMBD gene, JW0528 gene, JW0529 gene, VREN_LAMBD gene, emrE gene, ybcK gene, ybcL gene, ybcM gene, ybcN gene, ninE gene, ybcO gene, rus gene, ybcQ gene, JW0539.5 gene, nmpC gene, vlyS gene, ybcS gene, ybcT gene, vboR gene, ybcV gene, ybcW gene, nohB gene, ybcX gene, ybcY gene, JW0552 gene, appY gene, ompT gene, envY gene, ybcH gene, nfrA gene, nfrB gene, cusS gene, cusR gene, cusC gene, ylcC gene, cusB gene, cusA gene, pheP gene, ybdG gene, nfnB gene, ybdF gene, ybdJ gene, ybdK gene, hokE gene, yi81 gene, yi82 gene, ymcC gene, ymcD gene, cspH gene, cspG gene, sfa gene, yccL gene, yccM gene, torS gene, torT gene, torR gene, torC gene, torA gene, torD gene, yccD gene, cbpA gene, yccE gene, agp gene, yccJ gene, wrbA gene, yciG gene, ycdG gene, JW0992 gene, JW0993 gene, JW0994 gene, ycdK gene, JW0996 gene, JW0997 gene, ycdC gene, putA gene, yzpU gene, putP gene, JW1002 gene, ycdO gene, ycdB gene, phoH gene, ycdP gene, ycdQ gene, ycdR gene, ycdR gene, ycdS gene, ycdT gene, JW1012 gene, JW1013 gene, JW1014 gene, ycdU gene, ymfD gene, ymfE gene, lit gene, intE gene, VXIS_BPP21 gene, ymfH gene, ymfI gene, ymfJ gene, RPC2_BPP22 gene, JW1132 gene, ymfL gene, ymfM gene, ymfN gene, ymfR gene, ymfO gene, JW1138 gene, JW1139 gene, ycfK gene, ymfS gene, ycfA gene, ycfE gene, pin gene, mcrA gene, JW1146 gene, ycgW gene, ycgX gene, ycgE gene, JW1150 gene, ycgZ gene, ymgA gene, JW 1153 gene, ymgC gene, yahA gene, JW1156 gene, JW1157 gene, aidA gene, ymgD gene, JW1160 gene, JW1161 gene, JW1162 gene, ydfH gene, ydfZ gene, ydfI gene, shiA gene, JW1537 gene, tnpR gene, ydfM gene, Y314_LAMBD gene, nohA gene, ydfO gene, yccL gene, cspI gene, ydfR gene, vlyS gene, cspB gene, cspF gene, regQ_LAMBD gene, JW1552 gene, rem gene, hokD gene, relE gene, relB gene, ydfV gene, flxA gene, ydfW gene, ydfX gene, dicC gene, sdaC gene, yqeH gene, yqeI gene, yqeJ gene, yqeK gene, ygeF gene, ygeG gene, ygeH gene, ygeI gene, JW2822 gene, ygeK gene, JW2824 gene, JW2825 gene, JW2830 gene, ygeP gene, JW2832 gene, nlpD gene, yagR gene, ygeT gene, JW2836 gene, ygeV gene, ygeW gene, dpaL gene, ygeY gene, ygeZ gene, arcL gene, yqeB gene, JW2844 gene, ygfJ gene, gltD gene, JW2847 gene, ygfM gene, yagR gene, ygfO gene, ygfP gene, ygfQ gene, ygfR gene, ygfS gene, ygfT gene, pbuX gene, idi gene, epsM gene, exeL gene, exeC gene, JW2938 gene, JW2939 gene, JW2940 gene, JW2941 gene, yghK gene, glcB gene, glcG gene, glcF gene, glcD gene, glcC gene, JW2948 gene, yghQ gene, yghR gene, yghS gene, yghT gene, yihL gene, yihM gene, yihN gene, yshA gene, yihO gene, yihP gene, yihQ gene, yihR gene, yihS gene, yihT gene, yihU gene, yihV gene, yihW gene, yihX gene, rbn gene, yihZ gene, yiiD gene, yiiE gene, yiiF gene, yiiG gene, frvR gene, frvX gene, frvB gene, frvA gene, yiiL gene, rhaD gene, rhaA gene, rhaB gene, rhaS gene, rhaR gene, rhaT gene, ptsA gene, JW3920 gene, frwC gene, frwB gene, pflD gene, pflC gene, frwD gene, yijO gene, yijP gene, JW3986 gene, yjbF gene, yjbG gene, yjbH gene, JW3989.5 gene, yjbA gene, xylE gene, malG gene, malF gene, malE gene, malK gene, lamB gene, malM gene, yjbl gene, yjcP gene, yjcQ gene, yjcR gene, yjcS gene, alsK gene, alsE gene, alsC gene, alsA gene, alsB gene, phnQ gene, phnP gene, phnO gene, phnN gene, phnM gene, phnL gene, phnK gene, phnJ gene, phnl gene, phnH gene, phnG gene, phnF gene, phnE gene, phnD gene, phnC gene, phnB gene, phnA gene, yjdA gene, yjcZ gene, yidF gene, yidG gene, yidH gene, yidI gene, yidJ gene, yidK gene, yidL gene, JW3657 gene, glvG gene, glvB gene, glvC gene, yidP gene, yidE gene, yidQ gene, yidR gene, yidS gene, dgoT gene, dgoA gene, dgoK gene, dgoR gene, yidW gene, yidX gene, yidA gene, yidB gene, asnA gene, yieM gene, JW3724 gene, yieN gene, kup gene, rbsD gene, rbsA gene, rbsC gene, rbsB gene, rbsK gene, rbsR gene, yieO gene, yecT gene, flhE gene, flhA gene, flhB gene, cheZ gene, cheY gene, cheB gene, cheR gene, tap gene, tar gene, cheW gene, cheA gene, motB gene, motA gene, flhC gene, flhD gene, JW1882 gene, JW1883 gene, yecG gene, otsA gene, otsB gene, araH gene, araG gene, araF gene, yecI gene, yecJ gene, JW1892 gene, ftn gene, yecH gene, tyrP gene, uvrC gene, uvrY gene, yecF gene, sdiA gene, yecC gene, yecS gene, yedO gene, fliY gene, fliZ gene, fliA gene, fliC gene, fliD gene, fliS gene, fliT gene, amyA gene, yedD gene, yedE gene, yedF gene, yedK gene, yedL gene, yedN gene, yedM gene, fliE gene, fliF gene, fliG gene, fliH gene, fliI gene, fliJ gene, fliK gene, fliL gene, fliM gene, fliN gene, fliO gene, fliP gene, fliQ gene, fliR gene, rcsA gene, dsrB gene, JW1937 gene, yedA gene, vsr gene, dcm gene, yedJ gene, JW1946 gene, ompC gene, JW1948 gene, yedS gene, yedU gene, yedV gene, copR gene, yedX gene, JW1954 gene, JW1955 gene, JW1956 gene, JW1974 gene, JW1979 gene, JW1980 gene, yeeP gene, flu gene, JW1983 gene, yeeS gene, yeeT gene, yeeU gene, yeeV gene, yeeW gene, yefJ gene, JW2013 gene, yefJ gene, yefI gene, wbbJ gene, yefG gene, rfc gene, glf gene, rfbX gene, rfbC gene, rfbA gene, rfbD gene, rfbB gene, intA gene, yfjH gene, alpA gene, yfjI gene, JW2606 gene, yfjJ gene, yfjK gene, yfjL gene, yfjM gene, yfjN gene, yfjO gene, yfjP gene, yfjQ gene, yfjR gene, ypjK gene, yfjS gene, yfjT gene, yfjU gene, yfjV gene, JW2622 gene, yfjW gene, JW2623.5 gene, yfjX gene, yfjY gene, yfjZ gene, ypjF gene, ypjA gene, JW2629 gene, JW2630 gene, JW2631 gene, JW2632 gene, JW2633 gene, JW2634 gene, ygaE gene, yzzM gene, yqaE gene, JW2642 gene, ygaP gene, stpA gene, JW2645 gene, ygaC gene, ygaM gene, nrdH gene, nrdI gene, nrdE gene, proV gene, proW gene, proX gene, JW2655 gene, JW2656 gene, ygaZ gene, ygaH gene, srlA gene, srlB gene, srlD gene, gutM gene, srlR gene, gutQ gene, gltF gene, yhcA gene, yhcD gene, yhcE gene, yhcE gene, yhcF gene, yhcG gene, yhcH gene, nanK gene, nanE gene, yegE gene, alkA gene, yegD gene, JW2055 gene, JW2056 gene, JW2057 gene, JW2058 gene, yegM gene, yegN gene, yegO gene, yegB gene, baeS gene, baeR gene, JW2065 gene, yegQ gene, ogrK gene, JW2068 gene, JW2069 gene, yegS gene, gatR gene, JW2072 gene, JW2073 gene, gatR gene, gatD gene, gatC gene, gatB gene, gatA gene, gatZ gene, gatY gene, fbaB gene, xegT gene, yegU gene, yegV gene, yegW gene, yegX gene, molR gene, yehl gene, yehK gene, yehL gene, yehO gene, yehP gene, yehQ gene, yehR gene, yehS gene, yehT gene, yehU gene, yehV gene, yehW gene, yehX gene, yehY gene, yehZ gene, bglX gene, dld gene, pbpG gene, yohC gene, yeiI gene, yeiJ gene, yeiK gene, yeiL gene, yeiM gene, yeiN gene, yeiC gene, cmtA gene, cmtB gene, intB gene, JW4228 gene, yjgW gene, yjgX gene, yjgY gene, yjgZ gene, yi41 gene, yi42 gene, yjhB gene, yjhC gene, yjhD gene, yjhE gene, yi91 gene, JW4244 gene, JW4245 gene, JW4246 gene, yjhU gene, yjhF gene, yjhG gene, yjhH gene, yjhI gene, sgcR gene, sgcE gene, sgcA gene, sgcQ gene, sgcC gene, JW4266.5 gene, sgcX gene, yjhP gene, yjhQ gene, JW4269.5 gene, JW4270 gene, JW4270.5 gene, yjhR gene, yjhS gene, yjhT gene, yjhA gene, fimB gene, fimE gene, fimA gene, fimI gene, fimC gene, fimD gene, fimF gene, fimG gene, fimH gene, gntP gene, uxuA gene, uxuB gene, uxuR gene, yjiC gene, yjiD gene, yjiE gene, iadA gene, yjiG gene, yjiH gene, yjiI gene, yjiJ gene, yjiK gene, JW4296.5 gene, yjiL gene, yjiM gene, yjiN gene, yjiO gene, yjiP gene, yjiQ gene, yjiR gene, yjiS gene, yjiT gene, yjiV gene, mcrD gene, mcrC gene, mcrB gene, yjiW gene, hsdS gene, hsdM gene, hsdR gene, mrr gene, yjiA gene, yjiX gene, yjiY gene, tsr gene, yjjL gene, JW4320 gene, yjjM gene, yjjN gene, mdoB gene, yjjA gene, kdpF gene, ybfA gene, rhsC gene, JW0690 gene, ybfB gene, rhsA gene, ybfC gene, ybfL gene, ybfD gene, ybgA gene, yjfI gene, yjfJ gene, yjfK gene, yjfL gene, yjfM gene, yjfC gene, aidB gene, yjfN gene, yjfO gene, yjfP gene, yjfQ gene, yjfR gene, sgaT gene, sgaB gene, sgaA gene, sgaH gene, sgaU gene, rhsB gene, JW3448 gene, yhhH gene, yibA gene, yhhI gene, yhhJ gene, yhiH gene, yhiI gene, yhiJ gene, yhiK gene, yhiL gene, yhiM gene, yhiN gene, JW0782 gene, ybiA gene, dinG gene, ybiB gene, ybiC gene, ybiJ gene, ybiI gene, ybiX gene, ybiL gene, ybiM gene, ybiN gene, ybiO gene, glnQ gene, glnP gene, glnH gene, yliA gene, yliB gene, yliC gene, yliD gene, JW0817 gene, JW0818 gene, yliG gene, yliH gene, yliI gene, yliJ gene, dacC gene, deoR gene, ybjG gene, ybjC gene, mdaA gene, rimK gene, ybjN gene, potF gene, potG gene, potH gene, potI gene, ybjO gene, ybjF gene, artJ gene, artM gene, artQ gene, artI gene, artP gene, ybjP gene, JW0850 gene, JW0851 gene, JW0852 gene, ybjT gene and ltaA gene, or homologous gene thereof.
(26) The *E*. *coli* mutant strain according to any one of (23) to (25) comprising chromosomal DNA that does not comprise DNA having the nucleotide sequences as shown in [1] to [43] of the nucleotide sequence as shown in SEQ ID NO: 1 or DNA equivalent thereto.
[1] a nucleotide sequence of nucleotides 243965 to 253410
[2] a nucleotide sequence of nucleotides 261964 to 387531
[3] a nucleotide sequence of nucleotides 389139 to 403703
[4] a nucleotide sequence of nucleotides 518030 to 532711
[5] a nucleotide sequence of nucleotides 535505 to 550215
[6] a nucleotide sequence of nucleotides 563941 to 608118
[7] a nucleotide sequence of nucleotides 728821 to 739593
[8] a nucleotide sequence of nucleotides 832552 to 848089
[9] a nucleotide sequence of nucleotides 867638 to 883473
[10] a nucleotide sequence of nucleotides 890998 to 909379
[11] a nucleotide sequence of nucleotides 1048888 to 1097019
[12] a nucleotide sequence of nucleotides 1198106 to 1225146
[13] a nucleotide sequence of nucleotides 1630912 to 1641038
[14] a nucleotide sequence of nucleotides 1642632 to 1650222
[15] a nucleotide sequence of nucleotides 1964524 to 1993075
[16] a nucleotide sequence of nucleotides 1996662 to 2028209
[17] a nucleotide sequence of nucleotides 2033449 to 2044376
[18] a nucleotide sequence of nucleotides 2069149 to 2081926
[19] a nucleotide sequence of nucleotides 2104393 to 2116064
[20] a nucleotide sequence of nucleotides 2146264 to 2187855
[21] a nucleotide sequence of nucleotides 2200669 to 2229850
[22] a nucleotide sequence of nucleotides 2255895 to 2263491
[23] a nucleotide sequence of nucleotides 2755677 to 2789480
[24] a nucleotide sequence of nucleotides 2795174 to 2800890
[25] a nucleotide sequence of nucleotides 2804308 to 2810147
[26] a nucleotide sequence of nucleotides 2825314 to 2830262
[27] a nucleotide sequence of nucleotides 2985362 to 3033128
[28] a nucleotide sequence of nucleotides 3077085 to 3078916
[29] a nucleotide sequence of nucleotides 3110110 to 3134341
[30] a nucleotide sequence of nucleotides 3360737 to 3370547
[31] a nucleotide sequence of nucleotides 3618576 to 3636916
[32] a nucleotide sequence of nucleotides 3854662 to 3865831
[33] a nucleotide sequence of nucleotides 3867102 to 3877010
[34] a nucleotide sequence of nucleotides 3926659 to 3938878
[35] a nucleotide sequence of nucleotides 4059898 to 4079659
[36] a nucleotide sequence of nucleotides 4086451 to 4099982
[37] a nucleotide sequence of nucleotides 4139232 to 4149709
[38] a nucleotide sequence of nucleotides 4235359 to 4251735
[39] a nucleotide sequence of nucleotides 4298977 to 4311511
[40] a nucleotide sequence of nucleotides 4313318 to 4329721
[41] a nucleotide sequence of nucleotides 4409533 to 4423151
[42] a nucleotide sequence of nucleotides 4496202 to 4509534
[43] a nucleotide sequence of nucleotides 4517876 to 4599637

(27) The *E. coli* mutant strain according to any one of (23) to (26) comprising chromosomal DNA that does not comprise ygiS gene, ygiT gene, ygiU gene, JW2991 gene, ygiW gene, ygiX gene, ygiY gene, ygiZ gene, mdaB gene and ygiN gene, or homologous gene thereof.
(28) The *E*. *coli* mutant strain according to any one of (23) to (27) comprising chromosomal DNA that does not comprise DNA having a nucleotide sequence comprising nucleotides 3165628 to 3172940 of the nucleotide sequence as shown in SEQ ID NO: 1 or DNA equivalent thereto.
(29) The *E*. *coli* mutant strain according to any one of (23) to (28) comprising chromosomal DNA that does not comprise ybeL gene, ybeQ gene, ybeR gene, ybeS gene, ybeT gene, ybeU gene, ybeV gene, ybeW gene, ybeK gene, gltL gene, gltK gene, gltJ gene, ybeJ gene and JW0651 gene, or homologous gene thereof.
(30) The *E*. *coli* mutant strain according to any one of (23) to (29) comprising chromosomal DNA that does not comprise DNA having a nucleotide sequence comprising nucleotides 675104 to 689374 of the nucleotide sequence as shown in SEQ ID NO: 1 or DNA equivalent thereto.
(31) The *E*. *coli* mutant strain according to any one of (23) to (30) comprising chromosomal DNA that does not comprise yfcC gene, JW2296 gene, yfcE gene, yfcF gene, yfcG gene, folX gene, JW2301 gene, yfcI gene, hisP gene, hisM gene, hisQ gene, hisJ gene, argT gene, yfdB gene, JW2346 gene, yfdH gene, JW2348 gene, yfdK gene, yfdL gene, yfdM gene, xylU gene, yfdN gene, yfdO gene, JW2356 gene, JW2357 gene, JW2358 gene, JW2359 gene, JW2360 gene, JW2378 gene, yqhT gene, ptwC gene andptwB gene, or homologous gene thereof.
(32) The *E. coli* mutant strain according to any one of (23) to (31) comprising chromosomal DNA that does not comprise DNA having the nucleotide sequences as shown in [1] to [3] below of the nucleotide sequence as shown in SEQ ID NO: 1 or DNA equivalent thereto.
[1] a nucleotide sequence of nucleotides 2423365 to 2434098
[2] a nucleotide sequence of nucleotides 2472852 to 2482482
[3] a nucleotide sequence of nucleotides 2505075 to 2514557

(33) The *E*. *coli* mutant strain according to any one of (23) to (32) comprising chromosomal DNA that does not comprise pinO gene, gspA gene, gspC gene, gspD gene, gspE gene, hofF gene, hofG gene, hofH gene, gspI gene, gspJ gene, gspK gene, gspL gene, pshM gene, hofD gene, bfr gene, yheA gene and chiA gene, or homologous gene thereof.
(34) The *E*. *coli* mutant strain according to any one of (23) to (33) comprising chromosomal DNA that does not comprise DNA having a nucleotide sequence comprising nucleotides 3453021 to 3469366 of the nucleotide sequence as shown in SEQ ID NO: 1 or DNA equivalent thereto.
(35) The *E*. *coli* mutant strain according to any one of (23) to (34) comprising chromosomal DNA that does not comprise elaB gene, elaA gene, elaC gene, elaD gene, yfbK gene, yfbL gene, yfbM gene, yfbN gene, JW2269 gene and JW2270 gene, or homologous gene thereof.
(36) The *E*. *coli* mutant strain according to any one of (23) to (35) comprising chromosomal DNA that does not comprise DNA having a nucleotide sequence comprising nucleotides 2386262 to 2395471 of the nucleotide sequence as shown in SEQ ID NO: 1 or DNA equivalent thereto.
(37) The *E*. *coli* mutant strain according to any one of (23) to (36) comprising chromosomal DNA that does not comprise yhdJ gene, yhdU gene, envR gene, acrE gene, envD gene, yhdV gene, yhdW gene, yhdX gene and yhdY gene, or homologous gene thereof.
(38) The *E*. *coli* mutant strain according to any one of (23) to (37) comprising chromosomal DNA that does not comprise DNA having a nucleotide sequence comprising nucleotides 3411160 to 3420878 of the nucleotide sequence as shown in SEQ ID NO: 1 or DNA equivalent thereto
(39) The *E. coli* mutant strain according to any one of (23) to (38) comprising chromosomal DNA that does not comprise chpA gene or homologous gene thereof.
(40) The *E. coli* mutant strain according to any one of (23) to (39) comprising chromosomal DNA that does not comprise DNA having a nucleotide sequence comprising nucleotides 2910274 to 2910573 of the nucleotide sequence as shown in SEQ ID NO: 1 or DNA equivalent thereto.
(41) The *E*. *coli* mutant strain according to any one of (23) to (40) comprising chromosomal DNA that does not comprise fhua gene, fhuC gene, fhuD gene fhuB gene, or homologous gene thereof.
(42) The *E*. *coli* mutant strain according to any one of (23) to (41) comprising chromosomal DNA that does not comprise DNA having a nucleotide sequence comprising nucleotides 167065 to 172979 of the nucleotide sequence as shown in SEQ ID NO: 1 or DNA equivalent thereto.
[Table 1]

**Table 1**

| Gene name | Coding strand | Start number in SEQ ID NO:1 (stop number when encoded by complementary strand) | Stop number in SEQ ID NO:1 (start number when encoded by complementary strand) |
|---|---|---|---|
| JW0001(thrA) | | 1 | 2463 |
| JW0002(thrB) | | 2465 | 3397 |
| JW0003(thrC) | | 3398 | 4684 |
| JW0004(101#4) | | 4898 | 5194 |
| JW0005(yaaA) | Complementary strand | 5347 | 6123 |
| JW0006(yaaJ) | Complementary strand | 6193 | 7623 |
| JW0007(taIB) | | 7902 | 8855 |
| JW0008(mog) | | 8970 | 9557 |
| JW0009(yaaH) | Complementary strand | 9592 | 10158 |
| JW0010(102#2.1) | Complementary strand | 10307 | 11020 |
| JW0011(htgA) | | 10389 | 10979 |
| JW0012(yaaI) | Complementary strand | 11046 | 11450 |
| JW0013(dnaK) | | 11827 | 13743 |
| JW0014(dnaJ) | | 13832 | 14962 |
| JW0015(yi81) | | 15109 | 16221 |
| JW0016(yi82) | Complementary strand | 15533 | 15841 |
| JW0017(hokC) | Complementary strand | 16415 | 16624 |
| JW0018(nhaA) | | 17153 | 18319 |
| JW0019(nhaR) | | 18379 | 19284 |
| JW0020(insB) | Complementary strand | 19475 | 19978 |
| JW0021(insA) | Complementary strand | 19897 | 20172 |
| JW0022(rpsT) | Complementary strand | 20479 | 20742 |
| JW0023(ribF) | | 21071 | 22012 |
| JW0024(ileS) | | 22055 | 24871 |
| JW0025(lspA) | | 24871 | 25365 |
| JW0026(fkpB) | | 25490 | 25939 |
| JW0027(lytB) | | 25941 | 26891 |
| JW0028(yaaF) | | 26957 | 27871 |
| JW0029(dapB) | | 28038 | 28859 |
| JW0030(carA) | | 29315 | 30463 |
| JW0031(carB) | | 30481 | 33702 |
| JW0032(yaaV) | Complementary strand | 33750 | 33929 |
| JW0033(caiF) | | 33964 | 34359 |
| JW0034(caiE) | Complementary strand | 34445 | 35056 |
| JW0035(caiD) | Complementary strand | 35041 | 35934 |
| JW0036(caiC) | Complementary strand | 35935 | 37503 |
| JW0037(caiB) | Complementary strand | 37562 | 38779 |
| JW0038(caiA) | Complementary strand | 38908 | 40050 |
| JW0039(caiT) | Complementary strand | 40081 | 41595 |
| JW0040(fixA) | | 42067 | 42837 |
| JW0041 (fixB) | | 42852 | 43793 |
| JW0042(fixC) | | 43844 | 45130 |
| JW0043(fixX) | | 45127 | 45414 |
| JW0044(yaaU) | | 45471 | 46802 |
| JW0045(yabF) | | 46910 | 47440 |
| JW0046(kefC) | | 47433 | 49295 |
| JW0047(foIA) | | 49487 | 49966 |
| JW0048(apaH) | Complementary strand | 50044 | 50886 |
| JW0049(apaG) | Complementary strand | 50893 | 51270 |
| JW0050(ksgA) | Complementary strand | 51273 | 52094 |
| JW0051(pdxA) | Complementary strand | 52091 | 53080 |
| JW0052(surA) | Complementary strand | 53080 | 54366 |
| JW0053(imp) | Complementary strand | 54419 | 56773 |
| JW0054(djlA) | | 57028 | 57843 |
| JW0055(yabP) | | 58138 | 58788 |
| JW0056(yabQ) | | 58785 | 58943 |
| JW0057(rluA) | Complementary strand | 59351 | 60010 |
| JW0058(hepA) | Complementary strand | 60022 | 62928 |
| JW0059(polB) | Complementary strand | 63093 | 65444 |
| JW0060(araD) | Complementary strand | 65519 | 66214 |
| JW0061 (araA) | Complementary strand | 66499 | 68001 |
| JW0062(araB) | Complementary strand | 68012 | 69712 |
| JW0063(araC) | | 70051 | 70929 |
| JW0064(yabI) | | 71066 | 71779 |
| JW0065(yabJ) | Complementary strand | 71893 | 72591 |
| JW0066(yabK) | Complementary strand | 72575 | 74185 |
| JW0067(tbpA) | Complementary strand | 74161 | 75144 |
| JW0068(yabN) | Complementary strand | 75308 | 76963 |
| JW0069(yabM) | | 77285 | 78463 |
| JW0070(leuD) | Complementary strand | 78512 | 79117 |
| JW0071 (leuC) | Complementary strand | 79128 | 80528 |
| JW0072(leuB) | Complementary strand | 80531 | 81622 |
| JW0073(leuA) | Complementary strand | 81622 | 83193 |
| JW0074(leuL) | Complementary strand | 83286 | 83372 |
| JW0075(leuO) | | 84032 | 84976 |
| JW0076(ilvl) | | 85294 | 87018 |
| JW0077(ilvH) | | 87021 | 87512 |
| JW0078(fruR) | | 87692 | 88696 |
| JW0079(yabB) | | 89298 | 89756 |
| JW0080(yabC) | | 89758 | 90699 |
| JW0081 (ftsL) | | 90696 | 91061 |
| JW0082(ftsI) | | 91077 | 92843 |
| JW0083(murE) | | 92830 | 94317 |
| JW0084(murF) | | 94314 | 95672 |
| JW0085(mraY) | | 95666 | 96748 |
| JW0086(murD) | | 96751 | 98067 |
| JW0087(ftsW) | | 98067 | 99311 |
| JW0088(murG) | | 99308 | 100375 |
| JW0089(murC) | | 100429 | 101904 |
| JW0090(ddlB) | | 101897 | 102817 |
| JW0091(ftsQ) | | 102819 | 103649 |
| JW0092(ftsA) | | 103646 | 104908 |
| JW0093(ftsZ) | | 104969 | 106120 |
| JW0094(lpxC) | | 106221 | 107138 |
| JW0095(yacA) | | 107438 | 107881 |
| JW0096(secA) | | 107943 | 110648 |
| JW0097(mutT) | | 110708 | 111097 |
| JW0098(yacG) | Complementary strand | 111313 | 111465 |
| JW0099(yacF) | Complementary strand | 111520 | 112263 |
| JW0100(yacE) | Complementary strand | 112263 | 112883 |
| JW0101(guaC) | | 113108 | 114151 |
| JW0102(hofC) | Complementary strand | 114186 | 115388 |
| JW0103(hofB) | Complementary strand | 115378 | 116763 |
| JW0104(ppdD) | Complementary strand | 116773 | 117213 |
| JW0105(nadC) | Complementary strand | 117416 | 118309 |
| JW0106(ampD) | | 118397 | 118948 |
| JW0107(ampE) | | 118945 | 119799 |
| JW0108(aroP) | Complementary strand | 119842 | 121215 |
| JW0109(pdhR) | | 121756 | 122520 |
| JW0110(aceE) | | 122681 | 125344 |
| JW0111(aceF) | | 125359 | 127251 |
| JW0112(IpdA) | | 127576 | 129000 |
| JW0113(yacH) | Complementary strand | 129071 | 130924 |
| JW0114(acnB) | | 131279 | 133876 |
| JW0115(yacL) | | 134052 | 134414 |
| JW0116(speD) | Complementary strand | 134452 | 135246 |
| JW0117(speE) | Complementary strand | 135262 | 136128 |
| JW0118(yacC) | Complementary strand | 136234 | 136581 |
| JW0119(yacK) | | 136747 | 138297 |
| JW0120(gcd) | Complementary strand | 138499 | 140889 |
| JW0121(hpt) | | 141083 | 141631 |
| JW0122(yadF) | Complementary strand | 141672 | 142334 |
| JW0123(yadG) | | 142443 | 143369 |
| JW0124(yadH) | | 143366 | 144136 |
| JW0125(yadI) | | 144241 | 144681 |
| JW0126(yadJ) | | 144745 | 145974 |
| JW0127(panD) | Complementary strand | 145978 | 146358 |
| JW0128(yadD) | | 146683 | 147534 |
| JW0129(panC) | Complementary strand | 147608 | 148459 |
| JW0130(panB) | Complementary strand | 148471 | 149265 |
| JW0131(yadC) | Complementary strand | 149379 | 150617 |
| JW0132(yadK) | Complementary strand | 150667 | 151263 |
| JW0133(yadL) | Complementary strand | 151290 | 151895 |
| JW0134(yadM) | Complementary strand | 151907 | 152476 |
| JW0135(htrE) | Complementary strand | 152493 | 155090 |
| JW0136(ecpD) | Complementary strand | 155125 | 155865 |
| JW0137(yadN) | Complementary strand | 155963 | 156547 |
| JW0138(folK) | Complementary strand | 156917 | 157396 |
| JW0139(pcnB) | Complementary strand | 157393 | 158811 |
| JW0140(yadB) | Complementary strand | 158850 | 159776 |
| JW0141(dksA) | Complementary strand | 159813 | 160268 |
| JW0142(sfsA) | Complementary strand | 160446 | 161150 |
| JW0143(ligT) | Complementary strand | 161165 | 161704 |
| JW0144(hrpB) | | 161769 | 164198 |
| JW0145(ponB) | | 164394 | 166928 |
| JW0146(fhuA) | | 167148 | 169391 |
| JW0147(fhuC) | | 169442 | 170239 |
| JW0148(fhuD) | | 170239 | 171129 |
| JW0149(fhuB) | | 171126 | 173108 |
| JW0150(hemL) | Complementary strand | 173266 | 174546 |
| JW0151(yadQ) | | 174882 | 176192 |
| JW0152(yadR) | | 176274 | 176618 |
| JW0153(yadS) | Complementary strand | 176665 | 177288 |
| JW0154(yadT) | Complementary strand | 177326 | 178126 |
| JW0155(pfs) | Complementary strand | 178119 | 178817 |
| JW0156(dgt) | | 178901 | 180418 |
| JW0157(htrA) | | 180548 | 181972 |
| JW0158(yaeG) | | 182109 | 183284 |
| JW0159(yaeN) | Complementary strand | 183373 | 183759 |
| JW0160(yaeI) | Complementary strand | 183921 | 184454 |
| JW0161(dapD) | Complementary strand | 184787 | 185611 |
| JW0162(glnD) | Complementary strand | 185642 | 188314 |
| JW0163(map) | Complementary strand | 188376 | 189170 |
| JW0164(rpsB) | | 189538 | 190263 |
| JW0164.5(120#5) | Complementary strand | 190215 | 190466 |
| JW0165(tsf) | | 190521 | 191372 |
| JW0166(smb) | | 191519 | 192244 |
| JW0167(rrf) | | 192536 | 193093 |
| JW0168(dxr) | | 193185 | 194381 |
| JW0169(rth) | | 194567 | 195328 |
| JW0170(cdsA) | | 195449 | 196198 |
| JW0171(yaeL) | | 196210 | 197562 |
| JW0172(yzzN) | | 197592 | 200024 |
| JW0173(hlpA) | | 200146 | 200631 |
| JW0174(IpxD) | | 200635 | 201660 |
| JW0175(fabZ) | | 201765 | 202220 |
| JW0176(lpxA) | | 202224 | 203012 |
| JW0177(lpxB) | | 203012 | 204160 |
| JW0178(rnhB) | | 204157 | 204753 |
| JW0179(dnaE) | | 204790 | 208272 |
| JW0180(accA) | | 208285 | 209244 |
| JW0181(ldcC) | | 209343 | 211484 |
| JW0182(yaeR) | | 211541 | 211930 |
| JW0183(mesJ) | | 211995 | 213293 |
| JW0184(rof) | Complementary strand | 213342 | 213596 |
| JW0185(yaeP) | Complementary strand | 213589 | 213789 |
| JW0186(yaeQ) | | 213955 | 214500 |
| JW0187(yaeJ) | | 214497 | 214919 |
| JW0188(cutF) | | 214933 | 215643 |
| JW0189(yaeF) | Complementary strand | 215843 | 216721 |
| JW0190(proS) | Complementary strand | 216721 | 218439 |
| JW0191(yaeB) | Complementary strand | 218551 | 219258 |
| JW0192(rcsF) | Complementary strand | 219255 | 219659 |
| JW0193(yaeC) | Complementary strand | 219777 | 220592 |
| JW0194(yaeE) | Complementary strand | 220632 | 221285 |
| JW0195(abc) | Complementary strand | 221278 | 222309 |
| JW0196(yaeD) | | 222497 | 223072 |
| JW0197(yafB) | | 228831 | 229634 |
| JW0198(yafC) | Complementary strand | 229631 | 230545 |
| JW0199(yafD) | | 230807 | 231586 |
| JW0200(yafE) | | 231590 | 232213 |
| JW0201 (dniR) | Complementary strand | 232261 | 232929 |
| JW0202(gloB) | Complementary strand | 233691 | 234446 |
| JW0203(yafS) | | 234480 | 235202 |
| JW0204(rnhA) | Complementary strand | 235199 | 235666 |
| JW0205(dnaQ) | | 235731 | 236462 |
| JW0206(yafT) | | 236999 | 237784 |
| JW0207(yafU) | Complementary strand | 238410 | 238748 |
| JW0208(ybfL) | | 238854 | 239042 |
| JW0209(yafV) | Complementary strand | 239083 | 239862 |
| JW0210(up18) | | 240007 | 240480 |
| JW0211(yafH) | Complementary strand | 240523 | 243045 |
| JW0212(gmhA) | | 243207 | 243785 |
| JW0213(yafJ) | | 243991 | 244758 |
| JW0214(yafK) | Complementary strand | 244729 | 245469 |
| JW0215(yafQ) | Complementary strand | 245625 | 245903 |
| JW0216(dinJ) | Complementary strand | 245906 | 246166 |
| JW0217(yafL) | | 246376 | 247125 |
| JW0218(yafM) | | 247301 | 247798 |
| JW0219(fhiA) | Complementary strand | 248022 | 249761 |
| JW0220(lafU) | | 249856 | 250491 |
| JW0221(dinP) | | 250562 | 251617 |
| JW0222(yafN) | | 251669 | 251962 |
| JW0223(yafO) | | 251965 | 252363 |
| JW0224(yafP) | | 252373 | 252825 |
| JW0225(127#12) | | 253131 | 253397 |
| JW0226(prfH) | | 253366 | 253866 |
| JW0227(pepD) | Complementary strand | 253923 | 255380 |
| JW0228(gpt) | | 255641 | 256099 |
| JW0229(yafA) | | 256191 | 257435 |
| JW0230(crl) | | 257493 | 257894 |
| JW0231 (phoE) | Complementary strand | 257933 | 258988 |
| JW0232(proB) | | 259276 | 260379 |
| JW0233(proA) | | 260391 | 261644 |
| JW0234(ykfI) | Complementary strand | 262216 | 262557 |
| JW0235(yafW) | Complementary strand | 262578 | 262895 |
| JW0235.5(yeeT) | Complementary strand | 262914 | 263135 |
| JW0236(ykfG) | Complementary strand | 263144 | 263620 |
| JW0237(yafX) | Complementary strand | 263636 | 264268 |
| JW0238(ykfF) | Complementary strand | 264192 | 264593 |
| JW0239(ykfB) | Complementary strand | 264508 | 264975 |
| JW0240(yafY) | Complementary strand | 264998 | 265855 |
| JW0241(ypjK) | Complementary strand | 265441 | 265617 |
| JW0242(yafZ) | Complementary strand | 266072 | 266893 |
| JW0243(ykfA) | Complementary strand | 266985 | 267848. |
| JW0244(perR) | Complementary strand | 268177 | 269070 |
| JW0245(129#3.1) | | 269166 | 269534 |
| JW0246(129#3.2) | | 269491 | 270642 |
| JW0247(129#5) | | 270718 | 271143 |
| JW0248(ykfC) | | 271750 | 272880 |
| JW0249(130#0.1) | | 272841 | 272951 |
| JW0250(is5B) | Complementary strand | 272989 | 274005 |
| JW0251 (yi51) | | 273325 | 273693 |
| JW0252(mmuP) | | 274189 | 275616 |
| JW0253(mmuM) | | 275603 | 276535 |
| JW0254(afuC) | Complementary strand | 276644 | 277690 |
| JW0255(130#4) | Complementary strand | 277702 | 278064 |
| JW0256(insB) | Complementary strand | 278066 | 278569 |
| JW0257(insA) | Complementary strand | 278488 | 278763 |
| JW0258(130#5) | Complementary strand | 278912 | 279250 |
| JW0259(yagB) | Complementary strand | 279273 | 279623 |
| JW0260(yagA) | Complementary strand | 279717 | 280871 |
| JW0261 (yagE) | | 281145 | 282074 |
| JW0262(yagF) | | 282089 | 284056 |
| JW0263(yagG) | | 284283 | 285665 |
| JW0264(yagH) | | 285677 | 287287 |
| JW0265(yagI) | Complementary strand | 287292 | 288050 |
| JW0266(argF) | Complementary strand | 288189 | 289193 |
| JW0267(yfjI) | | 289293 | 289526 |
| JW0268(insB) | Complementary strand | 289537 | 290040 |
| JW0269(insA) | Complementary strand | 289959 | 290234 |
| JW0270(yagJ) | | 290388 | 291119 |
| JW0271(yagK) | Complementary strand | 291210 | 291836 |
| JW0272(yagL) | Complementary strand | 292108 | 292806 |
| JW0273(yagM) | Complementary strand | 292833 | 293687 |
| JW0274(yagN) | Complementary strand | 294027 | 294467 |
| JW0275(intF) | Complementary strand | 294584 | 295984 |
| JW0276(yagP) | Complementary strand | 296269 | 296679 |
| JW0277(yagQ) | Complementary strand | 296658 | 297614 |
| JW0278(yagR) | Complementary strand | 297624 | 299822 |
| JW0279(yagS) | Complementary strand | 299819 | 300775 |
| JW0280(yagT) | Complementary strand | 300772 | 301461 |
| JW0281(yagU) | | 301879 | 302493 |
| JW0282(ykgJ) | Complementary strand | 302741 | 303070 |
| JW0283(yagV) | Complementary strand | 303383 | 304138 |
| JW0284(yagW) | Complementary strand | 304062 | 305705 |
| JW0285(yagX) | Complementary strand | 305695 | 308220 |
| JW0286(yagY) | Complementary strand | 308246 | 308914 |
| JW0287(yagZ) | Complementary strand | 308972 | 309559 |
| JW0288(ykgK) | Complementary strand | 309634 | 309885 |
| JW0289(ykgL) | | 310994 | 311227 |
| JW0290(ykgM) | Complementary strand | 311402 | 311668 |
| JW0291 (eaeH) | | 313245 | 314132 |
| JW0292(134#4.1) | | 314179 | 314478 |
| JW0293(134#4.2) | | 314475 | 315341 |
| JW0294(ykgA) | Complementary strand | 315338 | 316057 |
| JW0295(ykgB) | Complementary strand | 316614 | 317216 |
| JW0296(ykgI) | Complementary strand | 317219 | 317470 |
| JW0297(merA) | Complementary strand | 317564 | 318916 |
| JW0298(ykgD) | | 319115 | 319969 |
| JW0299(ykgE) | | 320562 | 321215 |
| JW0300(ykgF) | | 321226 | 322653 |
| JW0301(ykgG) | | 322652 | 323341 |
| JW0302(ykgH) | Complementary strand | 323584 | 324252 |
| JW0303(betA) | Complementary strand | 324465 | 326135 |
| JW0304(betB) | Complementary strand | 326149 | 327621 |
| JW0305(betI) | Complementary strand | 327635 | 328222 |
| JW0306(betT) | | 328351 | 330384 |
| JW0307(yahA) | | 331259 | 332347 |
| JW0308(yahB) | Complementary strand | 332389 | 333321 |
| JW0309(yahC) | Complementary strand | 333413 | 333910 |
| JW0310(yahD) | | 334168 | 334773 |
| JW0311(yahE) | | 334813 | 335676 |
| JW0312(yahF) | | 335666 | 337213 |
| JW0313(yahG) | | 337213 | 338631 |
| JW0314(yahH) | | 338552 | 338977 |
| JW0315(arcM) | | 339053 | 340003 |
| JW0316(yahJ) | | 340013 | 341395 |
| JW0317(yahK) | | 341772 | 342821 |
| JW0318(yahL) | | 343064 | 343879 |
| JW0319(yahM) | | 344262 | 344537 |
| JW0320(yahN) | Complementary strand | 344554 | 345225 |
| JW0321(yahO) | | 345372 | 345647 |
| JW0322(prpR) | Complementary strand | 345745 | 347331 |
| JW0323(bcpA) | | 347570 | 348460 |
| JW0324(prpC) | | 348900 | 350069 |
| JW0325(prpD) | | 350103 | 351554 |
| JW0326(prpE) | | 351594 | 353480 |
| JW0327(codB) | | 353810 | 355069 |
| JW0328(codA) | | 355059 | 356342 |
| JW0329(cynR) | Complementary strand | 356679 | 357578 |
| JW0330(cynT) | | 357687 | 358346 |
| JW0331(cynS) | | 358377 | 358847 |
| JW0332(cynX) | | 358880 | 360034 |
| JW0333(lacA) | Complementary strand | 360137 | 360748 |
| JW0334(lacY) | Complementary strand | 360814 | 362067 |
| JW0335(lacZ) | Complementary strand | 362119 | 365193 |
| JW0336(lacI) | Complementary strand | 365316 | 366398 |
| JW0337(mhpR) | Complementary strand | 366475 | 367422 |
| JW0338(mhpA) | | 367499 | 369163 |
| JW0339(mhpB) | | 369165 | 370109 |
| JW0340(mhpC) | | 370112 | 370993 |
| JW0341 (mhpD) | | 371003 | 371812 |
| JW0342(mhpF) | | 371809 | 372759 |
| JW0343(mhpE) | | 372756 | 373769 |
| JW0344(mhpT) | | 374302 | 375558 |
| JW0345(yaiL) | | 375660 | 376199 |
| JW0346(yaiM) | Complementary strand | 376423 | 377256 |
| JW0347(adhC) | Complementary strand | 377350 | 378459 |
| JW0348(yaiN) | Complementary strand | 378494 | 378769 |
| JW0349(yaiO) | Complementary strand | 378957 | 379730 |
| JW0350(140#9) | Complementary strand | 379732 | 379947 |
| JW0351 (yi2A) | | 380194 | 380604 |
| JW0352(yi22) | | 380562 | 381467 |
| JW0353(yi24) | Complementary strand | 380607 | 381038 |
| JW0354(yi23) | | 380635 | 380928 |
| JW0355(yaiP) | Complementary strand | 381627 | 382823 |
| JW0356(yaiS) | Complementary strand | 382947 | 383504 |
| JW0357(tauA) | | 384120 | 385082 |
| JW0358(tauB) | | 385095 | 385862 |
| JW0359(tauC) | | 385859 | 386686 |
| JW0360(ssiD) | | 386683 | 387534 |
| JW0361(hemB) | Complementary strand | 387641 | 388615 |
| JW0362(yaiT) | | 389139 | 390599 |
| JW0363(141#3.1) | Complementary strand | 390627 | 391493 |
| JW0364(141#3.2) | Complementary strand | 391490 | 391789 |
| JW0365(yaiU) | | 391909 | 393306 |
| JW0366(yaiV) | | 393349 | 394017 |
| JW0367(ampH) | Complementary strand | 394018 | 395148 |
| JW0368(sbmA) | | 395527 | 396747 |
| JW0369(yaiW) | | 396760 | 397854 |
| JW0370(yaiY) | Complementary strand | 397913 | 398221 |
| JW0371(yaiZ) | | 398412 | 398693 |
| JW0372(ddlA) | Complementary strand | 398717 | 399811 |
| JW0373(yaiB) | | 400274 | 400534 |
| JW0374(phoA) | | 400635 | 402050 |
| JW0375(psiF) | | 402151 | 402489 |
| JW0376(yaiC) | | 402591 | 403706 |
| JW0377(proC) | Complementary strand | 403723 | 404532 |
| JW0378(yaiI) | | 404652 | 405110 |
| JW0379(aroL) | | 405293 | 405817 |
| JW0380(yaiA) | | 405867 | 406058 |
| JW0381(aroM) | | 406316 | 406993 |
| JW0382(yaiE) | | 407065 | 407349 |
| JW0383(ykiA) | | 407557 | 407838 |
| JW0384(yaiD) | Complementary strand | 407996 | 408907 |
| JW0385(yajF) | | 409032 | 409940 |
| JW0386(araJ) | Complementary strand | 410185 | 411369 |
| JW0387(sbcC) | Complementary strand | 411495 | 414641 |
| JW0388(sbcD) | Complementary strand | 414638 | 415840 |
| JW0389(phoB) | | 416030 | 416719 |
| JW0390(phoR) | | 416777 | 418072 |
| JW0391(brnQ) | | 418479 | 419798 |
| JW0392(proY) | | 419871 | 421247 |
| JW0393(malZ) | | 421403 | 423220 |
| JW0394(yajH) | Complementary strand | 423225 | 423806 |
| JW0395(queA) | | 423899 | 424969 |
| JW0396(tgt) | | 425025 | 426152 |
| JW0397(yajC) | | 426175 | 426507 |
| JW0398(secD) | | 426535 | 428382 |
| JW0399(secF) | | 428393 | 429364 |
| JW0400(yajD) | | 429493 | 429840 |
| JW0401(tsx) | Complementary strand | 430017 | 430901 |
| JW0402(yajl) | Complementary strand | 431200 | 431799 |
| JW0403(ybaD) | | 431890 | 432339 |
| JW0404(ribD) | | 432343 | 433446 |
| JW0405(ribH) | | 433535 | 434005 |
| JW0406(nusB) | | 434025 | 434444 |
| JW0407(thiL) | | 434522 | 435499 |
| JW0408(pgpA) | | 435477 | 435995 |
| JW0409(yajO) | Complementary strand | 436049 | 437023 |
| JW0410(dxs) | Complementary strand | 437203 | 439065 |
| JW0411(ispA) | Complementary strand | 439090 | 439989 |
| JW0412(xseB) | Complementary strand | 439989 | 440231 |
| JW0413(thiI) | | 440437 | 441885 |
| JW0414(thiJ) | Complementary strand | 441939 | 442535 |
| JW0415(apbA) | Complementary strand | 442492 | 443403 |
| JW0416(yajQ) | | 443403 | 444062 |
| JW0417(yajR) | Complementary strand | 444190 | 445560 |
| JW0418(cyoE) | Complementary strand | 445703 | 446593 |
| JW0419(cyoD) | Complementary strand | 446605 | 446934 |
| JW0420(cyoC) | Complementary strand | 446934 | 447548 |
| JW0421(cyoB) | Complementary strand | 447538 | 449529 |
| JW0422(cyoA) | Complementary strand | 449551 | 450498 |
| JW0423(ampG) | Complementary strand | 450958 | 452433 |
| JW0424(yajG) | Complementary strand | 452477 | 453055 |
| JW0425(bolA) | | 453327 | 453677 |
| JW0426(tig) | | 454021 | 455319 |
| JW0427(clpP) | | 455565 | 456188 |
| JW0428(clpX) | | 456314 | 457588 |
| JW0429(lon) | | 457776 | 460130 |
| JW0430(hupB) | | 460339 | 460611 |
| JW0431 (ybaU) | | 460803 | 462674 |
| JW0432(ybaV) | | 462825 | 463196 |
| JW0433(ybaW) | | 463290 | 463688 |
| JW0434(ybaX) | Complementary strand | 463740 | 464435 |
| JW0435(ybaE) | Complementary strand | 464500 | 466200 |
| JW0436(cof) | | 466300 | 467118 |
| JW0437(ybaO) | | 467271 | 467729 |
| JW0438(mdIA) | | 467759 | 469531 |
| JW0439(mdIB) | | 469410 | 471305 |
| JW0440(gInK) | | 471486 | 471824 |
| JW0441(amtB) | | 471854 | 473140 |
| JW0442(tesB) | Complementary strand | 473189 | 474049 |
| JW0443(ybaY) | | 474267 | 474839 |
| JW0444(ybaZ) | Complementary strand | 474870 | 475259 |
| JW0445(ybaA) | | 475560 | 475913 |
| JW0446(ylaB) | Complementary strand | 475955 | 477511 |
| JW0447(ylaC) | Complementary strand | 477669 | 478178 |
| JW0448(maa) | Complementary strand | 478255 | 478806 |
| JW0449(hha) | Complementary strand | 478978 | 479196 |
| JW0450(ybaJ) | Complementary strand | 479222 | 479596 |
| JW0451(acrB) | Complementary strand | 480142 | 483291 |
| JW0452(acrA) | Complementary strand | 483314 | 484507 |
| JW0453(acrR) | | 484649 | 485296 |
| JW0454(aefA) | | 485424 | 488786 |
| JW0455(ybaM) | Complementary strand | 488998 | 489159 |
| JW0456(priC) | Complementary strand | 489173 | 489700 |
| JW0457(ybaN) | | 489770 | 490147 |
| JW0458(apt) | | 490300 | 490851 |
| JW0459(dnaX) | | 490980 | 492911 |
| JW0460(ybaB) | | 492964 | 493293 |
| JW0461 (recR) | | 493293 | 493898 |
| JW0462(htpG) | | 494008 | 495882 |
| JW0463(adk) | | 496063 | 496707 |
| JW0464(hemH) | | 496943 | 497905 |
| JW0465(aes) | Complementary strand | 497902 | 498861 |
| JW0466(gsk) | | 499013 | 500317 |
| JW0467(ybaL) | Complementary strand | 500450 | 502126 |
| JW0468(fsr) | Complementary strand | 502364 | 503584 |
| JW0469(ushA) | | 503802 | 505454 |
| JW0470(ybaK) | Complementary strand | 505491 | 505970 |
| JW0471(ybaP) | Complementary strand | 506174 | 506968 |
| JW0472(ybaQ) | | 507106 | 507447 |
| JW0473(ybaR) | Complementary strand | 507763 | 510267 |
| JW0474(ybaS) | | 510529 | 511461 |
| JW0475(ybaT) | | 511464 | 512756 |
| JW0476(cueR) | | 512881 | 513288 |
| JW0477(ybbJ) | Complementary strand | 513289 | 513747 |
| JW0478(ybbK) | Complementary strand | 513744 | 514661 |
| JW0479(ybbL) | | 514807 | 515484 |
| JW0480(ybbM) | | 515444 | 516250 |
| JW0481 (ybbN) | Complementary strand | 516313 | 517203 |
| JW0482(ybbO) | Complementary strand | 517228 | 518037 |
| JW0483(tesA) | Complementary strand | 518027 | 518653 |
| JW0484(ybbA) | | 518621 | 519307 |
| JW0485(ybbP) | | 519304 | 521718 |
| JW0486(rhsD) | | 522149 | 526429 |
| JW0487(ybbC) | | 526469 | 526837 |
| JW0488(rhsE) | | 526837 | 527547 |
| JW0489(ybbD) | | 527528 | 527788 |
| JW0490(156#1) | Complementary strand | 528533 | 528904 |
| JW0491 (ybbB) | Complementary strand | 529020 | 530114 |
| JW0492(ybbS) | Complementary strand | 530183 | 531109 |
| JW0493(ybbT) | | 531339 | 531821 |
| JW0494(ybbU) | | 531899 | 532714 |
| JW0495(gcl) | | 532804 | 534585 |
| JW0496(gip) | | 534598 | 535374 |
| JW0497(ybbQ) | | 535474 | 536352 |
| JW0498(ybbV) | | 536384 | 536662 |
| JW0499(aIIP) | | 536521 | 537975 |
| JW0500(aIIA) | | 538035 | 539396 |
| JW0501(ybbY) | | 539453 | 540754 |
| JW0502(ybbZ) | | 540776 | 541921 |
| JW0503(ylbA) | Complementary strand | 542149 | 542934 |
| JW0504(aIIC) | Complementary strand | 542945 | 544180 |
| JW0505(ylbC) | Complementary strand | 544202 | 545251 |
| JW0506(fdrA) | | 545568 | 547235 |
| JW0507(ylbE) | | 547245 | 547505 |
| JW0508(ylbE) | | 547502 | 548503 |
| JW0509(ylbF) | | 548514 | 549329 |
| JW0510(arcC) | | 549326 | 550219 |
| JW0511(purK) | Complementary strand | 550414 | 551481 |
| JW0512(purE) | Complementary strand | 551478 | 551987 |
| JW0513(ybbF) | Complementary strand | 552105 | 552827 |
| JW0514(ppiB) | Complementary strand | 552830 | 553324 |
| JW0515(cysS) | | 553498 | 554883 |
| JW0516(ybcI) | Complementary strand | 554919 | 555440 |
| JW0517(ybcJ) | Complementary strand | 555548 | 555814 |
| JW0518(folD) | Complementary strand | 555762 | 556628 |
| JW0519(sfmA) | | 557099 | 557641 |
| JW0520(sfmC) | | 557861 | 558553 |
| JW0521 (sfmD) | | 558584 | 561187 |
| JW0522(sfmH) | | 561076 | 562206 |
| JW0523(sfmF) | | 562091 | 562732 |
| JW0524(fimZ) | Complementary strand | 562735 | 563430 |
| JW0525(intD) | Complementary strand | 563702 | 564865 |
| JW0526(EXO_LAMBD) | Complementary strand | 564985 | 565248 |
| JW0527(VRPP_LAMBD) | | 565338 | 565574 |
| JW0528(159#4) | | 565729 | 566028 |
| JW0529(159#5) | | 566025 | 566891 |
| JW0530(VREN_LAMBD) | | 566949 | 567134 |
| JW0531(emrE) | | 567202 | 567534 |
| JW0532(ybcK) | | 567789 | 569315 |
| JW0533(ybcL) | | 569780 | 570331 |
| JW0534(ybcM) | | 570341 | 571138 |
| JW0535(ybcN) | | 571353 | 571808 |
| JW0536(ninE) | | 571808 | 571978 |
| JW0537(ybcO) | | 571971 | 572261 |
| JW0538(rus) | | 572258 | 572620 |
| JW0539(ybcQ) | | 572843 | 573226 |
| JW0539.5(160#10) | | 573368 | 573586 |
| JW0540(is5B) | Complementary strand | 573624 | 574640 |
| JW0541(yi51) | | 573960 | 574328 |
| JW0542(nmpC) | Complementary strand | 574645 | 575712 |
| JW0543(vlyS) | | 576285 | 576500 |
| JW0544(ybcS) | | 576500 | 576997 |
| JW0545(ybcT) | | 576982 | 577455 |
| JW0546(vboR) | Complementary strand | 577487 | 577780 |
| JW0547(ybcV) | Complementary strand | 578071 | 578604 |
| JW0548(ybcW) | | 578767 | 578973 |
| JW0549(nohB) | | 579721 | 580266 |
| JW0550(ybcX) | | 580241 | 580984 |
| JW0551(ybcY) | Complementary strand | 581039 | 581623 |
| JW0552(161#0.2) | | 581762 | 581947 |
| JW0553(appY) | | 582568 | 583317 |
| JW0554(ompT) | Complementary strand | 583567 | 584520 |
| JW0555(envY) | Complementary strand | 585034 | 585795 |
| JW0556(ybcH) | Complementary strand | 585978 | 586868 |
| JW0557(nfrA) | Complementary strand | 586869 | 589841 |
| JW0558(nfrB) | Complementary strand | 589828 | 592065 |
| JW0559(cusS) | Complementary strand | 592215 | 593669 |
| JW0560(cusR) | Complementary strand | 593647 | 594330 |
| JW0561(cusC) | | 594487 | 595860 |
| JW0562(ylcC) | | 596018 | 596350 |
| JW0563(cusB) | | 596366 | 597589 |
| JW0564(cusA) | | 597601 | 600744 |
| JW0565(pheP) | | 600846 | 602222 |
| JW0566(ybdG) | Complementary strand | 602303 | 603550 |
| JW0567(nfnB) | Complementary strand | 603658 | 604311 |
| JW0568(ybdF) | Complementary strand | 604405 | 604884 |
| JW0569(ybdJ) | Complementary strand | 604838 | 605086 |
| JW0570(ybdK) | Complementary strand | 605152 | 606270 |
| JW0571 (hokE) | | 606624 | 606875 |
| JW0572(yi81) | | 606952 | 608064 |
| JW0573(yi82) | Complementary strand | 607376 | 607684 |
| JW0574(entD) | Complementary strand | 608346 | 609116 |
| JW0575(fepA) | Complementary strand | 609141 | 611441 |
| JW0576(fes) | | 611702 | 612826 |
| JW0577(164#9) | | 612829 | 613047 |
| JW0578(entF) | | 613044 | 616925 |
| JW0579(fepE) | | 617141 | 618274 |
| JW0580(fepC) | Complementary strand | 618271 | 619086 |
| JW0581 (fepG) | Complementary strand | 619083 | 620075 |
| JW0582(fepD) | Complementary strand | 620072 | 621076 |
| JW0583(ybdA) | | 621187 | 622437 |
| JW0584(fepB) | Complementary strand | 622441 | 623397 |
| JW0584.5(165#7) | Complementary strand | 623401 | 623607 |
| JW0585(entC) | | 623772 | 624947 |
| JW0586(entE) | | 624957 | 626567 |
| JW0587(entB) | | 626581 | 627438 |
| JW0588(entA) | | 627438 | 628184 |
| JW0589(ybdB) | | 628187 | 628600 |
| JW0590(cstA) | | 628781 | 630886 |
| JW0591(ybdD) | | 631069 | 631266 |
| JW0592(ybdH) | Complementary strand | 631276 | 632364 |
| JW0593(ybdL) | | 632473 | 633633 |
| JW0594(ybdm) | Complementary strand | 633634 | 634263 |
| JW0595(ybdN) | Complementary strand | 634236 | 635456 |
| JW0596(ybdO) | Complementary strand | 635603 | 636505 |
| JW0597(dsbG) | Complementary strand | 636714 | 637460 |
| JW0598(ahpC) | | 637832 | 638395 |
| JW0599(ahpF) | | 638640 | 640205 |
| JW0600(ybdQ) | Complementary strand | 640326 | 640754 |
| JW0601(ybdR) | | 640975 | 642213 |
| JW0602(rnk) | Complementary strand | 642444 | 642854 |
| JW0603(rna) | Complementary strand | 643084 | 643890 |
| JW0604(citT) | Complementary strand | 644004 | 645467 |
| JW0605(citG) | Complementary strand | 645518 | 646396 |
| JW0606(citX) | Complementary strand | 646371 | 646922 |
| JW0607(citF) | Complementary strand | 646926 | 648500 |
| JW0608(citE) | Complementary strand | 648469 | 649377 |
| JW0609(citD) | Complementary strand | 649374 | 649670 |
| JW0610(citC) | Complementary strand | 649685 | 650743 |
| JW0611 (citA) | | 651122 | 652780 |
| JW0612(citB) | | 652749 | 653429 |
| JW0613(dcuC) | Complementary strand | 653470 | 653946 |
| JW0614(is5B) | | 653909 | 654925 |
| JW0615(yi51) | Complementary strand | 654221 | 654589 |
| JW0616(dcuC) | Complementary strand | 654963 | 656054 |
| JW0617(crcA) | | 656643 | 657203 |
| JW0618(cspE) | | 657378 | 657587 |
| JW0619(crcB) | Complementary strand | 657641 | 658024 |
| JW0620(ybeH) | | 658117 | 658344 |
| JW0621 (ybeM) | | 658341 | 658904 |
| JW0622(ybeC) | | 659033 | 659236 |
| JW0623(lipA) | Complementary strand | 659337 | 660302 |
| JW0624(ybeF) | Complementary strand | 660511 | 661464 |
| JW0625(lipB) | Complementary strand | 661723 | 662298 |
| JW0626(ybeD) | Complementary strand | 662465 | 662728 |
| JW0627(dacA) | Complementary strand | 662838 | 664049 |
| JW0628(rlpA) | Complementary strand | 664188 | 665276 |
| JW0629(mrdB) | Complementary strand | 665287 | 666399 |
| JW0630(mrdA) | Complementary strand | 666402 | 668303 |
| JW0631(ybeA) | Complementary strand | 668334 | 668801 |
| JW0632(ybeB) | Complementary strand | 668805 | 669014 |
| JW0633(phpB) | Complementary strand | 669382 | 669993 |
| JW0634(nadD) | Complementary strand | 670017 | 670658 |
| JW0635(holA) | Complementary strand | 670660 | 671691 |
| JW0636(rlpB) | Complementary strand | 671691 | 672272 |
| JW0637(leuS) | Complementary strand | 672287 | 674869 |
| JW0638(ybeL) | | 675104 | 675586 |
| JW0639(ybeQ) | Complementary strand | 675656 | 676639 |
| JW0640(ybeR) | | 676797 | 677504 |
| JW0641(ybeS) | | 677501 | 678928 |
| JW0642(ybeT) | Complementary strand | 678938 | 679492 |
| JW0643(ybeU) | | 679594 | 680301 |
| JW0644(ybeV) | | 680298 | 681749 |
| JW0645(ybeW) | Complementary strand | 681809 | 683479 |
| JW0646(ybeK) | Complementary strand | 683563 | 684498 |
| JW0647(gltL) | Complementary strand | 684616 | 685341 |
| JW0648(gltK) | Complementary strand | 685341 | 686015 |
| JW0649(gltJ) | Complementary strand | 686015 | 686755 |
| JW0650(ybeJ) | Complementary strand | 686925 | 687908 |
| JW0651(171#0.1) | | 687884 | 688045 |
| JW0652(is5B) | Complementary strand | 688083 | 689099 |
| JW0653(yi51) | | 688419 | 688787 |
| JW0654(lnt) | Complementary strand | 689429 | 690967 |
| JW0655(ybeX) | Complementary strand | 690992 | 691870 |
| JW0656(ybeY) | Complementary strand | 691960 | 692427 |
| JW0657(phoL) | Complementary strand | 692424 | 693503 |
| JW0658(yleA) | Complementary strand | 693617 | 695041 |
| JW0659(ubiF) | | 695187 | 696362 |
| JW0660(asnB) | Complementary strand | 697599 | 699263 |
| JW0661 (nagD) | Complementary strand | 699660 | 700412 |
| JW0662(nagC) | Complementary strand | 700460 | 701680 |
| JW0663(nagA) | Complementary strand | 701689 | 702837 |
| JW0664(nagB) | Complementary strand | 702897 | 703697 |
| JW0665(nagE) | | 704030 | 705976 |
| JW0666(glnS) | | 706179 | 707843 |
| JW0667(ybfM) | | 708420 | 709826 |
| JW0668(ybfN) | | 709876 | 710202 |
| JW0669(fur) | Complementary strand | 710286 | 710732 |
| JW0670(ybfJ) | | 710777 | 711031 |
| JW0671(fldA) | Complementary strand | 711021 | 711551 |
| JW0672(ybfE) | Complementary strand | 711691 | 712053 |
| JW0673(ybfF) | Complementary strand | 712124 | 712888 |
| JW0674(seqA) | | 713073 | 713618 |
| JW0675(pgm) | | 713644 | 715284 |
| JW0676(ybfP) | | 715498 | 715992 |
| JW0677(ybfG) | Complementary strand | 716033 | 716395 |
| JW0678(ybfH) | Complementary strand | 716474 | 716791 |
| JW0679(potE) | Complementary strand | 717032 | 718351 |
| JW0680(speF) | Complementary strand | 718348 | 720546 |
| JW0681(ybfK) | | 720669 | 720926 |
| JW0682(kdpE) | Complementary strand | 721142 | 721744 |
| JW0683(kdpD) | Complementary strand | 721816 | 724500 |
| JW0684(kdpC) | Complementary strand | 724493 | 725065 |
| JW0685(kdpB) | Complementary strand | 725074 | 727122 |
| JW0686(kdpA) | Complementary strand | 727145 | 728818 |
| JW0687(kdpF) | Complementary strand | 728818 | 728907 |
| JW0688(ybfA) | | 729220 | 729426 |
| JW0689(rhsC) | | 729669 | 733862 |
| JW0690(174#4.1) | Complementary strand | 732195 | 732614 |
| JW0691 (ybfB) | | 733862 | 734188 |
| JW0692(rhsA) | | 734306 | 735739 |
| JW0693(ybfC) | | 735736 | 736305 |
| JW0694(ybfD) | | 736531 | 736785 |
| JW0695(ybfL) | | 736986 | 738047 |
| JW0696(ybfD) | | 738178 | 738939 |
| JW0697(ybgA) | | 739087 | 739596 |
| JW0698(phrB) | | 739593 | 741011 |
| JW0699(ybgH) | Complementary strand | 741161 | 742642 |
| JW0700(ybgI) | | 742913 | 743656 |
| JW0701(ybgJ) | | 743679 | 744335 |
| JW0702(ybgK) | | 744329 | 745261 |
| JW0703(ybgL) | | 745251 | 745985 |
| JW0704(end8) | | 746021 | 746812 |
| JW0705(abrB) | Complementary strand | 746809 | 747900 |
| JW0706(ybgO) | Complementary strand | 748007 | 749095 |
| JW0707(ybgP) | Complementary strand | 749065 | 749793 |
| JW0708(ybgQ) | Complementary strand | 749808 | 752306 |
| JW0709(ybgD) | Complementary strand | 752315 | 752881 |
| JW0710(gltA) | Complementary strand | 753271 | 754554 |
| JW0711(sdhC) | | 755263 | 755652 |
| JW0712(sdhD) | | 755646 | 755993 |
| JW0713(sdhA) | | 755993 | 757759 |
| JW0714(sdhB) | | 757775 | 758491 |
| JW0715(sucA) | | 758792 | 761593 |
| JW0716(sucB) | | 761608 | 762825 |
| JW0717(sucC) | | 763100 | 764266 |
| JW0718(sucD) | | 764266 | 765135 |
| JW0719(farR) | Complementary strand | 765239 | 765961 |
| JW0720(hrsA) | | 766070 | 768046 |
| JW0721(ybgB) | | 768064 | 770697 |
| JW0722(cydA) | | 771544 | 773112 |
| JW0723(cydB) | | 773128 | 774267 |
| JW0724(ybgT) | | 774282 | 774395 |
| JW0725(ybgE) | | 774395 | 774688 |
| JW0726(ybgC) | | 774838 | 775242 |
| JW0727(tolQ) | | 775239 | 775931 |
| JW0728(toIR) | | 775935 | 776363 |
| JW0729(toIA) | | 776428 | 777693 |
| JW0730(toIB) | | 777823 | 779118 |
| JW0731(pal) | | 779153 | 779674 |
| JW0732(ybgF) | | 779684 | 780475 |
| JW0733(nadA) | | 782171 | 783214 |
| JW0734(pnuC) | | 783252 | 783971 |
| JW0735(czcD) | Complementary strand | 783968 | 784909 |
| JW0736(ybgS) | Complementary strand | 785023 | 785403 |
| JW0737(aroG) | | 785719 | 786771 |
| JW0738(gpmA) | Complementary strand | 786929 | 787681 |
| JW0739(galM) | Complementary strand | 787883 | 788923 |
| JW0740(galK) | Complementary strand | 788917 | 790065 |
| JW0741(galT) | Complementary strand | 790069 | 791115 |
| JW0742(galE) | Complementary strand | 791125 | 792141 |
| JW0743(modF) | Complementary strand | 792402 | 793874 |
| JW0744(modE) | Complementary strand | 793942 | 794730 |
| JW0745(180#2) | | 794853 | 795008 |
| JW0746(modA) | | 795175 | 795948 |
| JW0747(modB) | | 795948 | 796637 |
| JW0748(modC) | | 796640 | 797698 |
| JW0749(ybhA) | Complementary strand | 797699 | 798517 |
| JW0750(ybhE) | | 798672 | 799667 |
| JW0751(ybhD) | Complementary strand | 799708 | 800724 |
| JW0752(ybhH) | | 800845 | 801897 |
| JW0753(ybhI) | | 801973 | 803406 |
| JW0754(ybhJ) | | 803586 | 805850 |
| JW0755(ybhC) | Complementary strand | 806084 | 807367 |
| JW0756(ybhB) | Complementary strand | 807519 | 807995 |
| JW0757(bioA) | Complementary strand | 808054 | 809343 |
| JW0758(bioB) | | 809430 | 810470 |
| JW0759(bioF) | | 810467 | 811621 |
| JW0760(bioC) | | 811608 | 812363 |
| JW0761(bioD) | | 812356 | 813033 |
| JW0762(uvrB) | | 813612 | 815633 |
| JW0763(ybhK) | Complementary strand | 815825 | 816733 |
| JW0764(moaA) | | 817130 | 818119 |
| JW0765(moaB) | | 818141 | 818653 |
| JW0766(moaC) | | 818656 | 819141 |
| JW0767(moaD) | | 819134 | 819379 |
| JW0768(moaE) | | 819381 | 819833 |
| JW0769(ybhL) | | 819970 | 820674 |
| JW0770(ybhM) | | 820879 | 821592 |
| JW0771(ybhN) | Complementary strand | 821628 | 822584 |
| JW0772(ybhO) | Complementary strand | 822584 | 823825 |
| JW0773(ybhP) | Complementary strand | 823822 | 824583 |
| JW0774(ybhQ) | | 824716 | 825126 |
| JW0775(ybhR) | Complementary strand | 825088 | 825900 |
| JW0776(ybhR) | Complementary strand | 825837 | 826193 |
| JW0777(ybhS) | Complementary strand | 826204 | 827337 |
| JW0778(yhiG) | Complementary strand | 827330 | 829081 |
| JW0779(yhiI) | Complementary strand | 829059 | 830057 |
| JW0780(ybiH) | Complementary strand | 830057 | 830728 |
| JW0781(rhlE) | | 830957 | 832321 |
| JW0782(204#3.1) | | 832420 | 832566 |
| JW0783(ybiA) | Complementary strand | 832553 | 833035 |
| JW0784(dinG) | | 833155 | 835305 |
| JW0785(ybiB) | | 835333 | 836295 |
| JW0786(ybiC) | | 836436 | 837521 |
| JW0787(ybiJ) | Complementary strand | 837750 | 838010 |
| JW0788(ybil) | Complementary strand | 838275 | 838541 |
| JW0789(ybiX) | Complementary strand | 838615 | 839328 |
| JW0790(ybiL) | Complementary strand | 839334 | 841616 |
| JW0791(ybiM) | Complementary strand | 841881 | 842285 |
| JW0792(ybiN) | | 842336 | 843343 |
| JW0793(ybiO) | Complementary strand | 843340 | 845700 |
| JW0794(glnQ) | Complementary strand | 845826 | 846548 |
| JW0795(glnP) | Complementary strand | 846545 | 847204 |
| JW0796(glnH) | Complementary strand | 847343 | 848089 |
| JW0797(dps) | Complementary strand | 848493 | 848996 |
| JW0798(ybiF) | Complementary strand | 849295 | 850182 |
| JW0799(ompX) | | 850535 | 851050 |
| JW0800(ybiP) | Complementary strand | 851099 | 852682 |
| JW0801 (206#8) | | 853268 | 853735 |
| JW0802(ybiR) | | 853732 | 854850 |
| JW0803(yibS) | Complementary strand | 854909 | 855829 |
| JW0804(ybiT) | | 856048 | 857640 |
| JW0805(ybiU) | Complementary strand | 857881 | 859146 |
| JW0806(ybiV) | Complementary strand | 859298 | 860113 |
| JW0807(ybiW) | Complementary strand | 860259 | 862691 |
| JW0808(ybiY) | Complementary strand | 862697 | 863623 |
| JW0809(mipB) | | 863655 | 864389 |
| JW0810(moeB) | Complementary strand | 864465 | 865214 |
| JW0811(moeA) | Complementary strand | 865214 | 866449 |
| JW0812(ybiK) | | 866653 | 867618 |
| JW0813(yliA) | | 867497 | 869476 |
| JW0814(yliB) | | 869241 | 871034 |
| JW0815(yliC) | | 871052 | 871972 |
| JW0816(yliD) | | 871975 | 872886 |
| JW0817(209#3) | | 873064 | 875412 |
| JW0818(209#4) | | 875420 | 876748 |
| JW0819(yliG) | Complementary strand | 876795 | 878120 |
| JW0820(yliH) | | 878333 | 878716 |
| JW0821 (yliI) | | 878827 | 879942 |
| JW0822(yliJ) | Complementary strand | 879939 | 880565 |
| JW0823(dacC) | | 880812 | 882014 |
| JW0824(deoR) | Complementary strand | 882061 | 882819 |
| JW0825(ybjG) | Complementary strand | 882877 | 883488 |
| JW0826(mdfA) | | 883758 | 884990 |
| JW0827(ybjH) | Complementary strand | 885031 | 885315 |
| JW0828(210#3) | Complementary strand | 885401 | 886219 |
| JW0829(210#4) | Complementary strand | 886216 | 887424 |
| JW0830(210#5) | | 887478 | 888044 |
| JW0831(210#6) | Complementary strand | 888219 | 889904 |
| JW0832(ybjM) | | 890174 | 890551 |
| JW0833(grxA) | Complementary strand | 890581 | 890838 |
| JW0834(ybjC) | | 890998 | 891285 |
| JW0835(mdaA) | | 891269 | 891991 |
| JW0836(rimK) | | 892052 | 892954 |
| JW0837(ybjN) | | 893042 | 893518 |
| JW0838(potF) | | 893869 | 894981 |
| JW0839(potG) | | 894995 | 896209 |
| JW0840(potH) | | 896219 | 897172 |
| JW0841(potI) | | 897169 | 898014 |
| JW0842(ybjO) | | 898074 | 898562 |
| JW0843(ybjF) | | 898603 | 899730 |
| JW0844(artJ) | Complementary strand | 899929 | 900660 |
| JW0845(artM) | Complementary strand | 900951 | 901619 |
| JW0846(artQ) | Complementary strand | 901619 | 902335 |
| JW0847(artI) | Complementary strand | 902342 | 903073 |
| JW0848(artP) | Complementary strand | 903091 | 903819 |
| JW0849(ybjP) | Complementary strand | 904037 | 904552 |
| JW0850(211#9) | | 904678 | 905001 |
| JW0851(211#10) | | 904998 | 905828 |
| JW0852(211#11) | Complementary strand | 905825 | 906874 |
| JW0853(ybjT) | Complementary strand | 906937 | 908442 |
| JW0854(ltaA) | Complementary strand | 908378 | 909379 |
| JW0855(poxB) | Complementary strand | 909416 | 911134 |
| JW0856(ybjV) | Complementary strand | 911267 | 912241 |
| JW0857(ybjW) | Complementary strand | 912247 | 913899 |
| JW0858(ybjE) | Complementary strand | 914043 | 914942 |
| JW0859(aqpZ) | Complementary strand | 915437 | 916132 |
| JW0860(ybjD) | | 916558 | 918216 |
| JW0861(ybjX) | Complementary strand | 918213 | 919205 |
| JW0862(213#3) | | 919320 | 920435 |
| JW0863(ybjZ) | | 920432 | 922378 |
| JW0864(cspD) | Complementary strand | 922451 | 922675 |
| JW0865(ylaJ) | | 922998 | 923318 |
| JW0866(clpA) | | 923349 | 925625 |
| JW0867(infA) | Complementary strand | 926310 | 926528 |
| JW0868(aat) | Complementary strand | 926813 | 927517 |
| JW0869(cydC) | Complementary strand | 927559 | 929280 |
| JW0870(cydD) | Complementary strand | 929281 | 931047 |
| JW0871(trxB) | Complementary strand | 931170 | 932135 |
| JW0872(lrp) | | 932680 | 933174 |
| JW0873(ftsK) | | 933309 | 937298 |
| JW0874(lolA) | | 937454 | 938068 |
| JW0875(ycaJ) | | 938079 | 939422 |
| JW0876(serS) | | 939513 | 940805 |
| JW0877(dmsA) | | 941131 | 943488 |
| JW0878(dmsB) | | 943499 | 944116 |
| JW0879(dmsC) | | 944118 | 944981 |
| JW0880(ycaC) | Complementary strand | 945016 | 945642 |
| JW0881 (ycaD) | | 945956 | 947104 |
| JW0882(yjeM) | | 947122 | 948744 |
| JW0883(ycaN) | Complementary strand | 948745 | 949653 |
| JW0884(ycaK) | | 949753 | 950343 |
| JW0885(pflA) | Complementary strand | 950425 | 951165 |
| JW0886(pflB) | Complementary strand | 951357 | 953639 |
| JW0887(focA) | Complementary strand | 953694 | 954551 |
| JW0888(ycaO) | Complementary strand | 954957 | 956717 |
| JW0889(ycaP) | | 956847 | 957539 |
| JW0890(serC) | | 957738 | 958826 |
| JW0891 (aroA) | | 958897 | 960180 |
| JW0892(ycaL) | | 960349 | 961113 |
| JW0893(cmk) | | 961286 | 961969 |
| JW0894(rpsA) | | 962080 | 963753 |
| JW0895(himD) | | 963913 | 964197 |
| JW0896(ycaI) | | 964327 | 966669 |
| JW0897(msbA) | | 966706 | 968454 |
| JW0898(lpxK) | | 968451 | 969437 |
| JW0899(ycaQ) | | 969474 | 970706 |
| JW0900(ycaR) | | 970758 | 970940 |
| JW0901(kdsB) | | 970937 | 971683 |
| JW0902(218#5) | | 971837 | 972730 |
| JW0903(ycbC) | Complementary strand | 972707 | 973486 |
| JW0904(smtA) | | 973622 | 974407 |
| JW0905(mukF) | | 974404 | 975726 |
| JW0906(kicA) | | 975734 | 976411 |
| JW0907(mukB) | | 976411 | 980871 |
| JW0908(ycbB) | | 981132 | 982979 |
| JW0909(ycbK) | | 983160 | 983708 |
| JW0910(ycbL) | | 983735 | 984382 |
| JW0911(aspC) | Complementary strand | 984604 | 985794 |
| JW0912(ompF) | Complementary strand | 985979 | 987067 |
| JW0913(asnS) | Complementary strand | 987670 | 989070 |
| JW0914(pncB) | Complementary strand | 989239 | 990441 |
| JW0915(pepN) | | 990707 | 993319 |
| JW0916(ssuB) | Complementary strand | 993362 | 994129 |
| JW0917(ssuC) | Complementary strand | 994126 | 994962 |
| JW0918(ssuD) | Complementary strand | 994928 | 996073 |
| JW0919(ssuA) | Complementary strand | 996070 | 997029 |
| JW0920(ssuE) | Complementary strand | 997022 | 997597 |
| JW0921(ycbQ) | | 997944 | 998492 |
| JW0922(ycbR) | | 998575 | 999276 |
| JW0923(ycbS) | | 999301 | 1001901 |
| JW0924(ycbT) | | 1001892 | 1002962 |
| JW0925(221#10) | | 1002974 | 1003516 |
| JW0926(221#11) | | 1003476 | 1004039 |
| JW0927(ycbF) | | 1004005 | 1004742 |
| JW0928(pyrD) | | 1004853 | 1005863 |
| JW0929(ycbW) | | 1006001 | 1006579 |
| JW0930(221 #15) | Complementary strand | 1006576 | 1007643 |
| JW0931(ycbY) | | 1007929 | 1010037 |
| JW0932(uup) | | 1010049 | 1011956 |
| JW0933(pqiA) | | 1012086 | 1013339 |
| JW0934(pqiB) | | 1013344 | 1014984 |
| JW0935(ymbA) | | 1014981 | 1015544 |
| JW0936(rmf) | | 1015800 | 1015967 |
| JW0937(fabA) | Complementary strand | 1016037 | 1016555 |
| JW0938(222#8) | Complementary strand | 1016624 | 1018384 |
| JW0939(ycbG) | | 1018570 | 1019022 |
| JW0940(ompA) | Complementary strand | 1019098 | 1020138 |
| JW0941 (sulA) | Complementary strand | 1020495 | 1021004 |
| JW0942(yccR) | | 1021223 | 1021852 |
| JW0943(yccS) | Complementary strand | 1021815 | 1023977 |
| JW0944(yccF) | Complementary strand | 1023987 | 1024433 |
| JW0945(helD) | | 1024556 | 1026610 |
| JW0946(mgsA) | Complementary strand | 1026642 | 1027109 |
| JW0947(223#9) | Complementary strand | 1027196 | 1027858 |
| JW0948(223#10) | | 1027977 | 1028444 |
| JW0949(yccV) | Complementary strand | 1028489 | 1028857 |
| JW0950(yccW) | Complementary strand | 1028864 | 1030054 |
| JW0951 (acyP) | | 1029936 | 1030427 |
| JW0952(yccK) | Complementary strand | 1030424 | 1030753 |
| JW0953(yccA) | Complementary strand | 1030844 | 1031503 |
| JW0953.5(224#4) | Complementary strand | 1031705 | 1031881 |
| JW0954(hyaA) | | 1032224 | 1033342 |
| JW0955(hyaB) | | 1033339 | 1035132 |
| JW0956(hyaC) | | 1035151 | 1035858 |
| JW0957(hyaD) | | 1035855 | 1036442 |
| JW0958(hyaE) | | 1036439 | 1036837 |
| JW0959(hyaF) | | 1036834 | 1037691 |
| JW0960(appC) | | 1037825 | 1039369 |
| JW0961(appB) | | 1039381 | 1040517 |
| JW0962(yccB) | | 1040530 | 1040622 |
| JW0963(appA) | | 1040702 | 1042000 |
| JW0964(yccC) | Complementary strand | 1042115 | 1044295 |
| JW0965(yccY) | Complementary strand | 1044315 | 1044773 |
| JW0966(yccZ) | Complementary strand | 1044749 | 1045888 |
| JW0967(ymcA) | Complementary strand | 1045934 | 1048030 |
| JW0968(ymcB) | Complementary strand | 1048030 | 1048776 |
| JW0969(ymcC) | Complementary strand | 1048773 | 1049417 |
| JW0970(ymcD) | Complementary strand | 1049524 | 1049847 |
| JW0971 (insA) | | 1049918 | 1050193 |
| JW0972(insB) | | 1050112 | 1050615 |
| JW0973(cspH) | Complementary strand | 1051048 | 1051248 |
| JW0974(cspG) | | 1051546 | 1051758 |
| JW0975(sfa) | | 1051932 | 1052162 |
| JW0976(yccL) | | 1052152 | 1052325 |
| JW0977(yccM) | Complementary strand | 1052374 | 1053447 |
| JW0978(torS) | Complementary strand | 1053519 | 1056233 |
| JW0979(torT) | | 1056346 | 1057374 |
| JW0980(torR) | Complementary strand | 1057347 | 1058039 |
| JW0981(torC) | | 1058169 | 1059341 |
| JW0982(torA) | | 1059341 | 1061887 |
| JW0983(torD) | | 1061884 | 1062483 |
| JW0984(yccD) | Complementary strand | 1062635 | 1062940 |
| JW0985(cbpA) | Complementary strand | 1062940 | 1063860 |
| JW0986(yccE) | | 1064121 | 1065377 |
| JW0987(agp) | | 1065670 | 1066911 |
| JW0988(yccJ) | Complementary strand | 1066949 | 1067176 |
| JW0989(wrbA) | Complementary strand | 1067197 | 1067793 |
| JW0990(yciG) | | 1068136 | 1068339 |
| JW0991(ycdG) | Complementary strand | 1068596 | 1069990 |
| JW0992(228#5) | Complementary strand | 1069945 | 1070520 |
| JW0993(228#6) | Complementary strand | 1070450 | 1071040 |
| JW0994(228#7) | Complementary strand | 1071050 | 1071850 |
| JW0995(ycdK) | Complementary strand | 1071858 | 1072244 |
| JW0996(228#9) | Complementary strand | 1072256 | 1072951 |
| JW0997(228#10) | Complementary strand | 1072948 | 1074096 |
| JW0998(ycdC) | | 1074327 | 1074965 |
| JW0999(putA) | Complementary strand | 1075005 | 1078967 |
| JW1000(yzpU) | Complementary strand | 1079119 | 1079310 |
| JW1001(putP) | | 1079390 | 1080898 |
| JW1002(229#1) | | 1081539 | 1082270 |
| JW1003(ycdO) | | 1082328 | 1083455 |
| JW1004(ycdB) | | 1083461 | 1084732 |
| JW1005(phoH) | | 1085077 | 1086141 |
| JW1006(ycdP) | Complementary strand | 1086191 | 1086604 |
| JW1007(ycdQ) | Complementary strand | 1086606 | 1087931 |
| JW1008(ycdR) | Complementary strand | 1087924 | 1088235 |
| JW1009(ycdR) | Complementary strand | 1088400 | 1089941 |
| JW1010(ycdS) | Complementary strand | 1089950 | 1092373 |
| JW1011(ycdT) | | 1092888 | 1094318 |
| JW1012(230#3) | Complementary strand | 1094359 | 1095225 |
| JW1013(230#3.1) | Complementary strand | 1095222 | 1095521 |
| JW1014(230#4) | | 1095607 | 1095930 |
| JW1015(ycdU) | | 1095927 | 1096913 |
| JW1016(ycdW) | | 1097789 | 1098727 |
| JW1017(ycdX) | | 1098782 | 1099519 |
| JW1018(ycdY) | | 1099543 | 1100097 |
| JW1019(ycdZ) | | 1100151 | 1100690 |
| JW1020(csgG) | Complementary strand | 1100754 | 1101587 |
| JW1021(csgF) | Complementary strand | 1101614 | 1102030 |
| JW1022(csgE) | Complementary strand | 1102055 | 1102444 |
| JW1023(csgD) | Complementary strand | 1102449 | 1103099 |
| JW1024(csgB) | | 1103854 | 1104309 |
| JW1025(csgA) | | 1104350 | 1104805 |
| JW1026(csgC) | | 1104864 | 1105196 |
| JW1027(yi22) | Complementary strand | 1105295 | 1106200 |
| JW1028(yi24) | | 1105724 | 1106155 |
| JW1029(yi23) | Complementary strand | 1105834 | 1106127 |
| JW1030(yi2A) | Complementary strand | 1106158 | 1106568 |
| JW1031(sfaA) | | 1106653 | 1106964 |
| JW1032(232#3) | | 1107059 | 1107592 |
| JW1033(ymdC) | | 1107534 | 1109015 |
| JW1034(ymdD) | Complementary strand | 1109023 | 1110180 |
| JW1035(mdoG) | | 1110574 | 1112109 |
| JW1036(232#7) | Complementary strand | 1110676 | 1110933 |
| JW1037(mdoH) | | 1112102 | 1114645 |
| JW1038(yceK) | | 1114842 | 1115045 |
| JW1039(msyB) | Complementary strand | 1115046 | 1115420 |
| JW1040(yceE) | Complementary strand | 1115503 | 1116729 |
| JW1041 (htrB) | Complementary strand | 1116901 | 1117821 |
| JW1042(yceA) | | 1118046 | 1119098 |
| JW1043(yceI) | Complementary strand | 1119140 | 1119715 |
| JW1044(233#6) | Complementary strand | 1119719 | 1120285 |
| JW1045(233#6.1) | Complementary strand | 1120546 | 1120686 |
| JW1046(soIA) | Complementary strand | 1120707 | 1121825 |
| JW1047(yceP) | Complementary strand | 1121940 | 1122197 |
| JW1048(dinI) | Complementary strand | 1122481 | 1122726 |
| JW1049(pyrC) | Complementary strand | 1122800 | 1123846 |
| JW1050(yceB) | Complementary strand | 1123952 | 1124512 |
| JW1051(grxB) | Complementary strand | 1124646 | 1125293 |
| JW1052(yqjV) | Complementary strand | 1125357 | 1126565 |
| JW1053(rimJ) | | 1126801 | 1127385 |
| JW1054(yceH) | | 1127396 | 1128043 |
| JW1055(mviM) | | 1128045 | 1128968 |
| JW1056(mviN) | | 1129078 | 1130613 |
| JW1057(flgN) | Complementary strand | 1130653 | 1131069 |
| JW1058(flgM) | Complementary strand | 1131074 | 1131367 |
| JW1059(flgA) | Complementary strand | 1131443 | 1132102 |
| JW1060(flgB) | | 1132257 | 1132673 |
| JW1061(flgC) | | 1132677 | 1133081 |
| JW1062(flgD) | | 1133093 | 1133788 |
| JW1063(flgE) | | 1133813 | 1135021 |
| JW1064(flgF) | | 1135041 | 1135796 |
| JW1065(flgG) | | 1135968 | 1136750 |
| JW1066(flgH) | | 1136794 | 1137501 |
| JW1067(flgI) | | 1137513 | 1138610 |
| JW1068(flgJ) | | 1138610 | 1139551 |
| JW1069(flgK) | | 1139617 | 1141260 |
| JW1070(flgL) | | 1141272 | 1142225 |
| JW1071 (rne) | Complementary strand | 1142421 | 1145606 |
| JW1072(rluC) | | 1146179 | 1147138 |
| JW1073(yceF) | Complementary strand | 1147250 | 1147873 |
| JW1074(yceD) | | 1148033 | 1148554 |
| JW1075(rpmF) | | 1148606 | 1148779 |
| JW1076(plsX) | | 1148890 | 1149930 |
| JW1077(fabH) | | 1149998 | 1150951 |
| JW1078(fabD) | | 1150967 | 1151896 |
| JW1079(fabG) | | 1151909 | 1152643 |
| JW1080(acpP) | | 1152854 | 1153090 |
| JW1081(fabF) | | 1153178 | 1154419 |
| JW1082(pabC) | | 1154539 | 1155348 |
| JW1083(yceG) | | 1155351 | 1156373 |
| JW1084(tmk) | | 1156363 | 1157004 |
| JW1085(holB) | | 1157001 | 1158005 |
| JW1086(ycfH) | | 1158016 | 1158813 |
| JW1087(ptsG) | | 1159108 | 1160541 |
| JW1088(fhuE) | Complementary strand | 1160601 | 1162790 |
| JW1089(ycfF) | | 1163124 | 1163483 |
| JW1090(ycfL) | | 1163486 | 1163863 |
| JW1091 (ycfM) | | 1163874 | 1164518 |
| JW1092(ycfN) | | 1164499 | 1165323 |
| JW1093(nagZ) | | 1165334 | 1166359 |
| JW1094(ycfP) | | 1166325 | 1166924 |
| JW1095(ndh) | | 1167324 | 1168628 |
| JW1096(ycfJ) | | 1168838 | 1169377 |
| JW1097(ycfQ) | Complementary strand | 1169439 | 1170149 |
| JW1098(ycfR) | | 1170312 | 1170569 |
| JW1099(ycfS) | Complementary strand | 1170651 | 1171613 |
| JW1100(mfd) | Complementary strand | 1171757 | 1175203 |
| JW1101(ycfT) | Complementary strand | 1175331 | 1176404 |
| JW1102(ycfU) | | 1176615 | 1177865 |
| JW1103(ycfV) | | 1177720 | 1178559 |
| JW1104(ycfW) | | 1178559 | 1179803 |
| JW1105(ycfX) | | 1179832 | 1180743 |
| JW1106(238#9) | | 1180759 | 1181598 |
| JW1107(cobB) | Complementary strand | 1181718 | 1182506 |
| JW1108(ymfA) | Complementary strand | 1182503 | 1182973 |
| JW1109(potD) | Complementary strand | 1183022 | 1184068 |
| JW1110(potC) | Complementary strand | 1184065 | 1184859 |
| JW1111(potB) | Complementary strand | 1184856 | 1185683 |
| JW1112(potA) | Complementary strand | 1185697 | 1186833 |
| JW1113(pepT) | | 1187083 | 1188309 |
| JW1114(ycfD) | Complementary strand | 1188358 | 1189479 |
| JW1115(phoQ) | Complementary strand | 1189555 | 1191015 |
| JW1116(phoP) | Complementary strand | 1191015 | 1191686 |
| JW1117(purB) | Complementary strand | 1191855 | 1193225 |
| JW1118(ycfC) | Complementary strand | 1193229 | 1193876 |
| JW1119(trmU) | Complementary strand | 1193906 | 1195012 |
| JW1120(ymfB) | Complementary strand | 1195066 | 1195527 |
| JW1121 (ymfC) | Complementary strand | 1195537 | 1196190 |
| JW1122(icdA) | | 1196362 | 1197612 |
| JW1123(ymfD) | Complementary strand | 1198106 | 1198771 |
| JW1124(ymfE) | Complementary strand | 1198772 | 1199485 |
| JW1125(lit) | | 1199934 | 1200827 |
| JW1126(intE) | Complementary strand | 1200918 | 1202045 |
| JVJ1127(VXIS_BPP21) | Complementary strand | 1202026 | 1202271 |
| JW1128(ymfH) | Complementary strand | 1202308 | 1202619 |
| JW1129(ymfl) | | 1202691 | 1203077 |
| JW1130(ymfJ) | Complementary strand | 1203015 | 1203323 |
| JW1131(RPC2_BPP22) | Complementary strand | 1203498 | 1204172 |
| JW1132(241#2) | | 1203960 | 1204463 |
| JW1133(ymfL) | | 1204495 | 1205064 |
| JW1134(ymfM) | | 1205061 | 1205399 |
| JW1135(ymfN) | | 1205409 | 1206776 |
| JW1136(ymfR) | | 1206788 | 1206970 |
| JW1137(ymfO) | | 1206970 | 1207443 |
| JW1138(241 #8) | | 1207331 | 1208161 |
| JW1139(241 #9) | | 1208152 | 1208736 |
| JW1140(ycfK) | | 1208740 | 1209369 |
| JW1141(ymfS) | | 1209152 | 1209784 |
| JW1142(ycfA) | Complementary strand | 1209756 | 1210358 |
| JW1143(ycfE) | Complementary strand | 1210358 | 1210897 |
| JW1144(pin) | | 1210924 | 1211478 |
| JW1145(mcrA) | | 1211585 | 1212418 |
| JW1146(241#16) | | 1212652 | 1212816 |
| JW1147(ycgW) | Complementary strand | 1212919 | 1213242 |
| JW1148(ycgX) | Complementary strand | 1213942 | 1214346 |
| JW1149(ycgE) | Complementary strand | 1214567 | 1215298 |
| JW1150(242#1) | Complementary strand | 1215503 | 1216714 |
| JW1151(ycgZ) | | 1217028 | 1217264 |
| JW1152(ymgA) | | 1217307 | 1217579 |
| JW1153(242#1.4) | | 1217608 | 1217874 |
| JW1154(ymgC) | | 1217987 | 1218235 |
| JW1155(yahA) | | 1218525 | 1220090 |
| JW1156(243#0.1) | | 1220222 | 1220440 |
| JW1157(243#1) | | 1220840 | 1222360 |
| JW1158(aidA) | | 1222427 | 1223461 |
| JW1159(ymgD) | Complementary strand | 1223544 | 1223879 |
| JW1160(243#4) | Complementary strand | 1223883 | 1224227 |
| JW1161(243#5) | | 1224737 | 1224925 |
| JW1162(243#5.1) | | 1224934 | 1225146 |
| JW1163(minE) | Complementary strand | 1225518 | 1225784 |
| JW1164(minD) | Complementary strand | 1225788 | 1226600 |
| JW1165(minC) | Complementary strand | 1226624 | 1227319 |
| JW1166(ycgJ) | | 1227839 | 1228207 |
| JW1167(ycgK) | Complementary strand | 1228310 | 1228711 |
| JW1168(ycgL) | | 1228920 | 1229246 |
| JW1169(243#12) | | 1229318 | 1229977 |
| JW1170(ycgN) | | 1230039 | 1230515 |
| JW1171(clyA) | Complementary strand | 1230722 | 1231639 |
| JW1172(umuD) | | 1232006 | 1232425 |
| JW1173(umuC) | | 1232425 | 1233693 |
| JW1174(dsbB) | Complementary strand | 1233739 | 1234275 |
| JW1175(nhaB) | Complementary strand | 1234415 | 1235956 |
| JW1176(fadR) | | 1236177 | 1236896 |
| JW1177(ycgB) | Complementary strand | 1236948 | 1238480 |
| JW1178(dadA) | | 1238810 | 1240108 |
| JW1179(dadX) | | 1240118 | 1241188 |
| JW1180(ycgO) | Complementary strand | 1241574 | 1243184 |
| JW1181(ldcA) | Complementary strand | 1243405 | 1244319 |
| JW1182(mltE) | | 1244305 | 1245030 |
| JW1183(ycgR) | Complementary strand | 1245032 | 1245766 |
| JW1184(tag1) | | 1245967 | 1246221 |
| JW1185(ycgY) | | 1246399 | 1246839 |
| JW1186(treA) | Complementary strand | 1246918 | 1248615 |
| JW1187(isSB) | Complementary strand | 1248821 | 1249837 |
| JW1188(yi51) | | 1249157 | 1249525 |
| JW1189(ycgC) | Complementary strand | 1250134 | 1251555 |
| JW1190(ycgS) | Complementary strand | 1251563 | 1252270 |
| JW1191(ycgT) | Complementary strand | 1252206 | 1253306 |
| JW1192(dhaR) | | 1253495 | 1255423 |
| JW1193(246#4) | Complementary strand | 1255523 | 1258390 |
| JW1194(ychF) | Complementary strand | 1259159 | 1260250 |
| JW1195(pth) | Complementary strand | 1260367 | 1260951 |
| JW1196(ychH) | | 1261229 | 1261507 |
| JW1197(ychM) | Complementary strand | 1261562 | 1262887 |
| JW1198(prsA) | Complementary strand | 1263366 | 1264313 |
| JW1199(ychB) | Complementary strand | 1264464 | 1265315 |
| JW1200(hemM) | Complementary strand | 1265315 | 1265938 |
| JW1201(hemA) | | 1266152 | 1267408 |
| JW1202(prfA) | | 1267450 | 1268532 |
| JW1203(hemK) | | 1268532 | 1269365 |
| JW1204(248#3) | | 1269362 | 1269754 |
| JW1205(ychA) | | 1269758 | 1270567 |
| JW1206(kdsA) | | 1270603 | 1271457 |
| JW1207(chaA) | Complementary strand | 1273187 | 1274287 |
| JW1208(chaB) | | 1274557 | 1274787 |
| JW1209(chaC) | | 1274924 | 1275640 |
| JW1210(ychN) | Complementary strand | 1275684 | 1276037 |
| JW1211(ychO) | | 1276363 | 1277616 |
| JW1212(narL) | Complementary strand | 1277617 | 1278267 |
| JW1213(narX) | Complementary strand | 1278260 | 1280056 |
| JW1214(narK) | | 1280395 | 1281786 |
| JW1215(narG) | | 1282302 | 1286045 |
| JW1216(narH) | | 1286042 | 1287580 |
| JW1217(narJ) | | 1287577 | 1288287 |
| JW1218(narI) | | 1288287 | 1288964 |
| JW1219(tpr) | Complementary strand | 1289525 | 1289626 |
| JW1220(purU) | Complementary strand | 1290220 | 1291062 |
| JW1221(ychJ) | Complementary strand | 1291112 | 1291570 |
| JW1222(ychK) | | 1291644 | 1292588 |
| JW1223(sprE) | | 1292680 | 1293693 |
| JW1224(galU) | | 1293895 | 1294803 |
| JW1225(hns) | Complementary strand | 1294947 | 1295360 |
| JW1226(tdk) | | 1295965 | 1296582 |
| JW1227(ychG) | Complementary strand | 1296864 | 1297454 |
| JW1227.5(251 #6) | Complementary strand | 1297406 | 1297636 |
| JW1228(adhE) | Complementary strand | 1297884 | 1300559 |
| JW1229(ychE) | | 1301036 | 1301683 |
| JW1230(yi2A) | | 1301946 | 1302356 |
| JW1231(yi22) | | 1302314 | 1303219 |
| JW1232(yi24) | Complementary strand | 1302359 | 1302790 |
| JW1233(yi23) | | 1302387 | 1302680 |
| JW1234(251#8.5) | Complementary strand | 1303144 | 1303491 |
| JW1235(oppA) | | 1303757 | 1305388 |
| JW1236(oppB) | | 1305474 | 1306394 |
| JW1237(oppC) | | 1306409 | 1307317 |
| JW1238(oppD) | | 1307329 | 1308342 |
| JW1239(oppF) | | 1308339 | 1309343 |
| JW1240(yciU) | Complementary strand | 1309396 | 1309725 |
| JW1241(cls) | Complementary strand | 1309760 | 1311220 |
| JW1242(kch) | Complementary strand | 1311591 | 1312844 |
| JW1243(yciI) | Complementary strand | 1313144 | 1313440 |
| JW1244(tonB) | | 1313649 | 1314383 |
| JW1245(yciA) | Complementary strand | 1314423 | 1314821 |
| JW1246(yciB) | Complementary strand | 1314926 | 1315465 |
| JW1247(yciC) | Complementary strand | 1315495 | 1316238 |
| JW1248(yciD) | | 1316595 | 1317233 |
| JW1249(yciE) | Complementary strand | 1317293 | 1317799 |
| JW1250(yciF) | Complementary strand | 1317845 | 1318345 |
| JW1251 (yciG) | Complementary strand | 1318431 | 1318610 |
| JW1252(trpA) | Complementary strand | 1318991 | 1319797 |
| JW1253(trpB) | Complementary strand | 1319797 | 1320990 |
| JW1254(trpC) | Complementary strand | 1321002 | 1322360 |
| JW1255(trpD) | Complementary strand | 1322364 | 1323959 |
| JW1256(trpE) | Complementary strand | 1323959 | 1325521 |
| JW1257(trpL) | Complementary strand | 1325613 | 1325657 |
| JW1258(yciV) | | 1325795 | 1326676 |
| JW1259(yciO) | | 1326637 | 1327293 |
| JW1260(yciQ) | | 1327465 | 1329216 |
| JW1261(yciL) | | 1329427 | 1330302 |
| JW1262(btuR) | Complementary strand | 1330342 | 1330932 |
| JW1263(yciK) | Complementary strand | 1330929 | 1331687 |
| JW1264(sohB) | | 1331907 | 1332956 |
| JW1265(yciN) | Complementary strand | 1332992 | 1333243 |
| JW1266(topA) | | 1333623 | 1336220 |
| JW1267(cysB) | | 1336430 | 1337404 |
| JW1268(acnA) | | 1338406 | 1341081 |
| JW1269(ribA) | Complementary strand | 1341145 | 1341735 |
| JW1270(pgpB) | | 1341905 | 1342669 |
| JW1271(yciS) | | 1342818 | 1343126 |
| JW1272(yciM) | | 1343133 | 1344302 |
| JW1273(pyrF) | | 1344496 | 1345233 |
| JW1274(yciH) | | 1345233 | 1345559 |
| JW1275(osmB) | Complementary strand | 1345685 | 1345903 |
| JW1276(yciT) | Complementary strand | 1346172 | 1346921 |
| JW1277(256#1) | Complementary strand | 1347011 | 1347193 |
| JW1278(yciR) | Complementary strand | 1347332 | 1349317 |
| JW1279(rnb) | Complementary strand | 1349553 | 1351487 |
| JW1280(yciW) | Complementary strand | 1351555 | 1352760 |
| JW1281 (envM) | Complementary strand | 1352826 | 1353614 |
| JW1282(ycjD) | Complementary strand | 1353982 | 1354335 |
| JW1283(sapF) | Complementary strand | 1354403 | 1355209 |
| JW1284(sapD) | Complementary strand | 1355211 | 1356203 |
| JW1285(sapC) | Complementary strand | 1356203 | 1357093 |
| JW1286(sapB) | Complementary strand | 1357080 | 1358045 |
| JW1287(sapA) | Complementary strand | 1358042 | 1359685 |
| JW1288(ymjA) | Complementary strand | 1359998 | 1360243 |
| JW1289(yeeF) | Complementary strand | 1360377 | 1361762 |
| JW1290(ycjK) | Complementary strand | 1362065 | 1363561 |
| JW1291 (ycjL) | | 1363695 | 1364459 |
| JW1292(ycjC) | | 1364486 | 1365043 |
| JW1293(aldH) | | 1365318 | 1366805 |
| JW1294(ordL) | | 1366807 | 1368087 |
| JW1295(goaG) | | 1368125 | 1369390 |
| JW1296(pspF) | Complementary strand | 1369510 | 1370487 |
| JW1297(pspA) | | 1370654 | 1371322 |
| JW1298(pspB) | | 1371376 | 1371600 |
| JW1299(pspC) | | 1371600 | 1371959 |
| JW1300(pspD) | | 1371968 | 1372189 |
| JW1301(pspE) | | 1372264 | 1372578 |
| JW1302(sucP) | | 1372791 | 1374470 |
| JW1303(258#8) | | 1374484 | 1375776 |
| JW1304(ycjO) | | 1375797 | 1376678 |
| JW1305(ycjP) | | 1376665 | 1377507 |
| JW1306(ycjQ) | | 1377538 | 1378590 |
| JW1307(259#3) | | 1378600 | 1379397 |
| JW1308(ycjS) | | 1379407 | 1380462 |
| JW1309(ycjT) | | 1380459 | 1382726 |
| JW1310(ycjU) | | 1382723 | 1383382 |
| JW1311(ycjV) | | 1383396 | 1384364 |
| JW1312(ompG) | | 1384522 | 1385427 |
| JW1313(ycjW) | Complementary strand | 1385538 | 1386536 |
| JW1314(ycjX) | | 1386692 | 1388089 |
| JW1315(ycjF) | | 1388086 | 1389147 |
| JW1316(tyrR) | | 1389295 | 1390836 |
| JW1317(tpx) | Complementary strand | 1390880 | 1391386 |
| JW1318(ycjG) | | 1391505 | 1392470 |
| JW1319(ycjI) | Complementary strand | 1392445 | 1393233 |
| JW1319.5(260#10) | Complementary strand | 1393255 | 1393437 |
| JW1320(ycjY) | Complementary strand | 1393508 | 1394440 |
| JW1321(ycjZ) | | 1394566 | 1395465 |
| JW1321.5(261#1.1) | | 1395554 | 1395679 |
| JW1322(mppA) | | 1395802 | 1397415 |
| JW1323(ynaI) | Complementary strand | 1397466 | 1398497 |
| JW1324(is5B) | | 1398651 | 1399667 |
| JW1325(yi51) | Complementary strand | 1398963 | 1399331 |
| JW1326(ynaJ) | | 1399940 | 1400197 |
| JW1327(ydaA) | Complementary strand | 1400247 | 1401197 |
| JW1328(fnr) | Complementary strand | 1401349 | 1402101 |
| JW1329(ogt) | Complementary strand | 1402296 | 1402811 |
| JW1330(ydaH) | Complementary strand | 1402822 | 1404354 |
| JW1331(261#9) | Complementary strand | 1404385 | 1405830 |
| JW1332(ydaJ) | Complementary strand | 1405830 | 1407155 |
| JW1333(262#1) | | 1407316 | 1408224 |
| JW1334(ydaL) | | 1408554 | 1409117 |
| JW1335(262#3) | Complementary strand | 1409138 | 1410430 |
| JW1336(262#4) | | 1410625 | 1411608 |
| JW1336.5(262#5) | | 1411883 | 1412056 |
| JW1337(dbpA) | | 1412086 | 1413459 |
| JW1338(ydaO) | Complementary strand | 1413588 | 1414523 |
| JW1339(intR) | Complementary strand | 1414575 | 1415810 |
| JW1340(ydaQ) | Complementary strand | 1415812 | 1416051 |
| JW1341(ydaC) | Complementary strand | 1416106 | 1416315 |
| JW1342(lar) | Complementary strand | 1416308 | 1416541 |
| JW1343(recT) | Complementary strand | 1416559 | 1417368 |
| JW1344(recE) | Complementary strand | 1417361 | 1419961 |
| JW1345(racC) | Complementary strand | 1420063 | 1420338 |
| JW1346(ydaE) | Complementary strand | 1420413 | 1420583 |
| JW1347(kiI) | Complementary strand | 1420583 | 1420804 |
| JW1348(sieB) | | 1421123 | 1421734 |
| JW1349(ydaF) | Complementary strand | 1421731 | 1421886 |
| JW1350(263#6.1) | Complementary strand | 1421897 | 1422076 |
| JW1351(racR) | Complementary strand | 1422340 | 1422816 |
| JW1352(263#6.3) | | 1422940 | 1423236 |
| JW1353(ydaT) | | 1423259 | 1423681 |
| JW1354(ydaU) | | 1423694 | 1424551 |
| JW1355(dnaC) | | 1424558 | 1425304 |
| JW1356(ydaW) | | 1425276 | 1425887 |
| JW1357(enpP) | | 1425914 | 1426219 |
| JW1358(trkG) | | 1426357 | 1427814 |
| JW1359(ynaK) | | 1427952 | 1428215 |
| JW1360(ydaY) | | 1428196 | 1428555 |
| JW1361(VMTH_LAMBD) | | 1429029 | 1430057 |
| JW1362(VLOM_LAMBD) | | 1430033 | 1430188 |
| JW1363(is5B) | Complementary strand | 1430321 | 1431301 |
| JW1364(yi51) | | 1430657 | 1431025 |
| JW1365(VLOM_LAMBD) | | 1431098 | 1431559 |
| JW1366(Y401_LAMBD) | | 1431624 | 1434986 |
| JW1367(yzzL) | | 1434986 | 1435561 |
| JW1368(tnpO) | Complementary strand | 1435659 | 1436249 |
| JW1369(ynaE) | Complementary strand | 1436566 | 1436832 |
| JW1370(upo3) | Complementary strand | 1437760 | 1438194 |
| JW1371(ompN) | Complementary strand | 1438335 | 1439468 |
| JW1372(nifJ) | Complementary strand | 1439835 | 1443359 |
| JW1373(ydbJ) | | 1443744 | 1443899 |
| JW1374(hslJ) | Complementary strand | 1443896 | 1444318 |
| JW1375(ldhA) | Complementary strand | 1444429 | 1445418 |
| JW1376(ydbH) | | 1445626 | 1448265 |
| JW1377(ynbE) | | 1448262 | 1448447 |
| JW1378(ydbL) | | 1448374 | 1448781 |
| JW1379(feaR) | Complementary strand | 1448953 | 1449858 |
| JW1380(feaB) | | 1450094 | 1451593 |
| JW1381(tynA) | Complementary strand | 1451651 | 1453924 |
| JW1382(paaZ) | Complementary strand | 1454172 | 1456217 |
| JW1383(paaA) | | 1456502 | 1457431 |
| JW1384(paaB) | | 1457443 | 1457730 |
| JW1385(paaC) | | 1457739 | 1458485 |
| JW1386(paaD) | | 1458494 | 1458997 |
| JW1387(paaE) | | 1459005 | 1460075 |
| JW1388(paaF) | | 1460072 | 1460839 |
| JW1389(paaG) | | 1460839 | 1461627 |
| JW1390(paaH) | | 1461629 | 1463056 |
| JW1391 (paaI) | | 1463046 | 1463468 |
| JW1392(paaJ) | | 1463468 | 1464673 |
| JW1393(paaK) | | 1464670 | 1466013 |
| JW1394(paaX) | | 1466114 | 1467064 |
| JW1395(paaY) | | 1467046 | 1467636 |
| JWy 396(ydbA) | | 1467967 | 1470525 |
| JW1397(yi22) | Complementary strand | 1470496 | 1471401 |
| JW1398(yi24) | | 1470925 | 1471356 |
| JW1399(yi23) | Complementary strand | 1471035 | 1471328 |
| JW1400(yi2A) | Complementary strand | 1471359 | 1471769 |
| JW1401(268#0.5) | | 1471933 | 1473084 |
| JW1402(ydbB) | | 1473265 | 1476588 |
| JW1403(ydbC) | | 1476796 | 1477656 |
| JW1404(ydbD) | | 1477713 | 1480025 |
| JW1405(269#1) | | 1480196 | 1480801 |
| JW1406(ynbB) | | 1480801 | 1481697 |
| JW1407(269#3) | | 1481713 | 1483470 |
| JW1408(ynbD) | | 1483484 | 1484776 |
| JW1409(acpD) | Complementary strand | 1484830 | 1485435 |
| JW1410(hrpA) | | 1485693 | 1489538 |
| JW1411 (ydcF) | | 1489810 | 1490610 |
| JW1412(aldA) | | 1490807 | 1492246 |
| JW1413(gapC) | Complementary strand | 1492288 | 1492539 |
| JW1414(gapC) | Complementary strand | 1492536 | 1492940 |
| JW1415(cybB) | | 1493441 | 1494007 |
| JW1416(ydcA) | | 1494252 | 1494425 |
| JW1416.5(271#2) | | 1494569 | 1494721 |
| JW1417(trg) | | 1495045 | 1496685 |
| JW1418(ydcI) | Complementary strand | 1496723 | 1497787 |
| JW1419(271#5) | | 1497863 | 1499206 |
| JW1420(ydcG) | | 1499461 | 1501086 |
| JW1421(271#7) | Complementary strand | 1501007 | 1501198 |
| JW1422(ydcH) | | 1501283 | 1501450 |
| JW1423(rimL) | | 1501513 | 1502052 |
| JW1424(271#10) | Complementary strand | 1502044 | 1503024 |
| JW1425(tehA) | | 1503148 | 1504140 |
| JW1426(tehB) | | 1504137 | 1504730 |
| JW1427(272#1) | | 1505032 | 1505700 |
| JW1427.5(272#2) | Complementary strand | 1505937 | 1506224 |
| JW1428(272#3) | | 1506292 | 1507440 |
| JW1429(272#4) | Complementary strand | 1507480 | 1508778 |
| JW1430(272#5) | | 1508747 | 1509283 |
| JW1431(prtC) | | 1509356 | 1511317 |
| JW1432(yncJ) | Complementary strand | 1511409 | 1511639 |
| JW1433(ydcQ) | | 1512062 | 1512499 |
| JW1434(yjiR) | | 1512578 | 1513984 |
| JW1435(273#4) | | 1514229 | 1515374 |
| JW1436(potA) | | 1515392 | 1516405 |
| JW1437(273#6) | | 1516406 | 1517347 |
| JW1438(potC) | | 1517337 | 1518131 |
| JW1439(ydcW) | | 1518153 | 1519577 |
| JW1440(ydcX) | | 1519889 | 1520137 |
| JW1441(ydcY) | | 1520223 | 1520456 |
| JW1442(ydcZ) | Complementary strand | 1520457 | 1520906 |
| JW1443(274#1) | Complementary strand | 1520903 | 1521655 |
| JW1444(yncB) | | 1521578 | 1522639 |
| JW1445(yncC) | | 1522837 | 1523502 |
| JW1446(yncD) | Complementary strand | 1523538 | 1525640 |
| JW1447(274#5) | | 1525882 | 1526943 |
| JW1448(ansP) | Complementary strand | 1527056 | 1528606 |
| JW1449(275#2) | | 1528822 | 1529439 |
| JW1450(275#2.1) | | 1529602 | 1529727 |
| JW1451(rhsE) | | 1530465 | 1532513 |
| JW1452(ydcD) | | 1532497 | 1532979 |
| JW1453(yhhI) | | 1533161 | 1533907 |
| JW1454(276#2.1) | | 1534026 | 1534151 |
| JW1455(ydcC) | | 1534391 | 1535527 |
| JW1456(ydcE) | | 1535627 | 1535860 |
| JW1457(yddH) | Complementary strand | 1535857 | 1536426 |
| JW1458(nhoA) | | 1536599 | 1537444 |
| JW1459(yddE) | Complementary strand | 1537540 | 1538433 |
| JW1460(narV) | Complementary strand | 1538512 | 1539192 |
| JW1461(narW) | Complementary strand | 1539189 | 1539884 |
| JW1462(narY) | Complementary strand | 1539884 | 1541428 |
| JW1463(narZ) | Complementary strand | 1541425 | 1545165 |
| JW1464(narU) | Complementary strand | 1545247 | 1546635 |
| JW1465(277#1.1) | | 1545457 | 1545627 |
| JW1466(yddJ) | Complementary strand | 1546959 | 1547294 |
| JW1467(yddK) | Complementary strand | 1547333 | 1548289 |
| JW1468(277#2) | Complementary strand | 1548313 | 1548603 |
| JW1469(yddG) | Complementary strand | 1548863 | 1549687 |
| JW1470(fdnG) | | 1549976 | 1553023 |
| JW1471 (fdnH) | | 1553036 | 1553920 |
| JW1472(fdnI) | | 1553913 | 1554566 |
| JW1473(yddM) | Complementary strand | 1554973 | 1555335 |
| JW1474(adhP) | Complementary strand | 1555403 | 1556413 |
| JW1475(sfcA) | Complementary strand | 1556547 | 1558271 |
| JW1475.1 (rpsV) | Complementary strand | 1558401 | 1558538 |
| JW1476(yddX) | Complementary strand | 1558640 | 1558918 |
| JW1477(osmC) | | 1559200 | 1559631 |
| JW1478(yddO) | Complementary strand | 1559687 | 1560613 |
| JW1479(yddP) | Complementary strand | 1560606 | 1561592 |
| JW1480(yddQ) | Complementary strand | 1561589 | 1562485 |
| JW1481 (yddR) | Complementary strand | 1562482 | 1563504 |
| JW1482(yddS) | Complementary strand | 1563506 | 1565194 |
| JW1483(280#1) | Complementary strand | 1565070 | 1565651 |
| JW1484(dos) | Complementary strand | 1565909 | 1568308 |
| JW1485(280#3) | Complementary strand | 1568333 | 1569715 |
| JW1486(280#4) | Complementary strand | 1570079 | 1571398 |
| JW1487(xasA) | Complementary strand | 1571529 | 1573064 |
| JW1488(gadB) | Complementary strand | 1573220 | 1574620 |
| JW1489(pqqL) | Complementary strand | 1574982 | 1577777 |
| JW1490(yddB) | Complementary strand | 1577822 | 1580194 |
| JW1491(yddA) | Complementary strand | 1580232 | 1580963 |
| JW1492(chuR) | Complementary strand | 1582208 | 1583365 |
| JW1493(301#2) | Complementary strand | 1583417 | 1585132 |
| JW1494(ydeO) | Complementary strand | 1585501 | 1586262 |
| JW1495(301#4) | Complementary strand | 1586782 | 1589061 |
| JW1496(fimH) | Complementary strand | 1589395 | 1590309 |
| JW1497(fimG) | Complementary strand | 1590368 | 1590871 |
| JW1498(fimF) | Complementary strand | 1590884 | 1591414 |
| JW1499(fimD) | Complementary strand | 1591428 | 1592576 |
| JW1500(hipA) | Complementary strand | 1593429 | 1594751 |
| JW1501(hipB) | Complementary strand | 1594751 | 1595017 |
| JW1502(aidA) | Complementary strand | 1595240 | 1596640 |
| JW1503(ydeK) | Complementary strand | 1596684 | 1600661 |
| JW1504(ydeV) | Complementary strand | 1601192 | 1602784 |
| JW1505(ydeW) | Complementary strand | 1602863 | 1603816 |
| JW1506(rbsA) | | 1604065 | 1605600 |
| JW1507(ydeY) | | 1605594 | 1606622 |
| JW1508(ydeZ) | | 1606622 | 1607614 |
| JW1509(304#1) | | 1607626 | 1608648 |
| JW1510(yneB) | | 1608675 | 1609550 |
| JW1511(yneC) | | 1609574 | 1609864 |
| JW1512(tam) | | 1609921 | 1610679 |
| JW1513(304#5) | Complementary strand | 1610683 | 1611648 |
| JW1514(uxaB) | Complementary strand | 1611804 | 1613255 |
| JW1515(305#1) | Complementary strand | 1613482 | 1614381 |
| JW1516(yneG) | Complementary strand | 1614541 | 1614900 |
| JW1517(yneH) | Complementary strand | 1614900 | 1615826 |
| JW1518(yneI) | Complementary strand | 1615890 | 1617302 |
| JW1519(yneJ) | | 1617379 | 1618260 |
| JW1520(305#6) | | 1618338 | 1619453 |
| JW1521(ydeA) | | 1619603 | 1620793 |
| JW1522(marC) | Complementary strand | 1620818 | 1621483 |
| JW1523(marR) | | 1621752 | 1622129 |
| JW1524(marA) | | 1622143 | 1622532 |
| JW1525(marB) | | 1622564 | 1622782 |
| JW1526(ydeD) | Complementary strand | 1622813 | 1623613 |
| JW1527(ydeF) | | 1623907 | 1625094 |
| JW1528(ydeH) | Complementary strand | 1625535 | 1626425 |
| JW1529(ydeI) | Complementary strand | 1626680 | 1627072 |
| JW1530(ydeJ) | | 1627348 | 1627866 |
| JW1531 (dcp) | Complementary strand | 1627910 | 1629955 |
| JW1532(ydfG) | | 1630092 | 1630838 |
| JW1533(ydfH) | | 1630927 | 1631613 |
| JW1534(ydfZ) | | 1631790 | 1631993 |
| JW1535(ydfl) | Complementary strand | 1632028 | 1633488 |
| JW1536(shiA) | Complementary strand | 1633577 | 1634860 |
| JW1537(307#4.1) | | 1635614 | 1635880 |
| JW1538(tnpR) | | 1636197 | 1636787 |
| JW1539(ydfM) | Complementary strand | 1636885 | 1637460 |
| JW1540(Y314_LAMBD) | Complementary strand | 1637460 | 1638422 |
| JW1541(nohA) | Complementary strand | 1638373 | 1638942 |
| JW1542(ydfO) | | 1639607 | 1640032 |
| JW1543(yccL) | Complementary strand | 1640184 | 1640360 |
| JW1544(cspI) | Complementary strand | 1641030 | 1641242 |
| JW1545(308#2) | Complementary strand | 1641605 | 1642102 |
| JW1546(lycV) | Complementary strand | 1642099 | 1642632 |
| JW1547(ydfR) | Complementary strand | 1642629 | 1642940 |
| JW1548(vlyS) | Complementary strand | 1642945 | 1643235 |
| JW1549(cspB) | Complementary strand | 1643914 | 1644129 |
| JW1550(cspF) | | 1644430 | 1644642 |
| JW1551 (regQ_LAMBD) | Complementary strand | 1645064 | 1645816 |
| JW1552(309#4) | Complementary strand | 1645830 | 1646879 |
| JW1553(rem) | Complementary strand | 1647226 | 1647477 |
| JW1554(hokD) | Complementary strand | 1647694 | 1647849 |
| JW1555(relE) | Complementary strand | 1647921 | 1648208 |
| JW1556(relB) | Complementary strand | 1648208 | 1648447 |
| JW1557(ydfV) | | 1648472 | 1648777 |
| JW1558(flxA) | | 1648980 | 1649312 |
| JW1559(ydfW) | Complementary strand | 1649749 | 1649898 |
| JW1560(ydfX) | Complementary strand | 1649921 | 1650211 |
| JW1561(dicC) | Complementary strand | 1650195 | 1650425 |
| JW1562(dicA) | | 1650509 | 1650916 |
| JW1563(ydfA) | | 1651083 | 1651238 |
| JW1564(ydfB) | | 1651240 | 1651368 |
| JW1565(ydfC) | | 1651398 | 1651616 |
| JW1566(dicB) | | 1652184 | 1652372 |
| JW1567(ydfD) | | 1652369 | 1652560 |
| JW1568(ydfE) | | 1652653 | 1653573 |
| JW1569(yi24) | Complementary strand | 1653417 | 1653683 |
| JW1570(yi22) | | 1653456 | 1654112 |
| JW1571(309#17.2) | | 1654087 | 1655283 |
| JW1572(rspB) | Complementary strand | 1655471 | 1656490 |
| JW1573(rspA) | Complementary strand | 1656502 | 1657716 |
| JW1574(ynfA) | Complementary strand | 1657922 | 1658248 |
| JW1575(ynfB) | | 1658383 | 1658724 |
| JW1576(speG) | | 1658759 | 1659319 |
| JW1577(ynfC) | Complementary strand | 1659322 | 1660068 |
| JW1578(ynfD) | | 1660098 | 1660445 |
| JW1579(ynfE) | | 1660644 | 1663070 |
| JW1580(ynfF) | | 1663128 | 1665554 |
| JW1581(dmsB) | | 1665565 | 1666182 |
| JW1582(dmsC) | | 1665944 | 1667038 |
| JW1583(ynfI) | | 1667072 | 1667695 |
| JW1584(yadQ) | | 1667854 | 1669146 |
| JW1585(bioD) | Complementary strand | 1669099 | 1669806 |
| JW1586(mlc) | Complementary strand | 1669919 | 1671139 |
| JW1587(ynfL) | Complementary strand | 1671274 | 1672167 |
| JW1588(ynfM) | | 1672274 | 1673527 |
| JW1589(asr) | | 1673924 | 1674259 |
| JW1590(311#13) | | 1674535 | 1675356 |
| JW1591(312#0.1) | Complementary strand | 1675395 | 1675724 |
| JW1592(ebr) | Complementary strand | 1675711 | 1676076 |
| JW1593(yhhT) | | 1676488 | 1677522 |
| JW1594(pntB) | Complementary strand | 1677547 | 1678935 |
| JW1595(pntA) | Complementary strand | 1678946 | 1680478 |
| JW1596(33#2) | | 1681002 | 1681946 |
| JW1597(arcD) | | 1682132 | 1683514 |
| JW1598(ydgB) | | 1683551 | 1684273 |
| JW1599(ydgC) | Complementary strand | 1684270 | 1684605 |
| JW1600(rstA) | | 1684725 | 1685453 |
| JW1601(rstB) | | 1685457 | 1686758 |
| JW1602(tus) | | 1686834 | 1687763 |
| JW1603(fumC) | Complementary strand | 1687760 | 1689163 |
| JW1604(fumA) | Complementary strand | 1689306 | 1690952 |
| JW1605(manA) | | 1691151 | 1692326 |
| JW1606(ydgA) | | 1692427 | 1693935 |
| JW1607(uidC) | Complementary strand | 1694161 | 1695426 |
| JW1608(uidB) | Complementary strand | 1695465 | 1696838 |
| JW1609(uidA) | Complementary strand | 1696835 | 1698646 |
| JW1610(uidR) | Complementary strand | 1699037 | 1699627 |
| JW1611(hdhA) | Complementary strand | 1699848 | 1700615 |
| JW1612(malI) | Complementary strand | 1700727 | 1701755 |
| JW1613(malX) | | 1701930 | 1703522 |
| JW1614(malY) | | 1703532 | 1704704 |
| JW1615(add) | | 1704808 | 1705809 |
| JW1616(yhhX) | Complementary strand | 1705843 | 1706922 |
| JW1616.1(blr) | | 1707126 | 1707251 |
| JW1617(ydgT) | | 1707524 | 1707739 |
| JW1618(ydgK) | | 1707825 | 1708265 |
| JW1619(ydgQ) | | 1708342 | 1708923 |
| JW1620(ydgM) | | 1708923 | 1709501 |
| JW1621(316#6) | | 1709494 | 1711716 |
| JW1622(ydgO) | | 1711717 | 1712775 |
| JW1623(ydgP) | | 1712779 | 1713399 |
| JW1624(ydgQ) | | 1713403 | 1714098 |
| JW1625(nth) | | 1714098 | 1714733 |
| JW1626(ydgR) | | 1715344 | 1716846 |
| JW1627(gst) | | 1716952 | 1717557 |
| JW1628(pdxY) | Complementary strand | 1717601 | 1718464 |
| JW1629(tyrS) | Complementary strand | 1718523 | 1719797 |
| JW1630(pdxH) | Complementary strand | 1719926 | 1720582 |
| JW1631 (ydhA) | Complementary strand | 1720641 | 1720970 |
| JW1632(ydhH) | Complementary strand | 1721068 | 1722177 |
| JW1633(slyB) | | 1722451 | 1722918 |
| JW1634(slyA) | Complementary strand | 1722965 | 1723405 |
| JW1635(ydhI) | | 1723600 | 1723836 |
| JW1636(yhcQ) | | 1723839 | 1724696 |
| JW1637(yhcP) | | 1724696 | 1726708 |
| JW1638(sodC) | Complementary strand | 1726709 | 1727230 |
| JW1639(ydhF) | Complementary strand | 1727311 | 1728207 |
| JW1640(ydhL) | Complementary strand | 1728256 | 1728666 |
| JW1641(ydhM) | | 1728682 | 1729197 |
| JW1642(nemA) | | 1729234 | 1730331 |
| JW1643(gloA) | | 1730412 | 1730819 |
| JW1644(rnt) | | 1730922 | 1731569 |
| JW1645(lhr) | | 1731662 | 1736278 |
| JW1646(ydhD) | Complementary strand | 1736329 | 1736676 |
| JW1647(ydhO) | | 1737079 | 1737825 |
| JW1648(sodB) | | 1737953 | 1738534 |
| JW1649(araJ) | Complementary strand | 1738696 | 1739865 |
| JW1650(purR) | | 1740419 | 1741444 |
| JW1651(ydhB) | Complementary strand | 1741441 | 1742373 |
| JW1652(ydhC) | | 1742486 | 1743697 |
| JW1653(cfa) | | 1743988 | 1745136 |
| JW1654(ribE) | Complementary strand | 1745176 | 1745817 |
| JW1655(ydhE) | | 1746032 | 1747405 |
| JW1656(ag43) | Complementary strand | 1747446 | 1748702 |
| JW1657(319#9) | | 1749275 | 1749580 |
| JW1658(319#10) | | 1749706 | 1751310 |
| JW1659(319#11) | Complementary strand | 1751322 | 1752134 |
| JW1660(phsC) | Complementary strand | 1752138 | 1752923 |
| JW1661(nrfC) | Complementary strand | 1752920 | 1753639 |
| JW1662(319#14) | Complementary strand | 1753652 | 1754299 |
| JW1663(ydhV) | Complementary strand | 1754303 | 1756489 |
| JW1664(319#16) | Complementary strand | 1756426 | 1757052 |
| JW1665(319#17) | Complementary strand | 1757507 | 1757716 |
| JW1666(pykF) | | 1758273 | 1759685 |
| JW1667(lpp) | | 1759996 | 1760232 |
| JW1668(ynhG) | Complementary strand | 1760296 | 1761300 |
| JW1669(ynhA) | Complementary strand | 1761449 | 1761865 |
| JW1670(sufS) | Complementary strand | 1761878 | 1763098 |
| JW1671(ynhC) | Complementary strand | 1763095 | 1764366 |
| JW1672(ynhD) | Complementary strand | 1764341 | 1765087 |
| JW1673(ynhE) | Complementary strand | 1765097 | 1766623 |
| JW1674(ydiC) | Complementary strand | 1766593 | 1766961 |
| JW1675(ydiH) | Complementary strand | 1767509 | 1767778 |
| JW1676(ybdB) | Complementary strand | 1767797 | 1768207 |
| JW1677(ydiJ) | Complementary strand | 1768204 | 1771260 |
| JW1678(320#10) | | 1771649 | 1772761 |
| JW1679(ydiL) | | 1773190 | 1773546 |
| JW1680(ydiM) | | 1773646 | 1774860 |
| JW1681 (ydiN) | | 1775087 | 1776352 |
| JW1682(ydiB) | | 1776364 | 1777230 |
| JW1683(aroD) | | 1777261 | 1778019 |
| JW1684(ydiF) | | 1778162 | 1779757 |
| JW1685(ydiO) | | 1779717 | 1780922 |
| JW1686(321#8) | Complementary strand | 1780965 | 1781876 |
| JW1687(ydiQ) | | 1782093 | 1782956 |
| JW1688(ydiR) | | 1782976 | 1783914 |
| JW1689(ydiS) | | 1783970 | 1785259 |
| JW 1690(ydiT) | | 1785256 | 1785549 |
| JW1691(ydiD) | | 1785552 | 1787252 |
| JW1692(ppsA) | Complementary strand | 1787309 | 1789687 |
| JW1693(ydiA) | | 1790020 | 1790853 |
| JW1694(aroH) | | 1791010 | 1792056 |
| JW1695(ydiE) | | 1792188 | 1792379 |
| JW1696(ydiU) | Complementary strand | 1792383 | 1793819 |
| JW1697(323#1) | Complementary strand | 1793882 | 1794595 |
| JW1698(nlpC) | Complementary strand | 1794842 | 1795306 |
| JW1699(btuD) | Complementary strand | 1795384 | 1796133 |
| JW1700(btuE) | Complementary strand | 1796133 | 1796684 |
| JW1701(btuC) | Complementary strand | 1796747 | 1797727 |
| JW1702(himA) | Complementary strand | 1797828 | 1798127 |
| JW1703(pheT) | Complementary strand | 1798132 | 1800519 |
| JW1704(pheS) | Complementary strand | 1800534 | 1801529 |
| JW1705(pheM) | Complementary strand | 1801801 | 1801845 |
| JW1706(rplT) | Complementary strand | 1801968 | 1802324 |
| JW1707(rpmI) | Complementary strand | 1802377 | 1802574 |
| JW1708(infC) | Complementary strand | 1802671 | 1803105 |
| JW1709(thrS) | Complementary strand | 1803217 | 1805145 |
| JW1710(arp) | | 1806153 | 1807568 |
| JW1711(324#3) | Complementary strand | 1807900 | 1808658 |
| JW1712(pfkB) | | 1808942 | 1809874 |
| JW1713(ydiZ) | | 1809975 | 1810265 |
| JW1714(325#1) | | 1810371 | 1811231 |
| JW1715(yniB) | Complementary strand | 1811272 | 1811808 |
| JW1716(yniC) | | 1811955 | 1812623 |
| JW1717(325#4) | | 1812786 | 1813376 |
| JW1718(325#5) | | 1813509 | 1814900 |
| JW1719(ydjO) | Complementary strand | 1814904 | 1815719 |
| JW1720(cedA) | Complementary strand | 1815996 | 1816259 |
| JW1721 (katE) | | 1816442 | 1818703 |
| JW1722(ydjC) | Complementary strand | 1818961 | 1819710 |
| JW1723(celF) | Complementary strand | 1819723 | 1821075 |
| JW1724(celD) | Complementary strand | 1821180 | 1822022 |
| JW1725(celC) | Complementary strand | 1822030 | 1822380 |
| JW1726(celB) | Complementary strand | 1822431 | 1823789 |
| JW1727(celA) | Complementary strand | 1823874 | 1824194 |
| JW1728(osmE) | Complementary strand | 1824493 | 1824831 |
| JW1729(nadE) | | 1825033 | 1825860 |
| JW1730(327#4) | | 1826090 | 1826977 |
| JW1731(327#5) | Complementary strand | 1826937 | 1827512 |
| JW1732(spy) | Complementary strand | 1827715 | 1828200 |
| JW1733(astE) | Complementary strand | 1828530 | 1829498 |
| JW1734(astB) | Complementary strand | 1829491 | 1830834 |
| JW1735(astD) | Complementary strand | 1830831 | 1832366 |
| JW1736(astA) | Complementary strand | 1832306 | 1833340 |
| JW1737(cstC) | Complementary strand | 1833337 | 1834557 |
| JW1738(xthA) | | 1835003 | 1835809 |
| JW1739(yqeD) | | 1835976 | 1836686 |
| JW1740(ydjY) | | 1836529 | 1837368 |
| JW1741(ydjZ) | | 1837383 | 1838090 |
| JW1742(ynjA) | | 1838090 | 1838638 |
| JW1743(328#9) | | 1838645 | 1839814 |
| JW1744(ynjC) | | 1840282 | 1841322 |
| JW1745(328#11) | | 1841235 | 1841975 |
| JW1746(ynjE) | | 1842027 | 1843349 |
| JW1747(328#13) | Complementary strand | 1843358 | 1843978 |
| JW1748(328#14) | | 1844065 | 1844472 |
| JW1749(328#15) | Complementary strand | 1844438 | 1844710 |
| JW1750(gdhA) | | 1844946 | 1846289 |
| JW1751(329#1) | Complementary strand | 1846406 | 1847569 |
| JW1752(topB) | Complementary strand | 1847574 | 1849535 |
| JW1753(seID) | Complementary strand | 1849540 | 1850583 |
| JW1754(ydjA) | Complementary strand | 1850700 | 1851251 |
| JW1755(sppA) | | 1851412 | 1853268 |
| JW1756(ansA) | | 1853435 | 1854451 |
| JW1757(ydjB) | | 1854462 | 1855103 |
| JW1758(ydjE) | Complementary strand | 1855196 | 1856554 |
| JW1759(ydjF) | Complementary strand | 1856671 | 1857429 |
| JW1760(ydjG) | Complementary strand | 1857566 | 1858546 |
| JW1761(330#4) | Complementary strand | 1858556 | 1859524 |
| JW1762(330#5) | Complementary strand | 1859508 | 1860344 |
| JW1763(ydjJ) | Complementary strand | 1860365 | 1861408 |
| JW1764(ydjK) | Complementary strand | 1861425 | 1862732 |
| JW1765(tdh) | Complementary strand | 1862831 | 1863907 |
| JW1766(330#9) | Complementary strand | 1864277 | 1864549 |
| JW1767(yeaA) | Complementary strand | 1864591 | 1865004 |
| JW1768(gapA) | | 1865346 | 1866341 |
| JW1769(yzzQ) | | 1866425 | 1867309 |
| JW1770(331#2) | Complementary strand | 1867357 | 1868211 |
| JW1771(yeaF) | Complementary strand | 1868301 | 1869047 |
| JW1772(yeaG) | | 1869483 | 1871417 |
| JW1773(yeaH) | | 1871530 | 1872813 |
| JW1774(yeaI) | | 1872960 | 1874435 |
| JW1775(yeaJ) | | 1874436 | 1876106 |
| JW1776(yeaK) | | 1876149 | 1876652 |
| JW1777(332#0.1) | Complementary strand | 1876653 | 1876787 |
| JW1778(yeaL) | | 1876927 | 1877373 |
| JW1779(yeaM) | Complementary strand | 1877330 | 1878151 |
| JW1780(yeaN) | | 1878248 | 1879429 |
| JW1781(yeaO) | | 1879484 | 1879831 |
| JW1782(yoaF) | Complementary strand | 1879853 | 1880107 |
| JW1783(332#6) | | 1880161 | 1881315 |
| JW1784(333#1) | Complementary strand | 1881582 | 1881830 |
| JW1785(yoaG) | Complementary strand | 1881978 | 1882160 |
| JW1786(yeaR) | Complementary strand | 1882164 | 1882523 |
| JW1787(yeaS) | Complementary strand | 1882696 | 1883334 |
| JW1788(yeaT) | Complementary strand | 1883461 | 1884384 |
| JW1789(ttuC) | | 1884487 | 1885572 |
| JW1790(caiT) | | 1885769 | 1887208 |
| JW1791 (yeaW) | | 1887120 | 1888364 |
| JW1792(yeaX) | | 1888420 | 1889385 |
| JW1793(rnd) | Complementary strand | 1889439 | 1890566 |
| JW1794(fadD) | Complementary strand | 1890636 | 1892321 |
| JW1795(slp) | Complementary strand | 1892526 | 1893107 |
| JW1796(yeaZ) | Complementary strand | 1893147 | 1893842 |
| JW1797(yoaA) | Complementary strand | 1893900 | 1895810 |
| JW1798(yoaB) | | 1895939 | 1896286 |
| JW1799(334#5.1) | | 1896684 | 1897007 |
| JW1800(yoaH) | Complementary strand | 1897127 | 1897306 |
| JW1801(pabB) | | 1897380 | 1898741 |
| JW1802(yeaB) | | 1898745 | 1899323 |
| JW1803(sdaA) | | 1899507 | 1900871 |
| JW1804(rtn) | | 1901002 | 1902600 |
| JW1805(yoaE) | Complementary strand | 1902604 | 1904160 |
| JW1806(manX) | | 1904623 | 1905594 |
| JW1807(manY) | | 1905657 | 1906457 |
| JW1808(manZ) | | 1906461 | 1907321 |
| JW1809(335#5) | | 1907376 | 1907834 |
| JW1810(yebN) | | 1908209 | 1908829 |
| JW1811 (rrmA) | Complementary strand | 1908826 | 1909635 |
| JW1812(cspC) | Complementary strand | 1909801 | 1910010 |
| JW1813(335#9.1) | Complementary strand | 1910023 | 1910166 |
| JW1814(335#10) | Complementary strand | 1910836 | 1911123 |
| JW1815(335#10.1) | Complementary strand | 1911198 | 1911341 |
| JW1816(kdgR) | Complementary strand | 1911883 | 1912674 |
| JW1817(tcmA) | | 1912740 | 1914224 |
| JW1818(htpX) | Complementary strand | 1914270 | 1915151 |
| JW1819(prc) | Complementary strand | 1915343 | 1917391 |
| JW1820(yebJ) | Complementary strand | 1917411 | 1917530 |
| JW1821(336#2) | Complementary strand | 1918206 | 1918757 |
| JW1822(yebS) | | 1918833 | 1920116 |
| JW1823(pqiB) | | 1920085 | 1922718 |
| JW1824(yebU) | | 1922792 | 1924237 |
| JW1825(336#6) | | 1924340 | 1924591 |
| JW1826(336#7) | | 1924612 | 1924887 |
| JW1827(pphA) | Complementary strand | 1924888 | 1925544 |
| JW1828(336#9) | Complementary strand | 1925940 | 1926281 |
| JW1829(336#10) | Complementary strand | 1926294 | 1927166 |
| JW1830(336#11) | Complementary strand | 1927170 | 1927544 |
| JW1831(holE) | | 1927683 | 1927913 |
| JW1832(336#13) | | 1928015 | 1928671 |
| JW1833(exoX) | | 1928695 | 1929357 |
| JW1834(ptrB) | Complementary strand | 1929354 | 1931414 |
| JW1835(yebE) | Complementary strand | 1931623 | 1932282 |
| JW1836(yebF) | Complementary strand | 1932609 | 1932977 |
| JW1837(yebG) | Complementary strand | 1933032 | 1933322 |
| JW1838(purT) | | 1933456 | 1934634 |
| JW1839(eda) | Complementary strand | 1934690 | 1935331 |
| JW1840(edd) | Complementary strand | 1935368 | 1937179 |
| JW1841(zwf) | Complementary strand | 1937414 | 1938889 |
| JW1842(yebK) | | 1939227 | 1940096 |
| JW1843(pykA) | | 1940224 | 1941666 |
| JW1844(mlt) | Complementary strand | 1941797 | 1942768 |
| JW1845(yebA) | Complementary strand | 1942888 | 1944147 |
| JW1846(znuA) | Complementary strand | 1944226 | 1945212 |
| JW1847(znuC) | | 1945237 | 1945992 |
| JW1848(yebI) | | 1945989 | 1946774 |
| JW1849(ruvB) | Complementary strand | 1946921 | 1947931 |
| JW1850(ruvA) | Complementary strand | 1947940 | 1948551 |
| JW1851(yebB) | | 1948727 | 1949428 |
| JW1852(ruvC) | Complementary strand | 1949430 | 1949951 |
| JW1853(yebC) | Complementary strand | 1949986 | 1950726 |
| JW1854(ntpA) | Complementary strand | 1950755 | 1951207 |
| JW1855(aspS) | Complementary strand | 1951325 | 1953097 |
| JW1856(yecD) | | 1953374 | 1953973 |
| JW1857(yecE) | | 1953970 | 1954788 |
| JW1858(yecN) | | 1954682 | 1955236 |
| JW1859(yecO) | | 1955277 | 1956020 |
| JW1860(yecP) | | 1956017 | 1956988 |
| JW1861(bisZ) | Complementary strand | 1957153 | 1959582 |
| JW1862(torY) | Complementary strand | 1959607 | 1960707 |
| JW1863(cutC) | Complementary strand | 1961095 | 1961841 |
| JW1864(yecL) | Complementary strand | 1961855 | 1961989 |
| JW1865(argS) | | 1962637 | 1964370 |
| JW1866(yecT) | | 1964526 | 1965035 |
| JW1867(flhE) | Complementary strand | 1965155 | 1965547 |
| JW1868(flhA) | Complementary strand | 1965547 | 1967625 |
| JW1869(flhB) | Complementary strand | 1967618 | 1968766 |
| JW1870(cheZ) | Complementary strand | 1968968 | 1969612 |
| JW1871(cheY) | Complementary strand | 1969623 | 1970012 |
| JW1872(cheB) | Complementary strand | 1970027 | 1971076 |
| JW1873(cheR) | Complementary strand | 1971079 | 1971939 |
| JW1874(tap) | Complementary strand | 1971958 | 1973559 |
| JW1875(tar) | Complementary strand | 1973605 | 1975266 |
| JW1876(cheW) | Complementary strand | 1975411 | 1975914 |
| JW1877(cheA) | Complementary strand | 1975935 | 1977899 |
| JW1878(motB) | Complementary strand | 1977904 | 1978830 |
| JW1879(motA) | Complementary strand | 1978827 | 1979714 |
| JW1880(flhC) | Complementary strand | 1979841 | 1980419 |
| JW1881(flhD) | Complementary strand | 1980422 | 1980781 |
| JW1882(340#3.1) | Complementary strand | 1981216 | 1982232 |
| JW1883(340#3.2) | | 1981552 | 1981920 |
| JW1884(yecG) | | 1982751 | 1983179 |
| JW1885(otsA) | Complementary strand | 1983186 | 1984616 |
| JW1886(otsB) | Complementary strand | 1984585 | 1985385 |
| JW1887(araH) | Complementary strand | 1985552 | 1986541 |
| JW1888(araG) | Complementary strand | 1986553 | 1988067 |
| JW1889(araF) | Complementary strand | 1988137 | 1989126 |
| JW1890(yecI) | | 1989923 | 1990426 |
| JW1891(yecJ) | Complementary strand | 1990505 | 1990756 |
| JW1892(341#5) | | 1991220 | 1991543 |
| JW1893(ftn) | | 1991714 | 1992211 |
| JW1894(yecH) | Complementary strand | 1992249 | 1992488 |
| JW1895(tyrP) | | 1992679 | 1993890 |
| JW1896(yecA) | Complementary strand | 1993952 | 1994617 |
| JW1897(pgsA) | Complementary strand | 1995267 | 1995815 |
| JW1898(uvrC) | Complementary strand | 1995872 | 1997704 |
| JW1899(uvrY) | Complementary strand | 1997701 | 1998357 |
| JW1900(yecF) | | 1998816 | 1999040 |
| JW1901(sdiA) | Complementary strand | 1999108 | 1999830 |
| JW1902(yecC) | Complementary strand | 2000060 | 2000812 |
| JW1903(yecS) | Complementary strand | 2000809 | 2001477 |
| JW1904(yedO) | Complementary strand | 2001492 | 2002574 |
| JW1905(fliY) | Complementary strand | 2002583 | 2003383 |
| JW1906(fliZ) | Complementary strand | 2003471 | 2004022 |
| JW1907(fliA) | Complementary strand | 2004068 | 2004787 |
| JW1908(fliC) | Complementary strand | 2005108 | 2006604 |
| JW1909(fliD) | | 2006870 | 2008276 |
| JW1910(fliS) | | 2008301 | 2008711 |
| JW1911(fliT) | | 2008711 | 2009076 |
| JW1912(amyA) | | 2009154 | 2010641 |
| JW1913(yedD) | Complementary strand | 2010675 | 2011088 |
| JW1914(yedE) | | 2011275 | 2012480 |
| JW1915(yedF) | | 2012477 | 2012710 |
| JW1916(yedK) | | 2012819 | 2013487 |
| JW1917(yedL) | | 2013598 | 2014077 |
| JW1918(yedN) | Complementary strand | 2014346 | 2014537 |
| JW1919(yedN) | Complementary strand | 2014547 | 2014867 |
| JW1920(yedM) | Complementary strand | 2014999 | 2015349 |
| JW1921(fliE) | Complementary strand | 2015698 | 2016012 |
| JW1922(fliF) | | 2016227 | 2017885 |
| JW1923(fliG) | | 2017878 | 2018873 |
| JW1924(fliH) | | 2018866 | 2019552 |
| JW1925(fliI) | | 2019552 | 2020925 |
| JW1926(fliJ) | | 2020944 | 2021387 |
| JW1927(fliK) | | 2021384 | 2022511 |
| JW1928(fliL) | | 2022616 | 2023080 |
| JW1929(fliM) | | 2023085 | 2024089 |
| JW1930(fliN) | | 2024086 | 2024499 |
| JW1931(fliO) | | 2024562 | 2024867 |
| JW1932(fliP) | | 2024867 | 2025604 |
| JW1933(fliQ) | | 2025614 | 2025883 |
| JW1934(fliR) | | 2025891 | 2026676 |
| JW1935(rcsA) | | 2026966 | 2027589 |
| JW1936(dsrB) | Complementary strand | 2027633 | 2027821 |
| JW1937(344#6) | | 2027981 | 2028211 |
| JW1938(yedP) | | 2028509 | 2029324 |
| JW1939(344#8) | Complementary strand | 2029321 | 2031015 |
| JW1940(344#9) | Complementary strand | 2031186 | 2031368 |
| JW1941(yedI) | Complementary strand | 2031447 | 2032364 |
| JW1942(yedA) | | 2032537 | 2033457 |
| JW1943(vsr) | Complementary strand | 2033446 | 2033916 |
| JW1944(dcm) | Complementary strand | 2033897 | 2035315 |
| JW1945(yedJ) | Complementary strand | 2035382 | 2036077 |
| JW1946(344#15) | Complementary strand | 2036117 | 2036482 |
| JW1947(ompC) | | 2037019 | 2037534 |
| JW1948(344#17) | | 2037544 | 2037753 |
| JW1949(yedS) | | 2037837 | 2038241 |
| JW1950(yedU) | | 2038833 | 2039684 |
| JW1951(yedV) | Complementary strand | 2039792 | 2041150 |
| JW1952(copR) | Complementary strand | 2041150 | 2041932 |
| JW1953(yedX) | | 2041954 | 2042367 |
| JW1954(345#1) | | 2042476 | 2043480 |
| JW1955(345#2) | | 2043481 | 2044116 |
| JW1956(345#3) | | 2044373 | 2045023 |
| JW1957(yodB) | | 2045342 | 2045896 |
| JW1958(345#5) | | 2046649 | 2047446 |
| JW1959(eaeH) | | 2047909 | 2055012 |
| JW1960(347#0.1) | Complementary strand | 2055274 | 2055441 |
| JW1961(347#1) | Complementary strand | 2055622 | 2056326 |
| JW1962(shiA) | | 2056641 | 2057957 |
| JW1963(amn) | | 2058059 | 2059513 |
| JW1964(347#4) | | 2059856 | 2060572 |
| JW1965(yeeO) | Complementary strand | 2061201 | 2062844 |
| JW1966(cbl) | Complementary strand | 2062962 | 2063912 |
| JW1967(nac) | Complementary strand | 2064014 | 2064931 |
| JW1968(erfK) | Complementary strand | 2065389 | 2066321 |
| JW1969(cobT) | Complementary strand | 2066386 | 2067465 |
| JW1970(cobS) | Complementary strand | 2067477 | 2068220 |
| JW1971 (cobU) | Complementary strand | 2068217 | 2068762 |
| JW1972(is5B) | Complementary strand | 2069303 | 2070319 |
| JW1973(yi51) | | 2069711 | 2070007 |
| JW1974(348#3) | | 2071621 | 2072025 |
| JW1975(yi22) | Complementary strand | 2071950 | 2072855 |
| JW1976(yi24) | | 2072379 | 2072810 |
| JW1977(yi23) | Complementary strand | 2072489 | 2072782 |
| JW1978(yi2A) | Complementary strand | 2072813 | 2073223 |
| JW1979(348#3.5) | | 2073135 | 2073296 |
| JW1980(348#4) | | 2073242 | 2073502 |
| JW1981(yeeP) | | 2073499 | 2074209 |
| JW1982(flu) | | 2074537 | 2077656 |
| JW1983(349#2) | | 2077777 | 2079309 |
| JW1984(yeeS) | | 2079306 | 2079752 |
| JW1985(yeeT) | | 2079815 | 2080036 |
| JW1986(yeeU) | | 2080110 | 2080478 |
| JW1987(yeeV) | | 2080567 | 2080941 |
| JW1988(yeeW) | | 2080938 | 2081132 |
| JW1989(yeeX) | Complementary strand | 2082030 | 2082425 |
| JW1990(yeeA) | Complementary strand | 2082531 | 2083589 |
| JW1991(gyrI) | Complementary strand | 2083787 | 2084260 |
| JW1992(phsE) | Complementary strand | 2084379 | 2085551 |
| JW1993(sbcB) | | 2085754 | 2087181 |
| JW1994(yeeD) | Complementary strand | 2087224 | 2087451 |
| JW1995(yeeE) | Complementary strand | 2087465 | 2088523 |
| JW1996(yeeF) | Complementary strand | 2088702 | 2090066 |
| JW1997(yeeY) | Complementary strand | 2090327 | 2091277 |
| JW1998(yeeZ) | Complementary strand | 2091302 | 2092126 |
| JW1999(yefM) | Complementary strand | 2092460 | 2092711 |
| JW2000(hisL) | | 2092994 | 2093044 |
| JW2001(hisG) | | 2093190 | 2094089 |
| JW2002(hisD) | | 2094095 | 2095399 |
| JW2003(hisC) | | 2095396 | 2096466 |
| JW2004(hisB) | | 2096466 | 2097533 |
| JW2005(hisH) | | 2097533 | 2098123 |
| JW2006(hisA) | | 2098123 | 2098860 |
| JW2007(hisF) | | 2098842 | 2099618 |
| JW2008(hisI) | | 2099612 | 2100223 |
| JW2009(wzz) | Complementary strand | 2100319 | 2101299 |
| JW2010(ugd) | Complementary strand | 2101445 | 2102611 |
| JW2011 (gnd) | Complementary strand | 2102860 | 2104266 |
| JW2012(yefJ) | Complementary strand | 2104394 | 2104675 |
| JW2013(351#10) | | 2104805 | 2104855 |
| JW2014(is5B) | Complementary strand | 2104893 | 2105909 |
| JW2015(yis51) | | 2105229 | 2105597 |
| JW2016(yefJ) | Complementary strand | 2105914 | 2106387 |
| JW2017(yefl) | Complementary strand | 2106389 | 2107507 |
| JW2018(wbbJ) | Complementary strand | 2107492 | 2108082 |
| JW2019(yefG) | Complementary strand | 2108063 | 2109055 |
| JW2020(rfc) | Complementary strand | 2109058 | 2110224 |
| JW2021(glf) | Complementary strand | 2110224 | 2111327 |
| JW2022(rfbX) | Complementary strand | 2111335 | 2112582 |
| JW2023(rfbC) | Complementary strand | 2112579 | 2113136 |
| JW2024(rfbA) | Complementary strand | 2113136 | 2114017 |
| JW2025(rfbD) | Complementary strand | 2114075 | 2114974 |
| JW2026(rfbB) | Complementary strand | 2114974 | 2116059 |
| JW2027(galF) | Complementary strand | 2116432 | 2117325 |
| JW2028(wcaM) | Complementary strand | 2117500 | 2118894 |
| JW2029(wcaL) | Complementary strand | 2118905 | 2120125 |
| JW2030(wcaK) | Complementary strand | 2120122 | 2121402 |
| JW2031(wzxC) | Complementary strand | 2121678 | 2123156 |
| JW2032(wcaJ) | Complementary strand | 2123158 | 2124552 |
| JW2033(cpsG) | Complementary strand | 2124607 | 2125977 |
| JW2034(cpsB) | Complementary strand | 2126082 | 2127518 |
| JW2035(wcaI) | Complementary strand | 2127521 | 2128744 |
| JW2036(wcaH) | Complementary strand | 2128741 | 2129223 |
| JW2037(wcaG) | Complementary strand | 2129223 | 2130188 |
| JW2038(gmd) | Complementary strand | 2130191 | 2131312 |
| JW2039(wcaF) | Complementary strand | 2131338 | 2131886 |
| JW2040(wcaE) | Complementary strand | 2131902 | 2132648 |
| JW2041 (wcaD) | Complementary strand | 2132659 | 2133876 |
| JW2042(wcaC) | Complementary strand | 2133851 | 2135068 |
| JW2043(wcaB) | Complementary strand | 2135065 | 2135553 |
| JW2044(wcaA) | Complementary strand | 2135556 | 2136395 |
| JW2045(yccC) | Complementary strand | 2136488 | 2138650 |
| JW2046(wzb) | Complementary strand | 2138653 | 2139096 |
| JW2047(wza) | Complementary strand | 2139102 | 2140241 |
| JW2048(yegH) | | 2140861 | 2142483 |
| JW2049(asmA) | Complementary strand | 2142757 | 2144610 |
| JW2050(dcd) | Complementary strand | 2144632 | 2145213 |
| JW2051 (udk) | Complementary strand | 2145305 | 2145946 |
| JW2052(yegE) | | 2146264 | 2149581 |
| JW2053(alkA) | Complementary strand | 2149690 | 2150538 |
| JW2054(yegD) | | 2150672 | 2152024 |
| JW2055(356#6) | Complementary strand | 2152037 | 2153983 |
| JW2056(356#7) | | 2154183 | 2154644 |
| JW2057(356#8) | Complementary strand | 2154709 | 2155470 |
| JW2058(356#9) | Complementary strand | 2155467 | 2156126 |
| JW2059(yegM) | | 2156894 | 2158261 |
| JW2060(yegN) | | 2158261 | 2161383 |
| JW2061(yegO) | | 2161384 | 2164461 |
| JW2062(yegB) | | 2164462 | 2165877 |
| JW2063(baeS) | | 2165874 | 2167277 |
| JW2064(baeR) | | 2167274 | 2167996 |
| JW2065(358#3) | | 2168148 | 2168519 |
| JW2066(yegQ) | | 2168666 | 2170027 |
| JW2067(ogrK) | Complementary strand | 2170300 | 2170518 |
| JW2068(358#6) | Complementary strand | 2170600 | 2170746 |
| JW2069(358#6.1) | Complementary strand | 2170987 | 2171364 |
| JW2070(yegS) | | 2171710 | 2172609 |
| JW2071(gatR). | Complementary strand | 2172691 | 2173164 |
| JW2072(358#8.1) | | 2173234 | 2173533 |
| JW2073(358#8.2) | | 2173530 | 2174396 |
| JW2074(gatR) | Complementary strand | 2174393 | 2174731 |
| JW2075(gatD) | Complementary strand | 2174831 | 2175871 |
| JW2076(gatC) | Complementary strand | 2175919 | 2177274 |
| JW2077(gatB) | Complementary strand | 2177278 | 2177562 |
| JW2078(gatA) | Complementary strand | 2177593 | 2177913 |
| JW2079(is5B) | | 2177876 | 2178892 |
| JW2080(yi51) | Complementary strand | 2178188 | 2178556 |
| JW2081 (gatA) | Complementary strand | 2178930 | 2179244 |
| JW2082(gatZ) | Complementary strand | 2179254 | 2180516 |
| JW2083(gatY) | Complementary strand | 2180545 | 2181405 |
| JW2084(fbaB) | Complementary strand | 2181707 | 2182831 |
| JW2085(xegT) | | 2183016 | 2184293 |
| JW2086(yegU) | | 2184290 | 2185294 |
| JW2087(yegV) | | 2185291 | 2186256 |
| JW2088(yegW) | Complementary strand | 2186230 | 2186976 |
| JW2089(yegX) | Complementary strand | 2187028 | 2187855 |
| JW2090(thiD) | Complementary strand | 2187911 | 2188711 |
| JW2091(thiM) | Complementary strand | 2188708 | 2189496 |
| JW2092(yohL) | Complementary strand | 2189719 | 2189991 |
| JW2093(yohM) | | 2190112 | 2190936 |
| JW2094(360#4) | | 2190975 | 2191493 |
| JW2095(yehA) | Complementary strand | 2191575 | 2192609 |
| JW2096(yehB) | Complementary strand | 2192625 | 2195105 |
| JW2097(yehC) | Complementary strand | 2195121 | 2195840 |
| JW2098(yehD) | Complementary strand | 2195875 | 2196417 |
| JW2099(yehE) | Complementary strand | 2196710 | 2196991 |
| JW2100(mrp) | Complementary strand | 2197254 | 2198363 |
| JW2101(metG) | | 2198495 | 2200528 |
| JW2102(molR) | | 2200669 | 2201493 |
| JW2103(molR) | | 2201950 | 2203542 |
| JW2104(molR) | | 2204165 | 2204464 |
| JW2105(yehI) | | 2204474 | 2208106 |
| JW2106(yehK) | | 2208167 | 2208484 |
| JW2107(yehL) | | 2208725 | 2209879 |
| JW2108(yehO) | | 2209890 | 2212169 |
| JW2109(yehP) | | 2211934 | 2213298 |
| JW2110(yehQ) | | 2213295 | 2215139 |
| JW2111(yehR) | | 2215408 | 2215881 |
| JW2112(yehS) | Complementary strand | 2215921 | 2216391 |
| JW2113(yehT) | Complementary strand | 2216438 | 2217172 |
| JW2114(yehU) | Complementary strand | 2217154 | 2218854 |
| JW2115(yehV) | | 2219061 | 2219792 |
| JW2116(yehW) | Complementary strand | 2219940 | 2220671 |
| JW2117(yehX) | Complementary strand | 2220676 | 2221602 |
| JW2118(yehY) | Complementary strand | 2221595 | 2222752 |
| JW2119(yehZ) | Complementary strand | 2222759 | 2223676 |
| JW2120(bglX) | Complementary strand | 2223887 | 2226184 |
| JW2121(dld) | | 2226380 | 2228095 |
| JW2122(pbpG) | Complementary strand | 2228133 | 2229074 |
| JW2123(yohC) | Complementary strand | 2229239 | 2229850 |
| JW2124(yohD) | | 2229996 | 2230574 |
| JW2125(yohF) | Complementary strand | 2230704 | 2231465 |
| JW2126(yohG) | Complementary strand | 2231518 | 2232714 |
| JW2127(yohH) | Complementary strand | 2232744 | 2233034 |
| JW2128(yohI) | Complementary strand | 2233633 | 2234580 |
| JW2129(yohJ) | | 2234819 | 2235217 |
| JW2130(yohK) | | 2235214 | 2235909 |
| JW2131 (cdd) | | 2236039 | 2236923 |
| JW2132(sanA) | | 2237073 | 2237792 |
| JW2133(gltD) | | 2238228 | 2239466 |
| JW2134(yeiA) | | 2239460 | 2240695 |
| JW2135(mglC) | Complementary strand | 2240938 | 2241948 |
| JW2136(mglA) | Complementary strand | 2241964 | 2243484 |
| JW2137(mglB) | Complementary strand | 2243545 | 2244543 |
| JW2138(galS) | Complementary strand | 2244823 | 2245863 |
| JW2139(yeiB) | Complementary strand | 2246005 | 2247162 |
| JW2140(folE) | Complementary strand | 2247179 | 2247847 |
| JW2141(yeiG) | | 2248105 | 2248941 |
| JW2142(cirA) | Complementary strand | 2248973 | 2250964 |
| JW2143(lysP) | Complementary strand | 2251258 | 2252727 |
| JW2144(yeiE) | Complementary strand | 2252932 | 2253813 |
| JW2145(yeiH) | | 2253912 | 2254961 |
| JW2146(nfo) | | 2255035 | 2255892 |
| JW2147(yeil) | | 2255895 | 2256983 |
| JW2148(yeiJ) | Complementary strand | 2257090 | 2258340 |
| JW2149(yeiK) | Complementary strand | 2258440 | 2259381 |
| JW2150(yeiL) | | 2259550 | 2260209 |
| JW2151 (yeiM) | Complementary strand | 2260280 | 2261530 |
| JW2152(yeiN) | Complementary strand | 2261624 | 2262562 |
| JW2153(yeiC) | Complementary strand | 2262550 | 2263491 |
| JW2154(fruA) | Complementary strand | 2263914 | 2265605 |
| JW2155(fruK) | Complementary strand | 2265622 | 2266560 |
| JW2156(fruB) | Complementary strand | 2266560 | 2267690 |
| JW2157(yeiO) | | 2268058 | 2269239 |
| JW2158(369#3.1) | Complementary strand | 2269236 | 2269622 |
| JW2159(yeiP) | | 2269684 | 2270217 |
| JW2160(yeiQ) | | 2270440 | 2271906 |
| JW2161(yeiR) | | 2272024 | 2273010 |
| JW2162(yeiU) | | 2273049 | 2273762 |
| JW2163(spr) | | 2274174 | 2274740 |
| JW2164(rtn) | | 2274921 | 2276477 |
| JW2165(yejA) | | 2276559 | 2278373 |
| JW2166(yejB) | | 2278374 | 2279468 |
| JW2167(yejE) | | 2279468 | 2280493 |
| JW2168(yejF) | | 2280495 | 2282084 |
| JW2169(yejG) | Complementary strand | 2282088 | 2282432 |
| JW2170(bcr) | Complementary strand | 2282765 | 2283898 |
| JW2171 (rsuA) | Complementary strand | 2283983 | 2284678 |
| JW2172(yejH) | | 2284827 | 2286587 |
| JW2173(rplY) | | 2286712 | 2286996 |
| JW2174(yejK) | Complementary strand | 2287135 | 2288142 |
| JW2175(yejL) | | 2288324 | 2288551 |
| JW2176(yejM) | | 2288571 | 2290331 |
| JW2177(yejO) | Complementary strand | 2290585 | 2292807 |
| JW2178(371#9) | | 2293100 | 2293222 |
| JW2179(is5B) | Complementary strand | 2293260 | 2294276 |
| JW2180(yi51) | | 2293596 | 2293964 |
| JW2181(narP) | | 2294695 | 2295342 |
| JW2182(ccmH) | Complementary strand | 2295553 | 2296605 |
| JW2183(dsbE) | Complementary strand | 2296602 | 2297159 |
| JW2184(ccmF) | Complementary strand | 2297156 | 2299099 |
| JW2185(ccmE) | Complementary strand | 2299096 | 2299575 |
| JW2186(ccmD) | Complementary strand | 2299572 | 2299781 |
| JW2187(ccmC) | Complementary strand | 2299778 | 2300515 |
| JW2188(ccmB) | Complementary strand | 2300557 | 2301219 |
| JW2189(ccmA) | Complementary strand | 2301216 | 2301833 |
| JW2190(napC) | Complementary strand | 2301852 | 2302454 |
| JW2191(napB) | Complementary strand | 2302464 | 2302934 |
| JW2192(napH) | Complementary strand | 2302910 | 2303773 |
| JW2193(napG) | Complementary strand | 2303760 | 2304455 |
| JW2194(napA) | Complementary strand | 2304462 | 2306948 |
| JW2195(napD) | Complementary strand | 2306945 | 2307208 |
| JW2196(napF) | Complementary strand | 2307198 | 2307692 |
| JW2197(eco) | | 2308100 | 2308588 |
| JW2198(yojH) | Complementary strand | 2309303 | 2310949 |
| JW2199(yojI) | Complementary strand | 2311167 | 2312810 |
| JW2200(alkB) | Complementary strand | 2312886 | 2313536 |
| JW2201(ada) | Complementary strand | 2313536 | 2314600 |
| JW2202(apbE) | Complementary strand | 2314674 | 2315276 |
| JW2203(ompC) | Complementary strand | 2315841 | 2316944 |
| JW2204(yojN) | | 2317683 | 2320355 |
| JW2205(rcsB) | | 2320372 | 2321022 |
| JW2206(rcsC) | Complementary strand | 2321222 | 2321599 |
| JW2207(374#2.1) | | 2321622 | 2321741 |
| JW2208(yi22) | Complementary strand | 2321666 | 2322571 |
| JW2209(yi24) | | 2322095 | 2322526 |
| JW2210(yi23) | Complementary strand | 2322205 | 2322498 |
| JW2211(yi2A) | Complementary strand | 2322529 | 2322939 |
| JW2212(rcsC) | Complementary strand | 2322936 | 2325359 |
| JW2213(atoS) | | 2325574 | 2327400 |
| JW2214(atoC) | | 2327397 | 2328782 |
| JW2215(atoD) | | 2328978 | 2329640 |
| JW2216(atoA) | | 2329640 | 2330290 |
| JW2217(atoE) | | 2330287 | 2331609 |
| JW2218(atoB) | | 2331640 | 2332824 |
| JW2219(376#1) | Complementary strand | 2332898 | 2333674 |
| JW2220(376#2) | Complementary strand | 2333679 | 2335328 |
| JW2221(376#3) | Complementary strand | 2335329 | 2335814 |
| JW2222(376#4) | Complementary strand | 2335830 | 2339933 |
| JW2223(376#5) | Complementary strand | 2339867 | 2340490 |
| JW2224(yfaA) | Complementary strand | 2340487 | 2342175 |
| JW2225(gyrA) | Complementary strand | 2342324 | 2344951 |
| JW2226(ubiG) | | 2345098 | 2345820 |
| JW2227(yfaL) | Complementary strand | 2345948 | 2349700 |
| JW2228(nrdA) | | 2350396 | 2352681 |
| JW2229(nrdB) | | 2352915 | 2354045 |
| JW2230(yfaE) | | 2354045 | 2354299 |
| JW2231(inaA) | Complementary strand | 2354353 | 2355003 |
| JW2232(yfaH) | | 2355218 | 2355424 |
| JW2233(glpQ) | Complementary strand | 2355466 | 2356542 |
| JW2234(glpT) | Complementary strand | 2356547 | 2357905 |
| JW2235(glpA) | | 2358178 | 2359806 |
| JW2236(glpB) | | 2359796 | 2361055 |
| JW2237(glpC) | | 2361052 | 2362242 |
| JW2238(yfaD) | | 2362435 | 2363334 |
| JW2238.5(379#2) | | 2363347 | 2363532 |
| JW2239(yhaF) | Complementary strand | 2363573 | 2364376 |
| JW2240(379#4) | Complementary strand | 2364394 | 2365683 |
| JW2241(379#5) | Complementary strand | 2365740 | 2366957 |
| JW2242(yjhI) | Complementary strand | 2366960 | 2367742 |
| JW2243(cinA) | Complementary strand | 2367962 | 2369164 |
| JW2244(379#8) | Complementary strand | 2369264 | 2369773 |
| JW2245(yfaO) | | 2370085 | 2370510 |
| JW2246(ais) | Complementary strand | 2370549 | 2371151 |
| JW2247(ybfE) | | 2371426 | 2372598 |
| JW2248(yfibF) | | 2372602 | 2373570 |
| JW2249(yfibG) | | 2373570 | 2375552 |
| JW2250(380#2) | | 2375549 | 2376439 |
| JW2251(380#3) | | 2376439 | 2378091 |
| JW2252(380#4) | | 2378088 | 2378423 |
| JW2253(380#5) | | 2378141 | 2378809 |
| JW2254(pmrD) | Complementary strand | 2378803 | 2379069 |
| JW2255(menE) | Complementary strand | 2379179 | 2380534 |
| JW2256(menC) | Complementary strand | 2380531 | 2381493 |
| JW2257(menB) | Complementary strand | 2381493 | 2382350 |
| JW2258(yfbB) | Complementary strand | 2382365 | 2383123 |
| JW2259(menD) | Complementary strand | 2383120 | 2384994 |
| JW2260(menF) | Complementary strand | 2384879 | 2386174 |
| JW2261(elaB) | Complementary strand | 2386253 | 2386558 |
| JW2262(elaA) | Complementary strand | 2386613 | 2387074 |
| JW2263(elaC) | | 2387139 | 2388056 |
| JW2264(elaD) | | 2388247 | 2389455 |
| JW2265(yfbK) | Complementary strand | 2389526 | 2391253 |
| JW2266(yfbL) | | 2391391 | 2392362 |
| JW2267(yfbM) | | 2392465 | 2392968 |
| JW2268(yfbN) | Complementary strand | 2393241 | 2393957 |
| JW2269(403#0.1) | | 2394112 | 2394588 |
| JW2270(403#1) | | 2394644 | 2395495 |
| JW2271(nuoN) | Complementary strand | 2395579 | 2396856 |
| JW2272(nuoM) | Complementary strand | 2397043 | 2398572 |
| JW2273(nuoL) | Complementary strand | 2398736 | 2400577 |
| JW2274(nuoK) | Complementary strand | 2400574 | 2400876 |
| JW2275(nuoJ) | Complementary strand | 2400873 | 2401427 |
| JW2276(nuoI) | Complementary strand | 2401439 | 2401981 |
| JW2277(nuoH) | Complementary strand | 2401996 | 2402973 |
| JW2278(nuoG) | Complementary strand | 2402970 | 2405702 |
| JW2279(nuoF) | Complementary strand | 2405749 | 2407086 |
| JW2280(nuoE) | Complementary strand | 2407083 | 2407583 |
| JW2281(nuoD) | Complementary strand | 2407586 | 2408809 |
| JW2282(nuoB) | Complementary strand | 2409482 | 2410144 |
| JW2283(nuoA) | Complementary strand | 2410160 | 2410603 |
| JW2284(lrhA) | Complementary strand | 2411234 | 2412172 |
| JW2285(insB) | Complementary strand | 2412334 | 2412837 |
| JW2286(insA) | Complementary strand | 2412756 | 2413031 |
| JW2287(yfbQ) | | 2413868 | 2415085 |
| JW2288(404#8) | | 2415169 | 2415768 |
| JW2289(yfhS) | Complementary strand | 2415827 | 2417659 |
| JW2290(yfbT) | Complementary strand | 2417746 | 2418414 |
| JW2291(yfbU) | Complementary strand | 2418407 | 2418901 |
| JW2292(405#4) | Complementary strand | 2418984 | 2419439 |
| JW2293(ackA) | | 2419777 | 2420979 |
| JW2294(pta) | | 2421054 | 2423198 |
| JW2295(yfcC) | | 2423388 | 2424908 |
| JW2296(406#2) | Complementary strand | 2424941 | 2425483 |
| JW2297(yfcE) | Complementary strand | 2425541 | 2426092 |
| JW2298(yfcF) | Complementary strand | 2426148 | 2426792 |
| JW2299(yfcG) | | 2426928 | 2427575 |
| JW2300(folX) | | 2427632 | 2427994 |
| JW2301(406#7) | | 2428015 | 2428908 |
| JW2302(yfcl) | Complementary strand | 2428956 | 2429846 |
| JW2303(hisP) | Complementary strand | 2430043 | 2430816 |
| JW2304(hisM) | Complementary strand | 2430824 | 2431540 |
| JW2305(hisQ) | Complementary strand | 2431537 | 2432223 |
| JW2306(hisJ) | Complementary strand | 2432313 | 2433095 |
| JW2307(argT) | Complementary strand | 2433316 | 2434098 |
| JW2308(ubiX) | Complementary strand | 2434364 | 2434933 |
| JW2309(purF) | Complementary strand | 2435028 | 2436545 |
| JW2310(dedE) | Complementary strand | 2436582 | 2437070 |
| JW2311 (dedD) | Complementary strand | 2437329 | 2437964 |
| JW2312(folC) | Complementary strand | 2437981 | 2439249 |
| JW2313(accD) | Complementary strand | 2439319 | 2440233 |
| JW2314(dedA) | Complementary strand | 2440389 | 2441048 |
| JW2315(truA) | Complementary strand | 2441131 | 2441943 |
| JW2316(usg1) | Complementary strand | 2441943 | 2442956 |
| JW2317(pdxB) | Complementary strand | 2443022 | 2444158 |
| JW2318(div) | | 2444257 | 2445252 |
| JW2319(yhhS) | Complementary strand | 2445249 | 2446427 |
| JW2320(fabB) | Complementary strand | 2446692 | 2447912 |
| JW2321(ddpX) | | 2448011 | 2450077 |
| JW2322(408#3) | Complementary strand | 2450198 | 2450476 |
| JW2323(yfcM) | Complementary strand | 2450510 | 2451244 |
| JW2324(yfcA) | Complementary strand | 2451058 | 2451867 |
| JW2325(mepA) | Complementary strand | 2451867 | 2452691 |
| JW2326(aroC) | Complementary strand | 2452695 | 2453780 |
| JW2327(yfcB) | Complementary strand | 2453815 | 2455080 |
| JW2328(yfcN) | | 2454913 | 2455464 |
| JW2329(409#4) | Complementary strand | 2455535 | 2456356 |
| JW2330(409#5) | Complementary strand | 2456358 | 2456897 |
| JW2331(409#6) | Complementary strand | 2456894 | 2457382 |
| JW2332(409#7) | Complementary strand | 2457379 | 2457891 |
| JW2333(yfcS) | Complementary strand | 2457891 | 2458643 |
| JW2334(yfcU) | Complementary strand | 2458663 | 2459559 |
| JW2335(yfcU) | Complementary strand | 2459572 | 2461308 |
| JW2336(409#10) | Complementary strand | 2461390 | 2461953 |
| JW2337(sixA) | Complementary strand | 2462634 | 2463119 |
| JW2338(yfcX) | Complementary strand | 2463322 | 2465466 |
| JW2339(yfcY) | Complementary strand | 2465466 | 2466776 |
| JW2340(yfcZ) | Complementary strand | 2466957 | 2467241 |
| JW2341(fadL) | | 2467607 | 2468953 |
| JW2342(410#5.1) | | 2469319 | 2470377 |
| JW2343(vacJ) | Complementary strand | 2470559 | 2471314 |
| JW2344(yfdC) | | 2471608 | 2472540 |
| JW2345(yfdB) | | 2472852 | 2474009 |
| JW2346(411#1) | | 2474162 | 2474524 |
| JW2347(yfdH) | | 2474521 | 2475441 |
| JW2348(411#2.1) | Complementary strand | 2475431 | 2475565 |
| JW2349(ycfA) | | 2477068 | 2477412 |
| JW2350(yfdK) | Complementary strand | 2477384 | 2477824 |
| JW2351(yfdL) | Complementary strand | 2477851 | 2478543 |
| JW2352(yfdM) | Complementary strand | 2478419 | 2478694 |
| JW2353(xyIU) | | 2478738 | 2478923 |
| JW2354(yfdN) | Complementary strand | 2478694 | 2479182 |
| JW2355(yfdO) | Complementary strand | 2479185 | 2479553 |
| JW2356(411#10) | | 2479827 | 2480273 |
| JW2357(411#11) | | 2480339 | 2481163 |
| JW2358(411#12) | | 2481291 | 2481827 |
| JW2359(411#13) | | 2481818 | 2482180 |
| JW2360(411#14) | | 2482180 | 2482485 |
| JW2361(dsdC) | Complementary strand | 2483001 | 2483936 |
| JW2362(dsdX) | | 2484154 | 2485491 |
| JW2363(dsdA) | | 2485509 | 2486837 |
| JW2364(emrY) | Complementary strand | 2486945 | 2488483 |
| JW2365(emrK) | Complementary strand | 2488483 | 2489646 |
| JW2366(evgA) | | 2490062 | 2490676 |
| JW2367(evgS) | | 2490681 | 2494274 |
| JW2368(bifF) | Complementary strand | 2494330 | 2495475 |
| JW2369(413#2.1) | Complementary strand | 2495549 | 2496493 |
| JW2370(yfdU) | Complementary strand | 2496563 | 2498257 |
| JW2371(414#1) | Complementary strand | 2498311 | 2499561 |
| JW2372(414#2) | Complementary strand | 2500074 | 2500709 |
| JW2373(ypdI) | | 2501005 | 2501280 |
| JW2374(414#3) | Complementary strand | 2501357 | 2501599 |
| JW2375(ddg) | | 2501952 | 2502872 |
| JW2376(yfdZ) | Complementary strand | 2503364 | 2504602 |
| JW2377(yehU) | | 2504996 | 2506675 |
| JW2378(414#7) | | 2506690 | 2507424 |
| JW2379(yijO) | | 2507437 | 2508294 |
| JW2380(ptsA) | Complementary strand | 2508297 | 2510792 |
| JW2381(frvX) | Complementary strand | 2510817 | 2511854 |
| JW2382(yqhT) | Complementary strand | 2511854 | 2512939 |
| JW2383(ptwC) | Complementary strand | 2512954 | 2514201 |
| JW2384(ptwB) | Complementary strand | 2514223 | 2514600 |
| JW2385(glk) | Complementary strand | 2514768 | 2515733 |
| JW2386(416#3) | | 2515937 | 2517193 |
| JW2387(416#4) | | 2517308 | 2517634 |
| JW2388(mntH) | Complementary strand | 2517775 | 2519013 |
| JW2389(nupC) | | 2519349 | 2520551 |
| JW2390(yi81) | | 2520638 | 2521750 |
| JW2391 (yi82) | Complementary strand | 2521062 | 2521370 |
| JW2392(yffA) | Complementary strand | 2521950 | 2524199 |
| JW2393(yfeC) | | 2524774 | 2525118 |
| JW2394(yfeD) | | 2525120 | 2525512 |
| JW2395(gltX) | Complementary strand | 2525564 | 2526979 |
| JW2396(xapR) | Complementary strand | 2527900 | 2528784 |
| JW2397(xapB) | Complementary strand | 2529036 | 2530292 |
| JW2398(xapA) | Complementary strand | 2530352 | 2531185 |
| JW2399(yfeN) | | 2531434 | 2532198 |
| JW2400(yfeR) | Complementary strand | 2532237 | 2533163 |
| JW2401(yfeH) | | 2533253 | 2534299 |
| JW2402(417#10) | Complementary strand | 2534318 | 2534557 |
| JW2403(lig) | Complementary strand | 2534466 | 2536481 |
| JW2404(zipA) | Complementary strand | 2536552 | 2537538 |
| JW2405(417#12.1) | | 2537070 | 2537312 |
| JW2406(cysZ) | | 2537769 | 2538530 |
| JW2407(cysK) | | 2538715 | 2539686 |
| JW2408(ptsH) | | 2540070 | 2540327 |
| JW2409(ptsI) | | 2540372 | 2542099 |
| JW2410(gsr) | | 2542140 | 2542649 |
| JW2411 (pdxK) | Complementary strand | 2542692 | 2543543 |
| JW2412(yfeK) | | 2543648 | 2544022 |
| JW2413(419#2) | | 2544055 | 2544789 |
| JW2414(cysM) | Complementary strand | 2544978 | 2545889 |
| JW2415(cysA) | Complementary strand | 2546023 | 2547120 |
| JW2416(cysW) | Complementary strand | 2547110 | 2547985 |
| JW2417(cysU) | Complementary strand | 2547985 | 2548818 |
| JW2418(cysP) | Complementary strand | 2548818 | 2549834 |
| JW2419(ucpA) | Complementary strand | 2550138 | 2550995 |
| JW2420(yfeT) | Complementary strand | 2551058 | 2551915 |
| JW2421 (yfeU) | | 2552079 | 2552975 |
| JW2422(ptbA) | | 2552979 | 2554403 |
| JW2423(yfeW) | | 2554321 | 2555712 |
| JW2424(420#4) | Complementary strand | 2555952 | 2556851 |
| JW2425(420#5) | Complementary strand | 2556947 | 2557522 |
| JW2426(yfeZ) | Complementary strand | 2557583 | 2558032 |
| JW2427(420#7) | Complementary strand | 2558019 | 2558444 |
| JW2428(amiA) | | 2558658 | 2559527 |
| JW2429(hemF) | | 2559531 | 2560430 |
| JW2430(yfeG) | Complementary strand | 2560436 | 2561488 |
| JW2431 (cchA) | Complementary strand | 2561534 | 2562034 |
| JW2432(421 #4) | Complementary strand | 2562047 | 2562706 |
| JW2433(eutC) | Complementary strand | 2562716 | 2563603 |
| JW2434(eutB) | Complementary strand | 2563624 | 2564985 |
| JW2435(421 #7) | Complementary strand | 2564997 | 2566400 |
| JW2436(eutH) | Complementary strand | 2566397 | 2567623 |
| JW2437(eutG) | Complementary strand | 2567840 | 2569027 |
| JW2438(eutJ) | Complementary strand | 2569017 | 2569853 |
| JW2439(eutE) | Complementary strand | 2569864 | 2571267 |
| JW2440(cchB) | Complementary strand | 2571279 | 2571566 |
| JW2441(cchA) | Complementary strand | 2571673 | 2571966 |
| JW2442(eutI) | Complementary strand | 2572005 | 2573021 |
| JW2443(421#15) | Complementary strand | 2573018 | 2573821 |
| JW2444(421#16) | Complementary strand | 2573818 | 2574519 |
| JW2445(421#17) | Complementary strand | 2574494 | 2574973 |
| JW2446(ypfE) | Complementary strand | 2574986 | 2575321 |
| JW2447(421#19) | Complementary strand | 2575614 | 2577893 |
| JW2448(talA) | | 2578182 | 2579132 |
| JW2449(tktB) | | 2579152 | 2581155 |
| JW2450(ypfG) | Complementary strand | 2581250 | 2582293 |
| JW2451(yffH) | Complementary strand | 2582419 | 2582994 |
| JW2452(aegA) | Complementary strand | 2583062 | 2585041 |
| JW2453(narQ) | | 2585247 | 2586947 |
| JW2454(acrD) | | 2587111 | 2590224 |
| JW2455(yffB) | | 2590763 | 2591119 |
| JW2456(dapE) | | 2591123 | 2592250 |
| JW2457(423#3) | | 2592278 | 2592478 |
| JW2458(ypfH) | Complementary strand | 2592588 | 2593310 |
| JW2459(ypfI) | Complementary strand | 2593360 | 2595375 |
| JW2460(423#6) | Complementary strand | 2595390 | 2596253 |
| JW2461(purC) | Complementary strand | 2596421 | 2597134 |
| JW2462(nlpB) | Complementary strand | 2597347 | 2598381 |
| JW2463(dapA) | Complementary strand | 2598398 | 2599276 |
| JW2464(gcvR) | | 2599422 | 2599994 |
| JW2465(bcp) | | 2599994 | 2600464 |
| JW2466(hyfA) | | 2600717 | 2601334 |
| JW2467(hyfB) | | 2601334 | 2603352 |
| JW2468(hyfC) | | 2603363 | 2604310 |
| JW2469(hyfD) | | 2604327 | 2605766 |
| JW2470(hyfE) | | 2605778 | 2606428 |
| JW2471 (hyFF) | | 2606433 | 2608013 |
| JW2472(hyfG) | | 2608003 | 2609670 |
| JW2473(hyfH) | | 2609680 | 2610225 |
| JW2474(hyfI) | | 2610222 | 2610977 |
| JW2475(hyfJ) | | 2610974 | 2611387 |
| JW2476(hyfR) | | 2611417 | 2613429 |
| JW2477(focB) | | 2613451 | 2614299 |
| JW2478(perm) | Complementary strand | 2614337 | 2615398 |
| JW2479(426#2.1) | | 2615611 | 2617074 |
| JW2480(yfgD) | | 2617095 | 2617454 |
| JW2481(yfgE) | Complementary strand | 2617592 | 2618185 |
| JW2482(uraA) | Complementary strand | 2618388 | 2619677 |
| JW2483(upp) | Complementary strand | 2619763 | 2620389 |
| JW2484(purM) | | 2620714 | 2621751 |
| JW2485(purN) | | 2621751 | 2622389 |
| JW2486(ppk) | | 2622561 | 2624627 |
| JW2487(ppx) | | 2624632 | 2626173 |
| JW2488(427#1) | Complementary strand | 2626212 | 2628455 |
| JW2489(427#2) | | 2629312 | 2629827 |
| JW2490(427#3) | | 2629843 | 2630382 |
| JW2491(guaA) | Complementary strand | 2630475 | 2632052 |
| JW2492(guaB) | Complementary strand | 2632121 | 2633656 |
| JW2493(xseA) | | 2633749 | 2635119 |
| JW2494(yfgJ) | Complementary strand | 2635116 | 2635367 |
| JW2495(yfgK) | Complementary strand | 2635401 | 2636912 |
| JW2496(yfgL) | Complementary strand | 2636991 | 2638169 |
| JW2497(yfgM) | Complementary strand | 2638180 | 2638800 |
| JW2498(hisS) | Complementary strand | 2638818 | 2640092 |
| JW2499(gcpE) | Complementary strand | 2640203 | 2641321 |
| JW2500(yfgA) | Complementary strand | 2641348 | 2642361 |
| JW2501(yfgB) | Complementary strand | 2642646 | 2643800 |
| JW2502(ndk) | Complementary strand | 2643950 | 2644381 |
| JW2503(pbpC) | Complementary strand | 2644530 | 2646842 |
| JW2504(429#5) | Complementary strand | 2646843 | 2651804 |
| JW2505(sseA) | | 2652011 | 2652856 |
| JW2506(sseB) | Complementary strand | 2653674 | 2654315 |
| JW2507(pepB) | Complementary strand | 2654592 | 2655875 |
| JW2508(yfhJ) | Complementary strand | 2656053 | 2656253 |
| JW2509(fdx) | Complementary strand | 2656265 | 2656600 |
| JW2510(hscA) | Complementary strand | 2656602 | 2658452 |
| JW2511(hscB) | Complementary strand | 2658469 | 2658984 |
| JW2512(yfhF) | Complementary strand | 2659080 | 2659403 |
| JW2513(iscU) | Complementary strand | 2659420 | 2659806 |
| JW2514(iscS) | Complementary strand | 2659834 | 2661048 |
| JW2515(iscR) | Complementary strand | 2661160 | 2661648 |
| JW2516(yfhQ) | Complementary strand | 2662100 | 2662840 |
| JW2517(suhB) | | 2662959 | 2663762 |
| JW2518(431#2) | | 2663880 | 2664761 |
| JW2519(csiE) | | 2664952 | 2666343 |
| JW2520(yfhS) | Complementary strand | 2666225 | 2667364 |
| JW2521(yfhT) | Complementary strand | 2667524 | 2668414 |
| JW2522(dgxA) | | 2668550 | 2669911 |
| JW2523(dgxB) | | 2669908 | 2670426 |
| JW2524(hcaC) | | 2670426 | 2670746 |
| JW2525(hcaB) | | 2670743 | 2671555 |
| JW2526(hcaD) | | 2671565 | 2672767 |
| JW2527(yphA) | | 2672792 | 2673286 |
| JW2528(yphB) | Complementary strand | 2673334 | 2674206 |
| JW2529(yphC) | Complementary strand | 2674218 | 2675312 |
| JW2530(yphD) | Complementary strand | 2675345 | 2676343 |
| JW2531(yphE) | Complementary strand | 2676368 | 2677879 |
| JW2532(yphF) | Complementary strand | 2677902 | 2678885 |
| JW2533(yphG) | Complementary strand | 2678982 | 2682356 |
| JW2534(yphH) | | 2682375 | 2683574 |
| JW2535(glyA) | Complementary strand | 2683772 | 2685025 |
| JW2536(hmpA) | | 2685353 | 2686543 |
| JW2537(glnB) | Complementary strand | 2686588 | 2686926 |
| JW2538(yfhA) | Complementary strand | 2686987 | 2688321 |
| JW2539(yfhG) | Complementary strand | 2688311 | 2689024 |
| JW2540(yfhK) | Complementary strand | 2689189 | 2690571 |
| JW2541 (purL) | Complementary strand | 2691174 | 2695061 |
| JW2542(yfhD) | | 2695457 | 2696875 |
| JW2543(yfhC) | Complementary strand | 2696872 | 2697408 |
| JW2544(yfhB) | Complementary strand | 2697433 | 2698005 |
| JW2545(yfhH) | | 2698277 | 2699125 |
| JW2546(yfhL) | | 2699181 | 2699441 |
| JW2547(acpS) | Complementary strand | 2700136 | 2700516 |
| JW2548(pdxJ) | Complementary strand | 2700516 | 2701247 |
| JW2549(recO) | Complementary strand | 2701259 | 2701987 |
| JW2550(era) | Complementary strand | 2701999 | 2702904 |
| JW2551(rnc) | Complementary strand | 2702901 | 2703581 |
| JW2552(lepB) | Complementary strand | 2703853 | 2704827 |
| JW2553(lepA) | Complementary strand | 2704843 | 2706642 |
| JW2554(rseC) | Complementary strand | 2706840 | 2707319 |
| JW2555(rseB) | Complementary strand | 2707316 | 2708272 |
| JW2556(rseA) | Complementary strand | 2708272 | 2708922 |
| JW2557(rpoE) | Complementary strand | 2708955 | 2709530 |
| JW2558(nadB) | | 2709938 | 2711560 |
| JW2559(yfiC) | Complementary strand | 2711545 | 2712282 |
| JW2560(srmB) | | 2712414 | 2713748 |
| JW2561(yfiE) | Complementary strand | 2713957 | 2714838 |
| JW2562(yfiK) | | 2714941 | 2715528 |
| JW2563(yfiD) | Complementary strand | 2715584 | 2715967 |
| JW2564(ung) | | 2716272 | 2716961 |
| JW2565(yfiF) | Complementary strand | 2717009 | 2718046 |
| JW2566(trx2) | | 2718253 | 2718672 |
| JW2567(yfiP) | | 2718717 | 2719439 |
| JW2568(yfiQ) | | 2719471 | 2722131 |
| JW2569(pssA) | | 2722245 | 2723600 |
| JW2570(yfiM) | | 2723697 | 2723969 |
| JW2571(kgtP) | Complementary strand | 2723966 | 2725264 |
| JW2572(437#4) | | 2725443 | 2725505 |
| JW2573(clpB) | Complementary strand | 2731118 | 2733691 |
| JW2574(437#6.1) | | 2733777 | 2733875 |
| JW2575(yfiH) | Complementary strand | 2733821 | 2734552 |
| JW2576(rluD) | Complementary strand | 2734549 | 2735529 |
| JW2577(yfiO) | | 2735664 | 2736401 |
| JW2578(yfiA) | | 2736672 | 2737013 |
| JW2579(pheL) | | 2737117 | 2737164 |
| JW2580(pheA) | | 2737263 | 2738423 |
| JW2581 (tyrA) | Complementary strand | 2738466 | 2739587 |
| JW2582(aroF) | Complementary strand | 2739598 | 2740668 |
| JW2583(yfiL) | | 2740839 | 2741243 |
| JW2584(yfiR) | | 2741393 | 2741911 |
| JW2585(yfiN) | | 2741901 | 2743127 |
| JW2586(yfiB) | | 2743143 | 2743625 |
| JW2587(rpIS) | Complementary strand | 2743701 | 2744048 |
| JW2588(trmD) | Complementary strand | 2744090 | 2744857 |
| JW2589(rimM) | Complementary strand | 2744888 | 2745445 |
| JW2590(rpsP) | Complementary strand | 2745455 | 2745703 |
| JW2591(ffh) | Complementary strand | 2745952 | 2747058 |
| JW2592(439#12) | | 2747405 | 2748271 |
| JW2593(yfjD) | | 2748337 | 2749578 |
| JW2594(grpE) | Complementary strand | 2749633 | 2750226 |
| JW2595(439#15) | Complementary strand | 2750223 | 2750396 |
| JW2596(yfB) | | 2750349 | 2751227 |
| JW2597(recN) | | 2751313 | 2752974 |
| JW2598(smpA) | | 2753123 | 2753464 |
| JW2599(yfjF) | Complementary strand | 2753526 | 2753816 |
| JW2600(yfjG) | Complementary strand | 2753806 | 2754282 |
| JW2601 (smpB) | | 2754414 | 2754896 |
| JW2602(intA) | | 2755677 | 2756918 |
| JW2603(yfjH) | Complementary strand | 2757162 | 2758118 |
| JW2604(alpA) | | 2758162 | 2758374 |
| JW2605(yfjI) | | 2758503 | 2759912 |
| JW2606(440#5.1) | Complementary strand | 2759865 | 2760071 |
| JW2607(yfjJ) | | 2760065 | 2760691 |
| JW2608(yfjK) | Complementary strand | 2760869 | 2763058 |
| JW2609(yfjL) | Complementary strand | 2763055 | 2764671 |
| JW2610(yfjM) | Complementary strand | 2765031 | 2765294 |
| JW2611(yfjN) | | 2765436 | 2766509 |
| JW2612(yfjO) | | 2766553 | 2766873 |
| JW2613(yfjP) | | 2767222 | 2768091 |
| JW2614(yfjQ) | | 2768183 | 2769004 |
| JW2615(yfjR) | | 2769221 | 2769922 |
| JW2616(ypjK) | | 2769807 | 2770199 |
| JW2617(yfjS) | | 2769950 | 2770642 |
| JW2618(yfjT) | | 2770666 | 2771133 |
| JW2619(yfjU) | Complementary strand | 2771358 | 2771672 |
| JW2620(yfjV) | Complementary strand | 2771685 | 2772278 |
| JW2621 (yfjV) | Complementary strand | 2772354 | 2772554 |
| JW2622(442#2.2) | Complementary strand | 2772494 | 2772610 |
| JW2623(yfjW) | | 2772836 | 2774539 |
| JW2623.5(442#4) | | 2775237 | 2775440 |
| JW2624(yfjX) | | 2775437 | 2775895 |
| JW2625(yfjY) | | 2775904 | 2776386 |
| JW2626(yfjZ) | | 2776633 | 2776950 |
| JW2627(ypjF) | | 2776971 | 2777300 |
| JW2628(ypjA) | Complementary strand | 2777664 | 2782373 |
| JW2629(443#1.1) | Complementary strand | 2783156 | 2783947 |
| JW2630(443#1.2) | Complementary strand | 2784047 | 2784445 |
| JW2631(443#2) | | 2786266 | 2786952 |
| JW2632(443#2.1) | | 2787343 | 2787756 |
| JW2633(443#2.2) | | 2787895 | 2788167 |
| JW2634(443#3) | | 2788398 | 2789480 |
| JW2635(ygaF) | | 2789500 | 2790768 |
| JW2636(gabD) | | 2790791 | 2792239 |
| JW2637(gabT) | | 2792253 | 2793533 |
| JW2638(gabP) | | 2793771 | 2795171 |
| JW2639(ygaE) | | 2795192 | 2795854 |
| JW2640(yzzM) | Complementary strand | 2795855 | 2796304 |
| JW2641(yqaE) | Complementary strand | 2796388 | 2796546 |
| JW2642(445#2) | | 2796729 | 2797028 |
| JW2643(ygaP) | | 2797038 | 2797562 |
| JW2644(stpA) | Complementary strand | 2797609 | 2798013 |
| JW2645(445#5) | | 2798682 | 2799131 |
| JW2646(ygaC) | Complementary strand | 2799168 | 2799512 |
| JW2647(ygaM) | | 2799652 | 2799993 |
| JW2648(nrdH) | | 2800241 | 2800486 |
| JW2649(nrdI) | | 2800483 | 2800893 |
| JW2650(nrdE) | | 2800866 | 2803010 |
| JW2651(nrdF) | | 2803020 | 2803979 |
| JW2652(proV) | | 2804333 | 2805535 |
| JW2653(proW) | | 2805528 | 2806592 |
| JW2654(proX) | | 2806650 | 2807642 |
| JW2655(445#15) | | 2807834 | 2808100 |
| JW2656(445#17) | | 2808094 | 2809011 |
| JW2657(ygaZ) | | 2809135 | 2809872 |
| JW2658(ygaH) | | 2809862 | 2810197 |
| JW2659(emrR) | | 2810288 | 2810818 |
| JW2660(emrA) | | 2810945 | 2812117 |
| JW2661 (emrB) | | 2812134 | 2813672 |
| JW2662(ygaG) | Complementary strand | 2813736 | 2814251 |
| JW2663(gshA) | Complementary strand | 2814401 | 2815957 |
| JW2664(yqaA) | Complementary strand | 2816030 | 2816458 |
| JW2665(yqaB) | Complementary strand | 2816455 | 2817021 |
| JW2666(csrA) | Complementary strand | 2818479 | 2818664 |
| JW2667(alaS) | Complementary strand | 2818899 | 2821529 |
| JW2668(oraA) | Complementary strand | 2821657 | 2822157 |
| JW2669(recA) | Complementary strand | 2822226 | 2823287 |
| JW2670(ygaD) | Complementary strand | 2823367 | 2823864 |
| JW2671(mltB) | Complementary strand | 2824009 | 2825094 |
| JW2672(srlA) | | 2825350 | 2826870 |
| JW2673(srlB) | | 2826881 | 2827252 |
| JW2674(srlD) | | 2827256 | 2828035 |
| JW2675(gutM) | | 2828141 | 2828500 |
| JW2676(srlR) | | 2828567 | 2829340 |
| JW2677(gutQ) | | 2829372 | 2830298 |
| JW2678(ygaA) | Complementary strand | 2830295 | 2830807 |
| JW2679(ygaA) | Complementary strand | 2830827 | 2831885 |
| JW2680(448#2) | | 2831997 | 2833436 |
| JW2681(ygbD) | | 2833433 | 2834566 |
| JW2682(hypF) | Complementary strand | 2834694 | 2836946 |
| JW2683(hydN) | Complementary strand | 2837099 | 2837626 |
| JW2684(ascG) | Complementary strand | 2837775 | 2838785 |
| JW2685(ascF) | | 2839045 | 2840502 |
| JW2686(ascB) | | 2840511 | 2841935 |
| JW2687(hycI) | Complementary strand | 2842094 | 2842564 |
| JW2688(hycH) | Complementary strand | 2842557 | 2842967 |
| JW2689(hycG) | Complementary strand | 2842964 | 2843731 |
| JW2690(hycF) | Complementary strand | 2843731 | 2844273 |
| JW2691 (hycE) | Complementary strand | 2844283 | 2845992 |
| JW2692(hycD) | Complementary strand | 2846010 | 2846933 |
| JW2693(hycC) | Complementary strand | 2846936 | 2848762 |
| JW2694(hycB) | Complementary strand | 2848759 | 2849370 |
| JW2695(hycA) | Complementary strand | 2849495 | 2849956 |
| JW2696(hypA) | | 2850168 | 2850518 |
| JW2697(hypB) | | 2850522 | 2851394 |
| JW2698(hypC) | | 2851385 | 2851657 |
| JW2699(hypD) | | 2851657 | 2852778 |
| JW2700(hypE) | | 2852817 | 2853785 |
| JW2701(fhlA) | | 2853859 | 2855937 |
| JW2702(ygbA) | Complementary strand | 2855974 | 2856327 |
| JW2703(mutS) | | 2856614 | 2859175 |
| JW2704(prpB) | | 2859281 | 2859937 |
| JW2705(ygbI) | Complementary strand | 2859988 | 2860785 |
| JW2706(ygbJ) | | 2860952 | 2861860 |
| JW2707(ygbK) | | 2861857 | 2863023 |
| JW2708(ygbL) | | 2863115 | 2863753 |
| JW2709(ygbM) | | 2863758 | 2864534 |
| JW2710(ygbN) | | 2864623 | 2865987 |
| JW2711(rpoS) | Complementary strand | 2866081 | 2867073 |
| JW2712(nlpD) | Complementary strand | 2867136 | 2868275 |
| JW2713(pcm) | Complementary strand | 2868415 | 2869041 |
| JW2714(surE) | Complementary strand | 2869035 | 2869796 |
| JW2715(ygbO) | Complementary strand | 2869777 | 2870826 |
| JW2716(ygbB) | Complementary strand | 2870823 | 2871302 |
| JW2717(ygbP) | Complementary strand | 2871302 | 2872012 |
| JW2718(ygbQ) | Complementary strand | 2872031 | 2872342 |
| JW2719(ygbE) | Complementary strand | 2872534 | 2872857 |
| JW2720(cysC) | Complementary strand | 2872907 | 2873512 |
| JW2721(cysN) | Complementary strand | 2873512 | 2874939 |
| JW2722(cysD) | Complementary strand | 2874941 | 2875849 |
| JW2723(iap) | | 2876101 | 2877138 |
| JW2724(ygbF) | Complementary strand | 2878089 | 2878439 |
| JW2725(452#12) | Complementary strand | 2878375 | 2879292 |
| JW2726(ygcH) | Complementary strand | 2879308 | 2879907 |
| JW2727(452#14) | Complementary strand | 2879894 | 2880568 |
| JW2728(ygcJ) | Complementary strand | 2880571 | 2881662 |
| JW2729(ygcK) | Complementary strand | 2881675 | 2882157 |
| JW2730(453#2) | Complementary strand | 2882150 | 2883658 |
| JW2731 (ygcB) | Complementary strand | 2884073 | 2886739 |
| JW2732(cysH) | Complementary strand | 2887098 | 2887832 |
| JW2733(cysI) | Complementary strand | 2887907 | 2889619 |
| JW2734(cysJ) | Complementary strand | 2889619 | 2891418 |
| JW2735(ptpS) | | 2891734 | 2892099 |
| JW2736(ygcN) | | 2892177 | 2893448 |
| JW2737(ygcO) | | 2893439 | 2893699 |
| JW2738(ygcP) | | 2893716 | 2894291 |
| JW2739(ygcQ) | Complementary strand | 2894439 | 2895332 |
| JW2740(ygcR) | Complementary strand | 2895366 | 2896076 |
| JW2741(ygcS) | Complementary strand | 2896054 | 2897463 |
| JW2742(454#9) | Complementary strand | 2897485 | 2898279 |
| JW2743(ygcU) | Complementary strand | 2898282 | 2898647 |
| JW2744(ygcU) | Complementary strand | 2898656 | 2898937 |
| JW2745(ygcW) | Complementary strand | 2899007 | 2899867 |
| JW2746(yqcE) | | 2900111 | 2901388 |
| JW2747(ygcE) | | 2901769 | 2902893 |
| JW2748(ygcF) | Complementary strand | 2904267 | 2904938 |
| JW2749(ygcG) | | 2905162 | 2906103 |
| JW2750(eno) | Complementary strand | 2906163 | 2907461 |
| JW2751(pyrG) | Complementary strand | 2907549 | 2909186 |
| JW2752(mazG) | Complementary strand | 2909414 | 2910205 |
| JW2753(chpA) | Complementary strand | 2910276 | 2910611 |
| JW2754(chpR) | Complementary strand | 2910611 | 2910859 |
| JW2755(relA) | Complementary strand | 2910937 | 2913171 |
| JW2756(ygcA) | Complementary strand | 2913219 | 2914520 |
| JW2757(barA) | | 2914577 | 2917333 |
| JW2758(ygcX) | Complementary strand | 2917565 | 2918905 |
| JW2759(gud2) | Complementary strand | 2918926 | 2920266 |
| JW2760(gudT) | Complementary strand | 2920268 | 2921620 |
| JW2761(mioC) | Complementary strand | 2922055 | 2922504 |
| JW2762(yqcB) | Complementary strand | 2922522 | 2923304 |
| JW2763(yqcC) | Complementary strand | 2923304 | 2923633 |
| JW2764(syd) | Complementary strand | 2924255 | 2924800 |
| JW2765(yqcD) | | 2924868 | 2925716 |
| JW2766(ygdH) | | 2925828 | 2927192 |
| JW2767(sdaC) | | 2927749 | 2929038 |
| JW2768(sdaB) | | 2929096 | 2930463 |
| JW2769(exo) | | 2930575 | 2931330 |
| JW2770(fucO) | Complementary strand | 2931385 | 2932536 |
| JW2771(fucA) | Complementary strand | 2932561 | 2933208 |
| JW2772(fucP) | | 2933755 | 2935071 |
| JW2773(fucl) | | 2935104 | 2936879 |
| JW2774(fucK) | | 2936958 | 2938406 |
| JW2775(fucU) | | 2938408 | 2938830 |
| JW2776(fucR) | | 2938888 | 2939619 |
| JW2777(ygdE) | Complementary strand | 2939663 | 2940763 |
| JW2778(ygdD) | Complementary strand | 2940756 | 2941151 |
| JW2779(gcvA) | Complementary strand | 2941170 | 2942087 |
| JW2780(ygdl) | Complementary strand | 2942438 | 2942668 |
| JW2781 (nifS) | | 2942857 | 2944062 |
| JW2782(ygdK) | | 2944062 | 2944505 |
| JW2783(ygdL) | Complementary strand | 2944556 | 2945362 |
| JW2784(mltA) | Complementary strand | 2945601 | 2946698 |
| JW2785(amiC) | Complementary strand | 2947277 | 2948620 |
| JW2786(argA) | | 2948762 | 2950093 |
| JW2787(recD) | Complementary strand | 2950155 | 2951981 |
| JW2788(recB) | Complementary strand | 2951981 | 2955523 |
| JW2789(ptr) | Complementary strand | 2955516 | 2958404 |
| JW2790(recC) | Complementary strand | 2958580 | 2961948 |
| JW2791 (ppdC) | Complementary strand | 2961961 | 2962284 |
| JW2792(ygdB) | Complementary strand | 2962269 | 2962634 |
| JW2793(ppdB) | Complementary strand | 2962673 | 2963476 |
| JW2794(ppdA) | Complementary strand | 2963227 | 2963697 |
| JW2795(thyA) | Complementary strand | 2963881 | 2964675 |
| JW2796(lgt) | Complementary strand | 2964682 | 2965557 |
| JW2797(ptsP) | Complementary strand | 2965708 | 2967954 |
| JW2798(ygdP) | Complementary strand | 2967967 | 2968497 |
| JW2799(mutH) | | 2969182 | 2969871 |
| JW2800(ygdQ) | | 2969940 | 2970653 |
| JW2801(ygdR) | | 2970791 | 2971009 |
| JW2802(tas) | | 2971117 | 2972157 |
| JW2803(ygeD) | Complementary strand | 2972189 | 2973382 |
| JW2804(aas) | Complementary strand | 2973375 | 2975534 |
| JW2805(galR) | | 2976119 | 2977150 |
| JW2806(lysA) | Complementary strand | 2977157 | 2978419 |
| JW2807(lysR) | | 2978541 | 2979476 |
| JW2808(ygeA) | Complementary strand | 2979463 | 2980155 |
| JW2809(araE) | Complementary strand | 2980284 | 2981702 |
| JW2810(kduD) | Complementary strand | 2982017 | 2982778 |
| JW2811 (kdul) | Complementary strand | 2982808 | 2983644 |
| JW2812(yqeF) . | Complementary strand | 2983931 | 2985115 |
| JW2813(sdaC) | | 2985367 | 2986596 |
| JW2814(yqeH) | | 2987023 | 2987688 |
| JW2815(yqeI) | | 2988022 | 2988831 |
| JW2816(yqeJ) | | 2988992 | 2989306 |
| JW2817(yqeK) | Complementary strand | 2989455 | 2989880 |
| JW2818(ygeF) | | 2990074 | 2990520 |
| JW2819(ygeG) | | 2990788 | 2991279 |
| JW2820(ygeH) | | 2991614 | 2992990 |
| JW2821(ygeI) | | 2993128 | 2993376 |
| JW2822(x) | | 2993520 | 2993936 |
| JW2823(ygeK) | Complementary strand | 2993981 | 2994427 |
| JW2824(464#6) | Complementary strand | 2994458 | 2994613 |
| JW2825(464#7) | Complementary strand | 2995269 | 2995523 |
| JW2826(yi22) | Complementary strand | 2995893 | 2996798 |
| JW2827(yi24) | | 2996322 | 2996753 |
| JW2828(yi23) | Complementary strand | 2996432 | 2996725 |
| JW2829(yi2A) | Complementary strand | 2996756 | 2997166 |
| JW2830(465#0.1) | | 2997078 | 2997245 |
| JW2831 (ygeP) | Complementary strand | 2997210 | 2997509 |
| JW2832(465#1) | Complementary strand | 2997555 | 2998391 |
| JW2833(nlpD) | Complementary strand | 2998657 | 2999412 |
| JW2834(yagR) | | 2999827 | 3002124 |
| JW2835(ygeT) | | 3002135 | 3003013 |
| JW2836(465#5) | | 3003010 | 3003489 |
| JW2837(ygeV) | Complementary strand | 3003529 | 3005307 |
| JW2838(ygeW) | | 3005854 | 3006945 |
| JW2839(dpaL) | | 3007029 | 3008225 |
| JW2840(ygeY) | | 3008283 | 3009494 |
| JW2841(ygeZ) | | 3009547 | 3010932 |
| JW2842(arcL) | | 3010980 | 3011912 |
| JW2843(yqeB) | Complementary strand | 3012133 | 3013758 |
| JW2844(466#6) | Complementary strand | 3013806 | 3014477 |
| JW2845(ygfJ) | | 3014679 | 3015257 |
| JW2846(gltD) | | 3015579 | 3018677 |
| JW2847(467#1) | | 3018680 | 3020008 |
| JW2848(ygfM) | | 3020059 | 3020838 |
| JW2849(yagR) | | 3020835 | 3023705 |
| JW2850(ygfO) | | 3023870 | 3025270 |
| JW2851(ygfP) | | 3025165 | 3026604 |
| JW2852(ygfQ) | | 3026640 | 3027209 |
| JW2853(ygfR) | | 3027176 | 3028006 |
| JW2854(ygfS) | Complementary strand | 3028042 | 3028533 |
| JW2855(ygfT) | Complementary strand | 3028530 | 3030464 |
| JW2856(pbuX) | | 3030816 | 3032333 |
| JW2857(idi) | | 3032583 | 3033131 |
| JW2858(lysS) | Complementary strand | 3033175 | 3034692 |
| JW2859(prfB) | Complementary strand | 3034702 | 3035367 |
| JW2860(recJ) | Complementary strand | 3035891 | 3037624 |
| JW2861(dsbC) | Complementary strand | 3037630 | 3038340 |
| JW2862(xerD) | Complementary strand | 3038365 | 3039261 |
| JW2863(fldB) | | 3039373 | 3039894 |
| JW2864(ygfX) | Complementary strand | 3039934 | 3040341 |
| JW2865(ygfY) | Complementary strand | 3040322 | 3040588 |
| JW2866(up14) | | 3040831 | 3041811 |
| JW2867(yqfA) | Complementary strand | 3042007 | 3042666 |
| JW2868(yqfB) | Complementary strand | 3042830 | 3043141 |
| JW2869(bglA) | | 3043180 | 3044619 |
| JW2870(ygfF) | Complementary strand | 3044676 | 3045419 |
| JW2871(gcvP) | Complementary strand | 3045686 | 3048559 |
| JW2872(gcvH) | Complementary strand | 3048678 | 3049067 |
| JW2873(gcvT) | Complementary strand | 3049091 | 3050185 |
| JW2874(visC) | Complementary strand | 3050633 | 3051835 |
| JW2875(ubiH) | Complementary strand | 3051858 | 3053036 |
| JW2876(pepP) | Complementary strand | 3053033 | 3054358 |
| JW2877(ygfB) | Complementary strand | 3054384 | 3054968 |
| JW2878(ygfE) | | 3055130 | 3055459 |
| JW2879(ygfA) | | 3055759 | 3056307 |
| JW2880(serA) | Complementary strand | 3056696 | 3057928 |
| JW2881(rpiA) | Complementary strand | 3058184 | 3058879 |
| JW2882(yqfE) | Complementary strand | 3058899 | 3059129 |
| JW2883(iciA) | | 3059271 | 3060164 |
| JW2884(sbm) | | 3060368 | 3062512 |
| JW2885(argK) | | 3062505 | 3063500 |
| JW2886(ygfG) | | 3063511 | 3064296 |
| JW2887(ygfH) | | 3064320 | 3065798 |
| JW2888(ygfI) | Complementary strand | 3065795 | 3066706 |
| JW2889(yggE) | Complementary strand | 3066858 | 3067598 |
| JW2890(yggA) | Complementary strand | 3067691 | 3068326 |
| JW2891(yggB) | Complementary strand | 3068465 | 3069325 |
| JW2892(fba) | Complementary strand | 3069683 | 3070762 |
| JW2893(pgk) | Complementary strand | 3070977 | 3072140 |
| JW2894(epd) | Complementary strand | 3072190 | 3073209 |
| JW2895(yggC) | Complementary strand | 3073494 | 3074207 |
| JW2896(yggD) | Complementary strand | 3074204 | 3074713 |
| JW2897(yggF) | Complementary strand | 3074735 | 3075700 |
| JW2898(yggP) | Complementary strand | 3075697 | 3076686 |
| JW2899(yggP) | Complementary strand | 3076617 | 3076973 |
| JW2900(cmtA) | Complementary strand | 3076988 | 3078376 |
| JW2901 (cmtB) | Complementary strand | 3078404 | 3078847 |
| JW2902(tktA) | Complementary strand | 3079161 | 3081155 |
| JW2903(yggG) | | 3081435 | 3082193 |
| JW2904(speB) | Complementary strand | 3082399 | 3083319 |
| JW2905(speA) | Complementary strand | 3083457 | 3085433 |
| JW2906(yqgB) | Complementary strand | 3085442 | 3085588 |
| JW2907(yqgC) | | 3085709 | 3085924 |
| JW2908(yqgD) | Complementary strand | 3085921 | 3086172 |
| JW2909(metK) | | 3086228 | 3087382 |
| JW2910(galP) | | 3087805 | 3089199 |
| JW2911(sprT) | | 3089276 | 3089773 |
| JW2912(endA) | | 3089868 | 3090575 |
| JW2913(yggJ) | | 3090655 | 3091386 |
| JW2914(gshB) | | 3091399 | 3092349 |
| JW2915(yqgE) | | 3092458 | 3093021 |
| JW2916(yqgF) | | 3093021 | 3093437 |
| JW2917(yggR) | Complementary strand | 3093621 | 3094601 |
| JW2918(yggS) | | 3094619 | 3095323 |
| JW2919(yggT) | | 3095341 | 3095907 |
| JW2920(yggU) | | 3095892 | 3096194 |
| JW2921(yggV) | | 3096202 | 3096795 |
| JW2922(yggW) | | 3096788 | 3097924 |
| JW2923(yggM) | Complementary strand | 3098079 | 3099086 |
| JW2924(ansB) | Complementary strand | 3099203 | 3100249 |
| JW2925(yggN) | Complementary strand | 3100425 | 3101144 |
| JW2926(yggL) | Complementary strand | 3101327 | 3101653 |
| JW2927(yggH) | Complementary strand | 3101653 | 3102372 |
| JW2928(mutY) | | 3102533 | 3103585 |
| JW2929(yggX) | | 3103613 | 3103888 |
| JW2930(mltC) | | 3103950 | 3104489 |
| JW2931(mltC) | | 3104536 | 3105033 |
| JW2932(nupG) | | 3105235 | 3106491 |
| JW2933(speC) | Complementary strand | 3106541 | 3108736 |
| JW2934(yqgA) | | 3109073 | 3109780 |
| JW2935(epsM) | Complementary strand | 3110110 | 3110646 |
| JW2936(exeL) | Complementary strand | 3110648 | 3111490 |
| JW2937(exeC) | Complementary strand | 3111574 | 3112569 |
| JW2938(477#5) | Complementary strand | 3112587 | 3112997 |
| JW2939(477#6) | Complementary strand | 3113063 | 3113872 |
| JW2940(477#7) | Complementary strand | 3114070 | 3116616 |
| JW2941(478#1) | Complementary strand | 3116604 | 3118631 |
| JW2942(yghK) | Complementary strand | 3119116 | 3120798 |
| JW2943(glcB) | Complementary strand | 3121153 | 3123324 |
| JW2944(glcG) | Complementary strand | 3123346 | 3123750 |
| JW2945(glcF) | Complementary strand | 3123755 | 3126040 |
| JW2946(glcD) | Complementary strand | 3126040 | 3127539 |
| JW2947(glcC) | | 3127790 | 3128554 |
| JW2948(502#6) | Complementary strand | 3128561 | 3129733 |
| JW2949(is5B) | | 3129696 | 3130712 |
| JW2950(yi51) | Complementary strand | 3130008 | 3130376 |
| JW2951 (yghQ) | Complementary strand | 3130859 | 3131788 |
| JW2952(yghR) | Complementary strand | 3131972 | 3132730 |
| JW2953(yghS) | Complementary strand | 3132762 | 3133460 |
| JW2954(yghT) | | 3133649 | 3134341 |
| JW2955(pitB) | Complementary strand | 3134390 | 3135889 |
| JW2956(gsp) | Complementary strand | 3136181 | 3138040 |
| JW2957(yghU) | | 3138197 | 3139111 |
| JW2958(hybG) | Complementary strand | 3139234 | 3139482 |
| JW2959(hybF) | Complementary strand | 3139519 | 3139836 |
| JW2960(hybE) | Complementary strand | 3139829 | 3140317 |
| JW2961(hybD) | Complementary strand | 3140310 | 3140804 |
| JW2962(hybC) | Complementary strand | 3140804 | 3142507 |
| JW2963(hybB) | Complementary strand | 3142504 | 3143679 |
| JW2964(hybA) | Complementary strand | 3143669 | 3144655 |
| JW2965(hoxK) | Complementary strand | 3144658 | 3145776 |
| JW2966(yghW) | Complementary strand | 3145965 | 3146252 |
| JW2967(504#11) | Complementary strand | 3146371 | 3146781 |
| JW2968(504#12) | Complementary strand | 3146781 | 3147206 |
| JW2969(504#13) | Complementary strand | 3147112 | 3147300 |
| JW2970(yajO) | | 3147412 | 3148452 |
| JW2971(yqhA) | Complementary strand | 3148492 | 3148986 |
| JW2972(yghA) | | 3149177 | 3150061 |
| JW2973(exbD) | Complementary strand | 3150333 | 3150758 |
| JW2974(exbB) | Complementary strand | 3150765 | 3151499 |
| JW2975(metC) | | 3151751 | 3152938 |
| JW2976(yghB) | | 3153078 | 3153737 |
| JW2977(exsA) | Complementary strand | 3153776 | 3154903 |
| JW2978(yqhD) | | 3154869 | 3156032 |
| JW2979(yghE) | | 3156370 | 3156963 |
| JW2980(yqhG) | | 3157163 | 3157456 |
| JW2981 (yqhG) | | 3157414 | 3158091 |
| JW2982(yqhH) | | 3158142 | 3158399 |
| JW2983(ygiQ) | Complementary strand | 3158442 | 3159683 |
| JW2984(ygiR) | Complementary strand | 3159683 | 3160660 |
| JW2985(sufI) | Complementary strand | 3160771 | 3162183 |
| JW2986(plsC) | Complementary strand | 3162258 | 3162995 |
| JW2987(parC) | Complementary strand | 3163229 | 3165487 |
| JW2988(ygiS) | Complementary strand | 3165625 | 3167232 |
| JW2989(ygiT) | Complementary strand | 3167365 | 3167760 |
| JW2990(ygiU) | Complementary strand | 3167762 | 3168058 |
| JW2991(506#5) | Complementary strand | 3168263 | 3168745 |
| JW2992(ygiW) | Complementary strand | 3168798 | 3169190 |
| JW2993(ygiX) | | 3169342 | 3170001 |
| JW2994(ygiY) | | 3169998 | 3171347 |
| JW2995(ygiZ) | Complementary strand | 3171393 | 3171725 |
| JW2996(mdaB) | | 3172045 | 3172626 |
| JW2997(ygiN) | | 3172657 | 3172971 |
| JW2998(parE) | Complementary strand | 3173019 | 3174911 |
| JW2999(yqiA) | Complementary strand | 3174940 | 3175521 |
| JW3000(icc) | Complementary strand | 3175521 | 3176348 |
| JW3001(yqiB) | Complementary strand | 3176373 | 3176795 |
| JW3002(yqiE) | Complementary strand | 3176796 | 3177425 |
| JW3003(toIC) | | 3177624 | 3179111 |
| JW3004(507#7) | | 3179111 | 3179371 |
| JW3005(ygiB) | | 3179226 | 3179930 |
| JW3006(ygiC) | | 3179936 | 3181096 |
| JW3007(ygiD) | Complementary strand | 3181134 | 3181949 |
| JW3008(ygiE) | | 3182065 | 3182838 |
| JW3009(ribB) | Complementary strand | 3183328 | 3183981 |
| JW3010(yqiC) | | 3184295 | 3184645 |
| JW3011(ygiL) | | 3184929 | 3185480 |
| JW3012(yi2A) | | 3185657 | 3186067 |
| JW3013(yi22) | | 3186025 | 3186930 |
| JW3014(yi24) | Complementary strand | 3186070 | 3186501 |
| JW3015(yi23) | | 3186098 | 3186391 |
| JW3016(yqiG) | | 3186915 | 3187859 |
| JW3017(yqiG) | | 3187684 | 3189381 |
| JW3018(yqiH) | | 3189388 | 3190146 |
| JW3019(yqil) | | 3190148 | 3190855 |
| JW3020(yqiI) | | 3190791 | 3191213 |
| JW3021(glgS) | Complementary strand | 3191256 | 3191456 |
| JW3022(508#6) | | 3191725 | 3192354 |
| JW3023(508#7) | | 3192381 | 3194042 |
| JW3024(rfaE) | Complementary strand | 3194837 | 3196270 |
| JW3025(glnE) | Complementary strand | 3196318 | 3199158 |
| JW3026(ygiF) | Complementary strand | 3199181 | 3200482 |
| JW3027(ygiM) | | 3200724 | 3201344 |
| JW3028(cca) | | 3201408 | 3202646 |
| JW3029(bacA) | Complementary strand | 3202827 | 3203648 |
| JW3030(foIB) | Complementary strand | 3203738 | 3204106 |
| JW3031 (ygiH) | | 3204211 | 3204828 |
| JW3032(ygiP) | Complementary strand | 3204841 | 3205773 |
| JW3033(ttdA) | | 3205980 | 3206891 |
| JW3034(ttdB) | | 3206888 | 3207493 |
| JW3035(ygjE) | | 3207541 | 3209004 |
| JW3036(ygjD) | Complementary strand | 3209047 | 3210060 |
| JW3037(rpsU) | | 3210298 | 3210513 |
| JW3038(dnaG) | | 3210624 | 3212369 |
| JW3039(rpoD) | | 3212564 | 3214405 |
| JW3040(ygjF) | Complementary strand | 3214484 | 3214990 |
| JW3041 (yqjH) | Complementary strand | 3215244 | 3216008 |
| JW3042(yqjI) | | 3216296 | 3216919 |
| JW3043(aer) | Complementary strand | 3217073 | 3218593 |
| JW3044(ygjG) | | 3219101 | 3220390 |
| JW3045(ygjH) | Complementary strand | 3220432 | 3220764 |
| JW3046(ebgR) | | 3220983 | 3221966 |
| JW3047(ebgA) | | 3222150 | 3225242 |
| JW3048(ebgC) | | 3225239 | 3225688 |
| JW3049(ygjI) | | 3225751 | 3227184 |
| JW3050(ygjJ) | | 3227318 | 3228388 |
| JW3051(ygjK) | | 3228405 | 3230756 |
| JW3052(fadH) | | 3231182 | 3233200 |
| JW3053(ygjM) | Complementary strand | 3233245 | 3233661 |
| JW3054(ygjN) | Complementary strand | 3233658 | 3233972 |
| JW3055(ygjO) | Complementary strand | 3234256 | 3235422 |
| JW3056(ygjP) | | 3235441 | 3235980 |
| JW3057(ygjQ) | | 3236057 | 3236749 |
| JW3058(ygjR) | | 3236810 | 3237814 |
| JW3059(ygjT) | | 3238098 | 3239063 |
| JW3060(ygjU) | | 3239460 | 3240704 |
| JW3061(ygjV) | Complementary strand | 3240709 | 3241260 |
| JW3062(uxaA) | Complementary strand | 3241343 | 3242830 |
| JW3063(uxaC) | Complementary strand | 3242845 | 3244257 |
| JW3064(exuT) | | 3244620 | 3246038 |
| JW3065(exuR) | | 3246168 | 3246944 |
| JW3066(yqjA) | | 3247289 | 3247951 |
| JW3067(yqjB) | | 3247955 | 3248338 |
| JW3068(yqjC) | | 3248470 | 3248853 |
| JW3069(yqjD) | | 3248891 | 3249196 |
| JW3070(yqjE) | | 3249199 | 3249603 |
| JW3071(yqjK) | | 3249593 | 3249892 |
| JW3072(yqjF) | | 3249988 | 3250470 |
| JW3073(yqjG) | | 3250540 | 3251526 |
| JW3074(yhaH) | | 3251820 | 3252185 |
| JW3075(yhal) | | 3252427 | 3252783 |
| JW3076(yhaJ) | Complementary strand | 3252834 | 3253730 |
| JW3077(yhaK) | | 3253835 | 3254536 |
| JW3078(yhaL) | | 3254553 | 3254723 |
| JW3079(yhaM) | Complementary strand | 3254857 | 3255423 |
| JW3080(yhaN) | Complementary strand | 3255605 | 3256168 |
| JW3081(yhaO) | Complementary strand | 3256196 | 3257473 |
| JW3082(yhaP) | Complementary strand | 3257804 | 3258631 |
| JW3083(yhaQ) | Complementary strand | 3258751 | 3259173 |
| JW3084(yhaR) | Complementary strand | 3259239 | 3259691 |
| JW3085(tdcE) | Complementary strand | 3259695 | 3261935 |
| JW3086(tdcD) | Complementary strand | 3261969 | 3263177 |
| JW3087(tdcC) | Complementary strand | 3263203 | 3264534 |
| JW3088(tdcB) | Complementary strand | 3264556 | 3265545 |
| JW3089(tdcA) | Complementary strand | 3265644 | 3266582 |
| JW3090(tdcR) | | 3266816 | 3267115 |
| JW3091(yhaB) | | 3267371 | 3267910 |
| JW3092(yhaC) | | 3267932 | 3269119 |
| JW3093(yhaD) | Complementary strand | 3270142 | 3271368 |
| JW3094(yhaE) | Complementary strand | 3271384 | 3272283 |
| JW3095(yhaF) | Complementary strand | 3272304 | 3273074 |
| JW3096(yhaU) | Complementary strand | 3273090 | 3274424 |
| JW3097(yhaG) | | 3274799 | 3276370 |
| JW3098(sohA) | | 3276519 | 3276854 |
| JW3099(yhaV) | | 3276854 | 3277318 |
| JW3100(agaR) | Complementary strand | 3277373 | 3278182 |
| JW3101(agaZ) | | 3278431 | 3279711 |
| JW3102(agaV) | | 3279734 | 3280207 |
| JW3103(agaW) | | 3280218 | 3280619 |
| JW3104(agaA) | | 3280639 | 3281142 |
| JW3105(agaS) | | 3281493 | 3282647 |
| JW3106(agaY) | | 3282660 | 3283520 |
| JW3107(agaB) | | 3283687 | 3284163 |
| JW3108(agaC) | | 3284202 | 3285005 |
| JW3109(agaD) | | 3284995 | 3285786 |
| JW3110(agaI) | | 3285787 | 3286542 |
| JW3111(yraH) | | 3286943 | 3287527 |
| JW3112(yraI) | | 3287607 | 3288302 |
| JW3113(yraJ) | | 3288331 | 3290847 |
| JW3114(yraK) | | 3290858 | 3291949 |
| JW3115(yraL) | Complementary strand | 3291992 | 3292852 |
| JW3116(yraM) | | 3292917 | 3294953 |
| JW3117(yraN) | | 3294911 | 3295306 |
| JW3118(yraO) | | 3295326 | 3295916 |
| JW3119(yraP) | | 3295926 | 3296501 |
| JW3120(yraQ) | Complementary strand | 3296615 | 3297655 |
| JW3121(yraR) | Complementary strand | 3297728 | 3298408 |
| JW3122(yhbO) | | 3298449 | 3299009 |
| JW3123(yhbP) | Complementary strand | 3298989 | 3299432 |
| JW3124(yhbQ) | | 3299483 | 3299785 |
| JW3125(yhbS) | Complementary strand | 3299772 | 3300275 |
| JW3126(yhbT) | Complementary strand | 3300269 | 3300793 |
| JW3127(yhbU) | | 3301002 | 3301997 |
| JW3128(yhbV) | | 3301988 | 3302884 |
| JW3129(yhbW) | | 3302965 | 3303972 |
| JW3130(mtr) | Complementary strand | 3304090 | 3305334 |
| JW3131(deaD) | Complementary strand | 3305488 | 3307428 |
| JW3132(nlpI) | Complementary strand | 3307557 | 3308441 |
| JW3133(pnp) | Complementary strand | 3308550 | 3310685 |
| JW3134(rpsO) | Complementary strand | 3310932 | 3311201 |
| JW3135(truB) | Complementary strand | 3311350 | 3312294 |
| JW3136(rbfA) | Complementary strand | 3312294 | 3312695 |
| JW3137(infB) | Complementary strand | 3312859 | 3315531 |
| JW3138(nusA) | Complementary strand | 3315556 | 3317043 |
| JW3139(yhbC) | Complementary strand | 3317071 | 3317493 |
| JW3140(argG) | | 3318154 | 3319497 |
| JW3141(yhbX) | Complementary strand | 3319505 | 3321148 |
| JW3142(secG) | Complementary strand | 3321690 | 3322022 |
| JW3143(mrsA) | Complementary strand | 3322250 | 3323587 |
| JW3144(foIP) | Complementary strand | 3323580 | 3324428 |
| JW3145(ftsH) | Complementary strand | 3324518 | 3326452 |
| JW3146(ftsJ) | Complementary strand | 3326552 | 3327181 |
| JW3147(yhbY) | | 3327307 | 3327600 |
| JW3148(greA) | Complementary strand | 3327756 | 3328232 |
| JW3149(dacB) | | 3328480 | 3329913 |
| JW3150(yhbZ) | Complementary strand | 3330099 | 3331271 |
| JW3151 (yhbE) | Complementary strand | 3331287 | 3332252 |
| JW3152(rpmA) | Complementary strand | 3332379 | 3332636 |
| JW3153(rpIU) | Complementary strand | 3332657 | 3332968 |
| JW3154(ispB) | | 3333227 | 3334198 |
| JW3155(nlp) | | 3334426 | 3334704 |
| JW3156(murA) | Complementary strand | 3334752 | 3336011 |
| JW3157(yrbA) | Complementary strand | 3336066 | 3336320 |
| JW3158(yrbB) | Complementary strand | 3336480 | 3336869 |
| JW3159(yrbC) | Complementary strand | 3336773 | 3337408 |
| JW3160(yrbD) | Complementary strand | 3337427 | 3337978 |
| JW3161(yrbE) | Complementary strand | 3337983 | 3338765 |
| JW3162(yrbF) | Complementary strand | 3338773 | 3339582 |
| JW3163(yrbG) | | 3339792 | 3340769 |
| JW3164(yrbH) | | 3340783 | 3341769 |
| JW3165(yrbI) | | 3341790 | 3342356 |
| JW3166(yrbK) | | 3342353 | 3342928 |
| JW3167(yhbN) | | 3342897 | 3343454 |
| JW3168(yhbG) | | 3343461 | 3344186 |
| JW3169(rpoN) | | 3344234 | 3345667 |
| JW3170(yhbH) | | 3345690 | 3345977 |
| JW3171(ptsN) | | 3346095 | 3346586 |
| JW3172(yhbJ) | | 3346632 | 3347486 |
| JW3173(ptsO) | | 3347483 | 3347755 |
| JW3174(yrbL) | | 3347969 | 3348601 |
| JW3175(mtgA) | Complementary strand | 3348598 | 3349326 |
| JW3176(yhbL) | Complementary strand | 3349323 | 3349976 |
| JW3177(arcB) | Complementary strand | 3350206 | 3352536 |
| JW3178(yhcC) | Complementary strand | 3352632 | 3353561 |
| JW3179(gltB) | | 3354143 | 3358696 |
| JW3180(gltD) | | 3358709 | 3360127 |
| JW3181(gltF) | | 3360687 | 3361451 |
| JW3182(yhcA) | | 3361623 | 3362297 |
| JW3183(yhcD) | | 3362318 | 3364699 |
| JW3184(yhcE) | | 3364696 | 3365175 |
| JW3185(isSB) | Complementary strand | 3365213 | 3366229 |
| JW3186(yi51) | | 3365549 | 3365917 |
| JW3187(yhcE) | | 3366192 | 3366440 |
| JW3188(yhcF) | | 3366437 | 3367153 |
| JW3189(yhcG) | | 3367338 | 3368465 |
| JW3190(yhcH) | Complementary strand | 3368525 | 3368989 |
| JW3191 (nanK) | Complementary strand | 3368986 | 3369894 |
| JW3192(nanE) | Complementary strand | 3369858 | 3370547 |
| JW3193(nanT) | Complementary strand | 3370595 | 3372085 |
| JW3194(npl) | Complementary strand | 3372194 | 3373087 |
| JW3195(nanR) | Complementary strand | 3373209 | 3374000 |
| JW3196(dcuD) | | 3374380 | 3375747 |
| JW3197(sspB) | Complementary strand | 3375790 | 3376287 |
| JW3198(sspA) | Complementary strand | 3376293 | 3376931 |
| JW3199(rpsl) | Complementary strand | 3377326 | 3377718 |
| JW3200(rpIM) | Complementary strand | 3377734 | 3378162 |
| JW3201 (yhcM) | Complementary strand | 3378381 | 3379508 |
| JW3202(yhcB) | | 3379696 | 3380100 |
| JW3203(hhoA) | | 3380254 | 3381621 |
| JW3204(hhoB) | | 3381711 | 3382778 |
| JW3205(mdh) | Complementary strand | 3382841 | 3383779 |
| JW3206(argR) | | 3384214 | 3384684 |
| JW3207(yhcN) | | 3384998 | 3385312 |
| JW3208(yhcO) | Complementary strand | 3385368 | 3385640 |
| JW3209(yhcP) | Complementary strand | 3385732 | 3387699 |
| JW3210(yhcQ) | Complementary strand | 3387705 | 3388637 |
| JW3211 (yhcR) | Complementary strand | 3388645 | 3388917 |
| JW3212(yhcS) | | 3389031 | 3389960 |
| JW3213(tldD) | Complementary strand | 3390094 | 3391539 |
| JW3214(yhdP) | Complementary strand | 3391970 | 3394930 |
| JW3215(yhdR) | Complementary strand | 3394923 | 3395771 |
| JW3216(rng) | Complementary strand | 3395839 | 3397308 |
| JW3217(yhdE) | Complementary strand | 3397298 | 3397891 |
| JW3218(mreD) | Complementary strand | 3397900 | 3398388 |
| JW3219(mreC) | Complementary strand | 3398388 | 3399491 |
| JW3220(mreB) | Complementary strand | 3399557 | 3400600 |
| JW3221(yhdA) | Complementary strand | 3400905 | 3402845 |
| JW3222(yhdH) | | 3402997 | 3403971 |
| JW3222.5(529#3) | | 3404788 | 3404976 |
| JW3223(accB) | | 3404949 | 3405419 |
| JW3224(accC) | | 3405430 | 3406779 |
| JW3225(yhdT) | | 3406888 | 3407130 |
| JW3226(panF) | | 3407120 | 3408571 |
| JW3227(prmA) | | 3408583 | 3409464 |
| JW3228(yhdG) | | 3409793 | 3410758 |
| JW3229(fis) | | 3410784 | 3411080 |
| JW3230(yhdJ) | | 3411160 | 3412050 |
| JW3231(yhdU) | | 3412134 | 3412313 |
| JW3232(envR) | Complementary strand | 3412316 | 3412978 |
| JW3233(acrE) | | 3413377 | 3414534 |
| JW3234(envD) | | 3414546 | 3417650 |
| JW3235(yhdV) | | 3417903 | 3418124 |
| JW3236(yhdW) | | 3418662 | 3419579 |
| JW3237(yhdX) | | 3419629 | 3420735 |
| JW3238(yhdY) | | 3420834 | 3421940 |
| JW3239(yhdZ) | | 3421948 | 3422706 |
| JW3240(yrdA) | | 3428533 | 3429303 |
| JW3241(yrdB) | Complementary strand | 3429279 | 3429536 |
| JW3242(aroE) | Complementary strand | 3429533 | 3430351 |
| JW3243(yrdC) | Complementary strand | 3430356 | 3430928 |
| JW3244(yrdD) | Complementary strand | 3430933 | 3431442 |
| JW3245(smg) | Complementary strand | 3431505 | 3431978 |
| JW3246(smf) | Complementary strand | 3431950 | 3432327 |
| JW3247(smf) | Complementary strand | 3432312 | 3433073 |
| JW3248(def) | | 3433203 | 3433712 |
| JW3249(fmt) | | 3433727 | 3434674 |
| JW3250(sun) | | 3434720 | 3436009 |
| JW3251(trkA) | | 3436031 | 3437407 |
| JW3252(mscL) | | 3437537 | 3437947 |
| JW3253(yhdL) | Complementary strand | 3437944 | 3438162 |
| JW3254(zntR) | Complementary strand | 3438218 | 3438643 |
| JW3255(yhdN) | Complementary strand | 3438654 | 3439022 |
| JW3256(rplQ) | Complementary strand | 3439129 | 3439512 |
| JW3257(rpoA) | Complementary strand | 3439553 | 3440542 |
| JW3258(rpsD) | Complementary strand | 3440568 | 3441188 |
| JW3259(rpsK) | Complementary strand | 3441222 | 3441611 |
| JW3260(rpsM) | Complementary strand | 3441628 | 3441984 |
| JW3261(rpmJ) | Complementary strand | 3442131 | 3442247 |
| JW3262(secY) | Complementary strand | 3442279 | 3443610 |
| JW3263(rplO) | Complementary strand | 3443618 | 3444052 |
| JW3264(rpmD) | Complementary strand | 3444056 | 3444235 |
| JW3265(rpsE) | Complementary strand | 3444239 | 3444742 |
| JW3266(rplR) | Complementary strand | 3444757 | 3445110 |
| JW3267(rpIF) | Complementary strand | 3445120 | 3445653 |
| JW3268(rpsH) | Complementary strand | 3445666 | 3446058 |
| JW3269(rpsN) | Complementary strand | 3446092 | 3446397 |
| JW3270(rplE) | Complementary strand | 3446412 | 3446951 |
| JW3271(rpIX) | Complementary strand | 3446966 | 3447280 |
| JW3272(rpIN) | Complementary strand | 3447291 | 3447662 |
| JW3273(rpsQ) | Complementary strand | 3447827 | 3448081 |
| JW3274(rpmC) | Complementary strand | 3448081 | 3448272 |
| JW3275(rpIP) | Complementary strand | 3448272 | 3448682 |
| JW3276(rpsC) | Complementary strand | 3448695 | 3449396 |
| JW3277(rpIV) | Complementary strand | 3449414 | 3449746 |
| JW3278(rpsS) | Complementary strand | 3449761 | 3450039 |
| JW3279(rpIB) | Complementary strand | 3450056 | 3450877 |
| JW3280(rpIW) | Complementary strand | 3450895 | 3451197 |
| JW3281(rplD) | Complementary strand | 3451194 | 3451799 |
| JW3282(rplC) | Complementary strand | 3451810 | 3452439 |
| JW3283(rpsJ) | Complementary strand | 3452472 | 3452783 |
| JW3284(pinO) | Complementary strand | 3453021 | 3453440 |
| JW3285(gspA) | Complementary strand | 3453442 | 3454911 |
| JW3286(gspC) | | 3455091 | 3455906 |
| JW3287(gspD) | | 3455878 | 3457842 |
| JW3288(gspE) | | 3457852 | 3459333 |
| JW3289(hofF) | | 3459330 | 3460526 |
| JW3290(hofG) | | 3460536 | 3460973 |
| JW3291 (hofH) | | 3460981 | 3461490 |
| JW3292(gspI) | | 3461448 | 3461864 |
| JW3293(gspJ) | | 3461857 | 3462444 |
| JW3294(gspK) | | 3462437 | 3463420 |
| JW3295(gspL) | | 3463432 | 3464598 |
| JW3296(pshM) | | 3464571 | 3465056 |
| JW3297(hofD) | | 3465056 | 3465733 |
| JW3298(bfr) | Complementary strand | 3465762 | 3466238 |
| JW3299(yheA) | Complementary strand | 3466310 | 3466504 |
| JW3300(chiA) | Complementary strand | 3466673 | 3469366 |
| JW3301(tufA) | Complementary strand | 3469658 | 3470842 |
| JW3302(fusA) | Complementary strand | 3470913 | 3473027 |
| JW3303(rpsG) | Complementary strand | 3473055 | 3473594 |
| JW3304(rpsL) | Complementary strand | 3473691 | 3474065 |
| JW3305(yheL) | Complementary strand | 3474191 | 3474478 |
| JW3306(yheM) | Complementary strand | 3474486 | 3474845 |
| JW3307(yheN) | Complementary strand | 3474845 | 3475231 |
| JW3308(yheO) | Complementary strand | 3475231 | 3475965 |
| JW3309(fkpA) | Complementary strand | 3476120 | 3476932 |
| JW3310(slyX) | | 3477153 | 3477371 |
| JW3311(slyD) | Complementary strand | 3477420 | 3478010 |
| JW3312(625#3) | Complementary strand | 3478105 | 3478305 |
| JW3313(kefB) | Complementary strand | 3478315 | 3480120 |
| JW3314(yheR) | Complementary strand | 3480120 | 3480674 |
| JW3315(yheS) | | 3480802 | 3482715 |
| JW3316(yheT) | | 3482715 | 3483737 |
| JW3317(yheU) | | 3483731 | 3483949 |
| JW3318(prkB) | | 3484003 | 3484872 |
| JW3319(yhfA) | Complementary strand | 3484927 | 3485331 |
| JW3320(crp) | | 3485633 | 3486265 |
| JW3321(yhfK) | | 3486316 | 3488406 |
| JW3322(argD) | Complementary strand | 3488473 | 3489693 |
| JW3323(pabA) | Complementary strand | 3489779 | 3490342 |
| JW3324(fic) | Complementary strand | 3490374 | 3490976 |
| JW3325(yhfG) | Complementary strand | 3490966 | 3491133 |
| JW3326(ppiA) | Complementary strand | 3491238 | 3491810 |
| JW3327(yhfC) | | 3492081 | 3493262 |
| JW3328(nirB) | | 3493524 | 3496067 |
| JW3329(nirD) | | 3496064 | 3496390 |
| JW3330(nirC) | | 3496516 | 3497322 |
| JW3331(cysG) | | 3497341 | 3498714 |
| JW3332(yhfL) | | 3498961 | 3499128 |
| JW3333(yhfM) | | 3499423 | 3500760 |
| JW3334(yhfN) | | 3500760 | 3501803 |
| JW3335(yhfO) | | 3501766 | 3502215 |
| JW3336(yhfP) | | 3502280 | 3502684 |
| JW3337(yhfQ) | | 3502681 | 3503466 |
| JW3338(yhfR) | | 3503500 | 3504297 |
| JW3339(yhfS) | Complementary strand | 3504449 | 3505534 |
| JW3340(yhfT) | Complementary strand | 3505546 | 3506850 |
| JW3341 (yhfU) | Complementary strand | 3506862 | 3507254 |
| JW3342(php) | Complementary strand | 3507226 | 3508104 |
| JW3343(yhfW) | Complementary strand | 3508101 . | 3509327 |
| JW3344(yhfX) | Complementary strand | 3509327 | 3510490 |
| JW3345(yhfY) | Complementary strand | 3510574 | 3510978 |
| JW3346(yhfZ) | Complementary strand | 3510953 | 3511639 |
| JW3347(trpS) | Complementary strand | 3512148 | 3513152 |
| JW3348(gph) | Complementary strand | 3513145 | 3513903 |
| JW3349(rpe) | Complementary strand | 3513896 | 3514573 |
| JW3350(dam) | Complementary strand | 3514591 | 3515427 |
| JW3351 (damX) | Complementary strand | 3515534 | 3516820 |
| JW3352(aroB) | Complementary strand | 3516912 | 3518000 |
| JW3353(aroK) | Complementary strand | 3518057 | 3518578 |
| JW3354(hofQ) | Complementary strand | 3518979 | 3520217 |
| JW3355(yrfA) | Complementary strand | 3520129 | 3520572 |
| JW3356(yrfB) | Complementary strand | 3520523 | 3520963 |
| JW3357(yrfC) | Complementary strand | 3520947 | 3521486 |
| JW3358(yrfD) | Complementary strand | 3521486 | 3522292 |
| JW3359(mrcA) | | 3522385 | 3524937 |
| JW3360(yrfE) | Complementary strand | 3525103 | 3525663 |
| JW3361(yrfF) | | 3525983 | 3528118 |
| JW3362(yrfG) | | 3528138 | 3528851 |
| JW3363(yrfH) | | 3528862 | 3529263 |
| JW3364(yrfI) | | 3529282 | 3530166 |
| JW3365(yhgE) | Complementary strand | 3530229 | 3531953 |
| JW3366(pckA) | | 3532332 | 3533954 |
| JW3367(envZ) | Complementary strand | 3534030 | 3535382 |
| JW3368(ompR) | Complementary strand | 3535379 | 3536098 |
| JW3369(greB) | | 3536326 | 3536802 |
| JW3370(yhgF) | | 3536899 | 3539220 |
| JW3371 (feoA) | | 3539675 | 3539902 |
| JW3372(feoB) | | 3539919 | 3542240 |
| JW3373(yhgG) | | 3542240 | 3542476 |
| JW3374(yhgA) | | 3542679 | 3543557 |
| JW3375(bioH) | Complementary strand | 3543586 | 3544356 |
| JW3376(yhgH) | | 3544346 | 3545077 |
| JW3377(yhgI) | | 3545136 | 3545711 |
| JW3378(gntT) | | 3546071 | 3547384 |
| JW3379(maIQ) | Complementary strand | 3547495 | 3549579 |
| JW3380(maIP) | Complementary strand | 3549589 | 3551982 |
| JW3381(maIT) | | 3552594 | 3555299 |
| JW3382(rtcA) | Complementary strand | 3555342 | 3555947 |
| JW3383(rtcA) | Complementary strand | 3555920 | 3556360 |
| JW3384(rtcB) | Complementary strand | 3556361 | 3557587 |
| JW3385(rtcR) | | 3557776 | 3559374 |
| JW3386(glpR) | Complementary strand | 3559356 | 3560114 |
| JW3387(glpG) | Complementary strand | 3560131 | 3560961 |
| JW3388(glpE) | Complementary strand | 3561006 | 3581332 |
| JW3389(glpD) | | 3561522 | 3563027 |
| JW3390(yzgL) | Complementary strand | 3563233 | 3563514 |
| JW3391(glgP) | Complementary strand | 3563643 | 3566090 |
| JW3392(glgA) | Complementary strand | 3566109 | 3567542 |
| JW3393(glgC) | Complementary strand | 3567542 | 3568837 |
| JW3394(glgX) | Complementary strand | 3568855 | 3570828 |
| JW3395(glgB) | Complementary strand | 3570825 | 3573011 |
| JW3396(asd) | Complementary strand | 3573284 | 3574387 |
| JW3397(yhgN) | | 3574580 | 3575173 |
| JW3398(gntU) | Complementary strand | 3575230 | 3575565 |
| JW3399(gntU) | Complementary strand | 3575571 | 3576569 |
| JW3400(gntK) | Complementary strand | 3576573 | 3577100 |
| JW3401(gntR) | Complementary strand | 3577292 | 3578233 |
| JW3402(yhhW) | Complementary strand | 3578457 | 3579152 |
| JW3403(yhhX) | Complementary strand | 3579275 | 3580312 |
| JW3404(615#7.1) | | 3580259 | 3580411 |
| JW3405(yhhY) | | 3580645 | 3581133 |
| JW3406(yhhZ) | | 3581370 | 3582548 |
| JW3407(yrhA) | | 3582545 | 3582961 |
| JW3408(insA) | | 3582990 | 3583265 |
| JW3409(insB) | | 3583184 | 3583687 |
| JW3410(615#10.1) | | 3583950 | 3584066 |
| JW3411(yrhB) | | 3584266 | 3584550 |
| JW3412(ggt) | Complementary strand | 3584588 | 3586330 |
| JW3413(yhhA) | | 3586450 | 3586890 |
| JW3414(ugpQ) | Complementary strand | 3586877 | 3587620 |
| JW3415(ugpC) | Complementary strand | 3587617 | 3588687 |
| JW3416(ugpE) | Complementary strand | 3588689 | 3589534 |
| JW3417(ugpA) | Complementary strand | 3589531 | 3590418 |
| JW3418(ugpB) | Complementary strand | 3590516 | 3591832 |
| JW3418.5(613#2) | Complementary strand | 3592067 | 3592219 |
| JW3419(IivF) | Complementary strand | 3592231 | 3592944 |
| JW3420(livG) | Complementary strand | 3592946 | 3593713 |
| JW3421(livM) | Complementary strand | 3593710 | 3594987 |
| JW3422(livH) | Complementary strand | 3594984 | 3595910 |
| JW3423(livK) | Complementary strand | 3595958 | 3597067 |
| JW3424(yhhK) | | 3597491 | 3597874 |
| JW3425(livJ) | Complementary strand | 3598062 | 3599165 |
| JW3426(rpoH) | Complementary strand | 3599436 | 3600290 |
| JW3427(ftsX) | Complementary strand | 3600535 | 3601593 |
| JW3428(ftsE) | Complementary strand | 3601586 | 3602254 |
| JW3429(ftsY) | Complementary strand | 3602257 | 3603750 |
| JW3430(yhhF) | | 3603900 | 3604496 |
| JW3431(yhhL) | | 3604363 | 3604755 |
| JW3432(yhhM) | Complementary strand | 3604758 | 3605117 |
| JW3433(yhhN) | | 3605258 | 3605884 |
| JW3434(atzN) | | 3605958 | 3608156 |
| JW3435(yhhP) | Complementary strand | 3608258 | 3608503 |
| JW3436(yhhQ) | | 3608724 | 3609389 |
| JW3437(yhhR) | | 3609408 | 3610019 |
| JW3438(yhhS) | Complementary strand | 3610023 | 3611282 |
| JW3439(yhhT) | | 3611291 | 3612421 |
| JW3440(yhhU) | | 3612476 | 3613063 |
| JW3441(nikA) | | 3613174 | 3614748 |
| JW3442(nikB) | | 3614748 | 3615692 |
| JW3443(nikC) | | 3615689 | 3616522 |
| JW3444(nikD) | | 3616522 | 3617286 |
| JW3445(nikE) | | 3617283 | 3618089 |
| JW3446(yhhG) | | 3618095 | 3618496 |
| JW3447(rhsB) | | 3618699 | 3622934 |
| JW3448(610#6.1) | Complementary strand | 3621225 | 3621365 |
| JW3449(yhhH) | | 3622906 | 3623289 |
| JW3450(yibA) | | 3623379 | 3623639 |
| JW3451(yhhl) | | 3623885 | 3625021 |
| JW3452(yhhJ) | Complementary strand | 3625186 | 3626313 |
| JW3453(yhiH) | Complementary strand | 3626310 | 3628994 |
| JW3454(yhiI) | Complementary strand | 3629041 | 3630108 |
| JW3455(yhiJ) | Complementary strand | 3630474 | 3632096 |
| JW3456(yhiK) | Complementary strand | 3632358 | 3632729 |
| JW3457(yhiL) | Complementary strand | 3632726 | 3633964 |
| JW3458(yhiM) | | 3634248 | 3635399 |
| JW3459(yhiN) | Complementary strand | 3635714 | 3636916 |
| JW3460(pitA) | | 3637148 | 3638647 |
| JW3461(uspB) | Complementary strand | 3638891 | 3639226 |
| JW3462(uspA) | | 3639617 | 3640051 |
| JW3463(yhiP) | | 3640368 | 3641837 |
| JW3464(yhiQ) | Complementary strand | 3641886 | 3642743 |
| JW3465(prlC) | Complementary strand | 3642646 | 3644688 |
| JW3466(yhiR) | | 3644891 | 3645733 |
| JW3467(gor) | | 3645805 | 3647157 |
| JW3468(arsR) | | 3648034 | 3648387 |
| JW3469(arsB) | | 3648441 | 3649730 |
| JW3470(arsC) | | 3649743 | 3650168 |
| JW3471(yhiS) | | 3650797 | 3651579 |
| JW3472(is5B) | Complementary strand | 3651688 | 3652704 |
| JW3473(yi51) | | 3652024 | 3652392 |
| JW3474(slp) | | 3653467 | 3654033 |
| JW3475(yhiF) | | 3654189 | 3654719 |
| JW3476(yhiD) | Complementary strand | 3654761 | 3655408 |
| JW3477(hdeB) | Complementary strand | 3655472 | 3655810 |
| JW3478(hdeA) | Complementary strand | 3655914 | 3656246 |
| JW3479(hdeD) | | 3656501 | 3657073 |
| JW3480(yhiE) | | 3657872 | 3658399 |
| JW3481(yhiU) | | 3658738 | 3659895 |
| JW3482(yhiV) | | 3659920 | 3663033 |
| JW3483(yhiW) | Complementary strand | 3663396 | 3664124 |
| JW3484(yhiX) | Complementary strand | 3664492 | 3665316 |
| JW3485(gadA) | Complementary strand | 3665686 | 3667086 |
| JW3486(yhjA) | Complementary strand | 3667297 | 3668694 |
| JW3487(treF) | | 3669098 | 3670747 |
| JW3488(yhjB) | Complementary strand | 3670798 | 3671400 |
| JW3489(yhjC) | | 3671848 | 3672819 |
| JW3490(yhjD) | | 3672868 | 3673881 |
| JW3491(yhjE) | | 3674292 | 3675614 |
| JW3492(yhjG) | Complementary strand | 3675796 | 3677871 |
| JW3493(yhjH) | Complementary strand | 3677926 | 3678693 |
| JW3494(kdgK) | | 3678706 | 3679854 |
| JW3495(yhjJ) | Complementary strand | 3679950 | 3681446 |
| JW3496(dctA) | Complementary strand | 3681667 | 3682953 |
| JW3497(yhjK) | Complementary strand | 3683136 | 3685091 |
| JW3498(yhjL) | Complementary strand | 3685206 | 3688628 |
| JW3499(yhjM) | Complementary strand | 3688660 | 3689766 |
| JW3500(yhjN) | Complementary strand | 3689773 | 3692112 |
| JW3501(yhjO) | Complementary strand | 3692123 | 3694789 |
| JW3502(yhjQ) | Complementary strand | 3694738 | 3695466 |
| JW3503(yhjR) | Complementary strand | 3695502 | 3695690 |
| JW3504(yhjS) | | 3695963 | 3697534 |
| JW3505(yhjT) | | 3697534 | 3697722 |
| JW3506(yhjU) | | 3697719 | 3699398 |
| JW3507(603#8) | Complementary strand | 3699485 | 3699760 |
| JW3508(yhjV) | | 3700068 | 3701339 |
| JW3509(dppF) | Complementary strand | 3701369 | 3702373 |
| JW3510(dppD) | Complementary strand | 3702370 | 3703353 |
| JW3511(dppC) | Complementary strand | 3703364 | 3704266 |
| JW3512(dppB) | Complementary strand | 3704276 | 3705295 |
| JW3513(dppA) | Complementary strand | 3705603 | 3707210 |
| JW3514(yzpK) | Complementary strand | 3707725 | 3708072 |
| JW3515(yhjW) | Complementary strand | 3708289 | 3710013 |
| JW3516(yhjX) | Complementary strand | 3710304 | 3711512 |
| JW3517(yhjY) | Complementary strand | 3711741 | 3712445 |
| JW3518(tag) | | 3712597 | 3713160 |
| JW3519(yiaC) | | 3713157 | 3713597 |
| JW3520(bisC) | Complementary strand | 3713566 | 3715905 |
| JW3521(yiaD) | | 3716052 | 3716711 |
| JW3522(yiaE) | | 3716803 | 3717789 |
| JW3523(yiaF) | Complementary strand | 3717839 | 3718669 |
| JW3524(yiaG) | | 3718983 | 3719273 |
| JW3525(cspA) | | 3719554 | 3719766 |
| JW3526(yiaZ) | Complementary strand | 3719953 | 3720105 |
| JW3527(yi5A) | | 3720185 | 3720706 |
| JW3528(yi5B) | | 3720703 | 3721554 |
| JW3529(yi5C) | Complementary strand | 3720961 | 3721317 |
| JW3530(glyS) | Complementary strand | 3721833 | 3723902 |
| JW3531(glyQ) | Complementary strand | 3723912 | 3724823 |
| JW3532(601#3) | Complementary strand | 3724918 | 3725217 |
| JW3533(yiaH) | | 3725392 | 3726387 |
| JW3534(yiaA) | Complementary strand | 3726429 | 3726866 |
| JW3535(yiaB) | Complementary strand | 3726912 | 3727265 |
| JW3536(xylB) | Complementary strand | 3727422 | 3728876 |
| JW3537(xylA) | Complementary strand | 3728948 | 3730270 |
| JW3538(xylF) | | 3730636 | 3731628 |
| JW3539(xylG) | | 3731706 | 3733247 |
| JW3540(xylH) | | 3733225 | 3734406 |
| JW3541(xylR) | | 3734484 | 3735662 |
| JW3542(bax), | Complementary strand | 3735858 | 3736682 |
| JW3543(malS) | | 3737002 | 3739032 |
| JW3544(avtA) | | 3739210 | 3740463 |
| JW3545(ysaA) | Complementary strand | 3740614 | 3741087 |
| JW3546(yiaJ) | Complementary strand | 3741189 | 3742037 |
| JW3547(yiaK) | | 3742238 | 3743236 |
| JW3548(yiaL) | | 3743248 | 3743715 |
| JW3549(yiaM) | | 3743833 | 3744306 |
| JW3550(yiaN) | | 3744309 | 3745583 |
| JW3551(yiaO) | | 3745600 | 3746586 |
| JW3552(lyx) | | 3746590 | 3748086 |
| JW3553(sgbH) | | 3748083 | 3748745 |
| JW3554(sgbU) | | 3748705 | 3749598 |
| JW3555(sgbE) | | 3749592 | 3750287 |
| JW3556(yiaT) | Complementary strand | 3750634 | 3751374 |
| JW3557(yiaU) | | 3751498 | 3752472 |
| JW3558(yiaV) | Complementary strand | 3752469 | 3753605 |
| JW3559(yiaW) | Complementary strand | 3753611 | 3753934 |
| JW3560(577#1.1) | | 3754048 | 3754260 |
| JW3561 (aldB) | Complementary strand | 3754479 | 3756017 |
| JW3562(yiaY) | Complementary strand | 3756182 | 3757330 |
| JW3563(selB) | Complementary strand | 3757520 | 3759364 |
| JW3564(selA) | Complementary strand | 3759361 | 3760752 |
| JW3565(yibF) | Complementary strand | 3760850 | 3761458 |
| JW3566(rhsA) | | 3761686 | 3765819 |
| JW3567(576#2.1) | Complementary strand | 3764212 | 3764631 |
| JW3568(yibA) | | 3765840 | 3766682 |
| JW3569(yibJ) | | 3766724 | 3767425 |
| JW3570(yibG) | | 3767680 | 3768141 |
| JW3570.5(576#7) | | 3769491 | 3769649 |
| JW3571(yibH) | Complementary strand | 3769746 | 3770882 |
| JW3572(yibI) | Complementary strand | 3770885 | 3771247 |
| JW3573(mtlA) | | 3771784 | 3773697 |
| JW3574(mtlD) | | 3773927 | 3775075 |
| JW3575(mtlR) | | 3775075 | 3775662 |
| JW3576(576#14) | Complementary strand | 3775674 | 3775883 |
| JW3577(yibL) | | 3776168 | 3776530 |
| JW3578(lldP) | | 3776902 | 3778557 |
| JW3579(lldR) | | 3778557 | 3779333 |
| JW3580(lldD) | | 3779330 | 3780520 |
| JW3581(yibK) | | 3780718 | 3781191 |
| JW3582(cysE) | Complementary strand | 3781244 | 3782065 |
| JW3583(gpsA) | Complementary strand | 3782145 | 3783164 |
| JW3584(secB) | Complementary strand | 3783164 | 3783631 |
| JW3585(grxC) | Complementary strand | 3783694 | 3783945 |
| JW3586(yibN) | Complementary strand | 3784087 | 3784518 |
| JW3587(yibO) | | 3784763 | 3786307 |
| JW3588(yibP) | | 3786317 | 3787600 |
| JW3589(yibQ) | | 3787730 | 3788563 |
| JW3590(yibD) | Complementary strand | 3788550 | 3789584 |
| JW3591 (tdh) | Complementary strand | 3789823 | 3790848 |
| JW3592(kbl) | Complementary strand | 3790858 | 3792054 |
| JW3593(htrL) | Complementary strand | 3792329 | 3793201 |
| JW3594(rfaD) | | 3793490 | 3794422 |
| JW3595(rfaF) | | 3794432 | 3795478 |
| JW3596(rfaC) | | 3795482 | 3796441 |
| JW3597(rfaL) | | 3796451 | 3797710 |
| JW3598(rfaK) | Complementary strand | 3797742 | 3798815 |
| JW3599(rfaZ) | Complementary strand | 3798848 | 3799699 |
| JW3600(rfaY) | Complementary strand | 3799770 | 3800468 |
| JW3601 (rfaJ) | Complementary strand | 3800486 | 3801502 |
| JW3602(rfaI) | Complementary strand | 3801542 | 3802561 |
| JW3603(rfaB) | Complementary strand | 3802561 | 3803670 |
| JW3604(rfaS) | Complementary strand | 3803684 | 3804619 |
| JW3605(rfaP) | Complementary strand | 3804656 | 3805453 |
| JW3606(rfaG) | Complementary strand | 3805446 | 3806570 |
| JW3607(rfaQ) | Complementary strand | 3806567 | 3807601 |
| JW3608(kdtA) | | 3808043 | 3809320 |
| JW3609(kdtB) | | 3809328 | 3809807 |
| JW3610(mutM) | Complementary strand | 3809846 | 3810655 |
| JW3611(rpmG) | Complementary strand | 3810753 | 3810920 |
| JW3612(rpmB) | Complementary strand | 3810941 | 3811177 |
| JW3613(radC) | Complementary strand | 3811394 | 3812068 |
| JW3614(dfp) | | 3812162 | 3813454 |
| JW3615(dut) | | 3813435 | 3813890 |
| JW3616(ttk) | | 3813955 | 3814593 |
| JW3617(pyrE) | Complementary strand | 3814630 | 3815271 |
| JW3618(rph) | Complementary strand | 3815366 | 3816052 |
| JW3619(yicC) | | 3816179 | 3817042 |
| JW3620(dinD) | | 3817263 | 3818087 |
| JW3621(yicG) | | 3818377 | 3818994 |
| JW3622(yicF) | Complementary strand | 3818991 | 3820673 |
| JW3623(gmk) | | 3820931 | 3821554 |
| JW3624(rpoZ) | | 3821609 | 3821884 |
| JW3625(spoT) | | 3821903 | 3824011 |
| JW3626(spoU) | | 3824018 | 3824707 |
| JW3627(recG) | | 3824713 | 3826794 |
| JW3628(gltS) | Complementary strand | 3826963 | 3828168 |
| JW3629(yicE) | | 3828448 | 3829839 |
| JW3630(yicH) | | 3829960 | 3831669 |
| JW3631(yicI) | Complementary strand | 3831722 | 3834040 |
| JW3632(yicJ) | Complementary strand | 3834050 | 3835432 |
| JW3633(yicK) | | 3836456 | 3837640 |
| JW3634(yicL) | | 3837751 | 3838674 |
| JW3635(nlpA) | Complementary strand | 3838678 | 3839496 |
| JW3636.1(569#3.2) | | 3839718 | 3840011 |
| JW3637(yicM) | Complementary strand | 3840052 | 3841290 |
| JW3638(yicN) | Complementary strand | 3841453 | 3841932 |
| JW3639(yicO) | Complementary strand | 3841958 | 3843370 |
| JW3640(yicP) | | 3843467 | 3845233 |
| JW3641 (uhpT) | Complementary strand | 3845279 | 3846670 |
| JW3642(uhpC) | Complementary strand | 3846808 | 3848127 |
| JW3643(uhpB) | Complementary strand | 3848137 | 3849639 |
| JW3644(uhpA) | Complementary strand | 3849639 | 3850229 |
| JW3645(ilvN) | Complementary strand | 3850305 | 3850595 |
| JW3646(ilvB) | Complementary strand | 3850599 | 3852287 |
| JW3647(ivbL) | Complementary strand | 3852393 | 3852491 |
| JW3648(567#1) | | 3853056 | 3853145 |
| JW3649(emrD) | | 3853419 | 3854609 |
| JW3650(yidF) | Complementary strand | 3854617 | 3855114 |
| JW3651 (yidG) | Complementary strand | 3855111 | 3855473 |
| JW3652(yidH) | Complementary strand | 3855463 | 3855810 |
| JW3653(yidI) | | 3855918 | 3856367 |
| JW3654(yidJ) | Complementary strand | 3856414 | 3857907 |
| JW3655(yidK) | Complementary strand | 3857904 | 3859619 |
| JW3656(yidL) | | 3859756 | 3860679 |
| JW3657(567#9.1) | Complementary strand | 3860676 | 3860888 |
| JW3658(glvG) | Complementary strand | 3860852 | 3861490 |
| JW3659(glvB) | Complementary strand | 3861490 . | 3861975 |
| JW3660(glvC) | Complementary strand | 3862000 | 3863106 |
| JW3661(yidP) | | 3863402 | 3864118 |
| JW3662(yidE) | Complementary strand | 3864115 | 3865776 |
| JW3663(ibpB) | Complementary strand | 3865972 | 3866400 |
| JW3664(ibpA) | Complementary strand | 3866512 | 3866925 |
| JW3665(yidQ) | | 3867156 | 3867563 |
| JW3666(yidR) | Complementary strand | 3867565 | 3868815 |
| JW3667(yidS) | | 3868859 | 3869944 |
| JW3668(dgoT) | Complementary strand | 3869941 | 3871278 |
| JW3669(dgoA) | Complementary strand | 3871353 | 3873116 |
| JW3670(dgoK) | Complementary strand | 3873100 | 3873978 |
| JW3671(dgoR) | Complementary strand | 3873975 | 3874271 |
| JW3672(yidW) | Complementary strand | 3874277 | 3874663 |
| JW3673(yidX) | | 3874998 | 3875669 |
| JW3674(yidA) | Complementary strand | 3875644 | 3876456 |
| JW3675(yidB) | Complementary strand | 3876571 | 3876969 |
| JW3676(gyrB) | Complementary strand | 3877209 | 3879623 |
| JW3677(recF) | Complementary strand | 3879652 | 3880725 |
| JW3678(dnaN) | Complementary strand | 3880725 | 3881825 |
| JW3679(dnaA) | Complementary strand | 3881830 | 3883233 |
| JW3680(rpmH) | | 3883841 | 3883981 |
| JW3681 (rnpA) | | 3883998 | 3884357 |
| JW3682(yidD) | | 3884321 | 3884578 |
| JW3683(yidC) | | 3884581 | 3886227 |
| JW3684(thdF) | | 3886333 | 3887697 |
| JW3685(tnaL) | | 3887940 | 3888014 |
| JW3686(tnaA) | | 3888235 | 3889650 |
| JW3687(tnaB) | | 3889741 | 3890988 |
| JW3688(yidY) | | 3891120 | 3892295 |
| JW3689(yidZ) | | 3892270 | 3893229 |
| JW3690(yieE) | | 3893374 | 3894135 |
| JW3691(yieF) | | 3894157 | 3894723 |
| JW3692(yieG) | Complementary strand | 3894777 | 3896114 |
| JW3693(yieH) | | 3896279 | 3896944 |
| JW3694(yiel) | | 3897011 | 3897478 |
| JW3695(yieJ) | | 3897527 | 3898114 |
| JW3696(yieK) | Complementary strand | 3898187 | 3898828 |
| JW3697(yieL) | Complementary strand | 3898912 | 3900114 |
| JW3698(yieC) | Complementary strand | 3900108 | 3901724 |
| JW3699(bglB) | Complementary strand | 3901793 | 3903205 |
| JW3700(bglF) | Complementary strand | 3903224 | 3905101 |
| JW3701(bglG) | Complementary strand | 3905235 | 3906071 |
| JW3702(phoU) | Complementary strand | 3906357 | 3907082 |
| JW3703(pstB) | Complementary strand | 3907097 | 3907870 |
| JW3704(pstA) | Complementary strand | 3908053 | 3908943 |
| JW3705(pstC) | Complementary strand | 3908943 | 3909902 |
| JW3706(pstS) | Complementary strand | 3909989 | 3911029 |
| JW3707(glmS) | Complementary strand | 3911343 | 3913172 |
| JW3708(glmU) | Complementary strand | 3913334 | 3914704 |
| JW3709(atpC) | Complementary strand | 3915057 | 3915476 |
| JW3710(uncD) | Complementary strand | 3915497 | 3916879 |
| JW3711(atpG) | Complementary strand | 3916906 | 3917769 |
| JW3712(atpA) | Complementary strand | 3917820 | 3919361 |
| JW3713(atpH) | Complementary strand | 3919374 | 3919907 |
| JW3714(atpF) | Complementary strand | 3919922 | 3920392 |
| JW3715(atpE) | Complementary strand | 3920454 | 3920693 |
| JW3716(atpB) | Complementary strand | 3920740 | 3921555 |
| JW3717(atpI) | Complementary strand | 3921564 | 3921956 |
| JW3718(gidB) | Complementary strand | 3922561 | 3923184 |
| JW3719(gidA) | Complementary strand | 3923248 | 3925137 |
| JW3720(mioC) | Complementary strand | 3925516 | 3925959 |
| JW3721(asnC) | Complementary strand | 3926049 | 3926507 |
| JW3722(asnA) | | 3926659 | 3927651 |
| JW3723(yieM) | Complementary strand | 3927656 | 3928939 |
| JW3724(559#4) | Complementary strand | 3928924 | 3929106 |
| JW3725(yieN) | Complementary strand | 3929100 | 3930596 |
| JW3726(kup) | | 3930819 | 3932378 |
| JW3727(rbsD) | | 3932817 | 3933272 |
| JW3728(rbsA) | | 3933280 | 3934785 |
| JW3729(rbsC) | | 3934790 | 3935755 |
| JW3730(rbsB) | | 3935780 | 3936670 |
| JW3731(rbsK) | | 3936796 | 3937725 |
| JW3732(rbsR) | | 3937729 | 3938721 |
| JW3733(yieO) | Complementary strand | 3938687 | 3940114 |
| JW3734(yieP) | Complementary strand | 3940137 | 3940682 |
| JW3735(yifA) | Complementary strand | 3946628 | 3947224 |
| JW3736(pssR) | Complementary strand | 3947065 | 3947466 |
| JW3737(yifE) | | 3947585 | 3947923 |
| JW3738(yifB) | Complementary strand | 3947948 | 3949498 |
| JW3739(ilvL) | | 3949821 | 3949919 |
| JW3740(ilvG) | | 3950059 | 3951042 |
| JW3741(ilvG) | | 3951122 | 3951703 |
| JW3742(ilvM) | | 3951700 | 3951963 |
| JW3743(ilvE) | | 3951983 | 3952912 |
| JW3744(ilvD) | | 3953008 | 3954825 |
| JW3745(ilvA) | | 3954828 | 3956372 |
| JW3746(ilvY) | Complementary strand | 3956424 | 3957317 |
| JW3747(ilvC) | | 3957467 | 3958942 |
| JW3748(ppiC) | Complementary strand | 3959026 | 3959307 |
| JW3749(yifN) | Complementary strand | 3959503 | 3959778 |
| JW3750(yifN) | Complementary strand | 3959733 | 3959951 |
| JW3751 (rep) | | 3960168 | 3962189 |
| JW3752(gppA) | Complementary strand | 3962236 | 3963720 |
| JW3753(rhIB) | Complementary strand | 3963856 | 3965121 |
| JW3754(trxA) | | 3965252 | 3965581 |
| JW3755(rhoL) | | 3965722 | 3965823 |
| JW3756(rho) | | 3965908 | 3967167 |
| JW3757(555#2.1) | | 3967177 | 3967395 |
| JW3758(rfe) | | 3967407 | 3968510 |
| JW3759(wzzE) | | 3968519 | 3969568 |
| JW3760(nfrC) | | 3969624 | 3970751 |
| JW3761(rffD) | | 3970748 | 3972010 |
| JW3762(rffG) | | 3972010 | 3973077 |
| JW3763(rffH) | | 3973096 | 3973977 |
| JW3764(yifH) | | 3974084 | 3974629 |
| JW3765(rffA) | | 3974634 | 3975764 |
| JW3766(wzxE) | | 3975766 | 3977016 |
| JW3767(554#5.1) | | 3977013 | 3977237 |
| JW3768(rffT) | | 3977152 | 3978093 |
| JW3769(rffT) | | 3978090 | 3979442 |
| JW3770(wecG) | | 3979445 | 3980185 |
| JW3771(yifK) | | 3980376 | 3981761 |
| JW3772(aslB) | | 3982447 | 3983682 |
| JW3773(aslA) | Complementary strand | 3983841 | 3985496 |
| JW3774(hemY) | Complementary strand | 3986175 | 3987371 |
| JW3775(hemX) | Complementary strand | 3987374 | 3988555 |
| JW3776(hemD) | Complementary strand | 3988577 | 3989317 |
| JW3777(hemC) | Complementary strand | 3989314 | 3990255 |
| JW3778(cyaA) | | 3990642 | 3993188 |
| JW3779(cyaY) | Complementary strand | 3993228 | 3993548 |
| JV3780(yzcX) | | 3993339 | 3993824 |
| JW3781 (yifL) | | 3994011 | 3994214 |
| JW3782(dapF) | | 3994248 | 3995075 |
| JW3783(yigA) | | 3995072 | 3995779 |
| JW3784(xerC) | | 3995776 | 3996672 |
| JW3785(yigB) | | 3996672 | 3997388 |
| JW3786(uvrD) | | 3997472 | 3999634 |
| JW3787(yigE) | Complementary strand | 3999781 | 4000158 |
| JW3788(yigE) | Complementary strand | 4000059 | 4000544 |
| JW3789(corA) | | 4000914 | 4001864 |
| JW3790(yigF) | Complementary strand | 4001907 | 4002287 |
| JW3791(yigG) | Complementary strand | 4002301 | 4002717 |
| JW3792(radD) | Complementary strand | 4002776 | 4003678 |
| JW3793(yigH) | Complementary strand | 4003717 | 4004202 |
| JW3794(pldA) | | 4004349 | 4005218 |
| JW3795(recQ) | | 4005345 | 4007177 |
| JW3796(yigJ) | | 4007355 | 4007522 |
| JW3797(yigJ) | | 4007393 | 4007860 |
| JW3798(yigK) | Complementary strand | 4007922 | 4008338 |
| JW3799(551#4) | Complementary strand | 4008335 | 4008541 |
| JW3800(pldB) | | 4008652 | 4009674 |
| JW3801(yigL) | | 4009556 | 4009822 |
| JW3802(yigL) | | 4009794 | 4010309 |
| JW3803(yigM) | | 4010542 | 4011441 |
| JW3804(metR) | Complementary strand | 4011329 | 4012282 |
| JW3805(metE) | | 4012519 | 4014780 |
| JW3806(ysgA) | Complementary strand | 4014609 | 4015433 |
| JW3807(551#11.1) | Complementary strand | 4014820 | 4014993 |
| JW3808(udp) | | 4015894 | 4016655 |
| JW3809(yigN) | | 4016796 | 4018223 |
| JW3810(ubiE) | | 4018318 | 4019073 |
| JW3811(yigP) | | 4019087 | 4019692 |
| JW3812(aarF) | | 4019689 | 4021329 |
| JW3813(yigT) | | 4021408 | 4021677 |
| JW3814(yigT) | | 4021761 | 4022198 |
| JW3815(tatC) | | 4022201 | 4022977 |
| JW3816(tatD) | | 4023007 | 4023627 |
| JW3817(yigW) | | 4023554 | 4023802 |
| JW3818(rfaH) | Complementary strand | 4023799 | 4024287 |
| JW3819(ubiD) | | 4024454 | 4025947 |
| JW3820(ubiB) | | 4025993 | 4026694 |
| JW3821(fadA) | Complementary strand | 4027075 | 4028238 |
| JW3822(fadB) | Complementary strand | 4028248 | 4030437 |
| JW3823(pepQ) | | 4030627 | 4031958 |
| JW3824(yigZ) | | 4031955 | 4032572 |
| JW3825(trkH) | | 4032611 | 4033909 |
| JW3826(trkH) | | 4033867 | 4034061 |
| JW3827(hemG) | | 4034073 | 4034618 |
| JW3828(mobB) | Complementary strand | 4040362 | 4040889 |
| JW3829(mobA) | Complementary strand | 4040871 | 4041455 |
| JW3830(yihD) | | 4041525 | 4041794 |
| JW3831 (yihE) | | 4041872 | 4042858 |
| JW3832(dsbA) | | 4042875 | 4043501 |
| JW3833(yihF) | | 4043613 | 4045085 |
| JW3834(yihG) | Complementary strand | 4045126 | 4046058 |
| JW3835(polA) | | 4046422 | 4049208 |
| JW3836(yihA) | Complementary strand | 4049589 | 4050188 |
| JW3837(yihI) | | 4050803 | 4051312 |
| JW3838(hemN) | | 4051501 | 4052874 |
| JW3839(ntrC) | Complementary strand | 4053325 | 4054734 |
| JW3840(ntrB) | Complementary strand | 4054746 | 4055795 |
| JW3841 (glnA) | Complementary strand | 4056081 | 4057490 |
| JW3842(yihK) | | 4057863 | 4059638 |
| JW3843(yihL) | | 4059902 | 4060612 |
| JW3844(yihM) | | 4060620 | 4061600 |
| JW3845(yihN) | | 4061702 | 4062967 |
| JW3846(yshA) | Complementary strand | 4063058 | 4063750 |
| JW3847(yihO) | Complementary strand | 4063765 | 4065228 |
| JW3848(yihP) | Complementary strand | 4065265 | 4066671 |
| JW3849(yihQ) | Complementary strand | 4066696 | 4068732 |
| JW3850(yihR) | Complementary strand | 4068931 | 4069857 |
| JW3851(yihS) | Complementary strand | 4069971 | 4071227 |
| JW3852(yihT) | Complementary strand | 4071229 | 4072107 |
| JW3853(yihU) | Complementary strand | 4072131 | 4073027 |
| JW3854(yihV) | | 4073189 | 4074091 |
| JW3855(yihW) | | 4074101 | 4074910 |
| JW3856(yihX) | | 4074988 | 4075608 |
| JW3857(rbn) | | 4075602 | 4076474 |
| JW3858(yihZ) | | 4076471 | 4076908 |
| JW3859(yiiD) | | 4076905 | 4077894 |
| JW3860(yiiE) | | 4078720 | 4078965 |
| JW3861 (yiiF) | | 4079183 | 4079425 |
| JW3862(fdhE) | Complementary strand | 4079755 | 4080684 |
| JW3863(fdoI) | Complementary strand | 4080681 | 4081316 |
| JW3864(fdoH) | Complementary strand | 4081313 | 4082215 |
| JW3865(fdoG) | Complementary strand | 4082228 | 4085278 |
| JW3866(fdhD) | | 4085472 | 4086305 |
| JW3867(yiiG) | | 4086458 | 4087513 |
| JW3868(frvR) | Complementary strand | 4087564 | 4089312 |
| JW3869(frvX) | Complementary strand | 4089312 | 4090382 |
| JW3870(frvB) | Complementary strand | 4090372 | 4091829 |
| JW3871(frvA) | Complementary strand | 4091834 | 4092280 |
| JW3872(yiiL) | Complementary strand | 4092581 | 4092895 |
| JW3873(rhaD) | Complementary strand | 4092905 | 4093729 |
| JW3874(rhaA) | Complementary strand | 4094180 | 4095439 |
| JW3875(rhaB) | Complementary strand | 4095436 | 4096905 |
| JW3876(rhaS) | | 4097193 | 4098029 |
| JW3877(rhaR) | | 4098013 | 4098951 |
| JW3878(rhaT) | Complementary strand | 4098948 | 4099982 |
| JW3879(sodA) | | 4100267 | 4100887 |
| JW3880(kdgT) | | 4101138 | 4102130 |
| JW3881(yiiM) | | 4102249 | 4102953 |
| JW3882(cpxA) | Complementary strand | 4103059 | 4104432 |
| JW3883(cpxR) | Complementary strand | 4104429 | 4105127 |
| JW3884(540#3.1) | | 4105274 | 4105423 |
| JW3885(cpxP) | | 4105408 | 4105776 |
| JW3886(yiiP) | | 4105925 | 4106827 |
| JW3887(pfkA) | | 4107008 | 4107970 |
| JW3888(sbp) | | 4108290 | 4109279 |
| JW3889(cdh) | | 4109386 | 4110141 |
| JW3890(tpiA) | Complementary strand | 4110196 | 4110963 |
| JW3891(yiiQ) | Complementary strand | 4111071 | 4111670 |
| JW3892(yiiR) | | 4111771 | 4112211 |
| JW3893(yiiS) | | 4112423 | 4112722 |
| JW3894(yiiT) | | 4112749 | 4113177 |
| JW3895(fpr) | Complementary strand | 4113182 | 4113928 |
| JW3896(glpX) | Complementary strand | 4114025 | 4115035 |
| JW3897(glpK) | Complementary strand | 4115170 | 4116678 |
| JW3898(glpF) | Complementary strand | 4116701 | 4117546 |
| JW3899(yiiU) | | 4117971 | 4118216 |
| JW3900(menG) | Complementary strand | 4118301 | 4118786 |
| JW3901(menA) | Complementary strand | 4118879 | 4119805 |
| JW3902(hslU) | Complementary strand | 4119872 | 4121203 |
| JW3903(hsIV) | Complementary strand | 4121213 | 4121743 |
| JW3904(ftsN) | Complementary strand | 4121836 | 4122795 |
| JW3905(cytR) | Complementary strand | 4122887 | 4123912 |
| JW3906(priA) | Complementary strand | 4124068 | 4126266 |
| JW3907(rpmE) | | 4126469 | 4126681 |
| JW3908(yiiX) | Complementary strand | 4126742 | 4127350 |
| JW3909(metJ) | Complementary strand | 4127534 | 4127851 |
| JW3910(metB) | | 4128128 | 4129288 |
| JW3911(metL) | | 4129291 | 4131723 |
| JW3912(537#1.1) | Complementary strand | 4131862 | 4132410 |
| JW3913(metF) | | 4132072 | 4132962 |
| JW3914(katG) | | 4133291 | 4135471 |
| JW3915(yijE) | | 4135531 | 4136469 |
| JW3916(yijF) | Complementary strand | 4136496 | 4137113 |
| JW3917(gldA) | Complementary strand | 4137388 | 4138530 |
| JW3918(talC) | Complementary strand | 4138502 | 4139164 |
| JW3919(ptsA) | Complementary strand | 4139176 | 4141311 |
| JW3920(536#1) | | 4141169 | 4141642 |
| JW3921(frwC) | | 4141985 | 4143064 |
| JW3922(frwB) | | 4143079 | 4143399 |
| JW3923(pflD) | | 4143450 | 4145747 |
| JW3924(pflC) | | 4145713 | 4146591 |
| JW3925(frwD) | | 4146593 | 4146934 |
| JW3926(yijO) | Complementary strand | 4146921 | 4147772 |
| JW3927(yijP) | Complementary strand | 4147987 | 4149720 |
| JW3928(ppc) | Complementary strand | 4149902 | 4152553 |
| JW3929(argE) | Complementary strand | 4153151 | 4154302 |
| JW3930(argC) | | 4154456 | 4155460 |
| JW3931(argB) | | 4155468 | 4156244 |
| JW3932(argH) | | 4156305 | 4157678 |
| JW3933(oxyR) | | 4157945 | 4158862 |
| JW3934(udhA) | Complementary strand | 4158845 | 4160179 |
| JW3935(yijC) | | 4160522 | 4161226 |
| JW3936(yijD) | | 4161226 | 4161585 |
| JW3937(trmA) | Complementary strand | 4161625 | 4162725 |
| JW3938(btuB) | | 4163094 | 4164938 |
| JW3939(murI) | | 4164871 | 4165740 |
| JW3940(murB) | | 4171512 | 4172540 |
| JW3941 (birA) | | 4172537 | 4173502 |
| JW3942(coaA) | Complementary strand | 4173531 | 4174481 |
| JW3943(tufB) | | 4175399 | 4176583 |
| JW3944(secE) | | 4176813 | 4177196 |
| JW3945(nusG) | | 4177198 | 4177743 |
| JW3946(rplK) | | 4177901 | 4178329 |
| JW3947(rplA) | | 4178333 | 4179037 |
| JW3948(rplJ) | | 4179450 | 4179947 |
| JW3949(rplL) | | 4180014 | 4180379 |
| JW3950(rpoB) | | 4180699 | 4184727 |
| JW3951(rpoC) | | 4184804 | 4189027 |
| JW3952(htrC) | | 4189240 | 4189779 |
| JW3953(thiH) | Complementary strand | 4190189 | 4191322 |
| JW3954(thiG) | Complementary strand | 4191319 | 4192164 |
| JW3955(thiS) | Complementary strand | 4192091 | 4192291 |
| JW3956(thiF) | Complementary strand | 4192275 | 4193030 |
| JW3957(thiE) | Complementary strand | 4193023 | 4193658 |
| JW3958(thiC) | Complementary strand | 4193658 | 4195553 |
| JW3959(rsd) | Complementary strand | 4195786 | 4196262 |
| JW3960(yjaD) | | 4196357 | 4197130 |
| JW3961(hemE) | | 4197170 | 4198234 |
| JW3962(nfi) | | 4198238 | 4198915 |
| JW3963(yjaG) | | 4198958 | 4199548 |
| JW3964(hupA) | | 4199735 | 4200007 |
| JW3965(yjaH) | | 4200020 | 4200715 |
| JW3966(yjaI) | Complementary strand | 4200717 | 4201283 |
| JW3967(hydH) | | 4201380 | 4202777 |
| JW3968(hydG) | | 4202774 | 4204099 |
| JW3969(purD) | Complementary strand | 4204096 | 4205385 |
| JW3970(purH) | Complementary strand | 4205397 | 4206986 |
| JW3971(yjaA) | | 4212689 | 4213072 |
| JW3972(yjaB) | Complementary strand | 4213135 | 4213578 |
| JW3973(metA) | | 4213735 | 4214664 |
| JW3974(aceB) | | 4214933 | 4216534 |
| JW3975(aceA) | | 4216564 | 4217868 |
| JW3976(aceK) | | 4218051 | 4219787 |
| JW3977(arp) | Complementary strand | 4219756 | 4221942 |
| JW3978(iclR) | Complementary strand | 4222259 | 4223083 |
| JW3979(metH) | | 4223283 | 4226966 |
| JW3980(yjbB) | | 4227186 | 4228817 |
| JW3981 (pepE) | Complementary strand | 4228908 | 4229597 |
| JW3982(yjbC) | | 4229809 | 4230681 |
| JW3983(yjbD) | Complementary strand | 4230814 | 4231086 |
| JW3984(lysC) | Complementary strand | 4231339 | 4232688 |
| JW3985(pgi) | | 4233213 | 4234862 |
| JW3986(633#6) | | 4235361 | 4235603 |
| JW3987(yjbF) | | 4235687 | 4236355 |
| JW3988(yjbG) | | 4236352 | 4237089 |
| JW3989(yjbH) | | 4237089 | 4239185 |
| JW3989.5(633#10) | Complementary strand | 4239232 | 4239510 |
| JW3990(yjbA) | | 4239780 | 4240190 |
| JW3991(xylE) | Complementary strand | 4240234 | 4241709 |
| JW3992(malG) | Complementary strand | 4242081 | 4242971 |
| JW3993(malF) | Complementary strand | 4242986 | 4244530 |
| JW3994(malE) | Complementary strand | 4244684 | 4245874 |
| JW3995(malK) | | 4246239 | 4247354 |
| JW3996(lamB) | | 4247426 | 4248766 |
| JW3997(malM) | | 4249009 | 4249929 |
| JW3998(yjbl) | | 4250410 | 4251738 |
| JW3999(ubiC) | | 4251850 | 4252458 |
| JW4000(ubiA) | | 4252471 | 4253343 |
| JW4001(plsB) | Complementary strand | 4253498 | 4255921 |
| JW4002(dgkA) | | 4256092 | 4256460 |
| JW4003(lexA) | | 4256570 | 4257178 |
| JW4004(dinF) | | 4257197 | 4258576 |
| JW4005(yjbJ) | | 4258692 | 4258901 |
| JW4006(yjbK) | Complementary strand | 4258943 | 4259518 |
| JW4007(yjbL) | | 4259776 | 4260030 |
| JW4008(yjbM) | | 4260054 | 4260761 |
| JW4009(yjbN) | | 4261124 | 4262161 |
| JW4010(yjbO) | | 4262085 | 4262537 |
| JW4011 (qor) | Complementary strand | 4262703 | 4263686 |
| JW4012(dnaB) | | 4263769 | 4265184 |
| JW4013(alr) | | 4265237 | 4266316 |
| JW4014(tyrB) | | 4266569 | 4267762 |
| JW4015(napA) | | 4268869 | 4269582 |
| JW4016(hobH) | Complementary strand | 4269310 | 4269537 |
| JW4017(yjbQ) | | 4269693 | 4270109 |
| JW4018(yjbR) | | 4270113 | 4270469 |
| JW4019(uvrA) | Complementary strand | 4270504 | 4273326 |
| JW4020(ssb) | | 4273580 | 4274116 |
| JW4021(yjcB) | Complementary strand | 4274215 | 4274565 |
| JW4022(yjcC) | | 4274926 | 4276512 |
| JW4023(soxS) | Complementary strand | 4276515 | 4276838 |
| JW4024(soxR) | | 4276924 | 4277388 |
| JW4025(yjcD) | | 4277934 | 4279283 |
| JW4026(yjcE) | | 4279435 | 4281084 |
| JW4027(yjcF) | Complementary strand | 4281238 | 4282530 |
| JW4028(yjcG) | Complementary strand | 4282708 | 4284357 |
| JW4029(yjcH) | Complementary strand | 4284354 | 4284668 |
| JW4030(acs) | Complementary strand | 4284868 | 4286826 |
| JW4031 (nrfA) | | 4287219 | 4288655 |
| JW4032(nrfB) | | 4288700 | 4289266 |
| JW4033(nrfC) | | 4289263 | 4289934 |
| JW4034(nrfD) | | 4289931 | 4290887 |
| JW4035(nrfE) | | 4290967 | 4292625 |
| JW4036(nrfF) | | 4292618 | 4293001 |
| JW4037(nrfG) | | 4292998 | 4293594 |
| JW4038(gltP) | | 4293936 | 4295249 |
| JW4039(yjcO) | Complementary strand | 4295891 | 4296580 |
| JW4040(fdhF) | Complementary strand | 4296674 | 4298821 |
| JW4041(yjcP) | Complementary strand | 4299019 | 4300485 |
| JW4042(yjcQ) | Complementary strand | 4300482 | 4302533 |
| JW4043(yjcR) | Complementary strand | 4302533 | 4303564 |
| JW4044(yjcS) | Complementary strand | 4304067 | 4306064 |
| JW4045(alsK) | Complementary strand | 4306325 | 4307254 |
| JW4046(alsE) | Complementary strand | 4307238 | 4307933 |
| JW4047(alsC) | Complementary strand | 4307944 | 4308924 |
| JW4048(alsA) | Complementary strand | 4308903 | 4310435 |
| JW4049(alsB) | Complementary strand | 4310562 | 4311497 |
| JW4050(alsR) | Complementary strand | 4311556 | 4312446 |
| JW4051(rpiB) | | 4312805 | 4313254 |
| JW4052(phnQ) | Complementary strand | 4313265 | 4313672 |
| JW4053(phnP) | Complementary strand | 4313798 | 4314556 |
| JW4054(phnO) | Complementary strand | 4314558 | 4314992 |
| JW4055(phnN) | Complementary strand | 4314979 | 4315536 |
| JW4056(phnM) | Complementary strand | 4315536 | 4316672 |
| JW4057(phnL) | Complementary strand | 4316669 | 4317349 |
| JW4058(phnK) | Complementary strand | 4317460 | 4318218 |
| JW4059(phnJ) | Complementary strand | 4318215 | 4319060 |
| JW4060(phnI) | Complementary strand | 4319053 | 4320117 |
| JW4061(phnH) | Complementary strand | 4320117 | 4320701 |
| JW4062(phnG) | Complementary strand | 4320698 | 4321150 |
| JW4063(phnF) | Complementary strand | 4321151 | 4321876 |
| JW4064(phnE) | Complementary strand | 4321897 | 4322265 |
| JW4065(phnE) | Complementary strand | 4322115 | 4322735 |
| JW4066(phnD) | Complementary strand | 4322790 | 4323806 |
| JW4067(phnC) | Complementary strand | 4323831 | 4324619 |
| JW4068(phnB) | Complementary strand | 4324752 | 4325195 |
| JW4069(phnA) | Complementary strand | 4325853 | 4326188 |
| JW4070(yjdA) | | 4326589 | 4328817 |
| JW4071(yjcZ) | | 4328847 | 4329692 |
| JW4072(proP) | | 4329956 | 4331458 |
| JW4073(basS) | Complementary strand | 4331635 | 4332726 |
| JW4074(basR) | Complementary strand | 4332736 | 4333404 |
| JW4075(yjdB) | Complementary strand | 4333401 | 4335074 |
| JW4076(yjdE) | Complementary strand | 4335148 | 4336485 |
| JW4077(adiY) | Complementary strand | 4336622 | 4337383 |
| JW4078(adi) | Complementary strand | 4337708 | 4339975 |
| JW4079(melR) | Complementary strand | 4340174 | 4341082 |
| JW4080(melA) | | 4341365 | 4342720 |
| JW4081 (melB) | | 4342835 | 4344244 |
| JW4082(yjdF) | Complementary strand | 4344383 | 4345012 |
| JW4083(fumB) | Complementary strand | 4345134 | 4346780 |
| JW4084(dcuB) | Complementary strand | 4346858 | 4348198 |
| JW4085(dcuR) | Complementary strand | 4348769 | 4349488 |
| JW4086(dcuS) | Complementary strand | 4349485 | 4351116 |
| JW4087(yidI) | | 4351297 | 4351527 |
| JW4088(yjdJ) | | 4351539 | 4351811 |
| JW4089(yjdK) | | 4352038 | 4352334 |
| JW4090(lysU) | Complementary strand | 4352654 | 4354171 |
| JW4091 (yjdL) | Complementary strand | 4354408 | 4355865 |
| JW4092(cadA) | Complementary strand | 4355924 | 4358071 |
| JW4093(cadB) | Complementary strand | 4358151 | 4359485 |
| JW4094(cadC) | Complementary strand | 4359850 | 4361388 |
| JW4095(cutA3) | Complementary strand | 4362187 | 4362786 |
| JW4096(cutA2) | Complementary strand | 4362799 | 4364268 |
| JW4097(cutA) | Complementary strand | 4364472 | 4364810 |
| JW4098(dcuA) | Complementary strand | 4364926 | 4366227 |
| JW4099(aspA) | Complementary strand | 4366345 | 4367781 |
| JW4100(fxsA) | | 4368118 | 4368594 |
| JW4101(yjeH) | Complementary strand | 4368610 | 4369866 |
| JW4102(groES) | | 4370142 | 4370435 |
| JW4103(groEL) | | 4370479 | 4372125 |
| JW4104(yjeI) | | 4372230 | 4372616 |
| JW4105(yjeJ) | Complementary strand | 4372819 | 4373688 |
| JW4106(yjeK) | Complementary strand | 4374083 | 4375111 |
| JW4107(efp) | | 4375153 | 4375719 |
| JW4108(ecnB) | | 4376007 | 4376153 |
| JW4109(sugE) | | 4376179 | 4376646 |
| JW4110(blc) | Complementary strand | 4376643 | 4377176 |
| JW4111(ampC) | Complementary strand | 4377265 | 4378398 |
| JW4112(frdD) | Complementary strand | 4378461 | 4378820 |
| JW4113(frdC) | Complementary strand | 4378831 | 4379226 |
| JW4114(frdB) | Complementary strand | 4379237 | 4379971 |
| JW4115(frdA) | Complementary strand | 4379964 | 4381772 |
| JW4116(genX) | | 4382097 | 4383074 |
| JW4117(yjeM) | | 4383251 | 4384795 |
| JW4118(yjeN) | | 4384847 | 4385161 |
| JW4119(yjeO) | | 4385158 | 4385472 |
| JW4120(yjeP) | Complementary strand | 4385501 | 4388824 |
| JW4121(psd) | Complementary strand | 4388846 | 4389814 |
| JW4122(yjeQ) | Complementary strand | 4389911 | 4390963 |
| JW4123(yjeR) | | 4390989 | 4391603 |
| JW4124(yjeS) | Complementary strand | 4392382 | 4393521 |
| JW4125(yjeF) | | 4393520 | 4395067 |
| JW4126(yjeE) | | 4395039 | 4395500 |
| JW4127(amiB) | | 4395519 | 4396856 |
| JW4128(mutL) | | 4396866 | 4398713 |
| JW4129(miaA) | | 4398706 | 4399656 |
| JW4130(hfq) | | 4399742 | 4400050 |
| JW4131(hflX) | | 4400126 | 4401406 |
| JW4132(hflK) | | 4401492 | 4402751 |
| JW4133(hflC) | | 4402754 | 4403758 |
| JW4134(yjeT) | | 4403840 | 4404037 |
| JW4135(purA) | | 4404141 | 4405439 |
| JW4136(yjeB) | | 4405644 | 4406069 |
| JW4137(vacB) | | 4406066 | 4408549 |
| JW4138(yjfH) | | 4408729 | 4409460 |
| JW4139(yjfI) | | 4409587 | 4409988 |
| JW4140(yjfJ) | | 4410007 | 4410705 |
| JW4141(yjfK) | | 4410756 | 4411415 |
| JW4142(yjfL) | | 4411433 | 4411831 |
| JW4143(yjfM) | | 4411841 | 4412479 |
| JW4144(yjfC) | | 4412482 | 4413645 |
| JW4145(aidB) | | 4413714 | 4415354 |
| JW4146(yjfN) | Complementary strand | 4415471 | 4415773 |
| JW4147(yjfO) | Complementary strand | 4415895 | 4416323 |
| JW4148(yjfP) | | 4416406 | 4417155 |
| JW4149(yjfQ) | Complementary strand | 4417152 | 4417907 |
| JW4150(yjfR) | Complementary strand | 4418015 | 4419085 |
| JW4151(sgaT) | | 4419377 | 4420831 |
| JW4152(sgaB) | | 4420847 | 4421152 |
| JW4153(sgaA) | | 4421162 | 4421626 |
| JW4154(sgaH) | | 4421640 | 4422290 |
| JW4155(sgaU) | | 4422300 | 4423154 |
| JW4156(sgaE) | | 4423154 | 4423840 |
| JW4157(yjfY) | Complementary strand | 4423970 | 4424245 |
| JW4158(rpsF) | | 4424572 | 4424967 |
| JW4159(priB) | | 4424974 | 4425288 |
| JW4160(rpsR) | | 4425293 | 4425520 |
| JW4161 (rpll) | | 4425562 | 4426011 |
| JW4162(yjfZ) | Complementary strand | 4426082 | 4426876 |
| JW4163(ytfA) | | 4427223 | 4427549 |
| JW4164(ytfB) | Complementary strand | 4427533 | 4428207 |
| JW4165(fklB) | | 4428230 | 4429009 |
| JW4166(cycA) | | 4429318 | 4430730 |
| JW4167(ytfE) | Complementary strand | 4430775 | 4431437 |
| JW4168(ytfF) | Complementary strand | 4431545 | 4432519 |
| JW4169(ytfG) | Complementary strand | 4432618 | 4433478 |
| JW4170(ytfH) | | 4433477 | 4433947 |
| JW4171(cpdB) | Complementary strand | 4434076 | 4436019 |
| JW4172(cysQ) | | 4436209 | 4436949 |
| JW4173(ytfI) | | 4437161 | 4437661 |
| JW4174.1(657#1.2) | | 4437652 | 4438098 |
| JW4175(ytfJ) | Complementary strand | 4438161 | 4438715 |
| JW4176(ytfK) | | 4439001 | 4439246 |
| JW4177(ytfL) | Complementary strand | 4439325 | 4440668 |
| JW4178(msrA) | Complementary strand | 4440991 | 4441629 |
| JW4179(ytfM) | | 4441835 | 4443568 |
| JW4180(ytfN) | | 4443565 | 4447344 |
| JW4181 (ytfP) | | 4447347 | 4447688 |
| JW4182(yzfA) | Complementary strand | 4447429 | 4447698 |
| JW4183(chpS) | | 4447894 | 4448151 |
| JW4184(chpB) | | 4448145 | 4448495 |
| JW4185(ppa) | Complementary strand | 4448575 | 4449105 |
| JW4186(ytfQ) | | 4449415 | 4450371 |
| JW4187(ytfR) | | 4450509 | 4451762 |
| JW4188(ytfS) | | 4451738 | 4452010 |
| JW4189(ytFT) | | 4452021 | 4453046 |
| JW4190(yjfF) . | | 4453057 | 4454028 |
| JW4191 (fbp) | Complementary strand | 4454061 | 4455059 |
| JW4192(mpI) | | 4455235 | 4456608 |
| JW4193(yjgA) | Complementary strand | 4456764 | 4457315 |
| JW4194(pmbA) | | 4457409 | 4458761 |
| JW4195(cybC) | | 4459003 | 4459305 |
| JW4196(nrdG) | Complementary strand | 4459350 | 4459814 |
| JW4197(nrdD) | Complementary strand | 4459972 | 4462110 |
| JW4198(treC) | Complementary strand | 4462504 | 4464159 |
| JW4199(treB) | Complementary strand | 4464209 | 4465630 |
| JW4200(treR) | Complementary strand | 4465749 | 4466696 |
| JW4201(mgtA) | | 4467075 | 4469771 |
| JW4202(yjgF) | Complementary strand | 4469977 | 4470402 |
| JW4203(pyrI) | Complementary strand | 4470436 | 4470897 |
| JW4204(pyrB) | Complementary strand | 4470910 | 4471845 |
| JW4205(pyrBI) | Complementary strand | 4471849 | 4471983 |
| JW4206(yjgH) | Complementary strand | 4472264 | 4472659 |
| JW4207(yjgI) | Complementary strand | 4472790 | 4473503 |
| JW4208(yjgJ) | | 4473574 | 4474167 |
| JW4209(yjgK) | | 4474303 | 4474764 |
| JW4210(yjgL) | | 4474851 | 4476701 |
| JW4211(argI) | Complementary strand | 4476746 | 4477750 |
| JW4212(yjgD) | | 4477912 | 4478328 |
| JW4213(yjgM) | Complementary strand | 4478473 | 4478766 |
| JW4214(yjgN) | | 4479186 | 4480367 |
| JW4215(valS) | Complementary strand | 4480426 | 4483281 |
| JW4216(holC) | Complementary strand | 4483281 | 4483724 |
| JW4217(xerB) | Complementary strand | 4483884 | 4485395 |
| JW4218(yjgP) | | 4485662 | 4486762 |
| JW4219(yjgQ) | | 4486759 | 4487844 |
| JW4220(yjgR) | Complementary strand | 4488005 | 4489507 |
| JW4221(idnR) | Complementary strand | 4489585 | 4490583 |
| JW4222(idnT) | Complementary strand | 4490650 | 4491969 |
| JW4223(idnO) | Complementary strand | 4492031 | 4492795 |
| JW4224(idnD) | Complementary strand | 4492819 | 4493850 |
| JW4225(idnK) | | 4494067 | 4494630 |
| JW4226(yjgB) | Complementary strand | 4494634 | 4495695 |
| JW4227(intB) | | 4496194 | 4497384 |
| JW4228(660#10.1) | Complementary strand | 4497565 | 4497705 |
| JW4229(yi2A) | | 4497671 | 4498081 |
| JW4230(yi22) | | 4498039 | 4498944 |
| JW4231 (yi24) | Complementary strand | 4498084 | 4498515 |
| JW4232(yi23) | | 4498112 | 4498405 |
| JW4233(yjgW) | | 4499043 | 4499378 |
| JW4234(yjgX) | Complementary strand | 4499487 | 4499933 |
| JW4235(yjgY) | Complementary strand | 4499876 | 4500325 |
| JW4236(yjgZ) | | 4500704 | 4501033 |
| JW4237(yi41) | Complementary strand | 4501547 | 4502893 |
| JW4238(yi42) | | 4502279 | 4502746 |
| JW4239(yjhB) | | 4503442 | 4504719 |
| JW4240(yjhC) | | 4504716 | 4505849 |
| JW4241(yjhD) | Complementary strand | 4506070 | 4506444 |
| JW4242(yjhE) | | 4506305 | 4506553 |
| JW4243(yi91) | | 4506605 | 4506907 |
| JW4244(662#3.2) | Complementary strand | 4506910 | 4508061 |
| JW4245(662#4) | | 4508402 | 4508998 |
| JW4246(662#5) | | 4509164 | 4509577 |
| JW4247(fecE) | Complementary strand | 4510134 | 4510901 |
| JW4248(fecD) | Complementary strand | 4510902 | 4511858 |
| JW4249(fecC) | Complementary strand | 4511855 | 4512853 |
| JW4250(fecB) | Complementary strand | 4512850 | 4513752 |
| JW4251 (fecA) | Complementary strand | 4513797 | 4516121 |
| JW4252(fecR) | Complementary strand | 4516208 | 4517161 |
| JW4253(fecI) | Complementary strand | 4517158 | 4517679 |
| JW4254(insA) | | 4517971 | 4518246 |
| JW4255(insB) | | 4518165 | 4518458 |
| JW4256(insB) | | 4518459 | 4518668 |
| JW4257(yjhU) | Complementary strand | 4518782 | 4519582 |
| JW4258(yjhF) | Complementary strand | 4520115 | 4521464 |
| JW4259(yjhG) | Complementary strand | 4521571 | 4523538 |
| JW4260(yjhH) | Complementary strand | 4523549 | 4524508 |
| JW4261(yjhI) | Complementary strand | 4524459 | 4525247 |
| JW4262(sgcR) | Complementary strand | 4525550 | 4526332 |
| JW4263(sgcE) | Complementary strand | 4526349 | 4526981 |
| JW4264(sgcA) | Complementary strand | 4526993 | 4527424 |
| JW4265(sgcQ) | Complementary strand | 4527555 | 4528361 |
| JW4266(sgcC) | Complementary strand | 4528374 | 4529687 |
| JW4266.5(664#6) | Complementary strand | 4529699 | 4529977 |
| JW4267(sgcX) | Complementary strand | 4529974 | 4531125 |
| JW4268(yjhP) | Complementary strand | 4531881 | 4532627 |
| JW4269(yjhQ) | Complementary strand | 4532683 | 4533228 |
| JW4269.5(664#11) | Complementary strand | 4533240 | 4533497 |
| JW4270(664#12) | Complementary strand | 4533988 | 4534119 |
| JW4270.5(664#13) | | 4534283 | 4534492 |
| JW4271 (yjhR) | | 4534459 | 4535475 |
| JW4272(yjhS) | Complementary strand | 4536058 | 4537038 |
| JW4273(yjhT) | Complementary strand | 4537103 | 4538317 |
| JW4274(yjhA) | Complementary strand | 4538229 | 4538954 |
| JW4275(fimB) | | 4540401 | 4541003 |
| JW4276(fimE) | | 4541481 | 4542077 |
| JW4277(fimA) | | 4542559 | 4543107 |
| JW4278(fimI) | | 4543064 | 4543711 |
| JW4279(fimC) | | 4543748 | 4544473 |
| JW4280(fimD) | | 4544541 | 4547177 |
| JW4281 (fimF) | | 4547187 | 4547717 |
| JW4282(fimG) | | 4547730 | 4548233 |
| JW4283(fimH) | | 4548253 | 4549155 |
| JW4284(gntP) | Complementary strand | 4549398 | 4550741 |
| JW4285(uxuA) | | 4551081 | 4552265 |
| JW4286(uxuB) | | 4552346 | 4553806 |
| JW4287(uxuR) | | 4554021 | 4554794 |
| JW4288(yjiC) | Complementary strand | 4554935 | 4555765 |
| JW4289(yjiD) | | 4556429 | 4556830 |
| JW4290(yjiE) | Complementary strand | 4556823 | 4557734 |
| JW4291 (iadA) | Complementary strand | 4557799 | 4558971 |
| JW4292(yjiG) | Complementary strand | 4558984 | 4559445 |
| JW4293(yjiH) | Complementary strand | 4559442 | 4560137 |
| JW4294(yjiI) | | 4560273 | 4560929 |
| JW4295(yjiJ) | Complementary strand | 4560942 | 4562120 |
| JW4296(yjiK) | Complementary strand | 4562188 | 4563159 |
| JW4296.5(668#5) | Complementary strand | 4563113 | 4563370 |
| JW4297(yjiL) | Complementary strand | 4563367 | 4564140 |
| JW4298(yjiM) | Complementary strand | 4564144 | 4565316 |
| JW4299(yjiN) | Complementary strand | 4565411 | 4566691 |
| JW4300(yjiO) | Complementary strand | 4566732 | 4567964 |
| JW4301 (yjiP) | | 4568443 | 4568754 |
| JW4302(yjiQ) | | 4568803 | 4569363 |
| JW4303(yjiR) | Complementary strand | 4569607 | 4571019 |
| JW4304(yjiS) | | 4571196 | 4571360 |
| JW4305(yjiT) | | 4571811 | 4573376 |
| JW4306(yjiV) | | 4573580 | 4575121 |
| JW4307(mcrD) | | 4575072 | 4576301 |
| JW4308(mcrC) | Complementary strand | 4576358 | 4577434 |
| JW4309(mcrB) | Complementary strand | 4577404 | 4578801 |
| JW431 0(yjiW) | Complementary strand | 4578945 | 4579343 |
| JW4311 (hsdS) | Complementary strand | 4579514 | 4580908 |
| JW4312(hsdM) | Complementary strand | 4580905 | 4582494 |
| JW4313(hsdR) | Complementary strand | 4582695 | 4586261 |
| JW4314(mrr) | | 4586395 | 4587309 |
| JW4315(yjiA) | Complementary strand | 4587355 | 4588209 |
| JW4316(yjiX) | Complementary strand | 4588322 | 4588525 |
| JW4317(yjiY) | Complementary strand | 4588575 | 4590740 |
| JW4318(tsr) | | 4591103 | 4592758 |
| JW4319(yjjL) | Complementary strand | 4592807 | 4594168 |
| JW4320(671#8) | | 4592916 | 4593062 |
| JW4321(yjjM) | Complementary strand | 4594383 | 4595189 |
| JW4322(yjjN) | | 4595420 | 4596457 |
| JW4323(mdoB) | Complementary strand | 4596595 | 4598847 |
| JW4324(yjjA) | Complementary strand | 4599140 | 4599637 |
| JW4325(dnaC) | Complementary strand | 4599683 | 4600420 |
| JW4326(dnaT) | Complementary strand | 4600423 | 4600962 |
| JW4327(yjjB) | Complementary strand | 4601069 | 4601395 |
| JW4328(yjjP) | Complementary strand | 4601533 | 4602366 |
| JW4329(yijQ) | | 4602922 | 4603647 |
| JW4330(bglJ) | | 4603605 | 4604282 |
| JW4331(fhuF) | Complementary strand | 4604320 | 4605108 |
| JW4332(yjjZ) | | 4605210 | 4605485 |
| JW4333(rsmC) | Complementary strand | 4606114 | 4607145 |
| JW4334(holD) | | 4607248 | 4607661 |
| JW4335(riml) | | 4607630 | 4608076 |
| JW4336(yijG) | | 4608091 | 4608768 |
| JW4337(prfC) | | 4608859 | 4610448 |
| JW4338(osmY) | | 4610841 | 4611446 |
| JW4339(674#2) | | 4611555 | 4611734 |
| JW4340(yjjU) | | 4611856 | 4612929 |
| JW4341(yjjV) | | 4612926 | 4613705 |
| JW4342(yjjW) | Complementary strand | 4614125 | 4614988 |
| JW4343(yjjI) | Complementary strand | 4614960 | 4616510 |
| JW4344(deoC) | | 4616768 | 4617547 |
| JW4345(deoA) | | 4617674 | 4618996 |
| JW4346(deoB) | | 4619048 | 4620271 |
| JW4347(deoD) | | 4620328 | 4621047 |
| JW4348(yjjJ) | | 4621214 | 4622545 |
| JW4349(lplA) | Complementary strand | 4622546 | 4623562 |
| JW4350(smp) | Complementary strand | 4623590 | 4624234 |
| JW4351(serB) | | 4624340 | 4625308 |
| JW4352(radA) | | 4625357 | 4626739 |
| JW4353(nadR) | | 4626739 | 4627992 |
| JW4354(yjjK) | Complementary strand | 4628300 | 4629967 |
| JW4355(slt) | | 4630178 | 4632115 |
| JW4356(trpR) | | 4632205 | 4632531 |
| JW4357(yjjX) | Complementary strand | 4632678 | 4633199 |
| JW4358(gpmG) | | 4633242 | 4633889 |
| JW4359(rob) | Complementary strand | 4633886 | 4634755 |
| JW4360(creA) | | 4634966 | 4635439 |
| JW4361(creB) | | 4635452 | 4636141 |
| JW4362(creC) | | 4636141 | 4637565 |
| JW4363(creD) | | 4637623 | 4638975 |
| JW4364(arcA) | Complementary strand | 4639035 | 4639751 |
| JW4365(yjjY) | | 4639847 | 4639987 |
| JW4366(lasT) | | 4640387 | 4641073 |
| JW4367(thrL) | | 4641287 | 4641352 |

Further, the *E*. *coli* mutant strain of the present invention includes;
(43) The *E. coli* mutant strain according to any one of (1) to (42) obtained via modification by a method selected from among the following [1] to [4]:
[1] a method for increasing the expression level of at least one enzyme associated with biosynthesis of the useful substance;
[2] a method for increasing the copy number of at least one enzyme gene associated with biosynthesis of the useful substance;
[3] a method for attenuating or blocking at least one metabolic pathway which branches from the biosynthetic pathway of the useful substance into metabolites other than the useful substance; or
[4] a method for selecting a strain that exhibits higher resistance to an analogue of a useful substance than a wild-type *E*. *coli* strain.

The *E. coli* mutant strains described in (1) to (42) above appear to lack parts of or entire genes that encode proteins that are not directly involved in biosynthesis of useful substances but indirectly suppress generation of such useful substances. Such mutant strains and the mutant strain (43) are useful for producing useful substances.

The *E*. *coli* mutant strain of the present invention may be any *E*. *coli* mutant strain, provided that it belongs to *Escherichia coli.* It is preferably a mutant strain of the *E*. *coli* K-12 strain, the *E*. *coli* B strain, or the *E*. *coli* W strain, more preferably a mutant strain of the *E. coli* K-12 strain, further preferably a mutant strain of the *E*. *coli* W3110 strain (ATCC27325) or the *E*. *coli* MG1655 strain (ATCC47076), and particularly preferably a mutant strain of the *E*. *coli* W3110 strain (ATCC27325), for example.

### 2. Method for producing the E. coli mutant strain of the present invention

The *E*. *coli* mutant strain of the present invention can be obtained by any method, provided that it allows such mutant strain to be obtained. For example, the following method wherein a given gene or DNA on *E. coli* chromosomal DNA is deleted may be employed.

A gene or DNA of *E. coli* chromosomal DNA can be deleted via a method utilizing homologous recombination, for example. An example of a method utilizing general homologous recombination is a method involving the use of plasmids for homologous recombination that can be prepared by ligating DNA fragments located at both outer sides of the gene or DNA that is intended to be deleted on *E. coli* chromosomal DNA to plasmid DNA having a drug-resistant gene that cannot be autonomously replicated in such *E. coli.*

After the plasmids for homologous recombination are introduced into a host cell by a conventional method, transformants into which the plasmids for homologous recombination have been integrated to chromosomal DNA by homologous recombination are selected using the drug resistance as an indicator. The obtained transformants are cultured in a medium that does not contain the aforementioned drug for several hours to 1 day, the cultures are applied to an agar medium that contains the drug and to an agar medium that does not contain the drug, and strains that do not grow in the former medium but grow in the latter medium are selected. Thus, strains in which the second homologous recombination has taken place on the chromosomal DNA can be obtained. By determining the nucleotide sequence of a region of the chromosomal DNA in which the gene or DNA to be deleted was present, the introduction of deletion of the target gene or DNA on chromosomal DNA can be confirmed.

An example of a method utilizing homologous recombination, and thereby effectively introducing deletion, substitution or addition of nucleotide(s) into multiple genes, is a method involving the use of a linear DNA.

Specifically, a linear DNA that contains DNA fragments existing at both outside regions of a gene or DNA to be deleted on chromosomal DNA is corporated into a cell to cause homologous recombination between the chromosomal DNA and the introduced linear DNA. This method can be applied to any microorganisms, provided that such *Escherichia coli* effectively incorporate a linear DNA. Such microorganisms are preferably *Escherichia coli* that expresses λ phage-derived recombinant proteins (Red recombination system).

An example of *Escherichia coli* that expresses the λRed recombination system is *Escherichia coli* JM101 comprising pKD46, which is plasmid DNA having the gene of the λ Red recombination system (available from the *E. coli* Genetic Stock Center, Yale University, U.S.A.).

Examples of DNAs that can be used for homologous recombination include:
(a) a linear DNA comprising, at both ends of the drug-resistant gene, DNA existing at both outside regions of a chromosomal DNA into which the introduction of deletion, substitution or addition of nucleotide(s) is intended, or DNA homologous thereto;
(b) a linear DNA to which DNA existing at both outside regions of a chromosomal DNA into which the introduction of deletion, substitution or addition of nucleotide(s) is intended, or DNA homologous thereto is directly ligated;
(c) a linear DNA comprising, at both ends of DNA to which a drug-resistant gene and a gene that can be used for negative selection are ligated, DNA existing at both outside regions of chromosomal DNA into which the introduction of deletion, substitution or addition of nucleotide(s) is intended, or DNA homologous thereto; and
(d) a linear DNA according to (a), which further comprises a nucleotide sequence which is recognized by yeast-derived Flp recombinase [Proc. Natl. Acad. Sci., U.S.A., 82, 5875, 1985] between a drug-resistant gene and DNA existing at both outside regions of a chromosomal DNA or DNA homologous thereto.

Any drug-resistant genes can be used, provided that such genes impart drug resistance to a drug to which *E. coli* shows sensitivity. For example, kanamycin-resistant genes, chloramphenicol-resistant genes, gentamicin-resistant genes, spectinomycin-resistant genes, tetracycline-resistant genes, or ampicillin-resistant genes can be used as drug-resistant genes.

The genes that can be used for negative selection are genes that are lethal to *E. coli* under given culture conditions, when such genes are expressed in *E. coli.* Examples of such genes include *sacB* genes derived from the microorganism which belongs to the genus *Bacillus* [Appl. Environ. Microbiol., 59, 1361-1366, 1993] and *rpsL* genes derived from the microorganism which belongs to the genus *Escherichia* [Genomics, 72, 99-104, 2001].

DNA that exists at both ends of the aforementioned linear DNA which is located at both outside regions of a chromosomal DNA into which introduction of substitution or deletion of nucleotide(s) is intended, or DNA homologous thereto, is oriented on the linear DNA in the same direction as the direction thereof on the chromosomal DNA. The length thereof is preferably about 10 bp to 100 bp, more preferably about 20 bp to 50 bp, and further preferably about 30 bp to 40 bp.

The nucleotide sequence that is recognized by yeast-derived Flp recombinase may be a nucleotide sequence that is recognized by yeast-derived Flp recombinase and has a nucleotide sequence which catalyze homologous recombination, provided that the aforementioned protein recognizes such nucleotide sequence and catalyzes homologous recombination of such nucleotide sequence.

"A DNA homologous thereto" refers to a DNA which has identity to the extent that homologous recombination takes place between the aforementioned linear DNA and a target region on a chromosomal DNA. Specifically, it refers to 80% or more, preferably 90% or more, more preferably 95% or more, and further preferably 100% homology.

Homology of the nucleotide sequences can be determined using the aforementioned programs such as BLAST or FASTA.

The aforementioned linear DNA can be prepared by PCR. Also, DNA containing the linear DNA can be constructed on the plasmid and the linear DNA of interest can then be obtained by restriction enzyme treatment.

Deletion, substitution, or addition of nucleotide(s) can be introduced into chromosomal DNAs of *E. coli* by any of the following methods 1 to 4:
Method 1: a method wherein the linear DNA (a) or (d) is introduced into a host microorganism, and a transformant, into which such linear DNA has been integrated into a chromosomal DNA by homologous recombination, is selected using drug resistance as an indicator;
Method 2: a method wherein a DNA to which DNA existing at both outside regions of a chromosomal DNA into which the introduction of deletion, substitution or addition of nucleotide(s) is intended, or DNA homologous thereto is directly ligated, is introduced into the transformant obtained by the above method 1, and the drug-resistant gene integrated into chromosomal DNA by this method is deleted so as to substitute or delete a region on a chromosomal DNA of a microorganism;
Method 3:
   [1] introducing the linear DNA (c) into a host microorganism and selecting a transformant into which the linear DNA has been integrated into chromosomal DNA by homologous recombination using drug resistance as an indicator,
   [2] synthesizing a DNA obtained by ligating DNAs homologous to DNAs located at both outside regions of a target region of substitution or deletion on a chromosomal DNA in the same direction on the chromosomal DNA and introducing the synthesized product into the transformant obtained in [1] above, and
   [3] culturing transformants subjected to the procedure [2] under conditions where the genes that can be used for negative selection are expressed, and selecting strains that can grow in the culture condition as strains in which the drug-resistant genes and genes that can be used for negative selection have been deleted from the chromosomal DNA; and
Method 4:
   [1] introducing the linear DNA (d) into a host microorganism and selecting a transformant into which the linear DNA has been integrated into chromosomal DNA by homologous recombination using drug resistance as an indicator, and
   [2] introducing Flp recombinase gene-expressing plasmid into the transformant obtained in the above [1] to express the Flp recombinase gene, and obtaining strains sensitive to the drug used in the above[1].

A method of introducing a linear DNA into a host microorganism that is employed in the above methods can be any method as long as the DNA can be introduced into the microorganism. Examples thereof include a method involving the use of calcium ions [Proc. Natl. Acad. Sci., U.S.A., 69, 2110, 1972], the protoplast method [Japanese Published Unexamined Patent Application No. 2483942/88(1988)], and electroporation [Nucleic Acids Res., 16, 6127, 1988].

By using the DNA comprising at a site around the center thereof any gene to be inserted into a chromosomal DNA as the DNA that is used in method 2 or method 3 [2], drug-resistant genes or the like can be deleted, and any genes can be simultaneously introduced into the chromosomal DNA.

According to the methods 2 to 4, foreign genes, such as drug-resistant genes and genes that can be used for negative selection, do not remain in the chromosomal DNA of the transformant which is finally obtained. With the utilization of such methods, accordingly, the same drug-resistant genes and genes that can be used for negative selection may be used, and the procedures of such methods are repeated. This enables the easy production of *E. coli* having deletion of gene or DNA at two or more different regions on a chromosomal DNA.

The amounts of the *E*. *coli* mutant strains obtained by such method are greater than those of wild-type *E*. *coli* strains after a given period of culture. This can be confirmed by, for example, culturing the wild-type parental *E*. *coli* strains and the mutant strains using M9 minimal medium that contains 1% glucose, 0.6% disodium phosphate, 0.3% potassium dihydrogenphosphate, 0.05% sodium chloride, 0.1% ammonium chloride, 2 mmol/l of magnesium sulfate heptahydrate, 10 mg/l of iron sulfate and 100 µmol/l of calcium chloride, or LB liquid medium containing 3% glucose (10 g/l of Bacto tryptone (Difco), 5 g/l of Bacto yeast extract (Difco), 5 g/l of sodium chloride, and 1 ml/l of 1 mol/l sodium hydroxide), and assaying absorption of culture products at 660 nm or 590 nm at a given frequency, or assaying an ATP content per unit of medium via a conventional technique.

Also, the *E. coli* mutant strain of the present invention can be obtained by a method wherein a gene or DNA of chromosomal DNA of *E*. *coli* capable of producing a useful substance is deleted or a method wherein the capacity for producing a useful substance is imparted to *E. coli* from which a gene or DNA of chromosomal DNA has been deleted.

*E. coli* capable of producing a useful substance may be any *E. coli,* provided that it is capable of producing one or more types of useful substances. Examples of such *E. coli* include a strain isolated from nature that is capable of producing a useful substance, and *E*. *coli* to which the ability to produce a desirable useful substance has been artificially imparted by conventional methods.

Examples of such conventional methods include:
(a) a method for reducing or removing at least one mechanism for regulating biosynthesis of a useful substance;
(b) a method for increasing the expression level of at least one enzyme associated with biosynthesis of a useful substance;
(c) a method for increasing the copy number of at least one enzyme gene associated with biosynthesis of a useful substance;
(d) a method for attenuating or blocking at least one metabolic pathway which branches from a biosynthetic pathway of a useful substance into metabolites other than the useful substance; and
(e) a method for selecting a strain that exhibits higher resistance to an analogue of a useful substance than a wild-type strain.
   Such conventional methods can be carried out alone or in combinations of two or more.

When useful substances are amino acids, for example, method (a) is described in Agric. Biol. Chem., 43, 105-111, 1979, J. Bacteriol., 110, 761-763, 1972, and Appl. Microbiol. Biotechnol., 39, 318-323, 1993; method (b) is described in Agric. Biol. Chem., 43, 105-111, 1979 and J. Bacteriol., 110, 761-763, 1972; method (c) is described in Appl. Microbiol. Biotechnol., 39, 318-323, 1993 and Agric. Biol. Chem., 39, 371-377, 1987; method (d) is described in Appl. Environ. Microbiol., 38, 181-190, 1979 and Agric. Biol. Chem., 42, 1773-1778, 1978; and method (e) is described in Agric. Biol. Chem., 36, 1675-1684, 1972, Agric. Biol. Chem., 41, 109-116, 1977, Agric. Biol. Chem., 37, 2013-2023, 1973, and Agric. Biol. Chem., 51, 2089-2094, 1987. With reference to these literatures, microorganisms capable of forming and accumulating various amino acids can be produced.

Also, many examples of a method for producing microorganisms capable of forming and accumulating amino acids by any of or two or more of methods (a) to (e) above are described in Biotechnology 2nd ed., vol. 6, Products of Primary Metabolism (VCH Verlagsgesellschaft mbH, Weinheim, 1996), section 14a or 14b, Advances in Biochemical Engineering/Biotechnology 79, 1-35, 2003, or Amino san Hakko (Amino acid fermentation), Japan Scientific Societies Press, Hiroshi Aida et al. (1986). In addition, many reports have been made concerning a specific method for producing microorganisms capable of forming and accumulating amino acids. Examples thereof include Japanese Published Unexamined Patent Application No. 2003-164297, Agric. Biol. Chem., 39, 153-160, 1975, Agric. Biol. Chem., 39, 1149-1153, 1975, Japanese Published Unexamined Patent Application No. 13599/83(1983), J. Gen. Appl. Microbiol., 4, 272-283, 1958, Japanese Published Unexamined Patent Application No. 94985/88(1988), Agric. Biol. Chem., 37, 2013-2023, 1973, WO 97/15673, Japanese Published Unexamined Patent Application No. 18596/81(1981), Japanese Published Unexamined Patent Application No. 144092/81(1981), and Japanese Published Unexamined Patent Application No. 2003-511086. With reference to these literatures, microorganisms capable of producing one or more kinds of amino acids can be produced. Also, *E*. *coli* capable of producing other useful substance can be produced by increase of biosynthetase of said useful substance, and improvement of biosynthesis reguratory mechanism, elimination mechanism and the like in the same way as in the production of *E. coli* mutant strain capable of producing an amino acid.

### 3. Method for producing useful substances of the present invention

The present invention relates to a method for producing useful substances comprising culturing the *E*. *coli* mutant strain of the present invention in a medium so as to generate and accumulate such useful substances in the culture and recovering the useful substances from the culture.

Useful substances are not particularly limited, provided that such substances can be produced with the use of the *E*. *coli* mutant strain of the present invention. Examples thereof include proteins, peptides, amino acids, nucleic acids, vitamins, saccharides, organic acids, and lipids. Examples of proteins include inosine kinase, glutamate 5-kinase (EC 2.7.2.11), glutamate-5-semialdehyde dehydrogenase (EC 1.2.1.41), pyrroline-5-carboxylate reductase (EC 1.5.1.2), and human granulocyte-colony-stimulating factors. An example of peptide is glutathione. Examples of amino acids include L-alanine, glycine, L-glutamine, L-glutamic acid, L-asparagine, L-aspartic acid, L-lysine, L-methionine, L-threonine, L-leucine, L-valine, L-isoleucine, L-proline, L-histidine, L-arginine, L-tyrosine, L-tryptophane, L-phenylalanine, L-serine, L-cysteine, L-3-hydroxyproline, and L-4-hydroxyproline. Examples of nucleic acids include inosine, guanosine, inosinic acid, and guanylic acid. Examples of vitamins include riboflavin, thiamine, and ascorbic acid. Examples of saccharides include xylose and xylitol. Examples of lipids include eicosapentaenoic acid (EPA) and docosahexaenoic acid (DHA).

The *E*. *coli* mutant strain that is used for the production method of the present invention can be cultured in accordance with a conventional technique as described below.

A medium for culturing *E. coli* mutant strain which is used for the production method of the present invention may be natural or synthetic medium as long as it contains carbon sources, nitrogen sources, inorganic salts, etc. assimilable by the microorganisms and the microorganisms can be efficiently cultured therein.

Any carbon sources assimilable by the microorganisms can be used. Examples thereof include: carbohydrates such as glucose, fructose, sucrose, molasses containing any of such substances, starch, and starch hydrolysate; organic acids such as acetic acid and propionic acid; and alcohols such as ethanol and propanol.

Examples of nitrogen sources include: ammonia; ammonium salts of inorganic or organic acids such as ammonium chloride, ammonium sulfate, ammonium acetate, and ammonium phosphate; peptone; meat extract; yeast extract; corn steep liquor; casein hydrolysate; soybean cake and hydrolysate of soybean cake; and various fermentation microorganisms and digests thereof.

Examples of inorganic salts include: monopotassium phosphate, dipotassium phosphate, magnesium phosphate, magnesium sulfate, sodium chloride, iron(I) sulfate, manganese sulfate, copper sulfate, and calcium carbonate.

Culturing is carried out under aerobic conditions such as shaking culture or submerged aeration agitation culture. Culturing temperature is preferably 15 to 40°C, and culture time is generally 16 hours to 7 days. During culturing, the pH is preferably maintained at 3.0 to 9.0. The pH is adjusted with an inorganic or organic acid, an alkali solution, urea, calcium carbonate, ammonia, or the like.

When useful substances are generated and accumulated extracellularly, precipitates such as cells are removed from the culture after the completion of culturing, and target useful substances can be isolated and purified from the culture via ion-exchange treatment, concentration, salting out, or other means. Such techniques can be employed in combination.

When useful substances are generated and accumulated intracellularly, cells are recovered from the culture after the completion of culturing and then disrupted via adequate means such as mechanical or chemical means. Target useful substances can be isolated and purified from the solution of disrupted cells via ion-exchange treatment, concentration, salting out, or other means, and such techniques can be employed in combination.

### Example 1

### Construction of mutant library of E. coli that partially lacks chromosomal DNA

(1) Preparation of *E*. *coli* capable of homologous recombination with linear DNA on chromosomal DNA
   The P1 phages were prepared from *E. coli* KM22 strains (Gene, 246, 321-330, 2000) capable of homologous recombination with linear DNA on chromosomal DNA in the following manner.
   The *E*. *coli* KM 22 strains were cultured in LB liquid medium containing 20 mg/l of kanamycin (10 g/l of Bacto tryptone (Difco), 5 g/l of Bacto yeast extract (Difco), 5 g/l of sodium chloride, and 1 ml/l of 1 mol/l sodium hydroxide) at 30°C overnight. Thereafter, 0.5 ml of culture solution, 1.4 ml of fresh LB liquid medium, and 100 µl of 0.1 mol/l calcium chloride solution were mixed.
   The resulting mixture was subjected to shaking culture at 30°C for 5 to 6 hours, and 400 µl of the mixture was transferred to a sterilized tube. A P1 phage stock solution (2 µl) was added thereto, the mixture was incubated at 37°C for 10 minutes, 3 ml of a soft agar solution maintained at 50°C (10 g/l of Bacto tryptone, 5 g/l of Bacto yeast extract, 5 mmol/l of calcium chloride, 1 ml/l of 1 mol/l sodium hydroxide, and 3 g/l of agar) was added thereto, the resultant was thoroughly agitated, and the resultant was then applied onto a Ca-LB agar medium plate (10 g/l of Bacto tryptone, 5 g/l of Bacto yeast extract, 5 mmol/l of calcium chloride, 1 ml/l of 1 mol/l sodium hydroxide, and 15 g/l of agar; plate diameter: 15 cm).
   The plate was subjected to culture at 37°C for 7 hours, the soft agar portion was finely ground using a spreader and recovered, and the resultant was centrifuged at 1,500 g at 4°C for 10 minutes. Chloroform (100 µl) was added to 1.5 ml of the supernatant, and the mixture was stored at 4°C overnight. On the following day, the solution was centrifuged at 15,000 g for 2 minutes, and the supernatant was stored at 4°C as a phage stock.
   Subsequently, the obtained phage was used to transform the *E. coli* W3110 strain. The culture solution (200 µl) of the *E. coli* W3110 strains that had been cultured in Ca-LB liquid medium at 30°C for 4 hours was fractionated, and 1 µl of phage stock was added. The mixture was incubated at 37°C for 10 minutes, 5 ml of LB liquid medium and 200 µl of 1 mol/l sodium citrate solution were added, and the mixture was agitated. The resultant was centrifuged at 1,500 g at 25°C for 10 minutes, the supernatant was discarded, 1 ml of LB liquid medium and 10 µl of 1 mol/l sodium citrate solution were added to the precipitated cells, and the resultant was incubated at 30°C for 2 hours. The solution (100 µl) was applied to an agar plate medium, Antibiotic Medium 3, containing 20 mg/l of kanamycin, and the plate was subjected to culture at 30°C overnight. Several colonies that had appeared were applied to an agar plate medium, Antibiotic Medium 3, containing 20 mg/l kanamycin (0.15% meat extract, 0.15% yeast extract, 0.5% peptone, 0.1% glucose, 0.35% sodium chloride, 0.132% dibasic potassium phosphate, and 1.5% agar; pH 7), the drug sensitivity thereof was inspected, and kanamycin-resistant strains were selected. Thus, *E*. *coli* W3110red strains into which a chromosomal DNA region (Δ(recC ptr recB recD)::Plac-red kan) capable of homologous recombination with linear DNA on chromosomal DNA had been transduced were obtained.
(2) Construction of mutant library of *E. coli* that lacks part ofchromosomal DNA
   (i) Preparation of competent cells
      The *E*. *coli* W3110red strains obtained in (1) above were cultured at 30°C until the OD at 660 nm reached 0.1 using 100 ml of LB liquid medium comprising 1 mmol/l of isopropyl-thio-β-D-galactopyranoside (IPTG). The following procedure was carried out at 4°C or with the use of an ice-cooled solution.
      The obtained culture was centrifuged, and the precipitated cells were suspended in 50 ml of a 20% glycerol solution. The glycerol solution was centrifuged, the precipitated cells were resuspended in a 20% glycerol solution, such procedure was repeated 3 or more times to wash the cells, and the cells were then suspended in 50 µl of a 10% glycerol solution. Thus, a solution of competent cells for electroporation was obtained.
   (ii) Preparation of DNA fragment used for introducing deletions into chromosomal DNA
      A DNA fragment used for introducing deletions of about 46 kbp DNA having a nucleotide sequence comprising nucleotides 4489585 to 4535472 of the nucleotide sequence as shown in SEQ ID NO: 1 into chromosomal DNA was produced in the following manner.
      DNA having the nucleotide sequence as shown in SEQ ID NO: 2 (DNA comprising a nucleotide sequence comprising nucleotides 4487585 to 4487605 of the nucleotide sequence as shown in SEQ ID NO: 1; hereafter referred to as "primer A"), DNA having the nucleotide sequence as shown in SEQ ID NO: 3 (DNA comprising a nucleotide sequence comprising at the 3' side of a 25-mer linker sequence a nucleotide sequence comprising nucleotides 4489584 to 4489561 of the nucleotide sequence as shown in SEQ ID NO:1; hereafter referred to as "primer B"), DNA having the nucleotide sequence as shown in SEQ ID NO: 4 (DNA comprising a nucleotide sequence comprising nucleotides 4537475 to 4537451 of the nucleotide sequence as shown in SEQ ID NO: 1; hereafter referred to as "primer C"), and DNA having the nucleotide sequence as shown in SEQ ID NO: 5 (DNA comprising a nucleotide sequence comprising at the 3' side of a 22-mer linker sequence a nucleotide sequence comprising nucleotides 4535473 to 4535496 of the nucleotide sequence as shown in SEQ ID NO: 1; hereafter referred to as "primer D") were synthesized.
      PCR was carried out using, as a template, chromosomal DNA of the *E*. *coli* W3110red strain and, as primer sets, a pair of primer A and primer B and a pair of primer C and primer D. PCR was carried out using LA-Taq (Takara Bio Inc.), and a reaction solution was prepared in accordance with the instructions included therein. Purified chromosomal DNA (100 ng) or small amounts of *E. coli* W3110red strains recovered with the use of a tip of a pick and 20 pmol of each primer DNA were added to the reaction solution, and PCR was then carried out. PCR was carried out by incubating at 94°C for 3 minutes, repeating a cycle of 94°C for 15 seconds, 55°C for 20 seconds, and 68°C for 1 minute and 30 seconds 30 times, and then incubating at 72°C for 10 minutes.
      The PCR-amplified DNA fragments were purified using a PCR Purification Kit (Qiagen) and dissolved in 50 µl of water. The DNA fragments were confirmed to be 1-kb DNA fragments homologous to nucleotide sequences at both ends of the target regions for deletion on chromosomal DNA. Hereafter, the DNA fragment amplified with the use of the pair of primer A and primer B is referred to as an upstream arm, and the DNA fragment amplified with the use of the pair of primer C and primer D is referred to as a downstream arm.
      Subsequently, PCR was carried out using, as a template, chromosomal DNA of the *Bacillus subtilis* 168 strain (ATCC23857) and, as a primer set, DNA having the nucleotide sequence as shown in SEQ ID NO: 6 and DNA having the nucleotide sequence as shown in SEQ ID NO: 7. PCR was carried out with the use of a reaction solution of the same composition as above under the same reaction conditions, except for the use of template DNA and primer DNA of different types.
      The PCR-amplified DNA fragments were purified in the same manner as described above and then dissolved in 50 µl of water. Such DNA fragments were confirmed to be sacB genes (Genbank accession no. X02730) derived from *Bacillus subtilis* that can be used for negative selection.
      Subsequently, PCR was carried out using, as a template, a pHSG399 plasmid DNA (Takara Bio Inc.) and, as a primer set, DNA having the nucleotide sequence as shown in SEQ ID NO: 8 and DNA having the nucleotide sequence as shown in SEQ ID NO: 9. PCR was carried out with the use of a reaction solution of the same composition as above under the same reaction conditions, except for the use of template DNA and primer DNA of different types.
      The PCR-amplified DNA fragments were separated by agarose gel electrophoresis, purified using the QIA quick Gel Extraction Kit (Qiagen), and dissolved in 50 µl of water. The DNA fragments were confirmed to comprise chloramphenicol-resistant genes (cat genes).
      Subsequently, PCR was carried out using as templates the DNA fragment comprising the sacB gene and the DNA fragment comprising the cat gene obtained above and, as a primer set, DNA having the nucleotide sequence as shown in SEQ ID NO: 10 and DNA having the nucleotide sequence as shown in SEQ ID NO: 11. PCR was carried out with the use of a reaction solution of the same composition as above under the same reaction conditions, except for the use of template DNA and primer DNA of different types.
      The PCR-amplified DNA fragments were separated by agarose gel electrophoresis, purified using the QIA quick Gel Extraction Kit, and dissolved in 50 µl of water. The DNA fragments were confirmed to comprise the sacB gene and the cat gene ligated in the same direction.
      Subsequently, PCR was carried out using, as template DNA, a mixture of 3 DNA fragments, i.e., the upstream arm, the downstream arm, and the DNA fragment comprising the sacB gene and the cat gene ligated thereto obtained above and, as a primer set, DNA having the nucleotide sequence as shown in SEQ ID NO: 12 (DNA comprising a nucleotide sequence comprising nucleotides 4488585 to 4488610 of the nucleotide sequence as shown in SEQ ID NO: 1) and DNA having the nucleotide sequence as shown in SEQ ID NO: 13 (DNA comprising a nucleotide sequence comprising nucleotides 4536475 to 4536451 of the nucleotide sequence as shown in SEQ ID NO: 1). PCR was carried out with the use of a reaction solution of the same composition as above under the same reaction conditions, except for the use of, as template DNA, 1 µl of a solution of upstream arm DNA, 1 µl of a solution of downstream arm DNA, and 1.5 µl of a DNA fragment comprising the sacB gene and the cat gene ligated thereto and, as a primer set, DNA having the nucleotide sequence as shown in SEQ ID NO: 12 and DNA having the nucleotide sequence as shown in SEQ ID NO: 13.
      The PCR-amplified DNA fragments were separated by agarose gel electrophoresis, purified using the QIA quick Gel Extraction Kit, and dissolved in 50 µl of water. The DNA fragments were confirmed to comprise DNA fragments homologous to both ends of the target region of deletion added to both ends of DNA to which the sacB gene and the cat gene have been ligated (hereafter referred to as a "DNA fragment for regional deletion").
   (iii) Preparation of mutant strain of *E*. *coli* that lacks part of chromosomal DNA
      The DNA fragment for regional deletion obtained above (0.1 µg) was added to 50 µl of a solution of *E*. *coli* 3110red competent cells obtained above, and electroporation was carried out using 0.1 cm cuvette (BioRad) at 1.8 kV and 25 µF. The transformants wherein the DNA fragment for regional deletion incorporated into the chromosomes of the *E*. *coli* W3110red strain were selected on an LBcm plate (10 g/l of Bacto tryptone (Difco), 5 g/l of Bacto yeast extract (Difco), 20 mg/l of kanamycin, 15 mg/l of chloramphenicol, and 1 ml/l of 1 mol/l NaOH). The colonies of the selected transformants were replicated on an suLB agar medium plate (10 g/l of Bacto tryptone (Difco), 5 g/l of Bacto yeast extract (Difco), 10% of sucrose, 20 mg/l of kanamycin, 15 mg/l of chloramphenicol, and 1 ml/l of 1 mol/l NaOH) to select strains exhibiting sucrose sensitivity.
      A nucleotide sequence comprising nucleotides 4489585 to 4535472 of the nucleotide sequence as shown in SEQ ID NO: 1, which is a target region for deletion on chromosomal DNA of the selected strain, had been substituted with DNA comprising the sacB gene and the cat gene ligated thereto. This substitution was confirmed in the following manner.
      PCR was carried out using, as a primer set, DNA comprising the nucleotide sequence as shown in SEQ ID NO: 2 (primer A), which has homologous nucleotide sequences in regions 1 kb upstream and downstream of the ends of the arm sequence of linear DNA used for transformation and DNA comprising the nucleotide sequence as shown in SEQ ID NO: 4 (primer C) and, as a template, chromosomal DNA of the strain selected above. PCR was carried out with the use of a reaction solution of the same composition as above under the same reaction conditions, except for the use of, as a template, small amounts of cell strains selected above (collected from the colonies with the tip of a pick) and, as a primer set, DNA having the nucleotide sequence as shown in SEQ ID NO: 3 and DNA having the nucleotide sequence as shown in SEQ ID NO: 5.
      Through the above PCR procedure, a DNA fragment of about 6.7 kb was amplified. A DNA fragment prepared by adding DNA of about 2 kb to both ends of a DNA fragment (2.7 kb) comprising the sacB gene and the cat gene ligated thereto is of about 6.7 kb. Accordingly, a transformant, which was an amplified fragment of about 6.7 kb, obtained via PCR involving the use of a primer set comprising primer A and primer C, was the transformant of interest. Thus, such transformant was designated as the ΔE_suc strain (a deleted region of 4489585 to 4535472 bp).
   (iv) Preparation of mutant strain of *E*. *coli* that lacks a partial region of chromosomal DNA
      Mutants that lack various regions of chromosomal DNAs of *E*. *coli* W3110red strains were prepared in the same manner used in the case of preparing the ΔE_suc strains.
      A DNA fragment comprising the cat gene and the sucB gene ligated thereto was eliminated from the ΔE_suc strain to prepare an *E*. *coli* mutant strain in the following manner. DNA comprising the nucleotide sequence as shown in SEQ ID NO: 14 (DNA comprising a nucleotide sequence to which nucleotides 4535496 to 4535476 and nucleotides 4489584 to 4489561 of the nucleotide sequence as shown in SEQ ID NO: 1 have been ligated; hereafter referred to as "primer E") and DNA comprising the nucleotide sequence as shown in SEQ ID NO: 15 (DNA comprising a nucleotide sequence to which nucleotides 4489561 to 4489584 and nucleotides 4535476 to 4535496 of the nucleotide sequence as shown in SEQ ID NO: 1 have been ligated; hereafter referred to as "primer F") were synthesized. PCR was carried out using, as a template, chromosomal DNA of the *E. coli* W3110 strain and, as primer sets, a pair of primer A and primer E and a pair of primer C and primer F. PCR was carried out with the use of a reaction solution of the same composition as above under the same reaction conditions, except for the use of a template and primer sets of different types.
      The PCR-amplified DNA fragments were purified using a PCR Purification Kit and dissolved in 50 µl of water. The DNA fragments were confirmed to comprise 2-kb nucleotide sequences homologous to the nucleotide sequences of chromosomal DNA at both ends of the target region for deletion. Hereafter, the DNA fragment amplified with the use of the pair of primer A and primer E is referred to as an upstream arm and the DNA fragment amplified with the use of the pair of primer C and primer F is referred to as a downstream arm.
      Subsequently, PCR was carried out using, as a template, a mixture of the upstream arm and the downstream arm obtained above and, as a primer set, DNA comprising the nucleotide sequence as shown in SEQ ID NO: 12 and DNA comprising the nucleotide sequence as shown in SEQ ID NO: 13. PCR was carried out with the use of a reaction solution of the same composition as above under the same reaction conditions, except for the use of, as templates, 1 µl of a solution of the upstream arm DNA and 1 µl of a solution of the downstream arm DNA obtained above and, as a primer set, DNA comprising the nucleotide sequence as shown in SEQ ID NO: 12 and DNA comprising the nucleotide sequence as shown in SEQ ID NO: 13.
      The PCR-amplified DNA fragments were separated by agarose gel electrophoresis, purified using the QIA quick Gel Extraction Kit, and dissolved in 50 µl of water. It was confirmed that a DNA fragment that is an amplification product of a 2-kb fragment comprising a 1-kb fragment in the deleted region of the both arms was obtained (hereafter referred to as a "DNA fragment for deleting the sacB-cat gene").
      In accordance with the method of (2) (i) above, a solution of ΔE_suc competent cells was prepared, the DNA fragment for gene deletion was added thereto, and such DNA fragment was introduced into the ΔE_suc cell via electroporation. SuLB agar medium was used as a selection medium. The colonies of the selected transformants were replicated on an LBcm agar plate to select strains exhibiting chloramphenicol sensitivity.
      Subsequently, PCR was carried out using, as a template, chromosomal DNA of the sucrose-resistant and chloramphenicol-sensitive strain selected above and, as primer set, the pair of primer A and primer C. PCR was carried out with the use of a reaction solution of the same composition as above under the same reaction conditions, except for the use of template DNA and primer DNA of different types.
      Through the above PCR procedure, an amplified fragment of about 4.0 kb was obtained. An amplified DNA fragment is obtained via PCR using, as a template, chromosomal DNA of the sucrose-resistant and chloramphenicol-sensitive strain into which substitution of interest has been introduced and from which the suc gene and the cat gene have been deleted. Since a region comprising an upstream 2-kbp region and a downstream 2-kbp region adjacent to the deleted region is amplified from such DNA fragment, a band of about 4 kb is amplified. Accordingly, it was confirmed that a partially deleted strain (the ΔE strain) of chromosomal DNA that lacks a specific region on chromosomal DNA so as not to leave the marker gene behind was prepared by the above method.
   (v) Preparation of mutant library of *E. coli* that lacks part of chromosomal DNA
      DNA that can be designed based on nucleotide sequences located at both ends of a target region of deletion on chromosomal DNA was used instead of the primers A to D above to prepare a mutant strain of *E. coli* that lacks an arbitrary region on chromosomal DNA of the *E. coli* W3110red strain in the same manner as described above.

### Example 2

### Growth test using minimal medium

Various mutant strains obtained in Example 1 were applied to an LB agar medium (which is a plate prepared with the addition of 15 g/l of agar to LB medium) and culture was conducted at 37°C overnight to obtain cell strains. Small amounts of such strains were introduced into 5 ml of LB medium containing 50 mg/l of kanamycin and culturing was carried out overnight to obtain culture products. The culture products (50 µl) were inoculated into 5 ml of M9 minimal medium that contained 1% glucose and 10 mg/l of iron sulfate, shaking culture was conducted at 37°C, and turbidity 0, 6, 10, 13, 16, 19, and 32 hours after the initiation of culture was measured based on absorption at 660 nm. Growth of the above mutants and that of their parental strains, *E*. *coli* W3110red strains, were compared, and mutants exhibiting growth curves substantially the same as those of the parental strains were selected.

The deleted regions of chromosomal DNA of the selected mutants and genes included in such regions are shown in Table 2.

### [Table 2]

**Table 2**

| Name of deleted region | Genes contained in deleted region |
|---|---|
| Δ 101B | JW0020(insB), JW0021 (insA) |
| Δ 102 | JW0065(yabJ), JW0066(yabK), JW0067(tbpA), JW0068(yabN) |
| Δ 103 | JW0146(fhuA), JW0147(fhuC), JW0148(fhuD), JW0149(fhuB) |
| Δ 001 | JW0213(yafJ), JW0214(yafK), JW0215(yafQ), JW0216(dinJ), JW0217(yafL), JW0218(yafM), JW0219(fhiA), JW0220(lafU), JW0221(dinP), JW0222(yafN), JW0223(yafO), JW0224(yafP), JW0225(127#12), JW0226(prfH) |
| Δ002 | JW0234(ykfl), JW0235(yafW), JW0235.5(yeeT), JW0236(ykfG), JW0237(yafX), JW0238(ykfF), JW0239(ykfB), JW0240(yafY), JW0241(ypjK), JW0242(yafZ), JW0243(ykfA), JW0244(perR), JW0245(129#3.1), JW0246(129#3.2), JW0247(129#5), JW0248(ykfC), JW0249(130#0.1), JW0250(is5B), JW0251(yi51), JW0252(mmuP), JW0253(mmuM), JW0254(afuC), JW0255(130#4), JW0256(insB), JW0257(insA), JW0258(130#5), JW0259(yagB), JW0260(yagA), JW0261(yagE), JW0262(yagF), JW0263(yagG), JW0264(yagH), JW0265(yagI), JW0266(argF), JW0267(yfjI), JW0268(insB), JW0269(insA), JW0270(yagJ), JW0271(yagK), JW0272(yagL), JW0273(yagM), JW0274(yagN), JW0275(intF), JW0276(yagP), JW0277(yagQ), JW0278(yagR), JW0279(yagS), JW0280(yagT), JW0281 (yagU), JW0282(ykgJ), JW0283(yagV), JW0284(yagW), JW0285(yagX), JW0286(yagY), JW0287(yagZ), JW0288(ykgK), JW0289(ykgL), JW0290(ykgM), JW0291 (eaeH), JW0292(134#4.1), JW0293(134#4.2), JW0294(ykgA), JW0295(ykgB), JW0296(ykgI), JW0297(merA), JW0298(ykgD), JW0299(ykgE), JW0300(ykgF), JW0301(ykgG), JW0302(ykgH), JW0303(betA), JW0304(betB), JW0305(betl), JW0306(betT), JW0307(yahA), JW0308(yahB), JW0309(yahC), JW0310(yahD), JW0311(yahE), JW0312(yahF), JW0313(yahG), JW0314(yahH), JW0315(arcM), JW0316(yahJ), JW0317(yahK), JW0318(yahL), JW0319(yahM), JW0320(yahN), JW0321 (yahO), JW0322(prpR), JW0323(bcpA), JW0324(prpC), JW0325(prpD), JW0326(prpE), JW0327(codB), JW0328(codA), JW0329(cynR), JW0330(cynT), JW0331(cynS), JW0332(cynX), JW0333(lacA), JW0334(lacY), JW0335(lacZ), JW0336(lacI), JW0337(mhpR), JW0338(mhpA), JW0339(mhpB), JW0340(mhpC), JW0341(mhpD), JW0342(mhpF), JW0343(mhpE), JW0344(mhpT), JW0345(yaiL), JW0346(yaiM), JW0347(adhC), JW0348(yaiN), JW0349(yaiO), JW0350(140#9), JW0351 (yi2A), JW0352(yi22), JW0353(yi24), JW0354(yi23), JW0355(yaiP), JW0356(yaiS), JW0357(tauA), JW0358(tauB), JW0359(tauC), JW0360(ssiD) |
| Δ 007 | JW0362(yaiT), JW0363(141#3.1), JW0364(141#3.2), JW0365(yaiU), JW0366(yaiV), JW0367(ampH), JW0368(sbmA), JW0369(yaiW), JW0370(yaiY), JW0371(yaiZ), JW0372(ddlA), JW0373(yaiB), JW0374(phoA), JW0375(psiF), JW0376(yaiC) |
| Δ 009B | JW0482(ybbO), JW0483(tesA), JW0484(ybbA), JW0485(ybbP), JW0486(rhsD), JW0487(ybbC), JW0488(rhsE), JW0489(ybbD), JW0490(156#1), JW0491(ybbB), JW0492(ybbS), JW0493(ybbT), JW0494(ybbU) |
| Δ 010 | JW0497(ybbQ), JW0498(ybbV), JW0499(allP), JW0500(allA), JW0501(ybbY), JW0502(ybbZ), JW0503(ylbA), JW0504(allC), JW0505(ylbC), JW0506(fdrA), JW0507(ylbE), JW0508(ylbE), JW0509(ylbF), JW0510(arcC), |
| Δ011 | JW0525(intD), JW0526(EXO_LAMBD), JW0527(VRPP_LAMBD), JW0528(159#4), JW0529(159#5), JW0530(VREN_LAMBD), JW0531(emrE), JW0532(ybcK), JW0533(ybcL), JW0534(ybcM), JW0535(ybcN), JW0536(ninE), JW0537(ybcO), JW0538(rus), JW0539(ybcQ), JW0539.5(160#10), JW0540(is5B), JW0541(yi51), JW0542(nmpC), JW0543(vlyS), JW0544(ybcS), JW0545(ybcT), JW0546(vboR), JW0547(ybcV), JW0548(ybcW), JW0549(nohB), JW0550(ybcX), JW0551(ybcY), JW0552(161#0.2), JW0553(appY), JW0554(ompT), JW0555(envY), JW0556(ybcH), JW0557(nfrA), JW0558(nfrB), JW0559(cusS), JW0560(cusR), JW0561(cusC), JW0562(ylcC), JW0563(cusB), JW0564(cusA), JW0565(pheP), JW0566(ybdG), JW0567(nfnB), JW0568(ybdF), JW0569(ybdJ), JW0570(ybdK), JW0571(hokE), JW0572(yi81), JW0573(yi82) |
| Δ012 | JW0638(ybeL), JW0639(ybeQ), JW0640(ybeR), JW0641(ybeS), JW0642(ybeT), JW0643(ybeU), JW0644(ybeV), JW0645(ybeW), JW0646(ybeK), JW0647(gltL), JW0648(gltK), JW0649(gltJ), JW0650(ybeJ), JW0651(171#0.1), JW0652(is5B), JW0653(yi51) |
| Δ013B | JW0681(ybfK), JW0682(kdpE), JW0683(kdpD), JW0684(kdpC), JW0685(kdpB), JW0686(kdpA), JW0687(kdpF), JW0688(ybfA), JW0689(rhsC), JW0690(174#4.1), JW0691(ybfB), JW0692(rhsA), JW0693(ybfC), JW0694(ybfD), JW0695(ybfL), JW0696(ybfD), JW0697(ybgA) |
| Δ013 | JW0687(kdpF), JW0688(ybfA), JW0689(rhsC), JW0690(174#4.1), JW0691(ybfB), JW0692(rhsA), JW0693(ybfC), JW0694(ybfD), JW0695(ybfL), JW0696(ybfD), JW0697(ybgA) |
| Δ 104 | JW0743(modF), JW0744(modE), JW0745(180#2), JW0746(modA), JW0747(modB), JW0748(modC) |
| Δ014 | JW0782(204#3.1), JW0783(ybiA), JW0784(dinG), JW0785(ybiB), JW0786(ybiC), JW0787(ybiJ), JW0788(ybiI), JW0789(ybiX), JW0790(ybiL), JW0791(ybiM), JW0792(ybiN), JW0793(ybiO), JW0794(glnQ), JW0795(glnP), JW0796(glnH) |
| Δ015 | JW0813(yliA), JW0814(yliB), JW0815(yliC), JW0816(yliD), JW0817(209#3), JW0818(209#4), JW0819(yliG), JW0820(yliH), JW0821(yliI), JW0822(yliJ), JW0823(dacC), JW0824(deoR), JW0825(ybjG) |
| Δ016 | JW0834(ybjC), JW0835(mdaA), JW0836(rimK), JW0837(ybjN), JW0838(potF), JW0839(potG), JW0840(potH), JW0841(potI), JW0842(ybjO), JW0843(ybjF), JW0844(artJ), JW0845(artM), JW0846(artQ), JW0847(artI), JW0848(artP), JW0849(ybjP), JW0850(211#9), JW0851(211#10), JW0852(211#11), JW0853(ybjT), JW0854(ltaA) |
| Δ017C | JW0916(ssuB), JW0917(ssuC), JW0918(ssuD), JW0919(ssuA) |
| Δ018 | JW0969(ymcC), JW0970(ymcD), JW0971(insA), JW0972(insB), JW0973(cspH), JW0974(cspG), JW0975(sfa), JW0976(yccL), JW0977(yccM), JW0978(torS), JW0979(torT), JW0980(torR), JW0981(torC), JW0982(torA), JW0983(torD), JW0984(yccD), JW0985(cbpA), JW0986(yccE), JW0987(agp), JW0988(yccJ), JW0989(wrbA), JW0990(yciG), JW0991(ycdG), JW0992(228#5), JW0993(228#6), JW0994(228#7), JW0995(ycdK), JW0996(228#9), JW0997(228#10), JW0998(ycdC), JW0999(putA), JW1000(yzpU), JW1001 (putP), JW1002(229#1), JW1003(ycdO), JW1004(ycdB), JW1005(phoH), JW1006(ycdP), JW1007(ycdQ), JW1008(ycdR), JW1009(ycdR), JW1010(ycdS), JW1011(ycdT), JW1012(230#3), JW1013(230#3.1), JW1014(230#4), JW1015(ycdU) |
| Δ090 | JW1105(ycfX), JW1106(238#9), JW1107(cobB), JW1108(ymfA), JW1109(potD), JW1110(potC), JW1111(potB), JW1112(potA), JW1113(pepT), JW1114(ycfD), JW1115(phoQ), JW1116(phoP) |
| Δ019 | JW1123(ymfD), JW1124(ymfE), JW1125(lit), JW1126(intE), JW1127(VXIS_BPP21), JW1128(ymfH), JW1129(ymfI), JW1130(ymfJ), JW1131 (RPC2_BPP22), JW1132(241#2), JW1133(ymfL), JW1134(ymflW), JW1135(ymfN), JW1136(ymfR), JW1137(ymfO), JW1138(241#8), JW1139(241#9), JW1140(ycfK), JW1141(ymfS), JW1142(ycfA), JW1143(ycfE), JW1144(pin), JW1145(mcrA), JW1146(241#16), JW1147(ycgW), JW1148(vcgX), JW1149(ycgE), JW1150(242#1), JW1151(ycgZ), JW1152(ymgA). JW1153(242#1.4), JW1154(ymgC), JW1155(yahA), JW1156(243#0.1), JW1157(243#1), JW1158(aidA), JW1159(ymgD), JW1160(243#4), JW1161(243#5), JW1162(243#5.1) |
| Δ020C | JW1166(ycgJ), JW1167(ycgK), JW1168(ycgL), JW1169(243#12), JW1170(ycgN), JW1171(clyA) |
| Δ 091 | JW1227(ychG), JW1227.5(251#6), JW1228(adhE), JW1229(ychE), JW1230(yi2A), JW1231(yi22), JW1232(yi24), JW1233(yi23), JW1234(251#8.5), JW1235(oppA), JW1236(oppB), JW1237(oppC), JW1238(oppD), JW1239(oppF), JW1240(yciU) |
| Δ 022 | JW1302(sucP), JW1303(258#8), JW1304(ycjO), JW1305(ycjP), JW1306(ycjQ), JW1307(259#3), JW1308(ycjS), JW1309(ycjT), JW1310(ycjU), JW1311(ycjV), JW1312(ompG), JW1313(ycjW), JW1314(ycjX), JW1315(ycjF), JW1316(tyrR), JW1317(tpx), JW1318(ycjG), JW1319(ycjl), JW1319.5(260#10), JW1320(ycjY), JW1321(ycjZ), JW1321.5(261#1.1), JW1322(mppA), JW1323(ynal), JW1324(is5B), JW1325(yi51), JW1326(ynaJ), JW1327(ydaA), JW1328(fnr), JW1329(ogt), JW1330(ydaH), JW1331(261#9), JW1332(ydaJ), JW1333(262#1), JW1334(ydaL), JW1335(262#3), JW1336(262#4), JW1336.5(262#5), JW1337(dbpA), JW1338(ydaO), JW1339(intR), JW1340(ydaQ), JW1341(ydaC), JW1342(lar), JW1343(recT), JW1344(recE), JW1345(racC), JW1346(ydaE), JW1347(kil), JW1348(sieB), JW1349(ydaF), JW1350(263#6.1), JW1351(racR), JW1352(263#6.3), JW1353(ydaT), JW1354(ydaU), JW1355(dnaC), JW1356(ydaW), JW1357(enpP), JW1358(trkG), JW1359(ynaK), JW1360(ydaY), JW1361(VMTH_LAMBD), JW1362(VLOM_LAMBD), JW1363(is5B), JW1364(yi51), JW1365(VLOM_LAMBD), JW1366(Y401_LAMBD), JW1367(yzzL), JW1368(tnpO), JW1369(ynaE), JW1370(upo3), |
| Δ 023 | JW1376(ydbH), JW1377(ynbE), JW1378(ydbL), JW1379(feaR), JW1380(feaB), JW1381(tynA), JW1382(paaZ), JW1383(paaA), JW1384(paaB), JW1385(paaC), JW1386(paaD), JW1387(paaE) |
| Δ 024 | JW1393(paaK), JW1394(paaX), JW1395(paaY), JW1396(ydbA), JW1397(yi22), JW1398(yi24), JW1399(yi23), JW1400(yi2A), JW1401(268#0.5), JW1402(ydbB), JW1403(ydbC), JW1404(ydbD), JW1405(269#1), JW1406(ynbB),JW1407(269#3), JW1408(ynbD) |
| Δ 025 | JW1416(ydcA), JW1416.5(271#2), JW1417(trg), JW1418(ydcI), JW1419(271#5), JW1420(ydcG), JW1421(271#7),JW1422(ydcH), JW1423(rimL) |
| Δ 026 | JW1427(272#1), JW1427.5(272#2), JW1428(272#3), JW1429(272#4), JW1430(272#5), JW1431 (prtC), JW1432(yncJ), JW1433(ydcQ), JW1434(yjiR), JW1435(273#4), JW1436(potA), JW1437(273#6), JW1438(potC), JW1439(ydcW), JW1440(ydcX), JW1441 (ydcY), JW1442(ydcZ/ydcZ/ydcZ/ydcZ), JW1443(274#1), JW1444(yncB), JW1445(yncC), JW1446(yncD), JW1447(274#5), JW1448(ansP), JW1449(275#2), JW1450(275#2.1), JW1451(rhsE), JW1452(ydcD), JW1453(yhhI), JW1454(276#2.1), JW1455(ydcC), JW1456(ydcE), JW1457(yddH), JW1458(nhoA), JW1459(yddE) |
| Δ 027 | JW1478(yddO), JW1479(yddP), JW1480(yddQ), JW1481(yddR), JW1482(yddS), JW1483(280#1), JW1484(dos), JW1485(280#3), JW1486(280#4), JW1487(xasA) |
| Δ 028B | JW1505(ydeW), JW1506(rbsA), JW1507(ydeY), JW1508(ydeZ), JW1509(304#1) |
| Δ 029B | JW1533(ydfH), JW1534(ydfZ), JW1535(ydfI), JW1536(shiA), JW1537(307#4.1), JW1538(tnpR), JW1539(ydfM), JW1540(Y314_LAMBD), JW1541(nohA), JW1542(ydfO), JW1543(yccL), JW1544(cspI) |
| Δ 030 | JW1547(ydfR), JW1548(vlyS), JW1549(cspB), JW1550(cspF), JW1551(regQ_LAMBD), JW1552(309#4), JW1553(rem), JW1554(hokD), JW1555(relE), JW1556(relB), JW1557(ydfV), JW1558(flxA), JW1559(ydfW), JW1560(ydfX), JW1561(dicC) |
| Δ 031C | JW1634(slyA), JW1635(ydhI), JW1636(yhcQ), JW1637(yhcP), JW1638(sodC), JW1639(ydhF), JW1640(ydhL), JW1641(ydhM), JW1642(nemA), JW1643(gloA) |
| Δ 105A | JW1699(btuD), JW1700(btuE), JW1701 (btuC) |
| Δ 032C | JW1722(ydjC), JW1723(celF), JW1724(celD), JW1725(celC), JW1726(celB), JW1727(celA), JW1728(osmE) |
| Δ 033C | JW1742(ynjA), JW1743(328#9), JW1744(ynjC), JW1745(328#11) |
| Δ034 | JW1771(yeaF), JW1772(yeaG), JW1773(yeaH), JW1774(yeal), JW1775(yeaJ), JW1776(yeaK), JW1777(332#0.1), JW1778(yeaL), JW1779(yeaM), JW1780(yeaN), JW1781(yeaO), JW1782(yoaF), JW1783(332#6), JW1784(333#1), JW1785(yoaG), JW1786(yeaR), JW1787(yeaS), JW1788(yeaT), JW1789(ttuC), JW1790(caiT), JW1791(yeaW), JW1792(yeaX) |
| Δ 092 | JW1806(manX), JW1807(manY), JW1808(manZ), JW1809(335#5), JW1810(yebN) |
| Δ036 | JW1866(yecT), JW1867(flhE), JW1868(flhA), JW1869(flhB), JW1870(cheZ), JW1871 (cheY), JW1872(cheB), JW1873(cheR), JW1874(tap), JW1875(tar), JW1876(cheW), JW1877(cheA), JW1878(motB), JW1879(motA), JW1880(flhC), JW1881(flhD), JW1882(340#3.1), JW1883(340#3.2), JW1884(yecG), JW1885(otsA), JW1886(otsB), JW1887(araH), JW1888(araG), JW1889(araF), JW1890(yecI), JW1891(yecJ), JW1892(341#5), JW1893(ftn), JW1894(yecH), JW1895(tyrP) |
| Δ 037 | JW1898(uvrC), JW1899(uvrY), JW1900(yecF), JW1901(sdiA), JW1902(yecC), JW1903(yecS), JW1904(yed0), JW1905(fliY), JW1906(fliZ), JW1907(fliA), JW1908(fliC), JW1909(fliD), JW1910(fliS), JW1911(fliT), JW1912(amyA), JW1913(yedD), JW1914(yedE), JW1915(yedF), JW1916(yedK), JW1917(yedL), JW1918(yedN), JW1919(yedN), JW1920(yedM), JW1921 (fliE), JW1922(fliF), JW1923(fliG), JW1924(fliH), JW1925(flil), JW1926(fliJ), JW1927(fliK), JW1928(fliL), JW1929(fliM), JW1930(fliN), JW1931 (fli0), JW1932(fliP), JW1933(fliQ), JW1934(fliR), JW1935(rcsA), JW1936(dsrB), JW1937(344#6) |
| Δ038 | JW1942(yedA), JW1943(vsr), JW1944(dcm), JW1945(yedJ), JW1946(344#15), JW1947(ompC), JW1948(344#17), JW1949(yedS), JW1950(yedU), JW1951(yedV), JW1952(copR), JW1953(yedX), JW1954(345#1), JW1955(345#2), JW1956(345#3) |
| Δ 039 | JW1972(is5B), JW1973(yi51), JW1974(348#3), JW1975(yi22), JW1976(yi24), JW1977(yi23), JW1978(yi2A), JW1979(348#3.5), JW1980(348#4), JW1981(yeeP), JW1982(flu), JW1983(349#2), JW1984(yeeS), JW1985(yeeT), JW1986(yeeU), JW1987(yeeV), JW1988(yeeW) |
| Δ 040 | JW2012(yefJ), JW2013(351 #10), JW2014(is5B), JW2015(yi51), JW2016(yefJ), JW2017(yelI), JW2018(wbbJ), JW2019(yefG), JW2020(rfc), JW2021 (glf), JW2022(rfbX), JW2023(rfbC), JW2024(rfbA), JW2025(rfbD), JW2026(rfbB) |
| Δ 041 | JW2052(yegE), JW2053(alkA), JW2054(yegD), JW2055(356#6), JW2056(356#7), JW2057(356#8), JW2058(356#9), JW2059(yegM), JW2060(yegN), JW2061(yegO). JW2062(yegB), JW2063(baeS), JW2064(baeR), JW2065(358#3), JW2066(yegQ), JW2067(ogrK), JW2068(358#6), JW2069(358#6.1), JW2070(yegS), JW2071(gatR), JW2072(358#8.1), JW2073(358#8.2), JW2074(gatR), JW2075(gatD), JW2076(gatC), JW2077(gatB), JW2078(gatA), JW2079(is5B), JW2080(yi51), JW2081(gatA), JW2082(gatZ), JW2083(gatY), JW2084(fbaB), JW2085(xegT), JW2086(yegU), JW2087(yegV), JW2088(yegW), JW2089(yegX) |
| Δ 042 | JW2102(molR), JW2103(molR), JW2104(molR), JW2105(yehI), JW2106(yehK), JW2107(yehL), JW2108(yehO), JW2109(yehP), JW2110(yehQ), JW2111(yehR), JW2112(yehS), JW2113(yehT), JW2114(yehU), JW2115(yehV), JW2116(yehW), JW2117(yehX), JW2118(yehY), JW2119(yehZ), JW2120(bglX), JW2121(dld), JW2122(pbpG), JW2123(yohC) |
| Δ 093 | JW2135(mglC), JW2136(mglA), JW2137(mglB), JW2138(galS) |
| Δ 088 | JW2147(yeiI), JW2148(yeiJ), JW2149(yeiK), JW2150(yeiL), JW2151(yeiM) JW2152(yeiN), JW2153(yeiC) |
| Δ 043 | JW2160(yeiQ), JW2161 (yeiR), JW2162(yeiU), JW2163(spr), JW2164(rtn), JW2165(yejA), JW2166(yejB), JW2167(yejE), JW2168(yejF), JW2169(yejG), JW2170(bcr), JW2171 (rsuA), JW2172(yejH) |
| Δ 106A | JW2185(ccmE), JW2186(ccmD), JW2187(ccmC), JW2188(ccmB), JW2189(ccmA) |
| Δ K | JW2213(atoS), JW2214(atoC), JW2215(atoD), JW2216(atoA), JW2217(atoE), JW2218(atoB), JW2219(376#1), JW2220(376#2), JW2221 (376#3), JW2222(376#4), JW2223(376#5) |
| Δ 045 | JW2261(elaB), JW2262(elaA), JW2263(elaC), JW2264(elaD), JW2265(yfbK), JW2266(yfbL), JW2267(yfbM), JW2268(yfibN), JW2269(403#0.1), JW2270(403#1) |
| Δ046 | JW2295(yfcC), JW2296(406#2), JW2297(yfcE), JW2298(yfcF), JW2299(yfcG), JW2300(folX), JW2301(406#7), JW2302(yfcI), JW2303(hisP), JW2304(hisM), JW2305(hisQ), JW2306(hisJ), JW2307(argT) |
| Δ047 | JW2345(yfdB), JW2346(411#1), JW2347(yfdH), JW2348(411#2.1), JW2349(ycfA), JW2350(yfdK), JW2351(yfdL), JW2352(yfdM), JW2353(xylU), JW2354(yfdN), JW2355(yfdO), JW2356(411#10), JW2357(411#11), JW2358(411#12), JW2359(411#13), JW2360(411#14) |
| Δ094 | JW2377(yehU), JW2378(414#7), JW2379(yijO), JW2380(ptsA), JW2381(frvX), JW2382(yqhT), JW2383(ptwC), JW2384(ptwB) |
| Δ095 | JW2527(yphA), JW2528(yphB), JW2529(yphC), JW2530(yphD), JW2531(yphE), JW2532(yphF), JW2533(yphG), JW2534(yphH) |
| Δ 050 | JW2602(intA), JW2603(yfjH), JW2604(alpA), JW2605(yfjI), JW2606(440#5.1), JW2607(yfjJ), JW2608(yfjK), JW2609(yfjL), JW2610(yfjM), JW2611(yfjN), JW2612(yfjO), JW2613(yfjP), JW2614(yfjQ), JW2615(yfjR), JW2616(ypjK), JW2617(yfjS), JW2618(yfjT), JW2619(yfjU), JW2620(yfjV), JW2621 (yFjV), JW2622(442#2.2), JW2623(yfjW), JW2623.5(442#4), JW2624(yfjX), JW2625(yfjY), JW2626(yfjZ), JW2627(ypjF), JW2628(ypjA), JW2629(443#1.1), JW2630(443#1.2), JW2631(443#2), JW2632(443#2.1), JW2633(443#2.2), JW2634(443#3) |
| Δ 051 | JW2639(ygaE), JW2640(yzzM), JW2641(yqaE), JW2642(445#2), JW2643(ygaP), JW2644(stpA), JW2645(445#5), JW2646(ygaC), JW2647(ygaM), JW2648(nrdH), JW2649(nrdI), JW2650(nrdE) |
| Δ096 | JW2652(proV), JW2653(proW), JW2654(proX), JW2655(445#15), JW2656(445#17), JW2657(ygaZ), JW2658(ygaH) |
| Δ097 | JW2672(srlA), JW2673(srlB), JW2674(srlD), JW2675(gutM), JW2676(srlR), JW2677(gutQ) |
| Δ 052 Δ 052 | JW2723(iap), JW2724(ygbF), JW2725(452#12), JW2726(ygcH), JW2727(452#14), JW2728(ygcJ), JW2729(ygcK), JW2730(453#2), JW2731(ygcB) |
| Δ 053 | JW2735(ptpS), JW2736(ygcN), JW2737(ygcO), JW2738(ygcP), JW2739(ygcQ), JW2740(ygcR), JW2741(ygcS), JW2742(454#9), JW2743(ygcU), JW2744(ygcU), JW2745(ygcW), JW2746(yqcE), JW2747(ygcE), JW2748(ygcF), JW2749(ygcG) |
| Δ 100 | JW2753(chpA) |
| Δ 054 | JW2813(sdaC), JW2814(yqeH), JW2815(yqeI), JW2816(yqeJ), JW2817(yqeK), JW2818(ygeF), JW2819(ygeG), JW2820(ygeH), JW2821(ygeI), JW2822(x), JW2823(ygeK), JW2824(464#6), JW2825(464#7), JW2826(yi22), JW2827(yi24), JW2828(yi23), JW2829(yi2A), JW2830(465#0.1), JW2831(ygeP), JW2832(465#1), JW2833(nlpD), JW2834(yagR), JW2835(ygeT), JW2836(465#5), JW2837(ygeV), JW2838(ygeW), JW2839(dpaL), JW2840(ygeY), JW2841 (ygeZ), JW2842(arcL), JW2843(yqeB), JW2844(466#6), JW2845(ygfJ), JW2846(gltD), JW2847(467#1), JW2848(ygfM), JW2849(yagR), JW2850(ygfO), JW2851(ygfP), JW2852(ygfQ), JW2853(ygfR), JW2854(ygfS), JW2855(ygfT), JW2856(pbuX), JW2857(idi) |
| Δ 098 | JW2900(cmtA), JW2901 (cmtB) |
| Δ 055 | JW2935(epsM), JW2936(exeL), JW2937(exeC), JW2938(477#5), JW2939(477#6), JW2940(477#7), JW2941 (478#1), JW2942(yghK), JW2943(glcB), JW2944(glcG), JW2945(glcF), JW2946(glcD), JW2947(glcC), JW2948(502#6), JW2949(is5B), JW2950(yi51), JW2951(yghQ), JW2952(yghR), JW2953(yghS), JW2954(yghT) |
| Δ056B | JW2988(ygiS), JW2989(ygiT), JW2990(ygiU), JW2991(506#5), JW2992(ygiW), JW2993(ygiX), JW2994(ygiY), JW2995(ygiZ), JW2996(mdaB), JW2997(ygiN) |
| Δ 059 | JW3046(ebgR), JW3047(ebgA), JW3048(ebgC), JW3049(ygjI), JW3050(ygjJ), JW3051(ygjK), JW3052(fadH), JW3053(ygjM), JW3054(ygjN), JW3055(ygjO), JW3056(ygjP), JW3057(ygjQ), JW3058(ygjR), JW3059(ygjT), JW3060(ygjU), JW3061(ygjV), JW3062(uxaA), JW3063(uxaC), JW3064(exuT), JW3065(exuR), JW3066(yqjA), JW3067(yqjB), JW3068(yqjC), JW3069(yqjD), JW3070(yqjE), JW3071(yqjK), JW3072(yqjF), JW3073(yqjG), JW3074(yhaH), JW3075(yhaI), JW3076(yhaJ), JW3077(yhaK), JW3078(yhaL), JW3079(yhaM), JW3080(yhaN), JW3081(yhaO), JW3082(yhaP), JW3083(yhaQ) |
| Δ 060B | JW3093(yhaD), JW3094(yhaE), JW3095(yhaF), JW3096(yhaU), JW3097(yhaG), JW3098(sohA), JW3099(yhaV), JW3100(agaR), JW3101(agaZ). JW3102(agaV), JW3103(agaW), JW3104(agaA), JW3105(agaS), JW3106(agaY), JW3107(agaB), JW3108(agaC), JW3109(agaD), JW3110(agaI), JW3111(yraH), JW3112(yraI), JW3113(yraJ), JW3114(yraK), JW3115(yraL), JW3116(yraM), JW3117(yraN), JW3118(yraO), JW3119(yraP), JW3120(yraQ) |
| Δ 061 C | JW3159(yrbC), JW3160(yrbD), JW3161 (yrbE), JW3162(yrbF) |
| Δ 062 | JW3181(gltF),JW3182(yhcA), JW3183(yhcD), JW3184(yhcE), JW3185(is5B), JW3186(yi51), JW3187(yhcE), JW3188(yhcF), JW3189(yhcG), JW3190(yhcH), JW3191(nanK), JW3192(nanE) |
| Δ 064 | JW3230(yhdJ), JW3231(yhdU), JW3232(envR), JW3233(acrE), JW3234(envD), JW3235(yhdV), JW3236(yhdW), JW3237(yhdX), JW3238(yhdY) |
| Δ 065 | JW3284(pinO), JW3285(gspA), JW3286(gspC), JW3287(gspD), JW3288(gspE), JW3289(hofF), JW3290(hofG), JW3291(hofH), JW3292(gspI), JW3293(gspJ), JW3294(gspK), JW3295(gspL), JW3296(pshM), JW3297(hofD), JW3298(bfr), JW3299(yheA), JW3300(chiA) |
| Δ 068C | JW3401(gntR), JW3402(yhhW), JW3403(yhhX), JW3404(615#7.1), JW3405(yhhY), JW3406(yhhZ), JW3407(yrhA), JW3408(insA), JW3409(insB), JW3410(615#10.1), JW3411(yrhB), JW3412(ggt), JW3413(yhhA) |
| Δ 068G | JW3419(livF), JW3420(livG), JW3421 (livM), JW3422(livH), JW3423(livK) |
| Δ 069 | JW3447(rhsB), JW3448(610#6.1), JW3449(yhhH), JW3450(yibA), JW3451(yhhI), JW3452(yhhJ), JW3453(yhiH), JW3454(yhil), JW3455(yhiJ), JW3456(yhiK), JW3457(yhiL), JW3458(yhiM), JW3459(yhiN) |
| Δ 070F | JW3509(dppF), JW3510(dppD), JW3511 (dppC), JW3512(dppB), JW3513(dppA) |
| Δ 071B | JW3536(xylB), JW3537(xylA), JW3538(xylF), JW3539(xylG), JW3540(xylH) |
| Δ 072 | JW3548(yiaL), JW3549(yiaM), JW3550(yiaN), JW3551(yiaO), JW3552(lyx), JW3553(sgbH), JW3554(sgbU), JW3555(sgbE), JW3556(yiaT), JW3557(yiaU), JW3558(yiaV), JW3559(yiaW), JW3560(577#1.1), JW3561(aldB), JW3562(yiaY), JW3563(selB), JW3564(selA), JW3565(yibF), JW3566(rhsA), JW3567(576#2.1), JW3568(yibA), JW3569(yibJ), JW3570(yibG), JW3570.5(576#7), JW3571(yibH), JW3572(yibI) |
| Δ074 | JW3650(yidF), JW3651(yidG), JW3652(yidH), JW3653(yidI), JW3654(yidJ), JW3655(yidK), JW3656(yidL), JW3657(567#9.1), JW3658(glvG), JW3659(glvB), JW3660(glvC), JW3661(yidP), JW3662(yidE) |
| Δ075 | JW3665(yidQ), JW3666(yidR), JW3667(yidS), JW3668(dgoT), JW3669(dgoA), JW3670(dgoK), JW3671(dgoR), JW3672(yidW), JW3673(yidX), JW3674(yidA), JW3675(yidB) |
| Δ076 | JW3722(asnA), JW3723(yieM), JW3724(559#4), JW3725(yieN), JW3726(kup), JW3727(rbsD), JW3728(rbsA), JW3729(rbsC), JW3730(rbsB), JW3731(rbsK), JW3732(rbsR), JW3733(yieO) |
| Δ078 | JW3843(yihL), JW3844(yihM), JW3845(yihN), JW3846(yshA), JW3847(yihO), JW3848(yihP), JW3849(yihQ), JW3850(yihR), JW3851(yihS), JW3852(yihT), JW3853(yihU), JW3854(yihV), JW3855(yihW), JW3856(yihX), JW3857(rbn), JW3858(yihZ), JW3859(yiiD), JW3860(yiiE), JW3861(yiiF) |
| Δ 079 | JW3867(yiiG), JW3868(frvR), JW3869(frvX), JW3870(frvB), JW3871(frvA), JW3872(yiiL), JW3873(rhaD), JW3874(rhaA), JW3875(rhaB), JW3876(rhaS), JW3877(rhaR), JW3878(rhaT) |
| Δ 080 | JW3919(ptsA), JW3920(536#1), JW3921(frwC). JW3922(frwB), JW3923(pflD), JW3924(pflC), JW3925(frwD), JW3926(yijO), JW3927(yijP) |
| Δ 081 | JW3986(633#6), JW3987(yjbF), JW3988(yjbG), JW3989(yjbH), JW3989.5(633#10), JW3990(yjbA), JW3991(xylE), JW3992(malG), JW3993(malF), JW3994(malE), JW3995(malK), JW3996(lamB), JW3997(malM), JW3998(yjbI) |
| Δ 082 | JW4041(yjcP), JW4042(yjcQ), JW4043(yjcR), JW4044(yjcS), JW4045(alsK), JW4046(alsE), JW4047(alsC), JW4048(alsA), JW4049(alsB) |
| Δ 083 | JW4052(phnQ), JW4053(phnP), JW4054(phnO), JW4055(phnN), JW4056(phnM), JW4057(phnL), JW4058(phnK), JW4059(phnJ), JW4060(phnI), JW4061(phnH), JW4062(phnG), JW4063(phnF), JW4064(phnE), JW4065(phnE), JW4066(phnD), JW4067(phnC), JW4068(phnB), JW4069(phnA), JW4070(yjdA), JW4071(yjcZ) |
| Δ 084 | JW4139(yjfI), JW4140(yjfJ), JW4141(yjfK), JW4142(yjfL), JW4143(yjfM), JW4144(yjfC), JW4145(aidB), JW4146(yjfN), JW4147(yjfO), JW4148(yjfP), JW4149(yjfQ), JW4150(yjfR), JW4151(sgaT), JW4152(sgaB), JW4153(sgaA), JW4154(sgaH), JW4155(sgaU) |
| Δ 108A | JW4186(ytfQ), JW4187(ytfR), JW4188(ytfS), JW4189(ytfT), JW4190(yjfF) |
| Δ 099 | JW4198(treC), JW4199(treB), JW4200(treR) |
| Δ 085 | JW4227(intB), JW4228(660#10.1), JW4229(yi2A), JW4230(yi22), JW4231(yi24), JW4232(yi23), JW4233(yjgW), JW4234(yjgX), JW4235(yjgY), JW4236(yjgZ), JW4237(yi41), JW4238(yi42), JW4239(yjhB), JW4240(yjhC), JW4241(yjhD), JW4242(yjhE), JW4243(yi91), JW4244(662#3.2), JW4245(662#4), JW4246(662#5) |
| Δ086 | JW4254(insA), JW4255(insB), JW4256(insB), JW4257(yjhU), JW4258(yjhF), JW4259(yjhG), JW4260(yjhH), JW4261(yjhI), JW4262(sgcR), JW4263(sgcE), JW4264(sgcA), JW4265(sgcQ), JW4266(sgcC), JW4266.5(664#6), JW4267(sgcX), JW4268(yjhP), JW4269(yjhQ), JW4269.5(664#11), JW4270(664#12), JW4270.5(664#13), JW4271(yjhR), JW4272(yjhS), JW4273(yjhT), JW4274(yjhA), JW4275(fimB), JW4276(fimE), JW4277(fimA), JW4278(fimI), JW4279(fimC), JW4280(fimD), JW4281(fimF), JW4282(fimG), JW4283(fimH), JW4284(gntP), JW4285(uxuA), JW4286(uxuB), JW4287(uxuR), JW4288(yjiC), JW4289(yjiD), JW4290(yjiE), JW4291(iadA), JW4292(yjiG), JW4293(yjiH), JW4294(yjiI), JW4295(yjiJ), JW4296(yjiK), JW4296.5(668#5), JW4297(yjiL), JW4298(yjiM), JW4299(yjiN), JW4300(yjiO), JW4301(yjiP), JW4302(yjiQ), JW4303(yjiR), JW4304(yjiS), JW4305(yjiT), JW4306(yjiV), JW4307(mcrD), JW4308(mcrC), JW4309(mcrB), JW4310(yjiW), JW4311 (hsdS), JW4312(hsdM), JW4313(hsdR), JW4314(mrr), JW4315(yjiA), JW4316(yjiX), JW4317(yjiY), JW4318(tsr), JW4319(yjjL), JW4320(671 #8), JW4321 (yjjM), JW4322(yjjN), JW4323(mdoB), JW4324(yjjA) |

### Example 3

### Preparation of mutant strain of E. coli having chromosomal DNA with multiple partial deletions

Mutants of *E*. *coli* having chromosomal DNA with multiple partial deletions were prepared using *E*. *coli* W3110red strains and accumulating the deleted regions that would not affect growth, which had been identified in Example 2, in *E*. *coli* W3110red strains by repeating the procedure of Example 1. Table 3 shows deleted regions of mutants of *E*. *coli* having chromosomal DNA with multiple partial deletions.

### Example 4

### Evaluation of mutant strain of E. coli having chromosomal DNA with multiple partial deletions by culturing (1)

Various mutants of *E*. *coli* having chromosomal DNA with multiple partial deletions obtained in Example 3 were applied to LB agar medium and cultured at 30°C overnight to obtain cell strains. Small amounts of such strains were inoculated into 1 ml of LB medium that contained 3% glucose, and culturing was carried out overnight to obtain culture products. The culture products (50 µl) were inoculated into 5 ml of LB medium that contained 3% glucose, and culturing was carried out at 30°C for 24 hours. After the completion of culturing, turbidity was measured based on absorption at 590 nm. The results are shown in Table 3.

**Table 3**

| Strain name | Deleted region | Total deleted length (kbp) | Finally reached turbidity (%) |
|---|---|---|---|
| W3110red | None | 0.0 | 100 |
| Step1 | Δ018 | 48.1 | 102 |
| Step2 | Deleted region possessed by Step1, Δ 055 | 72.4 | 106 |
| Step3 | Deleted region possessed by Step2, Δ 081 Δ 082, Δ 083 | 117.7 | 94 |
| Step4 | Deleted region possessed by Step3, Δ 019 | 144.7 | 98 |
| Step5 | Deleted region possessed by Step4, Δ 029B , Δ 030 | 162.4 | 99 |
| Step6 | Deleted region possessed by Step5, Δ009B Δ010, Δ011 | 236.0 | 96 |
| Step7 | Deleted region possessed by Step6, Δ078 Δ 079, Δ 080 | 279.8 | 106 |
| Step8 | Deleted region possessed by Step7, Δ 054 | 327.5 | 104 |
| Step9 | Deleted region possessed by Step8, Δ 001, Δ 002, Δ 007 | 477.1 | 112 |
| Step10 | Deleted region possessed by Step9, Δ074, Δ 075, Δ 077 | 510.4 | 123 |
| Step11 | Deleted region possessed by Step10, Δ036, Δ 037, Δ 038, Δ 039, Δ 040 | 605.9 | 123 |
| step 12 | Deleted region possessed by Step 11, Δ050, Δ051, Δ 096, Δ 097 | 656.2 | 153 |
| Step13 | Deleted region possessed by Step12, Δ 062 | 666.0 | 155 |
| Step14 | Deleted region possessed by Step13, Δ 041 Δ 042 Δ 043 Δ 088 | 744.4 | 189 |
| Step15 | Deleted region possessed by Step14, Δ098 | 746.2 | 178 |
| Step16 | Deleted region possessed by Step15, Δ 085, Δ 086 | 841.3 | 190 |
| Step17 | Deleted region possessed by Step16, Δ 013 | 852.1 | 180 |
| Step18 | Deleted region possessed by Step17, Δ084 | 865.7 | 195 |

As shown in Table 3, mutants of *E. coli* having chromosomal DNA with multiple partial deletions, the total extent of such deletion being 470 kbp or more, exhibited substantially increased turbidity 24 hours after culturing in relation to the total extent of deletion. Cell morphologies of *E*. *coli* mutants were the same as those of wild-type *E*. *coli* strains, and mutants of *E*. *coli* having chromosomal DNA with multiple partial deletions, and the total extent of such deletion being 470 kbp or more, were found to yield more cells via culturing than wild-type *E. coli* strains.

### Example 5

### Evaluation of mutant strain of E. coli having chromosomal DNA with multiple partial deletions by culturing (2)

In order to confirm that increase of cell mass confirmed in Example 4 does not solely result from the regions that had been newly deleted from various mutants, mutants resulting from deletions from the mutants shown in Table 3 were prepared in accordance with the methods described in Example 1 and Example 3, culturing was carried out in the same manner as in Example 4, and turbidity 24 hours after the initiation of culturing was measured. The results are shown in Table 4.

**Table 4**

| Strain name | Deleted region Deleted region | Total deleted length (kbp) | Finally reached turbidity (%) |
|---|---|---|---|
| W3110red | None | 0.0 | 100 |
| Unit1 | Δ018 | 48.1 | 102 |
| Unit2 | Δ 055 | 24.2 | 107 |
| Unit3 | Δ081, Δ 082, Δ083 | 45.3 | 93 |
| Unit4 | Δ 019 | 27.0 | 108 |
| Unit5 | Δ 029B, Δ 030 | 17.7 | 99 |
| Unit6 | Δ 009B, Δ 010, Δ 011 | 73.6 | 100 |
| Unit7 | Δ 078, Δ 079, Δ 080 | 43.8 | 101 |
| Unit8 | Δ 054 | 47.8 | 108 |
| Unit9 | Δ 001 Δ 002 Δ 007 | 149.6 | 101 |
| Unit10 | Δ 074 Δ 075 Δ 077 | 33.3 | 94 |
| Unit11 | Δ 036 Δ 037 Δ 038 Δ 039 Δ 040 | 95.5 | 110 |
| Unit12 | Δ 050 Δ 051 Δ 096 Δ 097 | 50.3 | 96 |
| Unit13 | Δ 062 | 9.8 | 100 |
| Unit14 | Δ 041 Δ 042 Δ 043 Δ 088 | 78.4 | 109 |
| Unit15 | Δ 098 | 1.8 | 98 |
| Unit16 | Δ 085 Δ 086 | 95.1 | 94 |
| Unit17 | Δ013 | 10.8 | 102 |
| Unit18 | Δ084 | 13.6 | 93 |

As shown in Table 4, new deletions would not result in increased turbidity. Accordingly, the increased cell mass observed in Example 4 was not attained by accumulation of deletions that would increase the amount of cells. However, it was demonstrated that such increase resulted from multiple and simultaneous occurrence of deletions of chromosomal DNA that would not solely affect growth.

### Example 6

### Preparation of mutant strain of E. coli having chromosomal DNA with multiple partial deletions (2)

The Step 18 strain shown in Table 3 was designated as a parent strain, and a strain to which deletion of the Δ043 region shown in Table 2 had been returned was prepared in the following manner.

In accordance with the method of Example 3, a strain that lacks Δ041 and Δ042 regions from the *E. coli* W3110red strain was obtained. Subsequently, a cassette comprising the sacB gene and the cat gene ligated thereto (hereafter referred to as "sacB_cat;" see Fig. 1) was introduced into the Δ088 region adjacent to the Δ043 region to obtain strains (hereafter referred to as the *"E. coli* W3110red Δ041, Δ042, and Δ088::aacB_cat strains").

P1-transducing phages were prepared from the *E. coli* W3110red Δ041, Δ042, and Δ088::sacB_cat strains in accordance with the method described in Example 1 (1), the Step18 strains were transformed with the P1 phages in accordance with the method described in Example 1 (1), and chloramphenicol-resistant strains were obtained. Thus, strains comprising the sacB/cat cassettes inserted into the Δ088 regions were selected.

Among the selected Step 18 transformants, strains to which the Δ043 regions have been returned, were selected in accordance with the method for detecting deletions described in Example 1 (2) (iii). sacB_cat on chromosomal DNA of the selected strain was eliminated by the method described in Example 1 (2) (iv) to obtain a Step18γ strain having chromosomal DNA that lacks regions shown in Table 5.

Subsequently, chromosomal DNA of the Step18γ strain was further subjected to deletion to prepare *E. coli* strains by the method described in Example 1 and Example 3. Table 5 shows mutants of *E. coli* having chromosomal DNA with multiple partial deletions.

**Table 5**

| Strain name | Deleted region | Total deletion length (kbp) | Turbidity of culture product (absolute value) | |
|---|---|---|---|---|
| | | | After 19 hours | After 36 hours |
| Step18 γ | Δ 001, Δ 002, Δ 007, Δ 009B, Δ 010, Δ011, Δ013, Δ018, Δ 019, Δ 029B, Δ 030, Δ 036, Δ 037, Δ038, Δ039, Δ040, Δ041, Δ042, Δ088, Δ050, Δ051, Δ 096, Δ 097, Δ054, Δ 098, Δ 055, Δ 062, Δ 074, Δ 075, Δ 077, Δ 078, Δ 079, Δ 080, Δ 081, Δ 082, Δ 083, Δ 084, Δ 085, Δ 086 | 865.7 | 5.54 | 6.64 |
| Step19 | Deleted region possessed by Step 18 γ, Δ 069 | 884.1 | 5.49 | 6.69 |
| Step20 | Deleted region possessed by Step 19, Δ 014, Δ 015, Δ 016 | 933.8 | 5.27 | 7.52 |
| Step21 | Deleted region possessed by Step20, Δ 056B | 941.1 | 5.25 | 7.85 |
| Step22 | Deleted region possessed by Step21, Δ 012 | 955.4 | 5.50 | 7.57 |
| Step23 | Deleted region possessed by Step22, Δ 046, Δ 047, Δ 094 | 985.2 | 5.35 | 7.61 |
| Step24 | Deleted region possessed by Step23, Δ 065 | 1001.6 | 5.61 | 8.01 |
| Step25 | Deleted region possessed by Step24, Δ 045 | 1010.8 | 5.43 | 7.69 |
| Step26 | Deleted region possessed by Step25, Δ 064 | 1020.5 | 5.62 | 7.87 |
| Step27 | Deleted region possessed by Step26, Δ 100 | 1020.8 | 5.52 | 7.90 |
| Step28 | Deleted region possessed by Step27, Δ 103 | 1026.7 | 5.36 | 7.61 |

### Example 7

### Evaluation of culture of mutant obtained in Example 6 by culturing

Various mutants obtained in Example 6 were applied to LB agar medium and cultured at 37°C overnight to obtain cell strains. Small amounts of such strains were inoculated into 5 ml of LB medium containing 50 mg/l of kanamycin and culturing was carried out overnight to obtain culture products. The culture products (50 µl) were inoculated into 5 ml of M9 medium that contained 1% glucose, 5 mg/l of iron sulfate and 20 g/l of calcium carbonate, shaking culture was conducted at 37°C, and 0, 6, 10, 13, 16, 19, and 32 hours after the initiation of culturing, turbidity was measured based on absorption at 660 nm.

Table 5 shows turbidity 19 and 32 hours after the initiation of culturing. Mutants with total deleted regions exceeding 933 kbp continuously exhibited increases in turbidity 19 hours after the initiation of culture and thereafter, at which point increase in turbidity of the parent strain appeared to have stopped and entered into a stationary phase. That is, mutants of *E*. *coli* strains having chromosomal DNA with total deleted regions of 933 kbp or more and with multiple partial deletions were found to yield a greater number of cells via culturing than wild-type *E. coli* strains.

### Example 8

### Preparation of substance using mutant strain of E. coli having chromosomal DNA with multiple partial deletions 1

(1) Preparation of threonine auxotrophic mutant
In accordance with the method of Example 1, threonine auxotrophic mutants that lack the threonine biosynthesis operon on chromosomal DNA of the *E*. *coli* W3110red strains (the thrABC gene comprising a nucleotide sequence comprising nucleotides 36 to 5175 of the nucleotide sequence as shown in SEQ ID NO: 1) were produced and designated as the *E*. *coli* W3110redΔT strains.
(2) Preparation of mutant that lacks metA gene and comprises threonine biosynthesis gene introduced therein
(i) Preparation of DNA fragment for metA gene deletion and thrABC gene introduction
   PCR was carried out using, as a template, chromosomal DNA of the *E*. *coli* W3110red strain and, as a primer set, DNA comprising the nucleotide sequence as shown in SEQ ID NO: 16 and DNA comprising the nucleotide sequence as shown in SEQ ID NO: 17. Thus, a DNA fragment having the nucleotide sequence in the 5' upstream region of the metA gene was amplified.
   PCR was carried out using LA-Taq, and a reaction solution was prepared in accordance with the instructions included therein. Purified chromosomal DNA (10 ng) and 20 pmol of each primer DNA were added to the reaction solution, and PCR was carried out. PCR was carried out by incubating at 94°C for 2 minutes, repeating a cycle of 94°C for 15 seconds, 55°C for 20 seconds, and 68°C for 90 seconds 25 times, and then incubating at 72°C for 10 minutes.
   The above PCR procedure verified that a target DNA fragment (fragment 1) had been obtained. By the similar method, a DNA fragment (fragment 2) comprising a nucleotide sequence of the 3' downstream region of the metA gene was obtained using DNA comprising the nucleotide sequence as shown in SEQ ID NO: 18 and DNA comprising the nucleotide sequence as shown in SEQ ID NO: 19 as primer DNAs.
   Subsequently, PCR was carried out using, as a template, chromosomal DNA of the *E*. *coli* W3110red strain and, as a primer set, DNA comprising the nucleotide sequence as shown in SEQ ID NO: 20 and DNA comprising the nucleotide sequence as shown in SEQ ID NO: 21 to amplify a DNA fragment containing threonine operon (thrABC gene).
   PCR was carried out using LA-Taq, and a reaction solution was prepared in accordance with the instructions included therein. Purified chromosomal DNA (10 ng) and 20 pmol of each primer DNA were added to the reaction solution, and PCR was carried out.
   PCR was carried out by incubating at 94°C for 2 minutes, repeating a cycle of 94°C for 15 seconds, 55°C for 20 seconds, and 68°C for 5 minutes 25 times, and then incubating at 72°C for 10 minutes.
   The above PCR procedure verified that a DNA fragment containing the thrABC gene (fragment 3) had been obtained.
   Also, PCR was carried out using, as a template, pHSG399 plasmid DNA (Takara Bio Inc.) and, as a primer set, DNA having the nucleotide sequence as shown in SEQ ID NO: 22 and DNA having the nucleotide sequence as shown in SEQ ID NO: 23 to amplify a DNA fragment containing a chloramphenicol-resistant gene (cat gene).
   PCR was carried out using LA-Taq, and a reaction solution was prepared in accordance with the instructions included therein. Plasmid DNA (40 ng) and 20 pmol of each primer DNA were added to the reaction solution, and PCR was carried out.
   PCR was carried out by incubating at 94°C for 2 minutes, repeating a cycle of 94°C for 15 seconds, 55°C for 20 seconds, and 68°C for 1 minute 25 times, and then heating at 72°C for 10 minutes.
   The above PCR procedure verified that a DNA fragment containing the chloramphenicol-resistant gene (fragment 4) had been obtained.
   The fragments 1 to 4 obtained by PCR were purified using the QIA quick Gel Extraction Kit (Qiagen) and dissolved in 50 µl of water to obtain DNA solutions. Primer sets of DNA as shown in SEQ ID NO: 17 and DNA as shown in SEQ ID NO: 22, DNA as shown in SEQ ID NO: 19 and DNA as shown in SEQ ID NO: 21, and DNA as shown in SEQ ID NO: 20 and DNA as shown in SEQ ID NO: 23 used in PCR each have an annelining sequence, i.e., a complementary sequence.
   PCR was carried out using as templates the fragments 1 to 4 obtained above and, as a primer set, DNA comprising the nucleotide sequence as shown in SEQ ID NO: 24 and DNA comprising the nucleotide sequence as shown in SEQ ID NO: 25 to amplify a DNA fragment comprising the threonine operon gene and the chloramphenicol-resistant gene ligated in the same transcription direction and comprising DNA having nucleotide sequences of the 5' upstream region and the 3' downstream region of the metA gene further ligated to both ends thereof.
   PCR was carried out using LA-Taq, and a reaction solution was prepared in accordance with the instructions included therein. The DNA solutions (1 µl each) and 20 pmol of each primer DNA were added to the reaction solution, and PCR was carried out.
   PCR was carried out by incubating at 94°C for 2 minutes, repeating a cycle of 94°C for 15 seconds, 55°C for 20 seconds, and 68°C for 7 minutes 25 times, and then incubating at 72°C for 10 minutes.
   The above PCR procedure verified that a target DNA fragment had been obtained (hereafter referred to as a "DNA fragment for threonine operon gene introduction"). The DNA fragment was purified using the QIA quick Gel Extraction Kit and dissolved in 50 µl of water to obtain a DNA solution.
(ii) Transformation of *E. coli* w3110redΔ strains
   The competent cells of the *E. coli* w3110redΔ strains obtained in (1) above were prepared in accordance with the method described in Example 1 (2) (i).
   The DNA fragment (0.1 µg) used for threonine operon gene introduction obtained in (2) (i) above was added to 50 µl of a solution of *E. coli* W3110redΔ competent cells, and electroporation was carried out using a 0.1-cm cuvette at 1.8 kV and 25 µF.
   Strains in which the metA gene region had been substituted with a DNA fragment for threonine operon gene introduction on chromosomal DNA were selected on an LBcm plate. From among the DNA fragments for threonine operon gene introduction obtained in (2)(i) above, strains into which mutation had been introduced at the time of PCR and into which threonine operon genes encoding inactive threonine biosynthesis proteins had been introduced were to be selected. To this end, strains that would grow on LB agar medium but would not grow on M9 met agar minimal medium (1% glucose, 0.6% sodium phosphate, 0.3% potassium dihydrogenphosphate, 0.05% sodium chloride, 0.1% ammonium chloride, 2 mmol/l of magnesium sulfate heptahydrate, 100 µmol/l of calcium chloride, 10 mg/l of ferric sulfate, and 100 mg/l of methionine), i.e., threonine auxotrophic mutants, were selected from among transformants selected on the LBcm plate. It was confirmed that such strains comprised inactive threonine biosynthesis genes inserted into the metA genes on chromosomal DNA and designated as the *E. coli* Thrl strains (W3110redΔT, ΔmetA::thrABC).
(iii) Preparation of threonine operon having desensitizing mutation and attenuator mutation
   PCR was carried out using, as a template, chromosomal DNA of the *E*. *coli* W3110red strain and, as a primer set, DNA comprising the nucleotide sequence as shown in SEQ ID NO: 26 and DNA comprising the nucleotide sequence as shown in SEQ ID NO: 27 to amplify a DNA fragment comprising part of the desensitizing thrA gene and a nucleotide sequence of the upstream region of the threonine operon. Specifically, DNA as shown in SEQ ID NO: 27 has the desensitizing mutation described in J. Bacteriol., 185, 5442-5461, 2003.
   PCR was carried out using LA-Taq and a reaction solution was prepared in accordance with the instructions included therein. Chromosomal DNA (10 ng) and 20 pmol of each primer DNA were added to the reaction solution and PCR was then carried out.
   PCR was carried out by incubating at 94°C for 2 minutes, repeating a cycle of 94°C for 15 seconds, 55°C for 20 seconds, and 68°C for 2 minutes and 30 seconds 25 times, and then incubating at 72°C for 10 minutes.
   The above PCR procedure verified that a target DNA fragment had been obtained. The DNA fragment was purified using the QIA quick Gel Extraction Kit and dissolved in 50 µl of water to obtain a DNA solution.
   Subsequently, PCR was carried out using, as a template, chromosomal DNA of the *E. coli* W3110red strain and, as a primer set, DNA comprising the nucleotide sequence as shown in SEQ ID NO: 20 and DNA comprising the nucleotide sequence as shown in SEQ ID NO: 28 to amplify a DNA fragment comprising part of the desensitising thrA gene and a nucleotide sequence downstream of the threonine operon. That is, DNA as shown in SEQ ID NO: 28 has a desensitizing mutation described in J. Bacteriol., 185, 5442-5461, 2003.
   PCR was carried out using LA-Taq and a reaction solution was prepared in accordance with the instructions included therein. Chromosomal DNA (10 ng) and 20 pmol of each primer DNA were added to the reaction solution and PCR was then carried out.
   PCR was carried out by incubating at 94°C for 2 minutes, repeating a cycle of 94°C for 15 seconds, 55°C for 20 seconds, and 68°C for 3 minutes and 30 seconds 25 times, and then incubating at 72°C for 10 minutes.
   The above PCR procedure verified that a target DNA fragment had been obtained. The DNA fragment was purified using the QIA quick Gel Extraction Kit and dissolved in 50 ml of water to obtain a DNA solution.
   PCR was carried out using, as a template, a DNA fragment comprising upstream and downstream nucleotide sequences of the threonine operon containing part of the desensitising thrA gene obtained via PCR above and, as a primer set, DNA comprising the nucleotide sequence as shown in SEQ ID NO: 20 and DNA comprising the nucleotide sequence as shown in SEQ ID NO: 29 to amplify a threonine operon DNA fragment containing the desensitising thrA gene.
   PCR was carried out using LA-Taq and a reaction solution was prepared in accordance with the instructions included therein. The DNA solution (1 µl each) and 20 pmol of each primer DNA were added to the reaction solution, and PCR was carried out.
   PCR was carried out by incubating at 94°C for 2 minutes, repeating a cycle of 94°C for 15 seconds, 55°C for 20 seconds, and 68°C for 5 minutes 25 times, and then incubating at 72°C for 10 minutes.
   The above PCR procedure verified that a target DNA fragment had been obtained. The DNA fragment was purified using the QIA quick Gel Extraction Kit and dissolved in 50 µl of water to obtain a DNA solution.
   Subsequently, PCR was carried out using, as a template, a threonine operon DNA fragment containing the desensitising thrA gene obtained above and, as a primer set, DNA comprising the nucleotide sequence as shown in SEQ ID NO: 29 and DNA comprising the nucleotide sequence as shown in SEQ ID NO: 30 to amplify a DNA fragment having a nucleotide sequence upstream of a threonine operon comprising an attenuator mutation. Specifically, DNA comprising the nucleotide sequence as shown in SEQ ID NO: 30 comprises an attenuator mutation of a threonine operon described in J. Mol. Biol., 183, 529-541, 1985.
   PCR was carried out using LA-Taq and a reaction solution was prepared in accordance with the instructions included therein. A DNA solution of a template DNA fragment (1 µl) and 20 pmol of each primer DNA were added to the reaction solution, and PCR was carried out.
   PCR was carried out by heating at 94°C for 2 minutes, repeating a cycle of 94°C for 15 seconds, 55°C for 20 seconds, and 68°C for 5 minutes 25 times, and then heating at 72°C for 10 minutes.
   The above PCR procedure verified that a target DNA fragment (fragment 5) had been obtained.
   Subsequently, PCR was carried out using, as a template, a threonine operon DNA fragment containing the desensitising thrA gene obtained above and, as a primer set, DNA comprising the nucleotide sequence as shown in SEQ ID NO: 31 and DNA comprising the nucleotide sequence as shown in SEQ ID NO: 20 to amplify a DNA fragment having a nucleotide sequence downstream of a threonine operon comprising an attenuator mutation. That is, DNA comprising the nucleotide sequence as shown in SEQ ID NO: 31 comprises an attenuator mutation of threonine operon described in J. Mol. Biol., 183, 529-541, 1985.
   PCR was carried out using LA-Taq and a reaction solution was prepared in accordance with the instructions included therein. A DNA solution of the template DNA fragment (1 µl) and 20 pmol of each primer DNA were added to the reaction solution, and PCR was carried out.
   PCR was carried out by incubating at 94°C for 2 minutes, repeating a cycle of 94°C for 15 seconds, 55°C for 20 seconds, and 68°C for 4 minutes and 30 seconds 25 times, and then incubating at 72°C for 10 minutes.
   The above PCR procedure verified that a target DNA fragment (fragment 6) had been obtained.
   Fragment 5 and fragment 6 obtained by the above PCR were purified using the QIA quick Gel Extraction Kit and dissolved in 50 µl of water to obtain DNA solutions.
   PCR was carried out using as templates fragment 5 and fragment 6 obtained above and, as a primer set, DNA comprising the nucleotide sequence as shown in SEQ ID NO: 20 and DNA comprising the nucleotide sequence as shown in SEQ ID NO: 21 to amplify a DNA fragment having a highly active threonine operon comprising the desensitising thrA gene and an attenuator mutation for increasing the expression level.
   PCR was carried out using LA-Taq and a reaction solution was prepared in accordance with the instructions included therein. The DNA solution containing fragment 5 and fragment 6 (1 µl each) and 20 pmol of each primer DNA were added to the reaction solution, and PCR was carried out.
   PCR was carried out by incubating at 94°C for 2 minutes, repeating a cycle of 94°C for 15 seconds, 55°C for 20 seconds, and 68°C for 5 minutes 25 times, and then incubating at 72°C for 10 minutes.
   The above PCR procedure verified that a target DNA fragment had been obtained. The DNA fragment was purified using the QIA quick Gel Extraction Kit and dissolved in 50 µl of water to obtain a DNA solution.
(iv) Preparation of *E. coli* strain possessing highly active threonine operon comprising desensitising thrA gene and attenuator mutation
   Competent cells of the *E. coli* Thr1 strains prepared in (ii) above were prepared in accordance with the method described in Example 1 (2) (i).
   A DNA fragment (0.1 µg) having the highly active threonine operon comprising the desensitising thrA gene and attenuator mutation obtained in (iii) above was added to 50 µl of a solution of competent cells of the *E. coli* Thr1 strain, and electroporation was carried out using a 0.1 cm cuvette at 1.8 kV and 25 µF.
   A transformant in which an inactive threonine operon has been substituted with a highly active threonine operon comprising the desensitising thrA gene and an attenuator mutation on chromosomal DNA was selected as a strain that would grow without exhibiting threonine auxotrophy using M9 met agar minimal medium. Such strain was confirmed to comprise the highly active threonine operon comprising the desensitising thrA gene and attenuator mutation inserted into the metA gene on chromosomal DNA and designated as the *E. coli* Thr2 strain.

(3) Fermentative production of threonine
In accordance with the method described in Example 1 (1), P1 transducing phages were prepared from the *E. coli* Thr2 strains. With the use of such phages, the *E. coli* W3110red strains and *E. coli* mutant strains comprising chromosomal DNAs with multiple partial deletions, which had been prepared in Example 3 and Example 6 (Step2, Step4, Step6, Step8, Step10, Step12, Step14, Step16, Step18y, Step20, Step22, Step24, Step26, and Step28), were subjected to P1 phage-mediated transduction in accordance with the method described in Example 1 (1). Strains in which a highly active threonine operon comprising the desensitising thrA gene and attenuator mutation derived from the *E*. *coli* Thr2 strain have been inserted into the metA gene of the mutant strains were selected as chloramphenicol-resistant strains and designated as W3110redT, Step2T, Step4T, Step6T, Step8T, Step10T, Step12T, Step14T, Step16T, Step18γT, Step20T, Step22T, Step24T, Step26T, and Step28T, respectively.
The transformants were applied to the LBcm plate described in Example 1 and cultured at 37°C overnight. Small amounts of cells were scraped from the plate and inoculated into a 300-ml conical flask with baffles containing 25 ml of threonine-producing medium (50 g/l of glucose, 20 g/l of ammonium sulfate, 1 g/l of monopotassium phosphate, 2 g/l of Bacto yeast extract, 0.5 g/l of magnesium sulfate heptahydrate, 5 mg/l of iron sulfate, 5 mg/l of manganese sulfate, 30 g/l of calcium carbonate, and 120 mg/l of methionine), followed by culturing in rotary shaker at 30°C and 250 rpm for 48 hours. During culturing, glucose was added thereto to a concentration of 20 g/l therein 24 hours after the initiation of culturing, and glucose was also added to culture solutions of Step10T, Step12T, Step14T, Step16T, Step18γT, Step20T, Step22T, Step24T, Step26T, and Step28T to a concentration of 20 g/l therein, 36 hours after the initiation of culturing.
After the completion of culturing, threonine that had been accumulated in the culture solution was quantified using an amino acid analyzer (Dionex). Glucose concentration in the culture solution was quantified using a reagent for quantifying glucose (Kyowa Medex Co., Ltd.). The results are shown in Table 6.

**Table 6**

| Strain name | Amount of strain at the completion of culture (OD660nm) | Amount of accumulated threonin (g/l) |
|---|---|---|
| W3110redT | 19.3 | 4.4 |
| Step2T | 19.5 | 2.2 |
| Step4T | 19.6 | 4.8 |
| Step6T | 20. 2 | 5. 7 |
| Step8T | 20. 3 | 3. 2 |
| Step10T | 22.3 | 7. 7 |
| Step12T | 22. 3 | 10.5 |
| Step14T | 24.3 | 9.5 |
| Step16T | 24.2 | 10.2 |
| Step18yT | 25.0 | 8.8 |
| Step20T | 24. 1 | 6. 1 |
| Step22T | 24. 7 | 11. 4 |
| Step24T | 23.5 | 11.2 |
| Step26T | 23. 6 | 10.1 |
| Step28T | 23.3 | 10. 6 |

As is apparent from Table 6, the Step 10 strain and the strains thereafter with the total length of chromosomal DNA deletion which exceeds 470 kb, in which the threonine biosynthetic gene has been reinforced, exhibits significant increase in the amount of threonine accumulated, as compared with a strain (W3110redT) in which the threonine biosynthetic gene of a strain comprising chromosomal DNA without deletions has been reinforced. Also, increase in the amount of threonine accumulated is equivalent to or higher than increase in cell mass increased. Accordingly, a mutant strain of *E. coli* comprising chromosomal DNA that is at least 470 kb shorter than that of a wild-type *E. coli* strain is useful as a host cell for substance production.

### Example 9

### Production of substances using mutant strain of E. coli comprising chromosomal DNA with multiple partial deletions 2

Glutathione is a peptidic substance comprising 3 types of amino acids, i.e., glutamic acid, cysteine, and glycine. The 3 aforementioned amino acids are condensed by the actions of γ-glutamyl cysteine synthetase and glutathione synthetase and glutathione is biosynthesized. ATP is a coenzyme that is indispensable for this condensation. Hereafter, the *E. coli* mutant strain of the present invention is used as an ATP source to produce glutathione.
(1) Obtaining of glutathione synthetase gene
   Plasmid DNA comprising the glutathione synthetase gene used in the experiment below was extracted from FERM BP-337 (Japanese Published Unexamined Patent Application No. 31690/90 (1990)).
   Plasmid DNA comprising the glutathione synthetase gene can also be obtained in the following manner.
   Specifically, in accordance with the methods described in Japanese Published Unexamined Patent Application No. 20196/83(1983) and J. Gen. Microbiol., 128, 1047-1052, 1982, the *E*. *coli* B strains were subjected to mutation by N-methyl-N'-nitrosoguanidine, mutants with attenuated glutathione synthesis were obtained, C912 was subjected to the same mutation, mutants with restored glutathione synthesis were obtained, and mutants in which γ-glutamyl cysteine synthetase inhibition by glutathione had been released are selected among therefrom (hereafter referred to as "inhibition-released strains").
   Subsequently, in accordance with the methods described in Japanese Published Unexamined Patent Application No. 31690/90 (1990) and Appl. Environ. Microbiol., 44, 1444-1448, 1982, the γ-glutamyl cysteine synthetase genes on the chromosome of the inhibition-released strain obtained by the above method were cloned into plasmid pBR322. Further, in accordance with the methods described in Japanese Published Unexamined Patent Application No. 31690/90(1990) and Agric. Biol. Chem., 47, 1381-1383, 1983, the glutathione synthetase genes on the chromosome of the inhibition-released strains were cloned into plasmid pBR322.
   Subsequently, in accordance with the methods described in Japanese Published Unexamined Patent Publication No. 31690/90 (1990) and Bioprocess Technol., 19, 159-183, 1994, the 2 above cloned fragments were accumulated in the vector plasmid pBR325. Thus, pBR325-gshI· II disclosed in Japanese Published Unexamined Patent Application No. 31690/90(1990) or plasmid DNA having a glutathione synthetase gene equivalent to that of the former plasmid DNA can be constructed.
   The pBR325-gshI· II obtained by the above method (hereafter referred to as "pGS600") was used to transform the *E*. *coli* C600 strain by the calcium chloride method. The transformant was selected using an LBcm plate containing 20 µg/ml of chloramphenicol and designated as *E*. *coli* C600/pGS600.
   The *E*. *coli* C600/pGS600 strain was subjected to shaking culture using 5 ml of LB liquid medium containing 3% glucose (LBG medium) at 30°C for 24 hours. Subsequently, 4 ml of the resulting culture product was introduced into a conical flask containing 400 ml of fresh LBG medium and the culture product was subjected to shaking culture at 30°C for 24 hours. The resulting culture product was centrifuged to precipitate cells, and the cells were washed twice with 100 ml of 100 mmol/l Tris-HCl buffer (0°C, pH 7.4).
   The washed cells were stored at -80°C, the cells were dissolved with the addition of 10 ml of 100 mmol/l Tris-HCl buffer (pH 7.4) immediately before use, and the resultant was used as an enzyme solution.
(2) Obtaining of *E*. *coli* W3110red cell strain and Step28 cell strain
   The W3110red strains and the Step28 strains were each independently subjected to shaking culture using 2.5 ml of LB liquid medium containing 3% glucose (LBG medium) at 30°C for 24 hours, and 200 µl of the resulting culture product was introduced into a conical flask containing 20 ml of fresh LBG medium, followed by shaking culture at 30°C for 24 hours. Table 7 shows turbidity (OD 660 nm) measured upon completion of culture.
   The resulting culture products were centrifuged to precipitate cells, and the cells were washed twice with 5 ml of 100 mmol/l Tris-HCl buffer (0°C, pH 7.4).
   To the resulting washed cells, 200 µl of a GT solution (20% glucose and 0.4% Triton X100) was added, and the resulting solution of cells was used as an ATP source.
(3) Production of glutathione
   To 900 µl of a reaction solution (2% glucose, 20 mmol/l of magnesium sulfate, 20 mmol/l of dipotassium sulfate, 0.22 mmol/l of oxidized nicotinamide adenine dinucleotide, 0.14 mmol/l of flavin mononucleotide, 5 mmol/l of glutamic acid, 5 mmol/l of cysteine, 5 mmol/l of glycine, 0.4% Triton X-100, 15 mmol/l of monopotassium phosphate, and 200 mmol/l of MOPS buffer; pH 7.8), 100 µl of the enzyme solution obtained in (1) above and 100 µl of the solution of the *E*. *coli* W3110red strains or Step28 strains obtained in (2) above were added, and the reaction was allowed to proceed while shaking culture was performed at 30°C for 90 minutes.

Glutathione that had been accumulated in the reaction solution was quantified using a glutathione quantification kit (Dojindo Laboratories). The results are shown in Table 7.

**Table 7**

| Strain name | Amount of strain at the completion of culture (turbidity 0D60nm) | Amount of accumulated glutathione (mmol/l) |
|---|---|---|
| W3110red | 2.2 | 0.18 |
| Step28 | 3.6 | 1.19 |

As shown in Table 7, the cell mass of the Step28 strains, which were subjected to the reaction as ATP sources and were mutants of *E*. *coli* strains having chromosomal DNA with multiple partial deletions, were 1.6 times (i.e., 3.6/2.2) those of the *E*. *coli* W3110red strains having chromosomal DNA without deletions; however, the figure for the amount of glutathione accumulated was 6.6 times (i.e., 1.19/0.18).

The *E*. *coli* mutant strain according to the present invention exhibits a higher activity for producing ATP per cell, i.e., a higher activity for reproducing ATP, than its parental strain, the *E*. *coli* W3110red strain without chromosomal DNA deletions. Therefore, the *E . coli* mutant strain according to the present invention was found to be useful for producing useful substances that require ATP in their biosynthetic pathways.

### Industrial Applicability

Use of the *E*. *coli* mutant strain of the present invention as a host cell enables effective production of useful substances.

### Free text of the Sequence Listing

SEQ ID NO:2 Explanation of Artificial sequence: synthesized DNA
SEQ ID NO:3 Explanation of Artificial sequence: synthesized DNA
SEQ ID NO:4 Explanation of Artificial sequence: synthesized DNA
SEQ ID NO:5 Explanation of Artificial sequence: synthesized DNA
SEQ ID NO:6 Explanation of Artificial sequence: synthesized DNA
SEQ ID NO:7 Explanation of Artificial sequence: synthesized DNA
SEQ ID NO:8 Explanation of Artificial sequence: synthesized DNA
SEQ ID NO:9 Explanation of Artificial sequence: synthesized DNA
SEQ ID NO: 10 Explanation of Artificial sequence: synthesized DNA
SEQ ID NO: 11 Explanation of Artificial sequence: synthesized DNA
SEQ ID NO:12 Explanation of Artificial sequence: synthesized DNA
SEQ ID NO:13 Explanation of Artificial sequence: synthesized DNA
SEQ ID NO:14 Explanation of Artificial sequence: synthesized DNA
SEQ ID NO:15 Explanation of Artificial sequence: synthesized DNA
SEQ ID NO: 16 Explanation of Artificial sequence: synthesized DNA
SEQ ID NO: 17 Explanation of Artificial sequence: synthesized DNA
SEQ ID NO:18 Explanation of Artificial sequence: synthesized DNA
SEQ ID NO: 19 Explanation of Artificial sequence: synthesized DNA
SEQ ID NO:20 Explanation of Artificial sequence: synthesized DNA
SEQ ID NO:21 Explanation of Artificial sequence: synthesized DNA
SEQ ID NO:22 Explanation of Artificial sequence: synthesized DNA
SEQ ID NO:23 Explanation of Artificial sequence: synthesized DNA
SEQ ID NO:24 Explanation of Artificial sequence: synthesized DNA
SEQ ID NO:25 Explanation of Artificial sequence: synthesized DNA
SEQ ID NO:26 Explanation of Artificial sequence: synthesized DNA
SEQ ID NO:27 Explanation of Artificial sequence: synthesized DNA
SEQ ID NO:28 Explanation of Artificial sequence: synthesized DNA
SEQ ID NO:29 Explanation of Artificial sequence: synthesized DNA
SEQ ID NO:30 Explanation of Artificial sequence: synthesized DNA
SEQ ID NO:31 Explanation of Artificial sequence: synthesized DNA

## Claims

1. An *E*. *coli* mutant strain comprising the following genes [1] and [2] or homologous genes thereof and comprising chromosomal DNA that is at least 470 kbp shorter than that of a wild-type *E*. *coli* strain, provided that when the following genes [1] and [2] or homologous genes thereof comprise genes that wild-type *E*. *coli* strains do not originally contain (hereafter referred to as "NC genes"), the aforementioned *E*. *coli* mutant strain derived from the wild-type *E*. *coli* strain does not necessarily have the NC gene.
[1] thrA gene, thrB gene, thrC gene, talB gene, mog gene, htgA gene, dnaK gene, dnaJ gene, hokC gene, nhaA gene, nhaR gene, rpsT gene, ribF gene, ileS gene, lspA gene, fkpB gene, lytB gene, dapB gene, carA gene, carB gene, caiF gene, caiE gene, caiD gene, caiC gene, caiB gene, caiA gene, caiT gene, fixA gene, fixB gene, fixC gene, fixX gene, kefC gene, folA gene, apaH gene, apaG gene, ksgA gene, pdxA gene, surA gene, imp gene, djlA gene, rluA gene, hepA gene, polB gene, araD gene, araA gene, araB gene, araC gene, tbpA gene, leuD gene, leuC gene, leuB gene, leuA gene, leuL gene, leuO gene, ilvI gene, ilvH gene, fruR gene, ftsL gene, ftsI gene, murE gene, murF gene, mraY gene, murD gene, ftsW gene, murG gene, murC gene, ddlB gene, ftsQ gene, ftsA gene, ftsZ gene, lpxC gene, secA gene, mutT gene, yacE gene, guaC gene, hofC gene, hofB gene, ppdD gene, nadC gene, ampD gene, ampE gene, aroP gene, pdhR gene, aceE gene, aceF gene, lpdA gene, acnB gene, speD gene, speE gene, gcd gene, hpt gene, yadF gene, panD gene, panC gene, panB gene, htrE gene, ecpD gene, folK gene, pcnB gene, dksA gene, sfsA gene, ligT gene, hrpB gene, ponB gene, hemL gene, pfs gene, dgt gene, htrA gene, dapD gene, glnD gene, map gene, rpsB gene, tsf gene, smb gene, rrf gene, dxr gene, rth gene, cdsA gene, yaeL gene, hlpA gene, lpxD gene, fabZ gene, lpxA gene, lpxB gene, rnhB gene, dnaE gene, accA gene, ldcC gene, mesJ gene, rof gene, cutF gene, proS gene, rcsF gene, abc gene, dniR gene, gloB gene, rnhA gene, dnaQ gene, up18 gene, gmhA gene, pepD gene, gpt gene, crl gene, phoE gene, proB gene, proA gene, hemB gene, proC gene, aroL gene, aroM gene, araJ gene, sbcC gene, sbcD gene, phoB gene, phoR gene, brnQ gene, proY gene, malZ gene, queA gene, tgt gene, secD gene, secF gene, tsx gene, ribD gene, ribH gene, nusB gene, thiL gene, pgpA gene, dxs gene, ispA gene, xseB gene, thiI gene, thiJ gene, apbA gene, cyoE gene, cyoD gene, cyoC gene, cyoB gene, cyoA gene, ampG gene, bolA gene, tig gene, clpP gene, clpX gene, lon gene, hupB gene, cof gene, mdlA gene, mdlB gene, glnK gene, amtB gene, tesB gene, maa gene, hha gene, acrB gene, acrA gene, acrR gene, aefA gene, priC gene, apt gene, dnaX gene, recR gene, htpG gene, adk gene, hemH gene, aes gene, gsk gene, fsr gene, ushA gene, cueR gene, gcl gene, gip gene, purK gene, purE gene, ybbF gene, ppiB gene, cysS gene, folD gene, sfmA gene, sfmC gene, sfmD gene, sfmH gene, sfmF gene, fimZ gene, entD gene, fepA gene, fes gene, entF gene, fepE gene, fepC gene, fepG gene, fepD gene, fepB gene, entC gene, entE gene, entB gene, entA gene, cstA gene, dsbG gene, ahpC gene, ahpF gene, rnk gene, rna gene, citT gene, citG gene, citX gene, citF gene, citE gene, citD gene, citC gene, citA gene, citB gene, dcuC gene, dcuC gene, crcA gene, cspE gene, crcB gene, lipA gene, lipB gene, dacA gene, rlpA gene, mrdB gene, mrdA gene, phpB gene, nadD gene, holA gene, rlpB gene, leuS gene, Int gene, phoL gene, ubiF gene, asnB gene, nagD gene, nagC gene, nagA gene, nagB gene, nagE gene, glnS gene, fur gene, fldA gene, seqA gene, pgm gene, potE gene, speF gene, kdpE gene, kdpD gene, kdpC gene, kdpB gene, kdpA gene, phrB gene, end8 gene, abrB gene, gltA gene, sdhC gene, sdhD gene, sdhA gene, sdhB gene, sucA gene, sucB gene, sucC gene, sucD gene, farR gene, hrsA gene, cydA gene, cydB gene, tolQ gene, tolR gene, tolA gene, tolB gene, pal gene, nadA gene, pnuC gene, czcD gene, aroG gene, gpmA gene, galM gene, galK gene, galT gene, galE gene, modF gene, modE gene, modA gene, modB gene, modC gene, bioA gene, bioB gene, bioF gene, bioC gene, bioD gene, uvrB gene, moaA gene, moaB gene, moaC gene, moaD gene, moaE gene, rhlE gene, dps gene, ompX gene, mipB gene, moeB gene, moeA gene, mdfA gene, grxA gene, poxB gene, aqpZ gene, cspD gene, clpA gene, infA gene, aat gene, cydC gene, cydD gene, trxB gene, lrp gene, ftsK gene, lolA gene, serS gene, dmsA gene, dmsB gene, dmsC gene, pflA gene, pflB gene, focA gene, serC gene, aroA gene, cmk gene, rpsA gene, himD gene, msbA gene, IpxK gene, kdsB gene, smtA gene, mukF gene, kicA gene, mukB gene, aspC gene, ompF gene, asnS gene, pncB gene, pepN gene, ssuB gene, ssuC gene, ssuD gene, ssuA gene, ssuE gene, pyrD gene, uup gene, pqiA gene, pqiB gene, rmf gene, fabA gene, ompA gene, sulA gene, helD gene, mgsA gene, acyP gene, hyaA gene, hyaB gene, hyaC gene, hyaD gene, hyaE gene, hyaF gene, appC gene, appB gene, appA gene, csgG gene, csgF gene, csgE gene, csgD gene, csgB gene, csgA gene, csgC gene, sfaA gene, mdoG gene, mdoH gene, msyB gene, htrB gene, solA gene, dinI gene, pyrC gene, grxB gene, rimJ gene, mviM gene, mviN gene, flgN gene, flgM gene, flgA gene, flgB gene, flgC gene, flgD gene, flgE gene, flgF gene, flgG gene, flgH gene, flgI gene, flgJ gene, flgK gene, flgL gene, rne gene, rluC gene, rpmF gene, plsX gene, fabH gene, fabD gene, fabG gene, acpP gene, fabF gene, pabC gene, tmk gene, holB gene, ptsG gene, fhuE gene, nagZ gene, ndh gene, mfd gene, ycfU gene, ycfV gene, ycfW gene, cobB gene, potD gene, potC gene, potB gene, potA gene, pepT gene, phoQ gene, phoP gene, purB gene, trmU gene, icdA gene, minE gene, minD gene, minC gene, clyA gene, umuD gene, umuC gene, dsbB gene, nhaB gene, fadR gene, dadA gene, dadX gene, IdcA gene, mltE gene, tag1 gene, treA gene, dhaR gene, pth gene, prsA gene, ychB gene, hemM gene, hemA gene, prfA gene, hemK gene, kdsA gene, chaA gene, chaB gene, chaC gene, narL gene, narX gene, narK gene, narG gene, narH gene, narJ gene, narI gene, tpr gene, purU gene, sprE gene, galU gene, hns gene, tdk gene, adhE gene, oppA gene, oppB gene, oppC gene, oppD gene, oppF gene, cls gene, kch gene, tonB gene, trpA gene, trpB gene, trpC gene, trpD gene, trpE gene, trpL gene, btuR gene, sohB gene, topA gene, cysB gene, acnA gene, ribA gene, pgpB gene, pyrF gene, osmB gene, rnb gene, envM gene, sapF gene, sapD gene, sapC gene, sapB gene, sapA gene, aldH gene, ordL gene, goaG gene, pspF gene, pspA gene, pspB gene, pspC gene, pspD gene, pspE gene, sucP gene, ompG gene, tyrR gene, tpx gene, mppA gene, fnr gene, ogt gene, dbpA gene, intR gene, lar gene, recT gene, recE gene, racC gene, kil gene, sieB gene, racR gene, dnaC gene, enpP gene, trkG gene, VMTH_LAMBD gene, VLOM_LAMBD gene, tnpO gene, upo3 gene, ompN gene, nifj gene, hslJ gene, IdhA gene, feaR gene, feaB gene, tynA gene, paaZ gene, paaA gene, paaB gene, paaC gene, paaD gene, paaE gene, paaF gene, paaG gene, paaH gene, paaI gene, paaJ gene, paaK gene, paaX gene, paaY gene, acpD gene, hrpA gene, aldA gene, gapC gene, gapC gene, cybB gene, trg gene, rimL gene, tehA gene, tehB gene, prtC gene, potA gene, potC gene, ansP gene, nhoA gene, narV gene, narW gene, narY gene, narZ gene, narU gene, fdnG gene, fdnH gene, fdnl gene, adhP gene, sfcA gene, rpsV gene, osmC gene, dos gene, xasA gene, gadB gene, pqqL gene, chuR gene, hipA gene, hipB gene, tam gene, uxaB gene, marC gene, marR gene, marA gene, marB gene, dcp gene, lycV gene, dicA gene, dicB gene, rspB gene, rspA gene, speG gene, dmsB gene, dmsC gene, bioD gene, mlc gene, asr gene, ebr gene, pntB gene, pntA gene, arcD gene, rstA gene, rstB gene, tus gene, fumC gene, fumA gene, manA gene, uidC gene, uidB gene, uidA gene, uidR gene, hdhA gene, malI gene, malX gene, malY gene, add gene, blr gene, nth gene, gst gene, pdxY gene, tyrS gene, pdxH gene, slyB gene, slyA gene, sodC gene, nemA gene, gloA gene, rnt gene, lhr gene, sodB gene, araJ gene, purR gene, cfa gene, ribE gene, ag43 gene, phsC gene, nrfC gene, pykF gene, lpp gene, sufS gene, aroD gene, ppsA gene, aroH gene, nlpC gene, btuD gene, btuE gene, btuC gene, himA gene, pheT gene, pheS gene, pheM gene, rplT gene, rpmI gene, infC gene, thrS gene, arp gene, pfkB gene, cedA gene, katE gene, celF gene, celD gene, celC gene, celB gene, celA gene, osmE gene, nadE gene, spy gene, astE gene, astB gene, astD gene, astA gene, cstC gene, xthA gene, gdhA gene, topB gene, selD gene, sppA gene, ansA gene, tdh gene, gapA gene, ttuC gene, caiT gene, rnd gene, fadD gene, slp gene, pabB gene, sdaA gene, manX gene, manY gene, manZ gene, rrmA gene, cspC gene, kdgR gene, tcmA gene, htpX gene, prc gene, pqiB gene, pphA gene, holE gene, exoX gene, ptrB gene, purT gene, eda gene, edd gene, zwf gene, pykA gene, mlt gene, znuA gene, znuC gene, ruvB gene, ruvA gene, ruvC gene, ntpA gene, aspS gene, bisZ gene, torY gene, cutC gene, argS gene, pgsA gene, amn gene, cbl gene, nac gene, erfK gene, cobT gene, cobS gene, cobU gene, gyrI gene, phsE gene, sbcB gene, hisL gene, hisG gene, hisD gene, hisC gene, hisB gene, hisH gene, hisA gene, hisF gene, hisI gene, wzz gene, ugd gene, gnd gene, galF gene, wcaM gene, wcaL gene, wcaK gene, wzxC gene, wcaJ gene, cpsG gene, cpsB gene, wcaI gene, wcaH gene, wcaG gene, gmd gene, wcaF gene, wcaE gene, wcaD gene, wcaC gene, wcaB gene, wcaA gene, wzb gene, wza gene, asmA gene, dcd gene, udk gene, thiD gene, thiM gene, mrp gene, metG gene, cdd gene, sanA gene, mglc gene, mglA gene, mglB gene, galS gene, folE gene, cirA gene, lysP gene, nfo gene, fruA gene, fruK gene, fruB gene, rplY gene, narP gene, ccmH gene, dsbE gene, ccmF gene, ccmE gene, ccmD gene, ccmC gene, ccmB gene, ccmA gene, napC gene, napB gene, napH gene, napG gene, napA gene, napD gene, napF gene, eco gene, alkB gene, ada gene, apbE gene, rcsB gene, rcsC gene, atoS gene, atoC gene, atoD gene, atoA gene, atoE gene, atoB gene, gyrA gene, ubiG gene, nrdA gene, nrdB gene, inaA gene, glpQ gene, glpT gene, glpA gene, glpB gene, glpC gene, cinA gene, ais gene, pmrD gene, menE gene, menC gene, menB gene, menD gene, menF gene, nuoN gene, nuoM gene, nuoL gene, nuoK gene, nuoJ gene, nuol gene, nuoH gene, nuoG gene, nuoF gene, nuoE gene, nuoD gene, nuoB gene, nuoA gene, lrhA gene, ackA gene, pta gene, ubiX gene, purF gene, dedE gene, dedD gene, folC gene, accD gene, dedA gene, truA gene, usgl gene, pdxB gene, div gene, fabB gene, ddpX gene, mepA gene, aroC gene, sixA gene, fadL gene, vacJ gene, dsdC gene, dsdX gene, dsdA gene, emrY gene, emrK gene, evgA gene, evgS gene, bifF gene, ddg gene, glk gene, mntH gene, nupC gene, gltX gene, xapR gene, xapB gene, xapA gene, lig gene, zipA gene, cysZ gene, cysK gene, ptsH gene, ptsI gene, gsr gene, pdxK gene, cysM gene, cysA gene, cysW gene, cysU gene, cysP gene, ucpA gene, ptbA gene, amiA gene, hemF gene, cchA gene, eutC gene, eutB gene, eutH gene, eutG gene, eutJ gene, eutE gene, cchB gene, eutI gene, talA gene, tktB gene, aegA gene, narQ gene, acrD gene, dapE gene, purC gene, nlpB gene, dapA gene, gcvR gene, bcp gene, hyfA gene, hyfB gene, hyfC gene, hyfD gene, hyfE gene, hyfF gene, hyfG gene, hyfH gene, hyfI gene, hyfJ gene, hyfR gene, focB gene, perM gene, yfgE gene, uraA gene, upp gene, purM gene, purN gene, ppk gene, ppx gene, guaA gene, guaB gene, xseA gene, hisS gene, gcpE gene, ndk gene, pbpC gene, sseA gene, sseB gene, pepB gene, fdx gene, hscA gene, hscB gene, iscU gene, iscS gene, iscR gene, suhB gene, csiE gene, dgxA gene, dgxB gene, hcaC gene, hcaB gene, hcaD gene, glyA gene, hmpA gene, glnB gene, purL gene, acpS gene, pdxJ gene, recO gene, era gene, rnc gene, lepB gene, lepA gene, rseC gene, rseB gene, rseA gene, rpoE gene, nadB gene, srmB gene, ung gene, trx2 gene, pssA gene, kgtP gene, clpB gene, rluD gene, pheL gene, pheA gene, tyrA gene, aroF gene, rplS gene, trmD gene, rimM gene, rpsP gene, ffh gene, grpE gene, recN gene, smpA gene, smpB gene, gabD gene, gabT gene, gabP gene, nrdF gene, emrR gene, emrA gene, emrB gene, gshA gene, csrA gene, alaS gene, oraA gene, recA gene, mltB gene, hypF gene, hydN gene, ascG gene, ascF gene, ascB gene, hyel gene, hycH gene, hycG gene, hycF gene, hycE gene, hycD gene, hycC gene, hycB gene, hycA gene, hypA gene, hypB gene, hypC gene, hypD gene, hypE gene, fhlA gene, mutS gene, prpB gene, rpoS gene, pcm gene, surE gene, ygbB gene, ygbP gene, ygbQ gene, cysC gene, cysN gene, cysD gene, iap gene, cysH gene, cysI gene, cysJ gene, ptpS gene, eno gene, pyrG gene, mazG gene, chpR gene, relA gene, barA gene, gud2 gene, gudT gene, mioC gene, syd gene, sdaB gene, exo gene, fucO gene, fucA gene, fucP gene, fucI gene, fucK gene, fucU gene, fucR gene, gcvA gene, nifS gene, mltA gene, amiC gene, argA gene, thyA gene, lgt gene, ptsP gene, mutH gene, tas gene, aas gene, galR gene, lysA gene, lysR gene, araE gene, kduD gene, kduI gene, lysS gene, prfB gene, recJ gene, dsbC gene, xerD gene, fldB gene, up14 gene, bglA gene, gcvP gene, gcvH gene, gcvT gene, visC gene, ubiH gene, pepP gene, serA gene, rpiA gene, iciA gene, sbm gene, argK gene, fba gene, pgk gene, epd gene, tktA gene, speB gene, speA gene, metK gene, galP gene, sprT gene, endA gene, gshB gene, yqgF gene, ansB gene, mutY gene, mltC gene, nupG gene, speC gene, pitB gene, gsp gene, hybG gene, hybF gene, hybE gene, hybD gene, hybC gene, hybB gene, hybA gene, hoxK gene, exbD gene, exbB gene, metC gene, exsA gene, sufI gene, plsC gene, parC gene, parE gene, icc gene, tolC gene, ribB gene, glgS gene, rfaE gene, glnE gene, cca gene, bacA gene, folB gene, ttdA gene, ttdB gene, ygjD gene, rpsU gene, dnaG gene, rpoD gene, aer gene, ebgR gene, ebgA gene, ebgC gene, fadH gene, uxaA gene, uxaC gene, exuT gene, exuR gene, tdcE gene, tdcD gene, tdcC gene, tdcB gene, tdcA gene, tdcR gene, sohA gene, agaR gene, agaZ gene, agaV gene, agaW gene, agaA gene, agaS gene, agaY gene, agaB gene, agaC gene, agaD gene, agaI gene, mtr gene, deaD gene, nlpI gene, pnp gene, rpsO gene, truB gene, rbfA gene, infB gene, nusA gene, argG gene, secG gene, mrsA gene, folP gene, ftsH gene, ftsJ gene, greA gene, dacB gene, yhbZ gene, rpmA gene, rplU gene, ispB gene, nlp gene, murA gene, yrbI gene, yrbK gene, rpoN gene, ptsN gene, ptsO gene, mtgA gene, arcB gene, gltB gene, nanT gene, npl gene, nanR gene, dcuD gene, sspB gene, sspA gene, rpsI gene, rplM gene, hhoA gene, hhoB gene, mdh gene, argR gene, tldD gene, rng gene, mreD gene, mreC gene, mreB gene, accB gene, accC gene, panF gene, prmA gene, fis gene, aroE gene, smg gene, smf gene, def gene, fmt gene, sun gene, trkA gene, mscL gene, zntR gene, rplQ gene, rpoA gene, rpsD gene, rpsK gene, rpsM gene, rpmJ gene, secY gene, rplO gene, rpmD gene, rpsE gene, rplR gene, rplF gene, rpsH gene, rpsN gene, rplE gene, rplX gene, rplN gene, rpsQ gene, rpmC gene, rplP gene, rpsC gene, rplV gene, rpsS gene, rplB gene, rplW gene, rplD gene, rplC gene, rpsJ gene, tufA gene, fusA gene, rpsG gene, rpsL gene, fkpA gene, slyX gene, slyD gene, kefB gene, prkB gene, crp gene, argD gene, pabA gene, fic gene, ppiA gene, nirB gene, nirD gene, nirC gene, cysG gene, php gene, trpS gene, gph gene, rpe gene, dam gene, damX gene, aroB gene, aroK gene, hofQ gene, mrcA gene, pckA gene, envZ gene, ompR gene, greB gene, feoA gene, feoB gene, bioH gene, gntT gene, malQ gene, malP gene, malT gene, rtcA gene, rtcB gene, rtcR gene, glpR gene, glpG gene, glpE gene, glpD gene, glgP gene, glgA gene, glgC gene, glgX gene, glgB gene, asd gene, gntU gene, gntU gene, gntK gene, gntR gene, ggt gene, ugpQ gene, ugpC gene, ugpE gene, ugpA gene, ugpB gene, livF gene, livG gene, livM gene, livH gene, livK gene, livJ gene, rpoH gene, ftsX gene, ftsE gene, ftsY gene, atzN gene, nikA gene, nikB gene, nikC gene, nikD gene, nikE gene, pitA gene, uspB gene, uspA gene, prlC gene, gor gene, arsR gene, arsB gene, arsC gene, slp gene, hdeB gene, hdeA gene, hdeD gene, gadA gene, treF gene, kdgK gene, dctA gene, dppF gene, dppD gene, dppC gene, dppB gene, dppA gene, tag gene, bisC gene, cspA gene, glyS gene, glyQ gene, xylB gene, xylA gene, xylF gene, xylG gene, xylH gene, xylR gene, bax gene, malS gene, avtA gene, lyx gene, sgbH gene, sgbU gene, sgbE gene, aldB gene, selB gene, selA gene, mtlA gene, mtlD gene, mtlR gene, lldP gene, lldR gene, lldD gene, cysE gene, gpsA gene, secB gene, grxC gene, tdh gene, kbl gene, htrL gene, rfaD gene, rfaF gene, rfaC gene, rfaL gene, rfaK gene, rfaZ gene, rfaY gene, rfaJ gene, rfaI gene, rfaB gene, rfaS gene, rfaP gene, rfaG gene, rfaQ gene, kdtA gene, kdtB gene, mutM gene, rpmG gene, rpmB gene, radC gene, dfp gene, dut gene, ttk gene, pyrE gene, rph gene, dinD gene, gmk gene, rpoZ gene, spoT gene, spoU gene, recG gene, gltS gene, nlpA gene, uhpT gene, uhpC gene, uhpB gene, uhpA gene, ilvN gene, ilvB gene, ivbL gene, emrD gene, ibpB gene, ibpA gene, gyrB gene, recF gene, dnaN gene, dnaA gene, rpmH gene, rnpA gene, yidC gene, thdF gene, tnaL gene, tnaA gene, tnaB gene, bglB gene, bglF gene, bglG gene, phoU gene, pstB gene, pstA gene, pstC gene, pstS gene, glmS gene, glmU gene, atpC gene, uncD gene, atpG gene, atpA gene, atpH gene, atpF gene, atpE gene, atpB gene, atpI gene, gidB gene, gidA gene, mioC gene, asnC gene, pssR gene, ilvL gene, ilvG gene, ilvM gene, ilvE gene, ilvD gene, ilvA gene, ilvY gene, ilvC gene, ppiC gene, rep gene, gppA gene, rhlB gene, trxA gene, rhoL gene, rho gene, rfe gene, wzzE gene, nfrC gene, rffD gene, rffG gene, rffH gene, rffA gene, wzxE gene, rffT gene, wecG gene, aslB gene, aslA gene, hemY gene, hemX gene, hemD gene, hemC gene, cyaA gene, cyaY gene, dapF gene, xerC gene, uvrD gene, corA gene, radD gene, pldA gene, recQ gene, pldB gene, metR gene, metE gene, udp gene, ubiE gene, aarF gene, tatC gene, tatD gene, rfaH gene, ubiD gene, ubiB gene, fadA gene, fadB gene, pepQ gene, trkH gene, trkH gene, hemG gene, mobB gene, mobA gene, dsbA gene, polA gene, yihA gene, hemN gene, ntrC gene, ntrB gene, glnA gene, fdhE gene, fdoI gene, fdoH gene, fdoG gene, fdhD gene, sodA gene, kdgT gene, cpxA gene, cpxR gene, cpxP gene, pfkA gene, sbp gene, cdh gene, tpiA gene, fpr gene, glpX gene, glpK gene, glpF gene, menG gene, menA gene, hslU gene, hslV gene, ftsN gene, cytR gene, priA gene, rpmE gene, metJ gene, metB gene, metL gene, metF gene, katG gene, gldA gene, talC gene, ppc gene, argE gene, argC gene, argB gene, argH gene, oxyR gene, udhA gene, trmA gene, btuB gene, murI gene, murB gene, birA gene, coaA gene, tufB gene, secE gene, nusG gene, rplK gene, rplA gene, rplJ gene, rplL gene, rpoB gene, rpoC gene, htrC gene, thiH gene, thiG gene, thiS gene, thiF gene, thiE gene, thiC gene, rsd gene, hemE gene, nfi gene, hupA gene, hydH gene, hydG gene, purD gene, purH gene, metA gene, aceB gene, aceA gene, aceK gene, arp gene, iclR gene, metH gene, pepE gene, lysC gene, pgi gene, ubiC gene, ubiA gene, plsB gene, dgkA gene, lexA gene, dinF gene, qor gene, dnaB gene, alr gene, tyrB gene, napA gene, hobH gene, uvrA gene, ssb gene, soxS gene, soxR gene, acs gene, nrfA gene, nrfB gene, nrfC gene, nrfD gene, nrfE gene, nrfF gene, nrfG gene, gltP gene, fdhF gene, alsR gene, rpiB gene, proP gene, basS gene, basR gene, adiY gene, adi gene, melR gene, melA gene, melB gene, fumB gene, dcuB gene, dcuR gene, dcuS gene, lysU gene, cadA gene, cadB gene, cadC gene, cutA3 gene, cutA2 gene, cutA gene, dcuA gene, aspA gene, fxsA gene, groES gene, groEL gene, efp gene, ecnB gene, sugE gene, blc gene, ampC gene, frdD gene, frdC gene, frdB gene, frdA gene, genX gene, psd gene, yjeQ gene, yjeE gene, amiB gene, mutL gene, miaA gene, hfq gene, hflX gene, hflK gene, hflC gene, purA gene, vacB gene, sgaE gene, rpsF gene, priB gene, rpsR gene, rplI gene, fldB gene, cycA gene, cpdB gene, cysQ gene, msrA gene, chpS gene, chpB gene, ppa gene, fbp gene, mpl gene, pmbA gene, cybC gene, nrdG gene, nrdD gene, treC gene, treB gene, treR gene, mgtA gene, pyrI gene, pyrB gene, pyrBI gene, argI gene, valS gene, holC gene, xerB gene, idnR gene, idnT gene, idnO gene, idnD gene, idnK gene, fecE gene, fecD gene, fecC gene, fecB gene, fecA gene, fecR gene, fecI gene, dnaC gene, dnaT gene, bglJ gene, fhuF gene, rsmC gene, holD gene, rimI gene, prfC gene, osmY gene, deoC gene, deoA gene, deoB gene, deoD gene, IplA gene, smp gene, serB gene, radA gene, nadR gene, slt gene, trpR gene, gpmG gene, rob gene, creA gene, creB gene, creC gene, creD gene, arcA gene, lasT gene, thrL gene;
[2] acrE gene, argT gene, artI gene, artJ gene, artM gene, artP gene, artQ gene, bfr gene, chiA gene, chpA gene, dacC gene, deoR gene, dinG gene, elaA gene, elaB gene, elaC gene, elaD gene, envD gene, envR gene, fhuA gene, fhuB gene, fhuC gene, fhuD gene, folX gene, glnH gene, glnP gene, glnQ gene, gltJ gene, gltK gene, gltL gene, gspA gene, gspC gene, gspD gene, gspE gene, gspl gene, gspJ gene, gspK gene, gspL gene, hisJ gene, hisM gene, hisP gene, hisQ gene, hofD gene, hofF gene, hofG gene, hofH gene, ltaA gene, mdaA gene, mdaB gene, pinO gene, potF gene, potG gene, potH gene, potI gene, pshM gene, ptwB gene, ptwC gene, rhsB gene, rimK gene, xylU gene.

2. The *E. coli* mutant strain according to claim 1 comprising chromosomal DNA that comprises DNA having the nucleotide sequences as shown in [1] to [69] below of the nucleotide sequence as shown in SEQ ID NO: 1 or DNA equivalent thereto.
[1] a nucleotide sequence of nucleotides 1 to 167064
[2] a nucleotide sequence of nucleotides 172980 to 243964
[3] a nucleotide sequence of nucleotides 253411 to 261963
[4] a nucleotide sequence of nucleotides 387532 to 389138
[5] a nucleotide sequence of nucleotides 403704 to 518029
[6] a nucleotide sequence of nucleotides 532712 to 535504
[7] a nucleotide sequence of nucleotides 550216 to 563940
[8] a nucleotide sequence of nucleotides 608119 to 675103
[9] a nucleotide sequence of nucleotides 689375 to 728820
[10] a nucleotide sequence of nucleotides 739594 to 832551
[11] a nucleotide sequence of nucleotides 848090 to 867637
[12] a nucleotide sequence of nucleotides 883474 to 890997
[13] a nucleotide sequence of nucleotides 909380 to 1048887
[14] a nucleotide sequence of nucleotides 1097020 to 1198105
[15] a nucleotide sequence of nucleotides 1225147 to 1630911
[16] a nucleotide sequence of nucleotides 1641039 to 1642631
[17] a nucleotide sequence of nucleotides 1650223 to 1964523
[18] a nucleotide sequence of nucleotides 1993076 to 1996661
[19] a nucleotide sequence of nucleotides 2028210 to 2033448
[20] a nucleotide sequence of nucleotides 2044377 to 2069148
[21] a nucleotide sequence of nucleotides 2081927 to 2104392
[22] a nucleotide sequence of nucleotides 2116065 to 2146263
[23] a nucleotide sequence of nucleotides 2187856 to 2200668
[24] a nucleotide sequence of nucleotides 2229851 to 2255894
[25] a nucleotide sequence of nucleotides 2263492 to 2270439
[26] a nucleotide sequence of nucleotides 2286589 to 2386261
[27] a nucleotide sequence of nucleotides 2395472 to 2423364
[28] a nucleotide sequence of nucleotides 2434099 to 2472851
[29] a nucleotide sequence of nucleotides 2482483 to 2505074
[30] a nucleotide sequence of nucleotides 2514558 to 2755676
[31] a nucleotide sequence of nucleotides 2789481 to 2795173
[32] a nucleotide sequence of nucleotides 2800891 to 2804307
[33] a nucleotide sequence of nucleotides 2810148 to 2825313
[34] a nucleotide sequence of nucleotides 2830263 to 2910273
[35] a nucleotide sequence of nucleotides 2910574 to 2985361
[36] a nucleotide sequence of nucleotides 3033129 to 3077084
[37] a nucleotide sequence of nucleotides 3078917 to 3110109
[38] a nucleotide sequence of nucleotides 3134342 to 3165627
[39] a nucleotide sequence of nucleotides 3172941 to 3360736
[40] a nucleotide sequence of nucleotides 3370548 to 3411159
[41] a nucleotide sequence of nucleotides 3420879 to 3453020
[42] a nucleotide sequence of nucleotides 3469367 to 3618575
[43] a nucleotide sequence of nucleotides 3636917 to 3854661
[44] a nucleotide sequence of nucleotides 3865832 to 3867101
[45] a nucleotide sequence of nucleotides 3877011 to 3926658
[46] a nucleotide sequence of nucleotides 3938879 to 4059897
[47] a nucleotide sequence of nucleotides 4079660 to 4086450
[48] a nucleotide sequence of nucleotides 4099983 to 4139231
[49] a nucleotide sequence of nucleotides 4149710 to 4235358
[50] a nucleotide sequence of nucleotides 4251736 to 4298976
[51] a nucleotide sequence of nucleotides 4311512 to 4313317
[52] a nucleotide sequence of nucleotides 4329722 to 4409532
[53] a nucleotide sequence of nucleotides 4423152 to 4496201
[54] a nucleotide sequence of nucleotides 4509535 to 4517875
[55] a nucleotide sequence of nucleotides 4599638 to 4641433
[56] a nucleotide sequence of nucleotides 167065 to 172979
[57] a nucleotide sequence of nucleotides 675104 to 689374
[58] a nucleotide sequence of nucleotides 832552 to 848089
[59] a nucleotide sequence of nucleotides 867638 to 883473
[60] a nucleotide sequence of nucleotides 890998 to 909379
[61] a nucleotide sequence of nucleotides 2386262 to 2395471
[62] a nucleotide sequence of nucleotides 2423365 to 2434098
[63] a nucleotide sequence of nucleotides 2472852 to 2482482
[64] a nucleotide sequence of nucleotides 2505075 to 2514557
[65] a nucleotide sequence of nucleotides 2910274 to 2910573
[66] a nucleotide sequence of nucleotides 3165628 to 3172940
[67] a nucleotide sequence of nucleotides 3411160 to 3420878
[68] a nucleotide sequence of nucleotides 3453021 to 3469366
[69] a nucleotide sequence of nucleotides 3618576 to 3636916

3. The *E. coli* mutant strain according to claim 1 or 2 comprising chromosomal DNA that does not comprise yafJ gene, yafK gene, yafQ gene, dinJ gene, yafL gene, yafM gene, fhiA gene, lafU gene, dinP gene, yafN gene, yafO gene, yafP gene, JW0225 gene, prfH gene, ykfI gene, yafW gene, yeeT gene, ykfG gene, yafX gene, ykfF gene, ykfB gene, yafY gene, ypjK gene, yafZ gene, ykfA gene, perR gene, JW0245 gene, JW0246 gene, JW0247 gene, ykfC gene, JW0249 gene, is5B gene, yi51 gene, mmuP gene, mmuM gene, afuC gene, JW0255 gene, insB gene, insA gene, JW0258 gene, yagB gene, yagA gene, yagE gene, yagF gene, yagG gene, yagH gene, yagI gene, argF gene, yfjI gene, yagJ gene, yagK gene, yagL gene, yagM gene, yagN gene, intF gene, yagP gene, yagQ gene, yagR gene, yagS gene, yagT gene, yagU gene, ykgJ gene, yagV gene, yagW gene, yagX gene, yagY gene, yagZ gene, ykgK gene, ykgL gene, ykgM gene, eaeH gene, JW0292 gene, JW0293 gene, ykgA gene, ykgB gene, ykgI gene, merA gene, ykgD gene, ykgE gene, ykgF gene; ykgG gene, ykgH gene, betA gene, betB gene, betI gene, betT gene, yahA gene, yahB gene, yahC gene, yahD gene, yahE gene, yahF gene, yahG gene, yahH gene, arcM gene, yahJ gene, yahK gene, yahL gene, yahM gene, yahN gene, yahO gene, prpR gene, bcpA gene, prpC gene, prpD gene, prpE gene, codB gene, codA gene, cynR gene, cynT gene, cynS gene, cynX gene, lacA gene, lacY gene, lacZ gene, lacI gene, mhpR gene, mhpA gene, mhpB gene, mhpC gene, mhpD gene, mhpF gene, mhpE gene, mhpT gene, yaiL gene, yaiM gene, adhC gene, yaiN gene, yaiO gene, JW0350 gene, yi2A gene, yi22 gene, yi24 gene, yi23 gene, yaiP gene, yaiS gene, tauA gene, tauB gene, tauC gene, ssiD gene, yaiT gene, JW0363 gene, JW0364 gene, yaiU gene, yaiV gene, ampH gene, sbmA gene, yaiW gene, yaiY gene, yaiZ gene, ddlA gene, yaiB gene, phoA gene, psiF gene, yaiC gene, ybbO gene, tesA gene, ybbA gene, ybbP gene, rhsD gene, ybbC gene, rhsE gene, ybbD gene, JW0490 gene, ybbB gene, ybbS gene, ybbT gene, ybbU gene, ybbQ gene, ybbV gene, allP gene, allA gene, ybbY gene, ybbZ gene, ylbA gene, allC gene, ylbC gene, fdrA gene, ylbE gene, ylbF gene, arcC gene, intD gene, EXO_LAMBD gene, VRPP_LAMBD gene, JW0528 gene, JW0529 gene, VREN_LAMBD gene, emrE gene, ybcK gene, ybcL gene, ybcM gene, ybcN gene, ninE gene, ybcO gene, rus gene, ybcQ gene, JW0539.5 gene, nmpC gene, vlyS gene, ybcS gene, ybcT gene, vboR gene, ybcV gene, ybcW gene, nohB gene, ybcX gene, ybcY gene, JW0552 gene, appY gene, ompT gene, envY gene, ybcH gene, nfrA gene, nfrb gene, cusS gene, cusR gene, cusC gene, ylcC gene, cusB gene, cusA gene, pheP gene, ybdG gene, nfnB gene, ybdF gene, ybdJ gene, ybdK gene, hokE gene, yi81 gene, yi82 gene, ymcC gene, ymcD gene, cspH gene, cspG gene, sfa gene, yccL gene, yccM gene, torS gene, torT gene, torR gene, torC gene, torA gene, torD gene, yccD gene, cbpA gene, yccE gene, agp gene, yccJ gene, wrbA gene, yciG gene, ycdG gene, JW0992 gene, JW0993 gene, JW0994 gene, ycdK gene, JW0996 gene, JW0997 gene, ycdC gene, putA gene, yzpU gene, putP gene, JW1002 gene, ycdO gene, ycdB gene, phoH gene, ycdP gene, ycdQ gene, ycdR gene, ycdS gene, ycdT gene, JW1012 gene, JW1013 gene, JW1014 gene, ycdU gene, ymfD gene, ymfE gene, lit gene, intE gene, VXIS_BPP21 gene, ymfH gene, ymfI gene, ymfJ gene, RPC2_BPP22 gene, JW1132 gene, ymfL gene, ymfM gene, ymfN gene, ymfR gene, ymfO gene, JW1138 gene, JW1139 gene, ycfK gene, ymfS gene, ycfA gene, ycfE gene, pin gene, mcrA gene, JW 1146 gene, ycgW gene, ycgX gene, ycgE gene, JW 1150 gene, ycgZ gene, ymgA gene, JW1153 gene, ymgC gene, yahA gene, JW1156 gene, JW1157 gene, aidA gene, ymgD gene, JW1160 gene, JW1161 gene, JW1162 gene, ydfH gene, ydfZ gene, ydfI gene, shiA gene, JW1537 gene, tnpR gene, ydfM gene, Y314_LAMBD gene, nohA gene, ydfO gene, yccL gene, cspI gene, ydfR gene, vlyS gene, cspB gene, cspF gene, regQ_LAMBD gene, JW1552 gene, rem gene, hokD gene, relE gene, relB gene, ydfV gene, flxA gene, ydfW gene, ydfX gene, dicC gene, sdaC gene, yqeH gene, yqeI gene, yqeJ gene, yqeK gene, ygeF gene, ygeG gene, ygeH gene, ygeI gene, JW2822 gene, ygeK gene, JW2824 gene, JW2825 gene, JW2830 gene, ygeP gene, JW2832 gene, nlpD gene, yagR gene, ygeT gene, JW2836 gene, ygeV gene, ygeW gene, dpaL gene, ygeY gene, ygeZ gene, arcL gene, yqeB gene, JW2844 gene, ygfJ gene, gltD gene, JW2847 gene, ygfM gene, yagR gene, ygfO gene, ygfP gene, ygfQ gene, ygfR gene, ygfs gene, ygfT gene, pbuX gene, idi gene, epsM gene, exeL gene, exeC gene, JW2938 gene, JW2939 gene, JW2940 gene, JW2941 gene, yghK gene, glcB gene, glcG gene, glcF gene, glcD gene, glcC gene, JW2948 gene, yghQ gene, yghR gene, yghS gene, yghT gene, yihL gene, yihM gene, yihN gene, yshA gene, yihO gene, yihP gene, yihQ gene, yihR gene, yihS gene, yihT gene, yihU gene, yihV gene, yihW gene, yihX gene, rbn gene, yihZ gene, yiiD gene, yiiE gene, yiiF gene, yiiG gene, frvR gene, frvX gene, frvB gene, frvA gene, yiiL gene, rhaD gene, rhaA gene, rhaB gene, rhaS gene, rhaR gene, rhaT gene, ptsA gene, JW3920 gene, frwC gene, frwB gene, pflD gene, pflC gene, frwD gene, yijO gene, yijP gene, JW3986 gene, yjbF gene, yjbG gene, yjbH gene, JW3989.5 gene, yjbA gene, xylE gene, malG gene, malF gene, malE gene, malK gene, lamB gene, malM gene, yjbI gene, yjcP gene, yjcQ gene, yjcR gene, yjcS gene, alsK gene, alsE gene, alsC gene, alsA gene, alsB gene, phnQ gene, phnP gene, phnO gene, phnN gene, phnM gene, phnL gene, phnK gene, phnJ gene, phnI gene, phnH gene, phnG gene, phnF gene, phnE gene, phnD gene, phnC gene, phnB gene, phnA gene, yjdA gene and yjcZ gene, or homologous gene thereof.

4. The *E. coli* mutant strain according to any one of claims 1 to 3 comprising chromosomal DNA that does not comprise DNA having the nucleotide sequences as shown in
[1] to [18] below of the nucleotide sequence as shown in SEQ ID NO: 1 or DNA equivalent thereto.
[1] a nucleotide sequence of nucleotides 243965 to 253410
[2] a nucleotide sequence of nucleotides 261964 to 387531
[3] a nucleotide sequence of nucleotides 389139 to 403703
[4] a nucleotide sequence of nucleotides 518030 to 532711
[5] a nucleotide sequence of nucleotides 535505 to 550215
[6] a nucleotide sequence of nucleotides 563941 to 608118
[7] a nucleotide sequence of nucleotides 1048888 to 1097019
[8] a nucleotide sequence of nucleotides 1198106 to 1225146
[9] a nucleotide sequence of nucleotides 1630912 to 1641038
[10] a nucleotide sequence of nucleotides 1642632 to 1650222
[11] a nucleotide sequence of nucleotides 2985362 to 3033128
[12] a nucleotide sequence of nucleotides 3110110 to 3134341
[13] a nucleotide sequence of nucleotides 4059898 to 4079659
[14] a nucleotide sequence of nucleotides 4086451 to 4099982
[15] a nucleotide sequence of nucleotides 4139232 to 4149709
[16] a nucleotide sequence of nucleotides 4235359 to 4251735
[17] a nucleotide sequence of nucleotides 4298977 to 4311511
[18] a nucleotide sequence of nucleotides 4313318 to 4329721

5. The *E*. *coli* mutant strain according to any one of claims 1 to 4 comprising chromosomal DNA that does not comprise yidF gene, yidG gene, yidH gene, yidI gene, yidJ gene, yidK gene, yidL gene, JW3657 gene, glvG gene, glvB gene, glvC gene, yidP gene, yidE gene, yidQ gene, yidR gene, yidS gene, dgoT gene, dgoA gene, dgoK gene, dgoR gene, yidW gene, yidX gene, yidA gene, yidB gene, asnA gene, yieM gene, JW3724 gene, yieN gene, kup gene, rbsD gene, rbsA gene, rbsC gene, rbsB gene, rbsK gene, rbsR gene and yieO gene, or homologous gene thereof.

6. The *E*. *coli* mutant strain according to any one of claims 1 to 5 comprising chromosomal DNA that does not comprise DNA having the nucleotide sequences as shown in
[1] to [3] below of the nucleotide sequence as shown in SEQ ID NO: 1 or DNA equivalent thereto.
[1] a nucleotide sequence of nucleotides3854662 to 3865831
[2] a nucleotide sequence of nucleotides 3867102 to 3877010
[3] a nucleotide sequence of nucleotides 3926659 to 3938878

7. The *E*. *coli* mutant strain according to any one of claims 1 to 6 comprising chromosomal DNA that does not comprise yecT gene, flhE gene, flhA gene, flhB gene, cheZ gene, cheY gene, cheB gene, cheR gene, tap gene, tar gene, cheW gene, cheA gene, motB gene, motA gene, flhC gene, flhD gene, JW1882 gene, JW1883 gene, yecG gene, otsA gene, otsB gene, araH gene, araG gene, araF gene, yecI gene, yecJ gene, JW1892 gene, ftn gene, yecH gene, tyrP gene, uvrC gene, uvrY gene, yecF gene, sdiA gene, yecC gene, yecS gene, yedO gene, fliY gene, fliZ gene, fliA gene, fliC gene, fliD gene, fliS gene, fliT gene, amyA gene, yedD gene, yedE gene, yedF gene, yedK gene, yedL gene, yedN gene, yedM gene, fliE gene, fliF gene, fliG gene, fliH gene, fliI gene, fliJ gene, fliK gene, fliL gene, fliM gene, fliN gene, fliO gene, fliP gene, fliQ gene, fliR gene, rcsA gene, dsrB gene, JW1937 gene, yedA gene, vsr gene, dcm gene, yedJ gene, JW1946 gene, ompC gene, JW1948 gene, yedS gene, yedU gene, yedV gene, copR gene, yedX gene, JW1954 gene, JW1955 gene, JW1956 gene, JW 1974 gene, JW1979 gene, JW1980 gene, yeeP gene, flu gene, JW1983 gene, yeeS gene, yeeU gene, yeeV gene, yeeW gene, yefJ gene, JW2013 gene, yefj gene, yefI gene, wbbJ gene, yefG gene, rfc gene, glf gene, rfbX gene, rfbC gene, rfbA gene, rfbD gene and rfbB gene, or homologous gene thereof.

8. The *E*. *coli* mutant strain according to any one of claims 1 to 7 comprising chromosomal DNA that does not comprise DNA having the nucleotide sequences as shown in
[1] to [5] below of the nucleotide sequence as shown in SEQ ID NO: 1 or DNA equivalent thereto.
[1] a nucleotide sequence of nucleotides1964524 to 1993075
[2] a nucleotide sequence of nucleotides 1996662 to 2028209
[3] a nucleotide sequence of nucleotides 2033449 to 2044376
[4] a nucleotide sequence of nucleotides 2069149t to 2081926
[5] a nucleotide sequence of nucleotides 2104393 to 2116064

9. The *E*. *coli* mutant strain according to any one of claims 1 to 8 comprising chromosomal DNA that does not comprise intA gene, yfjH gene, alpA gene, yfjI gene, JW2606 gene, yfjJ gene, yfjK gene, yfjL gene, yfjM gene, yfjN gene, yfjO gene, yfjP gene, yfjQ gene, yfjR gene, ypjK gene, yfjS gene, yfjT gene, yfjU gene, yfjV gene, JW2622 gene, yfjW gene, JW2623.5 gene, yfjX gene, yfjY gene, yfjZ gene, ypjF gene, ypjA gene, JW2629 gene, JW2630 gene, JW2631 gene, JW2632 gene, JW2633 gene, JW2634 gene, ygaE gene, yzzM gene, yqaE gene, JW2642 gene, ygaP gene, stpA gene, JW2645 gene, ygaC gene, ygaM gene, nrdH gene, nrdI gene, nrdE gene, proV gene, proW gene, proX gene, JW2655 gene, JW2656 gene, ygaZ gene, ygaH gene, srlA gene, srlB gene, srlD gene, gutM gene, srlR gene and gutQ gene, or homologous gene thereof.

10. The *E*. *coli* mutant strain according to any one of claims 1 to 9 comprising chromosomal DNA that does not comprise DNA having the nucleotide sequences as shown in
[1] to [4] below of the nucleotide sequence as shown in SEQ ID NO: 1 or DNA equivalent thereto.
[1] a nucleotide sequence of nucleotides 2755677 to 2789480
[2] a nucleotide sequence of nucleotides 2795174 to 2800890
[3] a nucleotide sequence of nucleotides 2804308 to 2810147
[4] a nucleotide sequence of nucleotides 2825314 to 2830262

11. The *E. coli* mutant strain according to any one of claims 1 to 10 comprising chromosomal DNA that does not comprise gltF gene, yhcA gene, yhcD gene, yhcE gene, yhcF gene, yhcG gene, yhcH gene, nanK gene and nanE gene, or homologous gene thereof.

12. The *E. coli* mutant strain according to any one of claims 1 to 11 comprising chromosomal DNA that does not comprise DNA having a nucleotide sequence comprising nucleotides 3360737 to 3370547 of the nucleotide sequence as shown in SEQ ID NO: 1 or DNA equivalent thereto.

13. The *E. coli* mutant strain according to any one of claims 1 to 12 comprising chromosomal DNA that does not comprise yegE gene, alkA gene, yegD gene, JW2055 gene, JW2056 gene, JW2057 gene, JW2058 gene, yegM gene, yegN gene, yegO gene, yegB gene, baeS gene, baeR gene, JW2065 gene, yegQ gene, ogrK gene, JW2068 gene, JW2069 gene, yegS gene, gatR gene, JW2072 gene, JW2073 gene, gatR gene, gatD gene, gatC gene, gatB gene, gatA gene, gatZ gene, gatY gene, fbaB gene, xegT gene, yegU gene, yegV gene, yegW gene, yegX gene, molR gene, yehI gene, yehK gene, yehL gene, yehO gene, yehP gene, yehQ gene, yehR gene, yehS gene, yehT gene, yehU gene, yehV gene, yehW gene, yehX gene, yehY gene, yehZ gene, bglX gene, dld gene, pbpG gene, yohC gene, yeiQ gene, yeiR gene, yeiU gene, spr gene, rtn gene, yejA gene, yejB gene, yejE gene, yejF gene, yejG gene, bcr gene, rsuA gene, yejH gene, yeiI gene, yeiJ gene, yeiK gene, yeiL gene, yeiM gene, yeiN gene and yeiC gene, or homologous gene thereof.

14. The *E. coli* mutant strain according to any one of claims 1 to 13 comprising chromosomal DNA that does not comprise DNA having any of the nucleotide sequences as shown in [1] to [4] below of the nucleotide sequence as shown in SEQ ID NO: 1 or DNA equivalent thereto.
[1] a nucleotide sequence of nucleotides2146264 to 2187855
[2] a nucleotide sequence of nucleotides 2200669 to 2229850
[3] a nucleotide sequence of nucleotides 2270440 to 2286588
[4] a nucleotide sequence of nucleotides 2255895 to 2263491

15. The *E*. *coli* mutant strain according to any one of claims 1 to 14 comprising chromosomal DNA that does not copmrise cmtA gene, cmtB gene, or homologous gene thereof.

16. The *E*. *coli* mutant strain according to any one of claims 1 to 15 comprising chromosomal DNA that does not comprise DNA having a nucleotide sequence comprising nucleotides 3077085 to 3078916 of the nucleotide sequence as shown in SEQ ID NO: 1 or DNA equivalent thereto.

17. The *E*. *coli* mutant strain according to any one of claims 1 to 16 comprising chromosomal DNA that does not comprise intB gene, JW4228 gene, yjgW gene, yjgX gene, yjgY gene, yjgZ gene, yi41 gene, yi42 gene, yjhB gene, yjhC gene, yjhD gene, yjhE gene, yi91 gene, JW4244 gene, JW4245 gene, JW4246 gene, yjhU gene, yjhF gene, yjhG gene, yjhH gene, yjhI gene, sgcR gene, sgcE gene, sgcA gene, sgcQ gene, sgcC gene, JW4266.5 gene, sgcX gene, yjhP gene, yjhQ gene, JW4269.5 gene, JW4270 gene, JW4270.5 gene, yjhR gene, yjhS gene, yjhT gene, yjhA gene, fimB gene, fimE gene, fimA gene, fimI gene, fimC gene, fimD gene, fimF gene, fimG gene, fimH gene, gntP gene, uxuA gene, uxuB gene, uxuR gene, yjiC gene, yjiD gene, yjiE gene, iadA gene, yjiG gene, yjiH gene, yjiI gene, yjiJ gene, yjiK gene, JW4296.5 gene, yjiL gene, yjiM gene, yjiN gene, yjiO gene, yjiP gene, yjiQ gene, yjiR gene, yjiS gene, yjiT gene, yjiV gene, mcrD gene, mcrC gene, mcrB gene, yjiW gene, hsdS gene, hsdM gene, hsdR gene, mrr gene, yjiA gene, yjiX gene, yjiY gene, tsr gene, yjjL gene, JW4320 gene, yjjM gene, yjjN gene, mdoB gene and yjjA gene, or homologous gene thereof.

18. The *E*. *coli* mutant strain according to any one of claims 1 to 17 comprising chromosomal DNA that does not comprise DNA having the nucleotide sequences as shown in
[1] and/or [2] below of the nucleotide sequence as shown in SEQ ID NO: 1 or DNA equivalent thereto.
[1] a nucleotide sequence of nucleotides 4496202 to 4509534
[2] a nucleotide sequence of nucleotides 4517876 to 4599637

19. The *E. coli* mutant strain according to any one of claims 1 to 18 comprising chromosomal DNA that does not comprise kdpF gene, ybfA gene, rhsC gene, JW0690 gene, ybfB gene, rhsA gene, ybfC gene, ybfD gene, ybfL gene, ybfD gene and ybgA gene, or homologous gene thereof.

20. The *E*. *coli* mutant strain according to any one of claims 1 to 19 comprising chromosomal DNA that does not comprise DNA having a nucleotide sequence comprising nucleotides 728821 to 739593 of the nucleotide sequence as shown in SEQ ID NO: 1 or DNA equivalent thereto.

21. The *E*. *coli* mutant strain according to any one of claims 1 to 20 comprising chromosomal DNA that does not comprise yjfI gene, yjfJ gene, yjfK gene, yjfL gene, yjfM gene, yjfC gene, aidB gene, yjfN gene, yjfO gene, yjfP gene, yjfQ gene, yjfR gene, sgaT gene, sgaB gene, sgaA gene, sgaH gene and sgaU gene, or homologous gene thereof.

22. The *E. coli* mutant strain according to any one of claims 1 to 21 comprising chromosomal DNA that does not comprise DNA having a nucleotide sequence comprising nucleotides 4409533 to 4423151 of the nucleotide sequence as shown in SEQ ID NO: 1 or DNA equivalent thereto.

23. An *E*. *coli* mutant strain comprising the gene (1) [1] above, spr gene, bcr gene, rsuA gene, or homologous gene thereof and comprising chromosomal DNA that is at least 933 kbp shorter than that of a wild-type *E. coli* strain.

24. The *E. coli* mutant strain according to claim 23 comprising chromosomal DNA that comprises DNA having the nucleotide sequences as shown in [1] to [55] of (2) above and DNA having a nucleotide sequence comprising nucleotides 2270440 to 2286588 of the nucleotide sequence as shown in SEQ ID NO: 1 or DNA equivalent thereto.

25. The *E. coli* mutant strain according to claim 23 or 24 comprising chromosomal DNA that does not comprise yafJ gene, yafK gene, yafQ gene, dinJ gene, yafL gene, yafM gene, fhiA gene, lafU gene, dinP gene, yafN gene, yafO gene, yafP gene, JW0225 gene, prfH gene, ykfI gene, yafW gene, yeeT gene, ykfG gene, yafX gene, ykfF gene, ykfB gene, yafY gene, ypjK gene, yafZ gene, ykfA gene, perR gene, JW0245 gene, JW0246 gene, JW0247 gene, ykfC gene, JW0249 gene, is5B gene, yi51 gene, mmuP gene, mmuM gene, afuC gene, JW0255 gene, insB gene, insA gene, JW0258 gene, yagB gene, yagA gene, yagE gene, yagF gene, yagG gene, yagH gene, yagI gene, argF gene, yfjI gene, yagJ gene, yagK gene, yagL gene, yagM gene, yagN gene, intF gene, yagP gene, yagQ gene, yagR gene, yagS gene, yagT gene, yagU gene, ykgJ gene, yagV gene, yagW gene, yagX gene, yagY gene, yagZ gene, ykgK gene, ykgL gene, ykgM gene, eaeH gene, JW0292 gene, JW0293 gene, ykgA gene, ykgB gene, ykgI gene, merA gene, ykgD gene, ykgE gene, ykgF gene, ykgG gene, ykgH gene, betA gene, betB gene, betI gene, betT gene, yahA gene, yahB gene, yahC gene, yahD gene, yahE gene, yahF gene, yahG gene, yahH gene, arcM gene, yahJ gene, yahK gene, yahL gene, yahM gene, yahN gene, yahO gene, prpR gene, bcpA gene, prpC gene, prpD gene, prpE gene, codB gene, codA gene, cynR gene, cynT gene, cynS gene, cynX gene, lacA gene, lacY gene, lacZ gene, lacI gene, mhpR gene, mhpA gene, mhpB gene, mhpC gene, mhpD gene, mhpF gene, mhpE gene, mhpT gene, yaiL gene, yaiM gene, adhC gene, yaiN gene, yaiO gene, JW0350 gene, yi2A gene, yi22 gene, yi24 gene, yi23 gene, yaiP gene, yaiS gene, tauA gene, tauB gene, tauC gene, ssiD gene, yaiT gene, JW0363 gene, JW0364 gene, yaiU gene, yaiV gene, ampH gene, sbmA gene, yaiW gene, yaiY gene, yaiZ gene, ddlA gene, yaiB gene, phoA gene, psiF gene, yaiC gene, ybbO gene, tesA gene, ybbA gene, ybbP gene, rhsD gene, ybbC gene, rhsE gene, ybbD gene, JW0490 gene, ybbB gene, ybbS gene, ybbT gene, ybbU gene, ybbQ gene, ybbV gene, allP gene, allA gene, ybbY gene, ybbZ gene, ylbA gene, allC gene, ylbC gene, fdrA gene, ylbE gene, ylbE gene, ylbE gene, ylbF gene, arcC gene, intD gene, EXO_LAMBD gene, VRPP_LAMBD gene, JW0528 gene, JW0529 gene, VREN_LAMBD gene, emrE gene, ybcK gene, ybcL gene, ybcM gene, ybcN gene, ninE gene, ybcO gene, rus gene, ybcQ gene, JW0539.5 gene, nmpC gene, vlyS gene, ybcS gene, ybcT gene, vboR gene, ybcV gene, ybcW gene, nohB gene, ybcX gene, ybcY gene, JW0552 gene, appY gene, ompT gene, envY gene, ybcH gene, nfrA gene, nfrB gene, cusS gene, cusR gene, cusC gene, ylcC gene, cusB gene, cusA gene, pheP gene, ybdG gene, nfnB gene, ybdF gene, ybdJ gene, ybdK gene, hokE gene, yi81 gene, yi82 gene, ymcC gene, ymcD gene, cspH gene, cspG gene, sfa gene, yccL gene, yccM gene, torS gene, torT gene, torR gene, torC gene, torA gene, torD gene, yccD gene, cbpA gene, yccE gene, agp gene, yccJ gene, wrbA gene, yciG gene, ycdG gene, JW0992 gene, JW0993 gene, JW0994 gene, ycdK gene, JW0996 gene, JW0997 gene, ycdC gene, putA gene, yzpU gene, putP gene, JW1002 gene, ycdO gene, ycdB gene, phoH gene, ycdP gene, ycdQ gene, ycdR gene, ycdR gene, ycdS gene, ycdT gene, JW1012 gene, JW1013 gene, JW1014 gene, ycdU gene, ymfD gene, ymfE gene, lit gene, intE gene, VXIS_BPP21 gene, ymfH gene, ymfI gene, ymfJ gene, RPC2_BPP22 gene, JW1132 gene, ymfL gene, ymfM gene, ymfN gene, ymfR gene, ymfO gene, JW1138 gene, JW1139 gene, ycfK gene, ymfS gene, ycfA gene, ycfE gene, pin gene, mcrA gene, JW1146 gene, ycgW gene, ycgX gene, ycgE gene, JW1150 gene, ycgZ gene, ymgA gene, JW1153 gene, ymgC gene, yahA gene, JW1156 gene, JW1157 gene, aidA gene, ymgD gene, JW1160 gene, JW1161 gene, JW1162 gene, ydfH gene, ydfZ gene, ydfI gene, shiA gene, JW1537 gene, tnpR gene, ydfM gene, Y314_LAMBD gene, nohA gene, ydfO gene, yccL gene, cspI gene, ydfR gene, vlyS gene, cspB gene, cspF gene, regq LAMBD gene, JW1552 gene, rem gene, hokD gene, relE gene, relB gene, ydfV gene, flxA gene, ydfW gene, ydfX gene, dicC gene, sdaC gene, yqeH gene, yqeI gene, yqeJ gene, yqeK gene, ygeF gene, ygeG gene, ygeH gene, ygeI gene, JW2822 gene, ygeK gene, JW2824 gene, JW2825 gene, JW2830 gene, ygeP gene, JW2832 gene, nlpD gene, yagR gene, ygeT gene, JW2836 gene, ygeV gene, ygeW gene, dpaL gene, ygeY gene, ygeZ gene, arcL gene, yqeB gene, JW2844 gene, ygfJ gene, gltD gene, JW2847 gene, ygfM gene, yagR gene, ygfO gene, ygfP gene, ygfQ gene, ygfR gene, ygfS gene, ygfT gene, pbuX gene, idi gene, epsM gene, exeL gene, exeC gene, JW2938 gene, JW2939 gene, JW2940 gene, JW2941 gene, yghK gene, glcB gene, glcG gene, glcF gene, glcD gene, glcC gene, JW2948 gene, yghQ gene, yghR gene, yghS gene, yghT gene, yihL gene, yihM gene, yihN gene, yshA gene, yihO gene, yihP gene, yihQ gene, yihR gene, yihS gene, yihT gene, yihU gene, yihV gene, yihW gene, yihX gene, rbn gene, yihZ gene, yiiD gene, yiiE gene, yiiF gene, yiiG gene, frvR gene, frvX gene, frvB gene, frvA gene, yiiL gene, rhaD gene, rhaA gene, rhaB gene, rhaS gene, rhaR gene, rhaT gene, ptsA gene, JW3920 gene, frwC gene, frwB gene, pflD gene, pflC gene, frwD gene, yijO gene, yijP gene, JW3986 gene, yjbF gene, yjbG gene, yjbH gene, JW3989.5 gene, yjbA gene, xylE gene, malG gene, malF gene, malE gene, malK gene, lamB gene, malM gene, yjbI gene, yjcP gene, yjcQ gene, yjcR gene, yjcS gene, alsK gene, alsE gene, alsC gene, alsA gene, alsB gene, phnQ gene, phnP gene, phnO gene, phnN gene, phnM gene, phnL gene, phnK gene, phnJ gene, phnI gene, phnH gene, phnG gene, phnF gene, phnE gene, phnD gene, phnC gene, phnB gene, phnA gene, yjdA gene, yjcZ gene, yidF gene, yidG gene, yidH gene, yidI gene, yidJ gene, yidK gene, yidL gene, JW3657 gene, glvG gene, glvB gene, glvC gene, yidP gene, yidE gene, yidQ gene, yidR gene, yidS gene, dgoT gene, dgoA gene, dgoK gene, dgoR gene, yidW gene, yidX gene, yidA gene, yidB gene, asnA gene, yieM gene, JW3724 gene, yieN gene, kup gene, rbsD gene, rbsA gene, rbsC gene, rbsB gene, rbsK gene, rbsR gene, yieO gene, yecT gene, flhE gene, flhA gene, flhB gene, cheZ gene, cheY gene, cheB gene, cheR gene, tap gene, tar gene, cheW gene, cheA gene, motB gene, motA gene, flhC gene, flhD gene, JW1882 gene, JW1883 gene, yecG gene, otsA gene, otsB gene, araH gene, araG gene, araF gene, yecI gene, yecJ gene, JW1892 gene, ftn gene, yecH gene, tyrP gene, uvrC gene, uvrY gene, yecF gene, sdiA gene, yecC gene, yecS gene, yedO gene, fliY gene, fliZ gene, fliA gene, fliC gene, fliD gene, fliS gene, fliT gene, amyA gene, yedD gene, yedE gene, yedF gene, yedK gene, yedL gene, yedN gene, yedM gene, fliE gene, fliF gene, fliG gene, fliH gene, fliI gene, fliJ gene, fliK gene, fliL gene, fliM gene, fliN gene, fliO gene, fliP gene, fliQ gene, fliR gene, rcsA gene, dsrB gene, JW1937 gene, yedA gene, vsr gene, dcm gene, yedJ gene, JW1946 gene, ompC gene, JW1948 gene, yedS gene, yedU gene, yedV gene, copR gene, yedX gene, JW1954 gene, JW1955 gene, JW1956 gene, JW1974 gene, JW1979 gene, JW1980 gene, yeeP gene, flu gene, JW1983 gene, yeeS gene, yeeT gene, yeeU gene, yeeV gene, yeeW gene, yefJ gene, JW2013 gene, yefj gene, yefl gene, wbbJ gene, yefG gene, rfc gene, glf gene, rfbX gene, rfbC gene, rfbA gene, rfbD gene, rfbB gene, intA gene, yfjH gene, alpA gene, yfjI gene, JW2606 gene, yfjJ gene, yfjK gene, yfjL gene, yfjM gene, yfjN gene, yfjO gene, yfjP gene, yfjQ gene, yfjr gene, ypjK gene, yfjS gene, yfjT gene, yfjU gene, yfjV gene, JW2622 gene, yfjW gene, JW2623.5 gene, yfjX gene, yfjY gene, yfjZ gene, ypjF gene, ypjA gene, JW2629 gene, JW2630 gene, JW2631 gene, JW2632 gene, JW2633 gene, JW2634 gene, ygaE gene, yzzM gene, yqaE gene, JW2642 gene, ygaP gene, stpA gene, JW2645 gene, ygaC gene, ygaM gene, nrdH gene, nrdI gene, nrdE gene, proV gene, proW gene, proX gene, JW2655 gene, JW2656 gene, ygaZ gene, ygaH gene, srlA gene, srlB gene, srlD gene, gutM gene, srlR gene, gutQ gene, gltF gene, yhcA gene, yhcD gene, yhcE gene, yhcE gene, yhcF gene, yhcG gene, yhcH gene, nanK gene, nanE gene, yegE gene, alkA gene, yegD gene, JW2055 gene, JW2056 gene, JW2057 gene, JW2058 gene, yegM gene, yegN gene, yegO gene, yegB gene, baeS gene, baeR gene, JW2065 gene, yegQ gene, ogrK gene, JW2068 gene, JW2069 gene, yegS gene, gatR gene, JW2072 gene, JW2073 gene, gatR gene, gatD gene, gatC gene, gatB gene, gatA gene, gatZ gene, gatY gene, fbaB gene, xegT gene, yegU gene, yegV gene, yegW gene, yegX gene, molR gene, yehI gene, yehK gene, yehL gene, yehO gene, yehP gene, yehQ gene, yehR gene, yehS gene, yehT gene, yehU gene, yehV gene, yehW gene, yehX gene, yehY gene, yehZ gene, bglX gene, dld gene, pbpG gene, yohC gene, yeiI gene, yeiJ gene, yeiK gene, yeiL gene, yeiM gene, yeiN gene, yeiC gene, cmtA gene, cmtB gene, intB gene, JW4228 gene, yjgW gene, yjgX gene, yjgY gene, yjgZ gene, yi41 gene, yi42 gene, yjhB gene, yjhC gene, yjhD gene, yjhE gene, yi91 gene, JW4244 gene, JW4245 gene, JW4246 gene, yjhU gene, yjhF gene, yjhG gene, yjhH gene, yjhI gene, sgcR gene, sgcE gene, sgcA gene, sgcQ gene, sgcC gene, JW4266.5 gene, sgcX gene, yjhP gene, yjhQ gene, JW4269.5 gene, JW4270 gene, JW4270.5 gene, yjhR gene, yjhS gene, yjhT gene, yjhA gene, fimB gene, fimE gene, fimA gene, fimI gene, fimC gene, fimD gene, fimF gene, fimG gene, fimH gene, gntP gene, uxuA gene, uxuB gene, uxuR gene, yjiC gene, yjiD gene, yjiE gene, iadA gene, yjiG gene, yjiH gene, yjiI gene, yjiJ gene, yjiK gene, JW4296.5 gene, yjiL gene, yjiM gene, yjiN gene, yjiO gene, yjiP gene, yjiQ gene, yjiR gene, yjiS gene, yjiT gene, yjiV gene, mcrD gene, mcrC gene, mcrB gene, yjiW gene, hsdS gene, hsdM gene, hsdR gene, mrr gene, yjiA gene, yjiX gene, yjiY gene, tsr gene, yjjL gene, JW4320 gene, yjjM gene, yjjN gene, mdoB gene, yjjA gene, kdpF gene, ybfA gene, rhsC gene, JW0690 gene, ybfB gene, rhsA gene, ybfC gene, ybfL gene, ybfD gene, ybgA gene, yjfI gene, yjfJ gene, yjfK gene, yjfL gene, yjfM gene, yjfC gene, aidB gene, yjfN gene, yjfO gene, yjfP gene, yjfQ gene, yjfR gene, sgaT gene, sgaB gene, sgaA gene, sgaH gene, sgaU gene, rhsB gene, JW3448 gene, yhhH gene, yibA gene, yhhI gene, yhhJ gene, yhiH gene, yhiI gene, yhiJ gene, yhiK gene, yhiL gene, yhiM gene, yhiN gene, JW0782 gene, ybiA gene, dinG gene, ybiB gene, ybiC gene, ybiJ gene, ybiI gene, ybiX gene, ybiL gene, ybiM gene, ybiN gene, ybiO gene, glnQ gene, glnP gene, glnH gene, yliA gene, yliB gene, yliC gene, yliD gene, JW0817 gene, JW0818 gene, yliG gene, yliH gene, yliI gene, yliJ gene, dacC gene, deoR gene, ybjG gene, ybjC gene, mdaA gene, rimK gene, ybjN gene, potF gene, potG gene, potH gene, potI gene, ybjO gene, ybjF gene, artJ gene, artM gene, artQ gene, artI gene, artP gene, ybjP gene, JW0850 gene, JW0851 gene, JW0852 gene, ybjT gene and ltaA gene, or homologous gene thereof.

26. The *E*. *coli* mutant strain according to any one of claims 23 to 25 comprising chromosomal DNA that does not comprise DNA having the nucleotide sequences as shown in
[1] to [43] of the nucleotide sequence as shown in SEQ ID NO: 1 or DNA equivalent thereto.
[1] a nucleotide sequence of nucleotides 243965 to 253410
[2] a nucleotide sequence of nucleotides 261964 to 387531
[3] a nucleotide sequence of nucleotides 389139 to 403703
[4] a nucleotide sequence of nucleotides 518030 to 532711
[5] a nucleotide sequence of nucleotides 535505 to 550215
[6] a nucleotide sequence of nucleotides 563941 to 608118
[7] a nucleotide sequence of nucleotides 728821 to 739593
[8] a nucleotide sequence of nucleotides 832552 to 848089
[9] a nucleotide sequence of nucleotides 867638 to 883473
[10] a nucleotide sequence of nucleotides 890998 to 909379
[11] a nucleotide sequence of nucleotides 1048888 to 1097019
[12] a nucleotide sequence of nucleotides 1198106 to 1225146
[13] a nucleotide sequence of nucleotides 1630912 to 1641038
[14] a nucleotide sequence of nucleotides 1642632 to 1650222
[15] a nucleotide sequence of nucleotides 1964524 to 1993075
[16] a nucleotide sequence of nucleotides 1996662 to 2028209
[17] a nucleotide sequence of nucleotides 2033449 to 2044376
[18] a nucleotide sequence of nucleotides 2069149 to 2081926
[19] a nucleotide sequence of nucleotides 2104393 to 2116064
[20] a nucleotide sequence of nucleotides 2146264 to 2187855
[21] a nucleotide sequence of nucleotides 2200669 to 2229850
[22] a nucleotide sequence of nucleotides 2255895 to 2263491
[23] a nucleotide sequence of nucleotides 2755677 to 2789480
[24] a nucleotide sequence of nucleotides 2795174 to 2800890
[25] a nucleotide sequence of nucleotides 2804308 to 2810147
[26] a nucleotide sequence of nucleotides 2825314 to 2830262
[27] a nucleotide sequence of nucleotides 2985362 to 3033128
[28] a nucleotide sequence of nucleotides 3077085 to 3078916
[29] a nucleotide sequence of nucleotides 3110110 to 3134341
[30] a nucleotide sequence of nucleotides 3360737 to 3370547
[31] a nucleotide sequence of nucleotides 3618576 to 3636916
[32] a nucleotide sequence of nucleotides 3854662 to 3865831
[33] a nucleotide sequence of nucleotides 3867102 to 3877010
[34] a nucleotide sequence of nucleotides 3926659 to 3938878
[35] a nucleotide sequence of nucleotides 4059898 to 4079659
[36] a nucleotide sequence of nucleotides 4086451 to 4099982
[37] a nucleotide sequence of nucleotides 4139232 to 4149709
[38] a nucleotide sequence of nucleotides 4235359 to 4251735
[39] a nucleotide sequence of nucleotides 4298977 to 4311511
[40] a nucleotide sequence of nucleotides 4313318 to 4329721
[41] a nucleotide sequence of nucleotides 4409533 to 4423151
[42] a nucleotide sequence of nucleotides 4496202 to 4509534
[43] a nucleotide sequence of nucleotides 4517876 to 4599637

27. The *E*. *coli* mutant strain according to any one of claims 23 to 26 comprising chromosomal DNA that does not comprise ygiS gene, ygiT gene, ygiU gene, JW2991 gene, ygiW gene, ygiX gene, ygiY gene, ygiZ gene, mdaB gene and ygiN gene, or homologous gene thereof.

28. The *E*. *coli* mutant strain according to any one of claims 23 to 27 comprising chromosomal DNA that does not comprise DNA having a nucleotide sequence comprising nucleotides 3165628 to 3172940 of the nucleotide sequence as shown in SEQ ID NO: 1 or DNA equivalent thereto.

29. The *E*. *coli* mutant strain according to any one of claims 23 to 28 comprising chromosomal DNA that does not comprise ybeL gene, ybeQ gene, ybeR gene, ybeS gene, ybeT gene, ybeU gene, ybeV gene, ybeW gene, ybeK gene, gltL gene, gltK gene, gltJ gene, ybeJ gene and JW0651 gene, or homologous gene thereof.

30. The *E*. *coli* mutant strain according to any one of claims 23 to 29 comprising chromosomal DNA that does not comprise DNA having a nucleotide sequence comprising nucleotides 675104 to 689374 of the nucleotide sequence as shown in SEQ ID NO: 1 or DNA equivalent thereto.

31. The *E. coli* mutant strain according to any one of claims 23 to 30 comprising chromosomal DNA that does not comprise yfcC gene, JW2296 gene, yfcE gene, yfcF gene, yfcG gene, folX gene, JW2301 gene, yfcI gene, hisP gene, hisM gene, hisQ gene, hisJ gene, argT gene, yfdB gene, JW2346 gene, yfdH gene, JW2348 gene, yfdK gene, yfdL gene, yfdM gene, xylU gene, yfdN gene, yfdO gene, JW2356 gene, JW2357 gene, JW2358 gene, JW2359 gene, JW2360 gene, JW2378 gene, yqhT gene, ptwC gene andptwB gene, or homologous gene thereof.

32. The *E*. *coli* mutant strain according to any one of claims 23 to 31 comprising chromosomal DNA that does not comprise DNA having the nucleotide sequences as shown in
[1] to [3] below of the nucleotide sequence as shown in SEQ ID NO: 1 or DNA equivalent thereto.
[1] a nucleotide sequence of nucleotides 2423365 to 2434098
[2] a nucleotide sequence of nucleotides 2472852 to 2482482
[3] a nucleotide sequence of nucleotides 2505075 to 2514557

33. The *E. coli* mutant strain according to any one of claims 23 to 32 comprising chromosomal DNA that does not comprise pinO gene, gspA gene, gspC gene, gspD gene, gspE gene, hofF gene, hofG gene, hofH gene, gspI gene, gspJ gene, gspK gene, gspL gene, pshM gene, hofD gene, bfr gene, yheA gene and chiA gene, or homologous gene thereof.

34. The *E. coli* mutant strain according to any one of claims 23 to 33 comprising chromosomal DNA that does not comprise DNA having a nucleotide sequence comprising nucleotides 3453021 to 3469366 of the nucleotide sequence as shown in SEQ ID NO: 1 or DNA equivalent thereto.

35. The *E*. *coli* mutant strain according to any one of claims 23 to 34 comprising chromosomal DNA that does not comprise elaB gene, elaA gene, elaC gene, elaD gene, yfbK gene, yfbL gene, yfbM gene, yfbN gene, JW2269 gene and JW2270 gene, or homologous gene thereof.

36. The *E. coli* mutant strain according to any one of claims 23 to 35 comprising chromosomal DNA that does not comprise DNA having a nucleotide sequence comprising nucleotides 2386262 to 2395471 of the nucleotide sequence as shown in SEQ ID NO: 1 or DNA equivalent thereto.

37. The *E*. *coli* mutant strain according to any one of claims 23 to 36 comprising chromosomal DNA that does not comprise yhdJ gene, yhdU gene, envR gene, acrE gene, envD gene, yhdV gene, yhdW gene, yhdX gene and yhdY gene, or homologous gene thereof.

38. The *E. coli* mutant strain according to any one of claims 23 to 37 comprising chromosomal DNA that does not comprise DNA having a nucleotide sequence comprising nucleotides 3411160 to 3420878 of the nucleotide sequence as shown in SEQ ID NO: 1 or DNA equivalent thereto.

39. The *E. coli* mutant strain according to any one of claims 23 to 38 comprising chromosomal DNA that does not comprise chpA gene or homologous gene thereof.

40. The *E. coli* mutant strain according to any one of claims 23 to 39 comprising chromosomal DNA that does not comprise DNA having a nucleotide sequence comprising nucleotides 2910274 to 2910573 of the nucleotide sequence as shown in SEQ ID NO: 1 or DNA equivalent thereto.

41. The *E*. *coli* mutant strain according to any one of claims 23 to 40 comprising chromosomal DNA that does not comprise fhuA gene, fhuC gene, fhuD gene fhuB gene, or homologous gene thereof.

42. The *E. coli* mutant strain according to any one of claims 23 to 41 comprising chromosomal DNA that does not comprise DNA having a nucleotide sequence comprising nucleotides 167065 to 172979 of the nucleotide sequence as shown in SEQ ID NO: 1 or DNA equivalent thereto.

43. The *E. coli* mutant strain according to any one of claims 1 to 42 obtained via modification by a method selected from among the following [1] to [5]:
[1] a method for reducing or removing at least one mechanism for regulating biosynthesis of the useful substance;
[2] a method for increasing the expression level of at least one enzyme associated with biosynthesis of the useful substance;
[3] a method for increasing the copy number of at least one enzyme gene associated with biosynthesis of the useful substance;
[4] a method for attenuating or blocking at least one metabolic pathway which branches from the biosynthetic pathway of the useful substance into metabolites other than the useful substance; or
[5] a method for selecting a strain that exhibits higher resistance to an analogue of a useful substance than a wild-type *E. coli* strain.

44. The *E*. *coli* mutant strain according to any one of claims 1 to 43, wherein the *E*. *coli* strain is the *E*. *coli* K-12, B, or W strain.

45. The *E*. *coli* mutant strain according to claim 44, wherein the *E. coli* K-12 strain is the *E*. *coli* W3110 strain (ATCC27325) or the *E. coli* MG1655 strain (ATCC47076).

46. A method for producing a useful substance comprising culturing an *E. coli* mutant strain according to any one of claims 1 to 45 in a medium so as to generate and accumulate such useful substance in the culture and recovering the useful substance from the culture.

47. The method according to any one of claims 43 to 46, wherein the useful substance is selected from the group consisting of proteins, peptides, amino acids, nucleic acids, vitamins, saccharides, organic acids, and lipids.
